(19) Europäisches Patentamt / European Patent Office / Office européen des brevets

(11) **EP 4 613 784 A1**

(12) **EUROPEAN PATENT APPLICATION**
published in accordance with Art. 153(4) EPC

(43) Date of publication:
**10.09.2025 Bulletin 2025/37**

(21) Application number: **23885712.2**

(22) Date of filing: **30.10.2023**

(51) International Patent Classification (IPC):
***C08F 20/18*** *(2006.01)* ***C07C 67/03*** *(2006.01)*
***C07C 69/54*** *(2006.01)* ***C08F 20/10*** *(2006.01)*

(52) Cooperative Patent Classification (CPC):
(C-Sets available)
**C07C 67/03; C07C 67/62; C08F 20/10; C08F 20/18**
(Cont.)

(86) International application number:
**PCT/JP2023/039088**

(87) International publication number:
**WO 2024/095957 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**31.10.2022 JP 2022174242**
**31.10.2022 JP 2022174342**
**31.10.2022 JP 2022174417**
**31.10.2022 JP 2022174460**
**31.10.2022 JP 2022174628**
**31.10.2022 JP 2022174760**

(71) Applicant: **Mitsubishi Chemical Corporation**
**Tokyo 100-8251 (JP)**

(72) Inventors:
- **AIZAWA Ryo**
  **Tokyo 100-8251 (JP)**
- **WATANABE Takeshi**
  **Tokyo 100-8251 (JP)**
- **ICHII Naoki**
  **Tokyo 100-8251 (JP)**

(74) Representative: **Hoffmann Eitle**
**Patent- und Rechtsanwälte PartmbB**
**Arabellastraße 30**
**81925 München (DE)**

(54) **ESTER COMPOUND-CONTAINING COMPOSITION, METHOD FOR PRODUCING SAME, POLYMERIZABLE COMPOSITION, (METH)ACRYLIC POLYMER, AND METHOD FOR PRODUCING SAME**

(57) An object of the present invention is to provide an ester compound-containing composition having excellent storage stability, a method for producing the same, a polymerizable composition, a (meth)acrylic polymer, and a method for producing the same.

A methacrylic acid-containing composition comprises the following ester compound (I) and the following component (A), in which the contained amount of the ester compound (I) is 95.00% to 99.99% by mass with respect to the total mass.

Ester compound (I): one or more compounds selected from the group consisting of an ester compound (1), an ester compound (2), a compound (3), an ester compound (4), and an ester compound (5)
Component (A): one or more components selected from the group consisting of a component A1, a component A2, a component A6, a component A7, a component A8, a component A9, and a component A10

EP 4 613 784 A1

(52) Cooperative Patent Classification (CPC): (Cont.)

C-Sets
**C07C 67/03, C07C 69/54;**
**C07C 67/62, C07C 69/54**

## Description

TECHNICAL FIELD

The present invention relates to an ester compound-containing composition, a method for producing the same, a polymerizable composition, a (meth)acrylic polymer, and a method for producing the same.

Priority is claimed on Japanese Patent Application No. 2022-174242, Japanese Patent Application No. 2022-174342, Japanese Patent Application No. 2022-174417, Japanese Patent Application No. 2022-174628, Japanese Patent Application No. 2022-174460, and Japanese Patent Application No. 2022-174760, filed October 31, 2022, the contents of which are incorporated herein by reference.

BACKGROUND ART

A (meth)acrylic polymer obtained from a (meth)acrylic acid ester is used in various fields such as a coating material, an adhesive, a resin reforming agent, artificial marble, and paper latex.

For example, a target (meth)acrylic acid alkyl ester can be produced by utilizing an ester exchange reaction between a (meth)acrylic acid alkyl ester and an alkyl alcohol as raw materials in the presence of $Ti(OR)_4$ (R is an alkyl group) as a catalyst (for example, Patent Document 1).

Citation List

Patent Document

Patent Document 1:Japanese Unexamined Patent Application, First Publication No. 2009-274986

SUMMARY OF INVENTION

Technical Problem

When an ester compound-containing composition comprising a (meth)acrylic acid ester is stored for a certain period after production, quality of the ester compound-containing composition may deteriorate.

An object of the present invention is to provide an ester compound-containing composition having excellent storage stability, a method for producing the same, a polymerizable composition obtained from the ester compound-containing composition, a (meth)acrylic polymer, and a method for producing the same.

Solution to Problem

The present inventors have conducted intensive studies. As a result, it is found that, when the ester compound-containing composition includes a specific component, generation of impurities such as a dimer of (meth)acrylic acid ester is suppressed, and thus the storage stability is improved.

According to one aspect of the present invention, the following ester compound-containing composition is provided.

An ester compound-containing composition comprising:

an ester compound (I) represented by Formula (I); and
one or more compounds selected from the group consisting of a compound represented by Formula (a1), a compound represented by Formula (a2), a compound represented by Formula (a6), a compound represented by Formula (a7), a compound represented by Formula (a8), a compound represented by Formula (a9), and a compound represented by Formula (a10),
wherein the contained amount of the ester compound (I) is 95.00% to 99.99% by mass,

$$CH_2=CR^{150}\text{-}C(=O)\text{-}O\text{-}R^{200} \cdots \qquad (I),$$

in Formula (I), $R^{150}$ is a hydrogen atom or a methyl group, and
$R^{200}$ is a monovalent hydrocarbon group having 2 to 20 carbon atoms, which may have a substituent, or a monovalent group having an ether bond and having 2 to 8 carbon atoms.

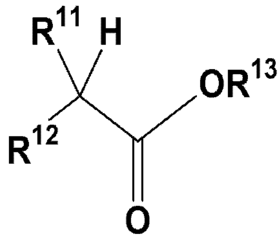

(a 1)

In Formula (a1), $R^{11}$, $R^{12}$, $R^{13}$ may be each independently a group selected from a hydrogen atom, an alkyl group, an alkenyl group, an aryl group, a hydroxy group, an alkoxy group, an amino group, a carbonyl group, and a monovalent group having an ether bond and/or a sulfide bond, or $R^{11}$, $R^{12}$, and $R^{13}$ may be each a divalent group in combination with any of the groups, which may have a substituent.

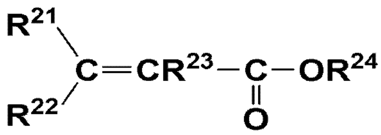

・・・(a 2)

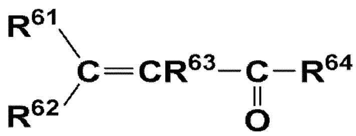

・・・(a 6)

In Formula (a2), $R^{21}$, $R^{22}$, and $R^{23}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{24}$ may be a hydrogen atom, an alkyl group, an alkenyl group, an aryl group, a hydroxy group, an alkoxy group, an amino group, a carbonyl group, or a monovalent group including an ether group, or $R^{21}$, $R^{22}$, $R^{23}$, and $R^{24}$ may be each a divalent group in combination with any of the groups, which may have a substituent,

provided that a case where $R^{21}$ = H, $R^{22}$ = H, $R^{23}$ = $R^{150}$, and $R^{24}$ = $R^{200}$, that is, a case where the compound represented by Formula (a2) is the same as the ester compound (I) is excluded.

In Formula (a6), $R^{61}$, $R^{62}$, and $R^{63}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{64}$ may be a monovalent group selected from an alkyl group, an alkenyl group, an aryl group, a hydroxy group, an alkoxy group, an amino group, a monovalent group including an ether group, an alkylthio group, or an arylthio group, or $R^{61}$, $R^{62}$, $R^{63}$, and $R^{64}$ may be each a divalent group in combination with any of the groups, which may have a substituent,

provided that a case where $R^{61}$ = H, $R^{62}$ = H, $R^{63}$ = $R^{150}$ and $R^{64}$ = $OR^{200}$, that is, a case where the compound represented by Formula (a6) is the same as the ester compound (I) is excluded.

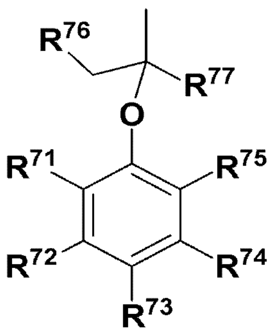

(a 7)

In Formula (a7), $R^{71}$, $R^{72}$, $R^{73}$, $R^{74}$, $R^{75}$, $R^{76}$, and $R^{77}$ are each independently a monovalent group selected from a hydrogen atom, an alkyl group, an alkenyl group, an aryl group, a hydroxy group, an alkoxy group, an amino group, a monovalent group including a carbonyl group, an alkylthio group, or an arylthio group.

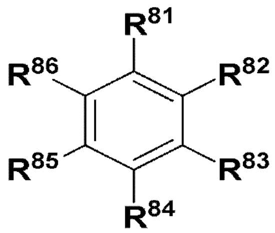

・・・(a8)

In Formula (a8), $R^{81}$, $R^{82}$, $R^{83}$, $R^{84}$, $R^{85}$, and $R^{86}$ are each independently a monovalent group selected from a hydrogen atom, an alkyl group, an alkenyl group, an aryl group, a hydroxy group, an alkoxy group, an amino group, a monovalent group including a carbonyl group, an alkylthio group, or an arylthio group, where the total number of carbon atoms in $R^{81}$ , $R^{82}$ , $R^{83}$ , $R^{84}$ , $R^{85}$ , and $R^{86}$ is 2 or more.

$$R^{91}-C\equiv N \quad \cdots (a9)$$

In Formula (a9), $R^{91}$ is an alkyl group having 1 to 5 carbon atoms, an alkenyl group having 2 to 5 carbon atoms, or an aryl group having 1 to 12 carbon atoms, each of which may have a substituent.

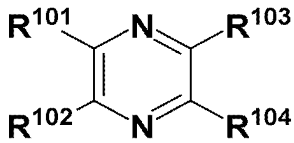

・・・(a10)

In Formula (a10), $R^{101}$, $R^{102}$, $R^{103}$, $R^{104}$ may be each independently a hydrogen atom or a monovalent group selected from an alkyl group, an alkenyl group, an aryl group, a hydroxy group, an alkoxy group, an amino group, a carbonyl group, a monovalent group including a heteroatom and/or an unsaturated bond, an alkylthio group, or an arylthio group, or $R^{101}$, $R^{102}$, $R^{103}$, and $R^{104}$ may be each a divalent group in combination with any of the groups, which may have a substituent and may have a heteroatom and/or an unsaturated bond, where the groups may further have a substituent.

According to another aspect of the present invention, a method for producing the ester compound-containing composition is provided, the production method comprising performing an ester exchange reaction between one or more alcohols selected from the group consisting of a monoalcohol having 2 to 20 carbon atoms, an ether bond-containing alcohol having 2 to 8 carbon atoms, a dialcohol having 2 to 8 carbon atoms, and a trialcohol having 2 to 8 carbon atoms, and methyl (meth)acrylate in a presence of at least one or more compounds selected from the group consisting of the compound represented by Formula (a1), the compound represented by Formula (a2), the compound represented by Formula (a6), the compound represented by Formula (a7), the compound represented by Formula (a8), the compound represented by Formula (a9), and the compound represented by Formula (a10).

According to still another aspect of the present invention, a polymerizable composition comprising the ester compound-containing composition is provided.

According to still another aspect of the present invention, a (meth)acrylic polymer obtained by polymerizing the polymerizable composition is provided.

According to still another aspect of the present invention, a method for producing a (meth)acrylic polymer, comprising polymerizing the polymerizable composition, is provided.

Advantageous Effects of Invention

According to the present invention, generation of a dimer of (meth)acrylic acid ester, a pyruvic acid ester, and the like during storage is suppressed. Therefore, it is possible to provide an ester compound-containing composition having excellent storage stability, a method for producing the same, a polymerizable composition obtained from the ester compound-containing composition, a (meth)acrylic polymer, and a method for producing the same.

DESCRIPTION OF EMBODIMENTS

Hereinafter, embodiments of the ester compound-containing composition according to the present invention will be described in detail. The following embodiments are merely examples for description, and the present invention is not limited to the embodiments. In addition, the present invention can be carried out in various aspects as long as the various aspects maintain the gist of the invention.

Meanings of the following terms are as follows.
"Monomer" means a compound having a polymerizable carbon-carbon double bond.
"(Meth)acrylate" is selected from "acrylate" and "methacrylate".
"(Meth)acrylic acid" is selected from "acrylic acid" and "methacrylic acid".
In addition, in the present specification, a numerical value range represented using "to" means a range including the numerical values listed before and after "to" as the lower limit value and the upper limit value. For example, "1 to 20" means 1 or more and 20 or less. The numerical value range described in the present specification can be any numerical value range of any combination of the lower limit value and the upper limit value thereof.

1. First aspect

Hereinafter, a first aspect of the embodiment will be described.
An ester compound-containing composition according to the first aspect comprises an ester compound (I) described later and a compound (component A1) described later. The contained amount of the ester compound (I) is 95.00% to 99.99% by mass.
The ester compound-containing composition according to the first aspect may further comprise a polymerization inhibitor (component B) described later, in addition to the ester compound (I).
As long as the effect of the present invention is not impaired, the ester compound-containing composition may further comprise the component B as necessary, in addition to the ester compound (I) and the component A1, and may further comprise at least one of a compound other than the ester compound (I), the component A1, and the component B (hereinafter, also referred to as "component C") or water.

(Ester compound (I))

The ester compound (I) is a compound represented by Formula (I).

$$CH_2=CR^{150}-C(=O)-O-R^{200} \cdots \qquad (I)$$

In Formula (I), $R^{150}$ is a hydrogen atom or a methyl group, and
$R^{200}$ is a monovalent hydrocarbon group having 2 to 20 carbon atoms, which may have a substituent, or a monovalent group having an ether bond and having 2 to 8 carbon atoms.
The ester compound (I) may be used alone or a combination of two or more kinds thereof may be used.
As the ester compound (I), one or more compounds selected from the group consisting of an ester compound (1) described later, an ester compound (2) described later, an ester compound (3) described later, an ester compound (4) described later, and an ester compound (5) described later are preferable. Hereinafter, the ester compounds will be described.
The ester compound (1) is a (meth)acrylic acid ester represented by Formula (1).

$$CH_2=CR^{1a}-C(=O)-O-R^{2a} \cdots \qquad (1)$$

In Formula (1), $R^{1a}$ is a hydrogen atom or a methyl group. $R^{2a}$ is a hydrocarbon group having 2 to 20 carbon atoms.
In the ester compound (1), the hydrocarbon group of $R^{2a}$ may be a saturated hydrocarbon group or an unsaturated hydrocarbon group.
In addition, the hydrocarbon group of $R^{2a}$ may be linear or branched, or may have a ring. When the hydrocarbon group of $R^{2a}$ has a ring, the ring may be an aliphatic ring or an aromatic ring.
In addition, the number of carbon atoms in the hydrocarbon group of $R^{2a}$ is 2 to 20, preferably 2 to 18 and more preferably 2 to 12.
Examples of the hydrocarbon group of $R^{2a}$ include an alkyl group having 2 to 20 carbon atoms, a cycloalkyl group having 3 to 20 carbon atoms, an alkenyl group having 2 to 20 carbon atoms, a cycloalkenyl group having 3 to 20 carbon atoms, an alkynyl group having 2 to 20 carbon atoms, an aryl group having 6 to 20 carbon atoms, and an aromatic alkyl group having 7 to 20 carbon atoms. The "aromatic alkyl group" means a group in which one or more hydrogen atoms of an alkyl group are substituted with an aryl group.
Examples of the alkyl group of $R^{2a}$ include an ethyl group, a propyl group, an isopropyl group, an n-butyl group, an isobutyl group, a t-butyl group, a 2-ethylhexyl group, a lauryl group, and a stearyl group.
Examples of the cycloalkyl group of $R^{2a}$ include a cyclopropyl group, a cyclohexyl group, and an isobornyl group.
Examples of the alkenyl group of $R^{2a}$ include a vinyl group and an allyl group.
Examples of the cycloalkenyl group of $R^{2a}$ include a cyclopentenyl group, a cyclopentadienyl group, and a

cyclohexenyl group.

Examples of the alkynyl group of $R^{2a}$ include a propynyl group.

Examples of the aryl group of $R^{2a}$ include a phenyl group and a naphthyl group.

Examples of the aromatic alkyl group of $R^{2a}$ include a benzyl group.

From the viewpoint that the ester compound (1) is relatively easy to obtain and is relatively easy to handle in terms of physical properties, $R^{2a}$ is preferably an alkyl group having 2 to 20 carbon atoms, a cycloalkyl group having 3 to 20 carbon atoms, an alkenyl group having 2 to 20 carbon atoms, an aryl group having 6 to 20 carbon atoms, or an aromatic alkyl group having 7 to 20 carbon atoms; and more preferably an ethyl group, an isopropyl group, an n-butyl group, an isobutyl group, a t-butyl group, a 2-ethylhexyl group, a lauryl group, a stearyl group, a cyclohexyl group, an isobonyl group, an allyl group, a phenyl group, or a benzyl group.

Examples of the ester compound (1) include ethyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, and benzyl (meth)acrylate.

As the ester compound (1), from the viewpoint that the ester compound (1) is relatively easy to obtain and is relatively easy to handle in terms of physical properties, an alkyl (meth)acrylate in which $R^{2a}$ is a linear or branched alkyl group having 2 to 20 carbon atoms, a cycloalkyl (meth)acrylate in which $R^{2a}$ is a cycloalkyl group having 3 to 20 carbon atoms, an alkenyl (meth)acrylate in which $R^{2a}$ is an alkenyl group having 2 to 20 carbon atoms, an aryl (meth)acrylate in which $R^{2a}$ is an aryl group having 6 to 20 carbon atoms, or an aromatic alkyl (meth)acrylate in which $R^{2a}$ is an aromatic alkyl group having 7 to 20 carbon atoms is preferable; and ethyl (meth)acrylate, isopropyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobonyl (meth)acrylate, allyl (meth)acrylate, phenyl (meth)acrylate, or benzyl (meth)acrylate is more preferable.

The ester compound (1) may be used alone or a combination of two or more kinds thereof may be used.

The ester compound (2) is a (meth)acrylic acid ester represented by Formula (2). The ester compound (3) is a (meth)acrylic acid ester represented by Formula (3). The ester compound (4) is a (meth)acrylic acid ester represented by Formula (4).

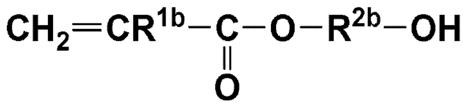
· · · (2)

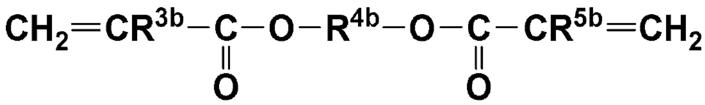
· · · (3)

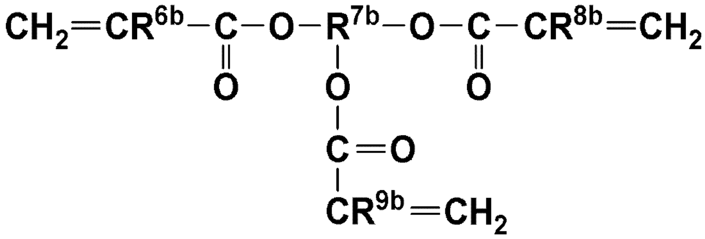
· · · (4)

In Formula (2), Formula (3), and Formula (4), $R^{1b}$, $R^{3b}$ $R^{5b}$, $R^{6b}$, $R^{8b}$, and $R^{9b}$ are each independently a hydrogen atom or a methyl group. $R^{2b}$ and $R^{4b}$ are each independently a linear or branched alkylene group or a hydroxyalkylene group, having 2 to 8 carbon atoms. $R^{7b}$ is a linear or branched trivalent hydrocarbon group having 2 to 8 carbon atoms.

In the ester compound (2) and the ester compound (3), the number of carbon atoms in the alkylene group or the hydroxyalkylene group of $R^{2b}$ and $R^{4b}$ is 2 to 8, preferably 2 to 6.

Examples of the alkylene group of $R^{2b}$ and $R^{4b}$ include an ethylene group, a propylene group, an isopropylene group, and a butylene group.

Examples of the hydroxyalkylene group of $R^{2b}$ and $R^{4b}$ include a hydroxyethylene group, a hydroxypropylene group, and a hydroxybutylene group.

The number of carbon atoms in the trivalent hydrocarbon group of $R^{7b}$ is 2 to 8, preferably 2 to 4.

Examples of the trivalent hydrocarbon group of $R^{7b}$ include $-(CH_2)-C(-CH_2-)(-CH_3)-CH_2-$.

Examples of the ester compound (2) include 2-hydroxyethyl (meth)acrylate, 2-hydroxypropyl (meth)acrylate, and 3-hydroxypropyl (meth)acrylate.

Examples of the ester compound (3) include ethylene glycol di(meth)acrylate, 1,3-propanediol di(meth)acrylate, 1,2-propanediol di(meth)acrylate, 1,3-butanediol di(meth)acrylate, and 1,6-hexanediol di(meth)acrylate.
Examples of the ester compound (4) include trimethylolpropane tri(meth)acrylate.
When the ester compound (I) includes the ester compounds (2) to (4), from the viewpoint of relatively easy availability, it is preferable that the ester compound (I) includes one or more selected from the group consisting of ethylene glycol di(meth)acrylate, 2-hydroxyethyl (meth)acrylate, 3-hydroxypropyl (meth)acrylate, and trimethylolpropane tri(meth)acrylate.
The ester compound (5) is a (meth)acrylic acid ester represented by Formula (5).

$$CH_2=CR^{1c}\text{-}C(=O)\text{-}O\text{-}R^{2c} \cdots \qquad (5)$$

In Formula (5), $R^{1c}$ is a hydrogen atom or a methyl group, and $R^{2c}$ is a monovalent group having an ether bond and having 2 to 8 carbon atoms.
In the ester compound (5), the number of etheric oxygens included in the monovalent group having 2 to 8 carbon atoms of $R^{2c}$ is preferably 1, but it is not limited thereto and may be 2 or more.
The monovalent group having 2 to 8 carbon atoms of $R^{2c}$ may be linear or branched, or may have a ring. When the monovalent group having 2 to 8 carbon atoms of $R^{2c}$ has a ring, the ring may or may not include the etheric oxygen.
The number of carbon atoms in the monovalent group having an ether bond of $R^{2c}$ is 2 to 8, preferably 2 to 7.
Examples of $R^{2c}$ include a 2-methoxyethyl group, a 2-(2-methoxyethoxy)ethyl group, a 2-[2-(2-methoxyethoxy)ethoxy]ethyl group, a glycidyl group, and a tetrahydrofurfuryl group.
As $R^{2c}$, from the viewpoint of relatively easy availability and relatively easy handling in terms of physical properties, a 2-methoxyethyl group, a glycidyl group, or a tetrahydrofurfuryl group is preferable.
Examples of the ester compound (5) include 2-methoxyethyl (meth)acrylate, glycidyl (meth)acrylate, 2-(2-methoxyethoxy)ethyl (meth)acrylate, 2-[2-(2-methoxyethoxy)ethoxy]ethyl (meth)acrylate, and tetrahydrofurfuryl (meth)acrylate.
As the ester compound (5), from the viewpoint of relatively easy handling in terms of physical properties, 2-methoxyethyl (meth)acrylate, glycidyl (meth)acrylate, or tetrahydrofurfuryl (meth)acrylate is preferable.

(Component A1)

The ester compound-containing composition according to the first aspect comprises a compound (component A1) represented by Formula (a1).

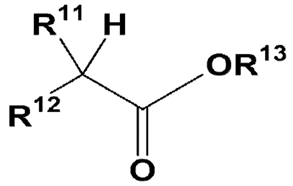

(a 1)

In Formula (a1), $R^{11}$, $R^{12}$, $R^{13}$ may be each independently a group selected from a hydrogen atom, an alkyl group, an alkenyl group, an aryl group, a hydroxy group, an alkoxy group, an amino group, a carbonyl group, and a monovalent group having an ether bond and/or a sulfide bond, or $R^{11}$, $R^{12}$, and $R^{13}$ may be each a divalent group in combination with any of the groups, which may have a substituent.
"α-hydrogen" in the component A1 represents a hydrogen atom bonded to a carbon atom adjacent to a carbon atom of a carbonyl group. In the ester compound-containing composition according to the first aspect, the presence of the component A1 makes it possible to suppress the generation of a dimer of (meth)acrylic acid ester and a pyruvic acid ester. In addition, by coexisting the component B (polymerization inhibitor) described later in addition to the ester compound (component A1) having an α-hydrogen, it is possible to more efficiently suppress the generation of a dimer of (meth)acrylic acid ester and a pyruvic acid ester. The mechanism can be estimated as follows.
The component B described later suppresses a polymerization reaction of the ester compound (I) by radical polymerization, by trapping radicals generated in the ester compound (I). However, a dimerization reaction of the ester compound (I) also proceeds by anionic polymerization under basic conditions. Since the ester compound having an α-hydrogen has weak acidity and can trap an anion which causes the anionic polymerization, the component A1 can suppress the dimerization reaction of the ester compound (I) by anionic polymerization. On the other hand, the pyruvic acid ester is produced by oxidizing the ester compound (I) with a hydroxyl radical and an oxygen molecule. The component B can trap the hydroxyl radical, and the component A1 can trap a radical intermediate generated by a reaction between the hydroxyl radical and the ester compound (I) and can return the intermediate to the ester compound (I). Therefore, it is

considered that the coexistence of the component A1 and the component B can efficiently suppress the generation of the pyruvic acid ester.

The molecular weight of the component A1 is preferably 1,000 or less. When the molecular weight of the component A1 is 1,000 or less, the number of α-hydrogens per unit mass in the component A1 can be increased, and thus the effect of the present invention can be obtained with a small mass. In addition, the molecular weight of the component A1 is more preferably 800 or less, still more preferably 600 or less, and particularly preferably 400 or less.

In Formula (a1), $R^{11}$, $R^{12}$, $R^{13}$ may be each independently a group selected from a hydrogen atom, an alkyl group, an alkenyl group, an aryl group, a hydroxy group, an alkoxy group, an amino group, a carbonyl group, and a monovalent group having an ether bond and/or a sulfide bond, or $R^{11}$, $R^{12}$, and $R^{13}$ may be each a divalent group in combination with any of the groups, which may have a substituent. $R^{11}$ and $R^{12}$, $R^{12}$ and $R^{13}$, or $R^{13}$ and $R^{11}$ may be the same or different from each other.

In general, the α-hydrogen of the ester compound has a property of reacting with an anion or a radical, but reactivity may be reduced depending on the type of a substituent. When $R^{11}$, $R^{12}$, and $R^{13}$ satisfy the above-described conditions, the reactivity of the α-hydrogen in the component A1 with an anion or a radical is maintained, and thus the effect of the present invention can be obtained.

$R^{11}$ and $R^{12}$ are preferably a hydrogen atom, an alkyl group having 1 to 5 carbon atoms, a hydroxy group, an alkoxy group having 1 to 6 carbon atoms, an amino group, a monovalent group including a carbonyl group, or an alkylthio group having 1 to 5 carbon atoms; more preferably a hydrogen atom or an alkyl group having 1 to 5 carbon atoms; and still more preferably a hydrogen atom, a methyl group, an ethyl group, an n-propyl group, or an isopropyl group. Since these groups are substituents having high stability, the component A1 can be prevented from being changed into other compounds during storage. In addition, since the above-described substituent has low electron-donating properties, the acidity of the α-hydrogen of the component A1 is improved.

$R^{13}$ is preferably a chain or cyclic alkyl group having 1 to 20 carbon atoms, a chain alkenyl group having 2 to 5 carbon atoms, an aryl group having 5 to 12 carbon atoms, a hydroxyalkylene group having 2 to 8 carbon atoms, an alkyl group having at least one acyloxy group having 2 to 20 carbon atoms, or a group having an etheric oxygen between carbon atoms of a hydrocarbon group having 2 to 8 carbon atoms; more preferably a chain alkyl group having 1 to 10 carbon atoms, an allyl group, a 2-hydroxyethyl group, a 2-hydroxypropyl group, a 2-methoxyethyl group, a glycidyl group, or a tetrahydrofurfuryl group; still more preferably a butyl group, an isobutyl group, a t-butyl group, an allyl group, or a 2-hydroxyethyl group; particularly preferably a butyl group or an isobutyl group; and most preferably a butyl group. Since these groups have substituents similar to those of the ester compound (I), when a (meth)acrylic polymer is produced by polymerization of the ester compound-containing composition, it is possible to prevent physical properties of the polymer from being adversely affected.

The alkyl group is a chain (linear or branched) alkyl group or a cyclic alkyl group. As the chain alkyl group, a chain alkyl group having 1 to 20 carbon atoms is preferable, a chain alkyl group having 1 to 10 carbon atoms is more preferable, and a chain alkyl group having 1 to 5 carbon atoms is still more preferable. Examples of the chain alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, and a decyl group; and a methyl group, an ethyl group, an n-propyl group, or an isopropyl group is preferable. In addition, as the cyclic alkyl group, a cyclic alkyl group having 3 to 20 carbon atoms is preferable, a cyclic alkyl group having 4 to 10 carbon atoms is more preferable, and a cyclic alkyl group having 5 to 7 carbon atoms is still more preferable. Examples of the cyclic alkyl group include a cyclopentyl group, a cyclohexyl group, and a cyclooctyl group. In addition, the cyclic alkyl group may be an aromatic alkyl group.

The alkenyl group is a chain (linear or branched) alkenyl group or a cyclic alkenyl group. As the chain alkenyl group, a chain alkenyl group having 2 to 20 carbon atoms is preferable, a chain alkenyl group having 2 to 10 carbon atoms is more preferable, and a chain alkenyl group having 2 to 5 carbon atoms is still more preferable. Examples of the chain alkenyl group include a vinyl group, a 1-propenyl group, an isopropenyl group, a 2-butenyl group, a 1,3-butadienyl group, a 2-pentenyl group, and a 2-hexenyl group. As the cyclic alkenyl group, a cyclic alkenyl group having 3 to 20 carbon atoms is preferable, a cyclic alkenyl group having 4 to 10 carbon atoms is more preferable, and a cyclic alkenyl group having 5 to 7 carbon atoms is still more preferable. Examples of the cyclic alkenyl group include a cyclopentenyl group and a cyclohexenyl group.

The aryl group is preferably an aryl group having 5 to 20 carbon atoms, and more preferably an aryl group having 5 to 12 carbon atoms. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a thiophenyl group, a triazolyl group, a tetrazolyl group, a pyridyl group, a pyrazinyl group, a pyrimidinyl group, a pyridazinyl group, a pyrazolyl group, a pyrrolyl group, a pyranyl group, a furyl group, a furazanyl group, an imidazolidinyl group, an isoquinolyl group, an isoindolyl group, an indolyl group,

a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

Examples of the monovalent group including a hydroxy group include a hydroxy group, a hydroxymethyl group, a 1-hydroxyethyl group, a 2-hydroxyethyl group, a 2-hydroxypropyl group, and a 3-hydroxypropyl group.

The alkoxy group is preferably an alkoxy group having 1 to 20 carbon atoms, more preferably an alkoxy group having 1 to 10 carbon atoms, and still more preferably an alkoxy group having 1 to 6 carbon atoms. Examples of the alkoxy group include a methoxy group, an ethoxy group, a butoxy group, and a phenoxy group.

The amino group includes an amino group ($-NH_2$) having no substituent on a nitrogen atom and an amino group in which a part or all of hydrogen atoms bonded to the nitrogen atom are substituted with carbon atoms. The number of carbon atoms in the amino group substituted with carbon atoms is preferably 1 to 20, more preferably 1 to 10, and still more preferably 1 to 5. Examples of the amino group include a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group include a formyl group, an acyl group, an acyloxy group, a carboxy group, an amide group, an alkoxycarbonyl group, a thiocarboxy group, and a thioester group.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6. Examples of the acyl group include an acetyl group, a propionyl group, a butyryl group, an isobutyryl group, a valeroyl group, an acryloyl group, a methacryloyl group, and a benzoyl group.

The acyloxy group is a substituent in which a carbonyl group is linked to an alkyl group, an alkenyl group, or an aryl group, and the carbonyl group is further linked to an ether bond. The total number of carbon atoms derived from the carbonyl group of the acyl group and of carbon atoms derived from the alkyl group, the alkenyl group, or the aryl group is preferably 2 to 20, more preferably 2 to 10, and still more preferably 2 to 6. Examples of the acyloxy group include an acetyloxy group, a propionyloxy group, a butyryloxy group, an isobutyryloxy group, a valeryloxy group, an acryloyloxy group, a methacryloyloxy group, and a benzoyloxy group.

Examples of the monovalent group including an acyloxy group include 2-acyloxyethyl, 2-acyloxypropyl, and 2,2-bis(acyloxymethyl)butyl.

The amide group includes an amide group ($-CONH_2$) having no substituent on a nitrogen atom and an amide group in which a part or all of hydrogen atoms bonded to the nitrogen atom are substituted with carbon atoms. As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group. The total number of carbon atoms derived from the carbonyl group and carbon atoms derived from the alkoxy group is preferably 2 to 20, more preferably 2 to 10, and still more preferably 2 to 6. Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to a sulfide bond and the sulfide bond is linked to an alkyl group or an aryl group. The total number of carbon atoms derived from the carbonyl group and carbon atoms derived from the alkyl group or the aryl group is preferably 2 to 20, more preferably 2 to 10, and still more preferably 2 to 6.

Examples of the thioester group include a methylthiocarbonyl group, an ethylthiocarbonyl group, a butylthiocarbonyl group, and a phenylthiocarbonyl group.

As an example, the monovalent group including a carbonyl group may be a substituent in which one or a plurality of hydrogens of an alkyl group are substituted with carbonyl groups. Examples of such a substituent include a 2-acetoxyethyl group, a 2-acetoethyl group, and a 2-(acetoacetoxy)ethyl group.

Examples of the monovalent group including an ether bond include a group having an etheric oxygen between two carbon atoms. The number of etheric oxygens included in the above-described group is preferably 1, but it is not limited thereto and may be 2 or more. In addition, the above-described group may be linear or branched, or may have a ring. When the above-described group has a ring, the ring may or may not include the etheric oxygen. The number of carbon atoms in the above-described group is preferably 2 to 8 and more preferably 2 to 7. Examples of such a substituent include a 2-methoxyethyl group, a 2-(2-methoxyethoxy)ethyl group, a 2-[2-(2-methoxyethoxy)ethoxy]ethyl group, a glycidyl group, and a tetrahydrofurfuryl group.

Examples of the monovalent group including a sulfide bond include an alkylthio group. The alkylthio group is a substituent in which an alkyl group is linked to a sulfide bond; and an alkylthio group having 1 to 20 carbon atoms is preferable, an alkylthio group having 1 to 10 carbon atoms is more preferable, and an alkylthio group having 1 to 5 carbon

atoms is still more preferable. Examples of the alkylthio group include a methylthio group, an ethylthio group, a propylthio group, and an isopropylthio group.

$R^{11}$ and $R^{12}$, $R^{12}$ and $R^{13}$, or $R^{13}$ and $R^{11}$ may be linked to each other to form a ring. Examples of a compound in which $R^{11}$ and $R^{12}$ are linked to each other to form a ring and a compound in which $R^{13}$ and $R^{11}$ are linked to each other to form a ring include methyl cyclohexanecarboxylate and methyl cyclopentanecarboxylate. Examples of a compound in which $R^{12}$ and $R^{13}$ are linked to each other to form a ring include α-methyl-δ-valerolactone and α-methyl-γ-butyrolactone.

Among the compounds satisfying the above-described conditions, from the viewpoint of quality stability of the ester compound-containing composition during storage, as the component A1, methyl isobutyrate, butyl isobutyrate, methyl butyrate, isobutyl butyrate, methyl propionate, ethyl propionate, butyl propionate, isobutyl propionate, butyl butyrate, isobutyl butyrate, ethyl isobutyrate, phenyl isobutyrate, isobutyl isobutyrate, methyl isovalerate, methyl 2-methylbutyrate, isoamyl isobutyrate, methyl lactate, methyl 2-methoxypropionate, N,N-dimethylglycine methyl ester, dimethyl malonate, methyl methylthioacetate, methyl 3-butenoate, methyl (R)-(-)-3-hydroxyisobutyrate, methyl acetate, ethyl acetate, butyl acetate, isobutyl acetate, phenyl acetate, methyl cyclohexanecarboxylate, methyl cyclopentanecarboxylate, α-methyl-δ-valerolactone, or α-methyl-γ-butyrolactone is preferable; butyl butyrate, isobutyl butyrate, butyl propionate, butyl acetate, methyl isobutyrate, methyl isovalerate, methyl 2-methylbutyrate, isoamyl isobutyrate, methyl lactate, methyl N,N-dimethylglycine, dimethyl malonate, methyl methylthioacetate, methyl 3-butenoate, methyl (R)-(-)-3-hydroxyisobutyrate, or methyl cyclohexanecarboxylate is more preferable; and methyl isobutyrate, methyl propionate, isobutyl isobutyrate, or methyl 2-methylbutyrate is still more preferable.

The component A1 may be one kind or two or more kinds.

(Polymerization inhibitor (component B))

The ester compound-containing composition according to the first aspect may further comprise a polymerization inhibitor (component B). The polymerization inhibitor means a compound having a function of suppressing the polymerization reaction of the ester compound (I).

When the ester compound-containing composition comprises the component B, the progress of the polymerization reaction of the ester compound (I) by radical polymerization mechanism during storage is suppressed. In addition, during storage, oxygen molecules in the ester compound-containing composition absorb ultraviolet rays derived from sunlight, thereby generating a hydroxyl radical. However, the polymerization inhibitor can trap the hydroxyl radical. Therefore, when the ester compound-containing composition comprises the component A1 and the component B, the amount of the hydroxyl radical can be reduced by two different mechanisms of suppressing the generation of the hydroxyl radical by the component A1 and removing the generated hydroxyl radical by the component B even when the hydroxyl radical is generated. Accordingly, the progress of dimerization of the ester compound (I) and the generation of the oxidative product can be suppressed more efficiently.

Examples of the polymerization inhibitor include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

In addition, as described above, by coexisting the component A1 and the component B, it is possible to efficiently suppress generation of methyl pyruvate due to the oxidation of the ester compound (I).

Examples of the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and

hydroquinone monobenzate.

Examples of the tocopherol include α-tocopherol and 2,3-dihydro-2,2-dimethyl-7-hydroxybenzofuran.

Examples of the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the nitrobenzene-based compound include nitrobenzene, o-dinitrobenzene, m-dinitrobenzene, p-dinitrobenzene, 2,4-dinitrobenzene, dinitrodurene, and 2,2-diphenyl-1-picrylhydrazine.

Examples of the N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethyl-piperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-t-butyl nitroxide, and di-t-amyl nitroxide.

Examples of the amine-based compound include N,N-diphenylamine, alkylated diphenylamine, 4,4'-dicamyldiphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl) phosphite, 4,4'-isopropylidenediphenol alkylphosphite, tris(2,4-di-t-butylphenyl)phosphite, tris(biphenyl)phosphite, distearyl pentaerythritol diphosphite, di(2,4-di-t-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-t-butylphenol) diphosphite, hexa(tridecyl)-1,1,3-tris(2-methyl-4-hydroxy-5-t-butylphenyl)butanetriphosphite, 3,5-dit-butyl-4-hydroxybenzyl phosphate diethyl ester, sodium-bis(4-t-butylphenyl)phosphate, sodium-2,2'-methylene-bis(4,6-di-t-butylphenyl)phosphate, and 1,3-bis(diphenoxyphosphoryloxy)benzene.

Examples of the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the iron-containing compound include iron (III) chloride.

Examples of the copper-containing compound include copper dimethyldithiocarbamate, copper diethylthiocarbamate, copper dibutylthiocarbamate, copper salicylate, copper acetate, copper thiocyanate, copper nitrate, copper chloride, copper carbonate, copper hydroxide, copper acrylate, and copper methacrylate.

Examples of the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

From the viewpoint of easily exhibiting the effect of further improving the storage stability of the ester compound-containing composition, as the component B, at least one polymerization inhibitor selected from the group consisting of a phenol-based compound, an N-oxyl-based compound, an amine-based compound, a phosphorus-containing compound, and a sulfur-containing compound is preferable; at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is more preferable; and at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, and phenothiazine is particularly preferable.

The component B may be used alone or a combination of two or more kinds thereof may be used.

When the ester compound-containing composition comprises a compound corresponding to both the component A1 and the component B, the compound is regarded as the component A1. When the ester compound-containing

composition comprises the component A1 and the component B, this means that the ester compound-containing composition further comprises the component B different from the compound.

When the ester compound-containing composition comprises two or more kinds of compounds corresponding to both the component A1 and the component B, a compound having the highest molar concentration in the ester compound-containing composition is regarded as the component A1, and the other compounds are regarded as the component B.

(Component C)

The component C is a compound other than the ester compound (I), the component A1, and the component B.

The ester compound-containing composition according to the embodiment of the present invention may comprise the component C as long as the contained amount of the ester compound (I) satisfies the range of 95.00% to 99.99% by mass with respect to the total mass of the ester compound-containing composition.

Examples of the component C include impurities generated in the process of producing the ester compound (I); and examples thereof include additives such as a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, and a fluorescent agent.

The ester compound-containing composition may comprise unreacted raw materials in the production of the ester compound-containing composition, such as methyl (meth)acrylate, alcohol, and (meth)acrylic acid.

In addition, the ester compound-containing composition may comprise impurities generated during the production of the ester compound-containing composition, such as diacetyl. The diacetyl may be contained as, for example, an impurity when an ester compound is produced by a C4 direct oxidation method. From the viewpoint of suppressing coloration of the ester compound-containing composition, it is preferable that diacetyl is not contained. However, when the diacetyl is contained as an unavoidable impurity, from the above-described viewpoint, the contained amount of the diacetyl per 1 L of the ester compound-containing composition is preferably 5 ppm by mass or less, more preferably 2 ppm by mass or less, still more preferably 1 ppm by mass or less, and particularly preferably 0.1 ppm by mass or less.

Furthermore, the ester compound-containing composition may comprise a (meth)acrylic acid ester other than the ester compound (I).

(Contained amount of ester compound (I))

The contained amount of the ester compound (I) in the ester compound-containing composition is 95.00% to 99.99% by mass with respect to the total mass of the ester compound-containing composition. When the contained amount of the ester compound (I) is the above-described lower limit value or more, the amount of impurities when the (meth)acrylic polymer is produced by polymerization of the ester compound-containing composition can be reduced, and thus it is possible to prevent physical properties of the polymer from being adversely affected. In addition, when the contained amount of the ester compound (I) is the above-described upper limit value or less, a purification cost can be suppressed. The contained amount of the ester compound (I) is preferably 96.00% by mass or more, more preferably 97.00% by mass or more, still more preferably 98.00% by mass or more, particularly preferably 99.00% by mass or more, and most preferably 99.50% by mass or more.

When the ester compound (I) includes the ester compound (1) and does not include the ester compound (2), the ester compound (3), the ester compound (4), and the ester compound (5), the contained amount of the ester compound (I) is the concentration of the ester compound (1).

When the ester compound (I) includes the ester compound (5) and does not include the ester compound (1), the ester compound (2), the ester compound (3), and the ester compound (4), the contained amount of the ester compound (I) is the concentration of the ester compound (5).

When the ester compound (I) includes at least one selected from the group consisting of the ester compound (2), the ester compound (3), and the ester compound (4), and does not include the ester compound (1) and the ester compound (5), the contained amount of the ester compound (I) is the concentration of at least one selected from the group consisting of the ester compound (2), the ester compound (3), and the ester compound (4).

The contained amount of the component A1 per 1 L of the ester compound-containing composition is not particularly limited, but is preferably 1 to 10,000 ppm by mass. When the contained amount of the component A1 is the above-described lower limit value or more, an effect of suppressing a decrease in purity of the ester compound (I) due to the dimerization of the ester compound (I) and the generation of the oxidative product is sufficiently obtained. When the contained amount of the component A1 is the above-described upper limit value or less, the amount of impurities generated when the (meth)acrylic polymer is produced by polymerization of the ester compound-containing composition can be suppressed, and thus it is possible to prevent physical properties of the polymer from being adversely affected.

The lower limit of the contained amount of the component A1 is more preferably 3 ppm by mass or more, still more preferably 5 ppm by mass or more, and particularly preferably 10 ppm by mass or more. In addition, the upper limit of the

contained amount of the component A1 is more preferably 7,500 ppm by mass or less, still more preferably 5,000 ppm by mass or less, particularly preferably 1,000 ppm by mass or less, and most preferably 500 ppm by mass or less.

The contained amount of the component B per 1 L of the ester compound-containing composition is not particularly limited, but is preferably 1 to 1,000 ppm by mass. When the contained amount of the component B is the above-described lower limit value or more, an effect of suppressing a decrease in purity of the ester compound (I) due to the dimerization of the ester compound (I) and the generation of the oxidative product is sufficiently obtained. When the contained amount of the component B is the above-described upper limit value or less, the amount of impurities when the (meth)acrylic polymer is produced by polymerization of the ester compound-containing composition can be reduced, and thus it is possible to prevent physical properties of the polymer from being adversely affected.

The contained amount of the component B is more preferably 3 ppm by mass or more, still more preferably 5 ppm by mass or more, and particularly preferably 10 ppm by mass or more. In addition, the contained amount of the component B is more preferably 750 ppm by mass or less, still more preferably 500 ppm by mass or less, even more preferably 250 ppm by mass or less, particularly preferably 100 ppm by mass or less, and most preferably 50 ppm by mass or less.

The moisture content of the ester compound-containing composition is preferably 5,000 ppm by mass or less, more preferably 4,000 ppm by mass or less, still more preferably 3,000 ppm by mass or less, particularly preferably 2,000 ppm by mass or less, and most preferably 1,000 ppm by mass or less with respect to the total mass of the ester compound-containing composition. When the moisture content of the ester compound-containing composition is the above-described upper limit value or less, the physical properties of the (meth)acrylic polymer obtained by polymerizing the ester compound-containing composition can be maintained more satisfactorily.

The lower limit value of the moisture content of the ester compound-containing composition is 0 ppm by mass.

[Analysis of ester compound-containing composition]

The component A1, the component B, the component C, and the water contained in the ester compound-containing composition can be confirmed by, for example, GC-MS measurement.

In a GC-MS chart of the ester compound-containing composition, when a peak is present at the same retention time as a sample of the component A1 and an m/z value detected in a mass spectrum of the peak matches exact mass of the component A1, it can be determined that the ester compound-containing composition comprises the component A1. When the sample of the component A1 cannot be obtained, when a pattern of the mass spectrum of the peak appearing in the GC-MS chart of the ester compound-containing composition and a pattern of a mass spectrum of the component A1 in mass spectrum database and match each other, it can be determined that the peak is the peak of the component A1 and the ester compound-containing composition comprises the component A1. Examples of the mass spectrum database include NIST 20, NIST 17, NIST 14, and NIST 14s. In addition, when volatility is low and the detection cannot be carried out by the GC-MS measurement, the detection can be carried out by LC-MS. The component B, the component C, and water can also be confirmed by the same method as that for the component A1.

The contained amount of the ester compound (I) can be calculated by performing GC-FID measurement of the ester compound-containing composition, quantifying by an area percentage method, and correcting the quantified moisture content using a Karl Fischer moisture meter.

The contained amount of the component A1 can be quantified, for example, by performing GC measurement of the ester compound-containing composition and using an internal standard method. When a sample of the component A1 cannot be obtained and the component A1 cannot be quantified by the internal standard method, the contained amount of the component A1 can be calculated using the following expression by performing GC-FID measurement on any organic compound having a known contained amount.

$$\text{Contained amount of component A1 (ppm by mass)} = \frac{N}{N_{A1}} \times \frac{S_{A1}}{S} \times M$$

In the expression, N is the number of carbon atoms in one molecule of the organic compound having a known concentration, $N_{A1}$ is the number of carbon atoms in one molecule of the component A1, $S_{A1}$ is a peak area of the component A1, S is a peak area of the organic compound, and M is a contained amount (ppm by mass) of any organic component. When the volatility is low and the quantification cannot be performed based on the GC area, the quantification can be performed using a chromatography method such as LC.

The contained amounts of the component B and the component C can also be calculated by the same method as that for the component A1.

The moisture content of the ester compound-containing composition can be confirmed by Karl Fischer method.

(Method for producing ester compound-containing composition)

A method for producing an ester compound-containing composition according to the first aspect is a method for producing an ester compound-containing composition comprising one or more ester compounds (I) selected from the group consisting of the ester compound (1), the ester compound (2), the ester compound (3), the ester compound (4), and the ester compound (5) described above.

The above-described ester compound-containing composition can be produced, for example, by performing an ester exchange reaction between methyl (meth)acrylate and various alcohols in the presence of the component A1 and the component B.

Among (meth)acrylic acid esters, the methyl (meth)acrylate tends to particularly easily undergo dimerization or oxidation to produce a pyruvic acid ester. However, by performing the ester exchange reaction in the presence of the component A1 and the component B, the dimerization of methyl (meth)acrylate or the generation of methyl pyruvate is suppressed, and as a result, the yield of the ester compound (I) is improved.

The method for producing an ester compound-containing composition according to the first aspect includes a step E of performing an ester exchange reaction between methyl (meth)acrylate and an alcohol (component D) in the presence of the component A1 and the component B.

When the ester compound (I) includes the ester compound (1), a monoalcohol having 2 to 20 carbon atoms is used in the step E as the component D.

When the ester compound (I) includes one or more selected from the group consisting of the ester compound (2), the ester compound (3), and the ester compound (4), one or more alcohols selected from the group consisting of a dialcohol having 2 to 8 carbon atoms and a trialcohol having 2 to 8 carbon atoms are used in the step E as the component D.

When the ester compound (I) includes the ester compound (5), an ether bond-containing alcohol having 2 to 8 carbon atoms is used in the step E as the component D.

For example, when obtaining the ester compound-containing composition comprising the ester compound (1) as the ester compound (I), in a reaction vessel, the methyl (meth)acrylate is subjected to an ester exchange with the monoalcohol having 2 to 20 carbon atoms as represented by Formula (II) in the presence of a catalyst, the component A1, and the component B.

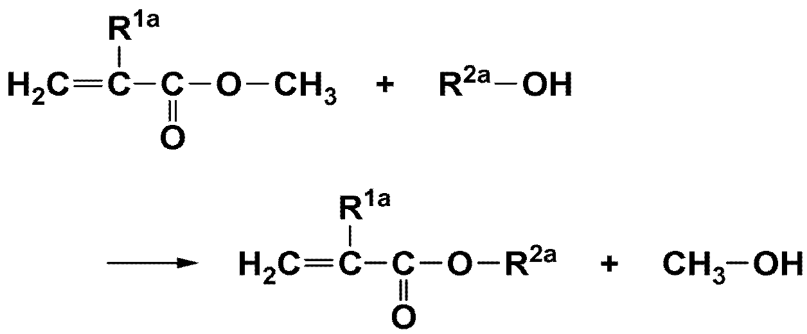

· · · (II)

$R^{1a}$ and $R^{2a}$ in Formula (II) are the same as $R^{1a}$ and $R^{2a}$ in Formula (1).

Examples of the monoalcohol having 2 to 20 carbon atoms include ethanol, n-butanol, isobutanol, t-butanol, 2-ethylhexanol, lauryl alcohol, stearyl alcohol, isobornyl alcohol, allyl alcohol, phenol, and benzyl alcohol.

The monoalcohol having 2 to 20 carbon atoms may be used alone or a combination of two or more kinds thereof may be used. The monoalcohol having 2 to 20 carbon atoms preferably includes a linear or branched monoalcohol having 2 to 20 carbon atoms, and more preferably includes n-butanol or isobutanol.

When obtaining the ester compound-containing composition comprising one or more selected from the group consisting of the ester compound (2), the ester compound (3), and the ester compound (4) as the ester compound (I), for example, in a reaction vessel, the methyl (meth)acrylate is subjected to an ester exchange with one or more alcohols selected from the group consisting of the dialcohol having 2 to 8 carbon atoms and the trialcohol having 2 to 8 carbon atoms in the presence of a catalyst, the component A1, and the component B.

For example, in the reaction vessel, an ester exchange reaction as represented by Formula (IV) and Formula (V) can be carried out in the presence of the catalyst, the component A1, and the component B.

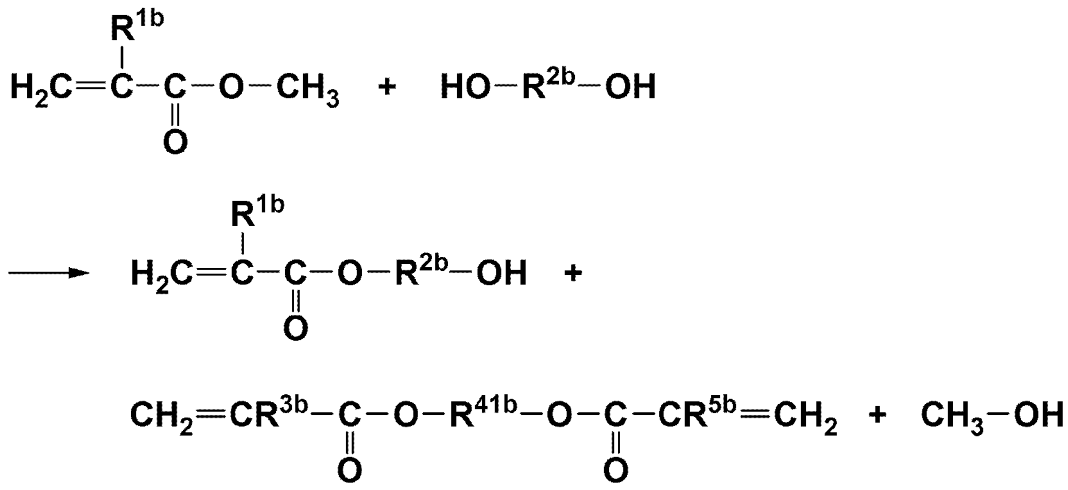

・・・(IV)

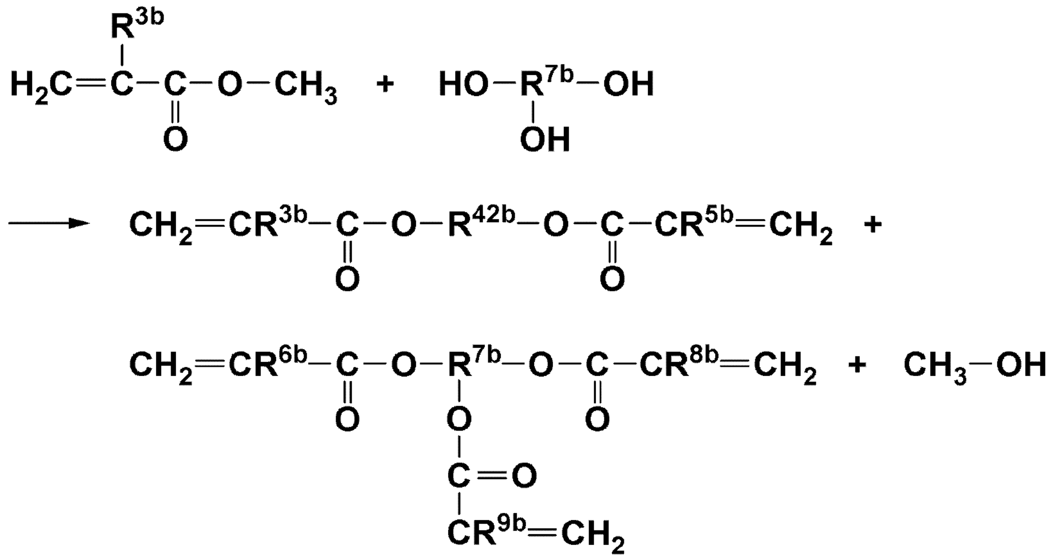

・・・(V)

$R^{1b}$, $R^{2b}$, $R^{3b}$, $R^{5b}$, $R^{6b}$, and $R^{7b}$ in Formula (IV) and Formula (V) are the same as $R^{1b}$, $R^{2b}$, $R^{3b}$ $R^{5b}$, $R^{6b}$ , and $R^{7b}$ in Formula (2), Formula (3), and Formula (4). In addition, $R^{41b}$ is a linear or branched alkylene group having 2 to 8 carbon atoms, and $R^{42b}$ is a linear or branched hydroxyalkylene group having 2 to 8 carbon atoms.

Examples of the dialcohol having 2 to 8 carbon atoms and the trialcohol having 2 to 8 carbon atoms include ethylene glycol, 1,2-propanediol, 1,3-propanediol, 1,3-butanediol, 1,6-hexanediol, and trimethylolpropane. Among these, it is preferable that the dialcohol having 2 to 8 carbon atoms and the trialcohol having 2 to 8 carbon atoms include one or more selected from the group consisting of ethylene glycol, 1,2-propanediol, 1,3-propanediol, and trimethylolpropane.

The dialcohol having 2 to 8 carbon atoms and the trialcohol having 2 to 8 carbon atoms may be used alone or a combination of two or more kinds thereof may be used.

When obtaining the ester compound-containing composition comprising the ester compound (5) as the ester compound (I), for example, in a reaction vessel, the methyl (meth)acrylate is subjected to an ester exchange with the ether bond-containing alcohol having 2 to 8 carbon atoms as represented by Formula (III) in the presence of a catalyst and the component A1.

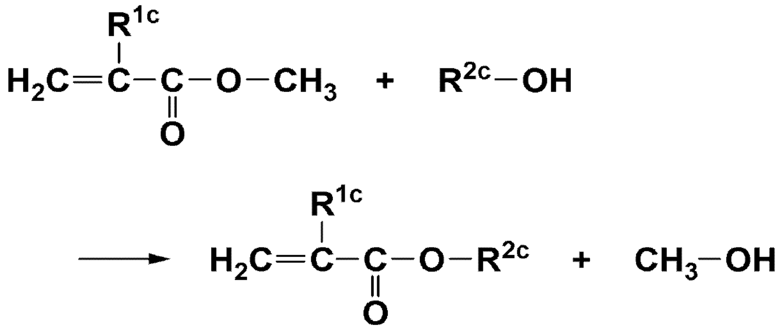

・・・(III)

$R^{1c}$ and $R^{2c}$ in Formula (III) are the same as $R^{1c}$ and $R^{2c}$ in Formula (5).

The number of ether bonds in the ether bond-containing alcohol having 2 to 8 carbon atoms is preferably 1, but the it is not limited thereto.

Examples of the ether bond-containing alcohol having 2 to 8 carbon atoms include 2-methoxyethanol, glycidyl alcohol, and tetrahydrofurfuryl alcohol. Among these, the ether bond-containing alcohol preferably includes one selected from 2-methoxyethanol, glycidyl alcohol, and tetrahydrofurfuryl alcohol.

The ether bond-containing alcohol having 2 to 8 carbon atoms may be used alone or a combination of two or more kinds thereof may be used.

The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic mixture with the methyl (meth)acrylate to the outside of the system.

Examples of the above-described reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

The ratio of the amount of the methyl (meth)acrylate to be charged and the amount of the alcohol to be charged can be appropriately determined. From the viewpoint of improving productivity, the ratio of the methyl (meth)acrylate to 1 mol of alcohol is preferably 0.1 to 10 mol, and more preferably 0.3 to 4 mol.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction product during the ester exchange reaction and high catalytic activity, an alkoxide of titanium, dibutyltin oxide, or dioctyltin oxide is preferable. The above-described catalyst may be used alone or a combination of two or more kinds thereof may be used.

The catalyst can be supplied alone to the reaction vessel. In addition, the catalyst can also be supplied to the reaction vessel in a state of being dissolved in the same alcohol as the alcohol as the raw material, or in a state of being dissolved in the methyl (meth)acrylate as the raw material. Examples thereof include a method of directly dissolving the catalyst in the total amount of the alcohol used in the reaction and supplying the catalyst to the reaction vessel, and a method of dissolving the catalyst in a part of the alcohol used in the reaction and supplying the catalyst to the reaction vessel.

The amount of the catalyst used is preferably 0.001 to 1 mol% and more preferably 0.01 to 0.1 mol% with respect to 1 mol of the alcohol.

A solvent may be used for the ester exchange reaction. When a solvent is used, it is preferable to use a solvent which forms an azeotropic composition with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or a combination of two or more kinds thereof may be used.

The reaction temperature for the ester exchange reaction varies depending on the type of the alcohol or the solvent, but is preferably 60°C to 150°C.

The reaction pressure for the ester exchange reaction is not particularly limited, and the reaction may be carried

out under any pressure of reduced pressure, normal pressure, or elevated pressure.

The form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, various methods such as distillation, crystallization, extraction, and column chromatography can be performed.

In the step E, the ester exchange reaction may be performed by blending the component B into the reaction solution. When the component B is present in addition to the component A1, the dimerization of methyl (meth)acrylate or the generation of methyl pyruvate is further suppressed, and thus an effect of improving the yield of the ester compound (I) is more easily obtained.

The component B may be blended into a solution containing the ester compound (I) after the ester exchange reaction and the component A1. In this case, a solution (B solution) containing the ester compound (I) and the component B is prepared separately from the solution (A solution) containing the ester compound (I) and the component A1, and the A solution and the B solution are mixed with each other to obtain the ester compound-containing composition. In addition, the ester compound (I), the A solution, and the B solution may be mixed with each other to obtain the ester compound-containing composition.

The method for producing an ester compound-containing composition is not limited to the above-described method.

For example, other (meth)acrylic acid esters other than the methyl (meth)acrylate may be used as the raw material instead of the methyl (meth)acrylate.

An esterification reaction between an alcohol and (meth)acrylic acid may be carried out to produce the ester compound (1).

(Evaluation method for storage stability and thermal stability of ester compound-containing composition)

The ester compound-containing composition has high quality stability during storage. Examples of an evaluation method of the quality stability of the ester compound-containing composition during storage include a method of actually storing the ester compound-containing composition for a long period of time and confirming the generation amount of the dimer of the ester compound (I), the generation amount of the oxidative product of the ester compound (I), and the contained amount of the ester compound (I). In addition, from the viewpoint of ease of work, a method of heating the ester compound-containing composition short period of time and confirming the generation amount of the dimer of the ester compound (I), the generation amount of the oxidative product of the ester compound (I), and the contained amount of the ester compound (I) may be used. When heating short period of time, the heating temperature is preferably 50°C to 100°C and a heating time is preferably 1 to 24 hours. When the ester compound-containing composition is stored at 25°C for 14 days or heated at 70°C for 7 to 20 hours, the quality stability of the ester compound-containing composition during storage is evaluated based on the generation amount of the dimer of the ester compound (I), the generation amount of the oxidative product of the ester compound (I), and the contained amount of the ester compound (I).

[Polymerizable composition]

A polymerizable composition according to the first aspect is a polymerizable composition for producing a (meth) acrylic polymer, and comprises the above-described ester compound-containing composition according to the first aspect. As an example, an ester compound-containing composition stored for 1 day or more after production can be used as the polymerizable composition.

The storage time of the ester compound-containing composition can be 1 day or more, 3 days or more, 7 days or more, or 14 days or more. In addition, the storage time of the ester compound-containing composition may be 180 days or less, 150 days or less, 120 days or less, or 90 days or less. The "storage time" means an elapsed time from immediately after the production, which is not limited to a time during which the product is left to stand under a specific environment and also includes a time of transportation or the like.

The material of a storage container for the ester compound-containing composition is not particularly limited, and for example, a metal container such as stainless steel, a resin container, or a glass container can be used. **In** this case, for example, a transparent container or an opaque container can be used.

The temperature at the time of storing the ester compound-containing composition is preferably -10°C or higher and more preferably 0°C or higher, and is preferably 60°C or lower and more preferably 50°C or lower. By setting the above-described storage temperature to -10°C or higher, a load on a cooling device can be reduced, and by setting the above-described storage temperature to 60°C or lower, it is easy to suppress the generation of the dimerized substance or the oxidative product of (meth)acrylic acid ester.

The oxygen concentration of a gas phase portion at the time of storing the ester compound-containing

composition is preferably 5% by volume or more and more preferably 7% by volume or more, and is preferably 30% by volume or less and more preferably 22% by volume or less. By setting the oxygen concentration of the gas phase portion during the storage to 5% by volume or more, it is easy to prevent the ester compound from being unintentionally polymerized by a polymerization prevention effect of oxygen, and by setting the oxygen concentration to 30% by volume or less, it is easy to suppress oxidation of the (meth)acrylic acid ester by oxygen and the generation of various impurities.

As an example, the ester compound-containing composition after the storage may be used in the polymerizable composition without further blending a monomer, or a monomer (hereinafter, also referred to as "other monomer") copolymerizable with the ester compound (1) may be further blended with the ester compound-containing composition after the storage to obtain the polymerizable composition.

As another example, the ester compound-containing composition after the production may be stored in a state in which the other monomer is blended, and used in the polymerizable composition. In this case, it is preferable that a polymerization initiator is not blended during the storage.

The contained amount of the ester compound (I) with respect to the total mass of monomers in the polymerizable composition is preferably 10% by mass or more and more preferably 20% by mass or more, and preferably 90% by mass or less and more preferably 80% by mass or less.

The above-described upper limit and lower limit of the contained amount of the ester compound (I) can be combined arbitrarily. For example, the contained amount of the ester compound (I) is preferably 10% to 90% by mass and more preferably 20% to 80% by mass.

Examples of the other monomer include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.

Examples of the unsaturated carboxylic acid include acrylic acid, methacrylic acid, maleic acid, and itaconic acid.

Examples of the unsaturated carboxylic acid anhydride include maleic acid anhydride and itaconic acid anhydride.

Examples of the maleimide include N-phenylmaleimide and N-cyclohexylmaleimide.

Examples of the hydroxy group-containing vinyl monomer include 2-hydroxyethyl acrylate, 2-hydroxyethyl methacrylate, and 2-hydroxypropyl methacrylate.

Examples of the vinyl ester include vinyl acetate and vinyl benzoate.

Examples of the nitrogen-containing vinyl monomer include methacrylamide and acrylonitrile.

Examples of the epoxy group-containing monomer include glycidyl acrylate and glycidyl methacrylate.

Examples of the aromatic vinyl monomer include styrene and a-methyl styrene.

Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.

Examples of the polyoxyalkylene glycol di(meth)acrylate include diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate.

Examples of the vinyl monomer having two or more ethylenically unsaturated bonds in the molecule include divinylbenzene.

As the other monomer, for example, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.

The other monomer may be used alone or a combination of two or more kinds thereof may be used.

When the component A1 is a monomer copolymerizable with the ester compound (I), the component A1 may be used as the monomer copolymerizable with the ester compound (I), or a monomer copolymerizable with the ester compound (I) may be used separately from the component A1.

The contained amount of the other monomer in the polymerizable composition is preferably 0 to 50 parts by mass with respect to 100 parts by mass of the ester compound (I). As a result, a (meth)acrylic polymer having high transparency can be obtained. The upper limit of the contained amount of the other monomer is more preferably 40 parts by mass or less and still more preferably 30 parts by mass or less with respect to 100 parts by mass of the ester compound (I). The lower limit of the contained amount of the other monomer is more preferably 0.01 parts by mass or more, still more preferably 0.1 parts by mass or more, and particularly preferably 1 part by mass or more with respect to 100 parts by mass of the ester compound (I).

The above-described upper limit and lower limit of the contained amount of the other monomer can be combined arbitrarily. For example, the contained amount of the other monomer is preferably 0 to 50 parts by mass, more preferably 0.01 to 40 parts by mass, still more preferably 0.1 to 30 parts by mass, and particularly preferably 1 to 30 parts by mass with respect to 100 parts by mass of the ester compound (I).

The polymerizable composition preferably comprises a polymerization initiator.

Examples of the polymerization initiator include an azo compound, an organic peroxide, a persulfate compound, and a redox polymerization initiator.

The polymerization initiator may be used alone or a combination of two or more kinds thereof may be used.

Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.

Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.

Examples of the persulfate compound include potassium persulfate.

The additional amount of the polymerization initiator is not particularly limited, and for example, can be 0.005 to 5 parts by mass with respect to 100 parts by mass of the total mass of the monomers in the polymerizable composition.

The polymerizable composition may further comprise, as necessary, other additives such as a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, and a fluorescent agent.

The other additives may be used alone or a combination of two or more kinds thereof may be used.

[Method for producing (meth)acrylic polymer]

A (meth)acrylic polymer can be produced by polymerizing the polymerizable composition.

The polymerization method is not particularly limited, and examples thereof include a bulk polymerization method, a solution polymerization method, an emulsion polymerization method, and a suspension polymerization method. Among these, from the viewpoint of environmental load due to the use of the solvent or the like and viewpoint of transparency of the obtained (meth)acrylic polymer, a bulk polymerization method is preferable.

The method of the bulk polymerization method is not particularly limited, and examples thereof include various casting polymerization methods such as a cell casting method and a continuous casting method.

The casting polymerization method is a method in which the (meth)acrylic polymer is obtained by casting and polymerizing the polymerizable composition in a mold made of two inorganic glass plates or metal plates (for example, SUS plates) arranged facing each other at a predetermined interval with the periphery sealed with a gasket such as a soft resin tube.

The mold for the casting polymerization is not particularly limited, and various molds can be used. Examples of a mold for cell casting include a mold in which two plate-shaped products such as an inorganic glass plate, a chromium-plated metal plate, and a stainless steel plate are arranged facing each other at a predetermined interval, and a gasket is disposed on edges of the plates to form a sealed space between the plate-shaped products and the gasket. Examples of the mold for continuous casting include a mold in which a sealed space is formed by opposing surfaces of a pair of endless belts running in the same direction at the same speed and gaskets running at the same speed as the endless belt on both sides of the endless belt.

A gap between cavities of the molds is appropriately adjusted to obtain a resin plate having a desired thickness, and is generally 1 to 30 mm.

The polymerization temperature is preferably 125°C to 210°C and more preferably 130°C to 180°C.

The polymerization time is preferably 0.5 to 24 hours.

The weight-average molecular weight (Mw) of the (meth)acrylic polymer is not particularly limited, and can be, for example, 100,000 to 1,000,000. As the Mw of the (meth)acrylic polymer is higher, solvent resistance and chemical resistance can be further improved.

The Mw of the (meth)acrylic polymer can be controlled by adjusting the polymerization temperature, the polymerization time, the addition amount of the polymerization initiator, and the like.

The (meth)acrylic polymer according to the first aspect has excellent heat resistance and excellent meltability. For example, among (meth)acrylic polymers, a granular (meth)acrylic polymer having a large amount of resin which can be stored per unit volume and having a small energy cost during storage and transportation needs to be dissolved or melted at the time of use. The (meth)acrylic polymer according to the first aspect is easily melted, has excellent kneading properties with other resins, and has excellent solubility in a monomer, a solvent, or the like.

2. Second aspect

Hereinafter, a second aspect of the embodiment will be described.

[Ester compound-containing composition]

An ester compound-containing composition according to the second aspect comprises an ester compound (I) described later, and at least one selected from the group consisting of a compound (component A2) described later and a compound (component A6) described later. The contained amount of the ester compound (I) is 95.00% to 99.99% by mass.

The ester compound-containing composition may further comprise a polymerization inhibitor (component B) described later, in addition to the ester compound (I) and the component A2 or the component A6.

As long as the effect of the present invention is not impaired, the ester compound-containing composition may further comprise the component B, other compounds (component C), or water as necessary.

(Ester compound (I))

The ester compound (I) is a compound represented by Formula (I).

$$CH_2=CR^{150}\text{-}C(=O)\text{-}O\text{-}R^{200} \cdots \quad (I)$$

In Formula (I), $R^{150}$ is a hydrogen atom or a methyl group, and
$R^{200}$ is a monovalent hydrocarbon group having 2 to 20 carbon atoms, which may have a substituent, or a group having an etheric oxygen between carbon atoms of a hydrocarbon group having 2 to 8 carbon atoms.

The ester compound (I) may be used alone or a combination of two or more kinds thereof may be used.

As the ester compound (I), one or more compounds selected from the group consisting of an ester compound (1) described later, an ester compound (2) described later, an ester compound (3) described later, an ester compound (4) described later, and an ester compound (5) described later are preferable. Hereinafter, the ester compounds will be described.

The ester compound (1) is a (meth)acrylic acid ester represented by Formula (1).

$$CH_2=CR^{1a}\text{-}C(=O)\text{-}O\text{-}R^{2a} \cdots \quad (1)$$

In Formula (1), $R^{1a}$ is a hydrogen atom or a methyl group. $R^{2a}$ is a hydrocarbon group having 2 to 20 carbon atoms.

The hydrocarbon group of $R^{2a}$ may be a saturated hydrocarbon group or an unsaturated hydrocarbon group.

The hydrocarbon group of $R^{2a}$ may be linear or branched, or may have a ring. When the hydrocarbon group of $R^{2a}$ has a ring, the ring may be an aliphatic ring or an aromatic ring.

The number of carbon atoms in the hydrocarbon group of $R^{2a}$ is 2 to 20, preferably 2 to 18 and more preferably 2 to 12.

Examples of the hydrocarbon group of $R^{2a}$ include an alkyl group having 2 to 20 carbon atoms, a cycloalkyl group having 3 to 20 carbon atoms, an alkenyl group having 2 to 20 carbon atoms, a cycloalkenyl group having 3 to 20 carbon atoms, an alkynyl group having 2 to 20 carbon atoms, an aryl group having 6 to 20 carbon atoms, and an aromatic alkyl group having 7 to 20 carbon atoms. The "aromatic alkyl group" means a group in which one or more hydrogen atoms of an alkyl group are substituted with an aryl group.

Examples of the alkyl group of $R^{2a}$ include an ethyl group, a propyl group, an isopropyl group, an n-butyl group, an isobutyl group, a t-butyl group, a 2-ethylhexyl group, a lauryl group, and a stearyl group.

Examples of the cycloalkyl group of $R^{2a}$ include a cyclopropyl group, a cyclohexyl group, and an isobornyl group.

Examples of the alkenyl group of $R^{2a}$ include a vinyl group and an allyl group.

Examples of the cycloalkenyl group of $R^{2a}$ include a cyclopentenyl group, a cyclopentadienyl group, and a cyclohexenyl group.

Examples of the alkynyl group of $R^{2a}$ include a propynyl group.

Examples of the aryl group of $R^{2a}$ include a phenyl group and a naphthyl group.

Examples of the aromatic alkyl group of $R^{2a}$ include a benzyl group.

From the viewpoint that the ester compound (1) is relatively easy to obtain and is relatively easy to handle in terms of physical properties, $R^{2a}$ is preferably an alkyl group having 2 to 20 carbon atoms, a cycloalkyl group having 3 to 20 carbon atoms, an alkenyl group having 2 to 20 carbon atoms, an aryl group having 6 to 20 carbon atoms, or an aromatic alkyl group having 7 to 20 carbon atoms; more preferably an ethyl group, an isopropyl group, an n-butyl group, an isobutyl

group, a t-butyl group, a 2-ethylhexyl group, a lauryl group, a stearyl group, a cyclohexyl group, an isobornyl group, an allyl group, a phenyl group, or a benzyl group; particularly preferably an n-butyl group or an isobutyl group; and most preferably an n-butyl group.

Examples of the ester compound (1) include ethyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, and benzyl (meth)acrylate.

As the ester compound (1), from the viewpoint that the ester compound (1) is relatively easy to obtain and is relatively easy to handle in terms of physical properties, an alkyl (meth)acrylate in which $R^{2a}$ is a linear or branched alkyl group having 2 to 20 carbon atoms, a cycloalkyl (meth)acrylate in which $R^{2a}$ is a cycloalkyl group having 3 to 20 carbon atoms, an alkenyl (meth)acrylate in which $R^{2a}$ is an alkenyl group having 2 to 20 carbon atoms, an aryl (meth)acrylate in which $R^{2a}$ is an aryl group having 6 to 20 carbon atoms, or an aromatic alkyl (meth)acrylate in which $R^{2a}$ is an aromatic alkyl group having 7 to 20 carbon atoms is preferable; ethyl (meth)acrylate, isopropyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, or benzyl (meth)acrylate is more preferable; butyl (meth)acrylate or isobutyl (meth)acrylate is particularly preferable; and butyl (meth)acrylate is most preferable.

The ester compound (1) may be used alone or a combination of two or more kinds thereof may be used.

The ester compound (2) is a (meth)acrylic acid ester represented by Formula (2). The ester compound (3) is a (meth)acrylic acid ester represented by Formula (3). The ester compound (4) is a (meth)acrylic acid ester represented by Formula (4).

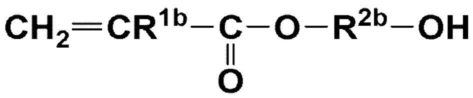
・・・(2)

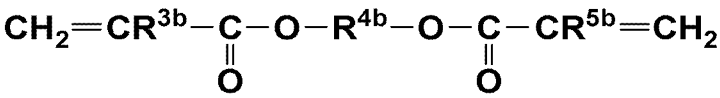
・・・(3)

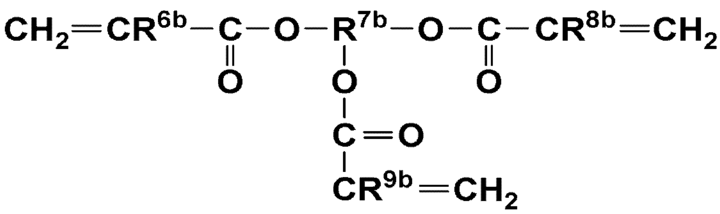
・・・(4)

In Formula (2), Formula (3), and Formula (4), $R^{1b}$, $R^{3b}$, $R^{5b}$, $R^{6b}$, $R^{8b}$, and $R^{9b}$ are each independently a hydrogen atom or a methyl group. $R^{2b}$ and $R^{4b}$ are each independently a linear or branched alkylene group or a hydroxyalkylene group, having 2 to 8 carbon atoms. $R^{7b}$ is a linear or branched trivalent hydrocarbon group having 2 to 8 carbon atoms.

The number of carbon atoms in the alkylene group or the hydroxyalkylene group of $R^{2b}$ and $R^{4b}$ is 2 to 8, preferably 2 to 6.

Examples of the alkylene group of $R^{2b}$ and $R^{4b}$ include an ethylene group, a propylene group, an isopropylene group, and a butylene group.

Examples of the hydroxyalkylene group of $R^{2b}$ and $R^{4b}$ include a hydroxyethylene group, a hydroxypropylene group, and a hydroxybutylene group.

The number of carbon atoms in the trivalent hydrocarbon group of $R^{7b}$ is 2 to 8, preferably 2 to 4.

Examples of the trivalent hydrocarbon group of $R^{7b}$ include $-(CH_2)-C(-CH_2-)(-CH_3)-CH_2-$.

Examples of the ester compound (2) include 2-hydroxyethyl (meth)acrylate, 2-hydroxypropyl (meth)acrylate, and 3-hydroxypropyl (meth)acrylate.

Examples of the ester compound (3) include ethylene glycol di(meth)acrylate, 1,3-propanediol di(meth)acrylate, 1,2-propanediol di(meth)acrylate, 1,3-butanediol di(meth)acrylate, and 1,6-hexanediol di(meth)acrylate.

Examples of the ester compound (4) include trimethylolpropane tri(meth)acrylate.

From the viewpoint of relatively easy availability, one or more selected from the group consisting of ethylene glycol di(meth)acrylate, 2-hydroxyethyl (meth)acrylate, 3-hydroxypropyl (meth)acrylate, and trimethylolpropane tri(meth) acrylate are preferable as the ester compound (2), the ester compound (3), and the ester compound (4).

The ester compound (2), the ester compound (3), and the ester compound (4) may be used alone or a

combination of two or more kinds thereof may be used.

The ester compound (5) is a (meth)acrylic acid ester represented by Formula (5).

$$CH_2=CR^{1c}-C(=O)-O-R^{2c} \cdots \quad (5)$$

In Formula (5), $R^{1c}$ is a hydrogen atom or a methyl group, and $R^{2c}$ is a monovalent group having an ether bond and having 2 to 8 carbon atoms.

The number of etheric oxygens included in the monovalent group having an ether bond and having 2 to 8 carbon atoms of $R^{2c}$ is preferably 1, but it is not limited thereto and may be 2 or more.

The monovalent group having 2 to 8 carbon atoms of $R^{2c}$ may be linear or branched, or may have a ring. When $R^{2c}$ has a ring, the ring may or may not include the etheric oxygen.

The number of carbon atoms in the monovalent group having an ether bond of $R^{2c}$ is 2 to 8, preferably 2 to 7.

Examples of $R^{2c}$ include a 2-methoxyethyl group, a 2-(2-methoxyethoxy)ethyl group, a 2-[2-(2-methoxyethoxy)ethoxy]ethyl group, a glycidyl group, and a tetrahydrofurfuryl group.

As $R^{2c}$, from the viewpoint of relatively easy availability and relatively easy handling in terms of physical properties, a 2-methoxyethyl group, a glycidyl group, or a tetrahydrofurfuryl group is preferable.

Examples of the ester compound (5) include 2-methoxyethyl (meth)acrylate, glycidyl (meth)acrylate, 2-(2-methoxyethoxy)ethyl (meth)acrylate, 2-[2-(2-methoxyethoxy)ethoxy]ethyl (meth)acrylate, and tetrahydrofurfuryl (meth)acrylate.

As the ester compound (5), from the viewpoint of relatively easy handling in terms of physical properties, 2-methoxyethyl (meth)acrylate, glycidyl (meth)acrylate, or tetrahydrofurfuryl (meth)acrylate is preferable.

The ester compound (5) may be used alone or a combination of two or more kinds thereof may be used.

(Component A2 and component A6)

Hereinafter, each component will be described.

The component A2 is a compound represented by Formula (a2).

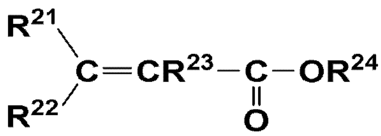

・・・(a 2)

**In** Formula (a2), $R^{21}$, $R^{22}$, and $R^{23}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{24}$ may be a hydrogen atom, an alkyl group, an alkenyl group, an aryl group, a hydroxy group, an alkoxy group, an amino group, a carbonyl group, or a monovalent group including an ether group, or $R^{21}$, $R^{22}$, $R^{23}$, and $R^{24}$ may be each a divalent group in combination with any of the groups, which may have a substituent,
provided that a case where $R^{21} = H$, $R^{22} = H$, $R^{23} = R^{150}$, and $R^{24} = R^{200}$, that is, a case where the compound represented by Formula (a2) is the same as the ester compound (I) is excluded. H represents a hydrogen atom, C represents a carbon atom, and O represents an oxygen atom.

The component A6 is a compound represented by Formula (a6).

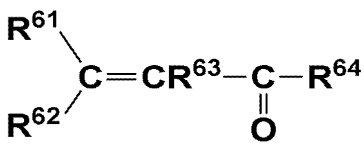

・・・(a 6)

In Formula (a6), $R^{61}$, $R^{62}$, and $R^{63}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{64}$ may be a monovalent group selected from an alkyl group, an alkenyl group, an aryl group, a hydroxy group, an alkoxy group, an amino group, a monovalent group including an ether group, an alkylthio group, or an arylthio group, or $R^{61}$, $R^{62}$, $R^{63}$, and $R^{64}$ may be each a divalent group in combination with any of the groups, which may have a substituent,
provided that a case where $R^{61} = H$, $R^{62} = H$, $R^{63} = R^{150}$, and $R^{64} = OR^{200}$, that is, a case where the compound represented by Formula (a6) is the same as the ester compound (I) is excluded.

Since the ester compound-containing composition according to the second aspect comprises one or more selected from the group consisting of the component A2 and the component A6, a dimerization reaction of the ester compound (I) and generation of an oxidative product of the ester compound (I) are suppressed during storage, and thus an ester compound-containing composition having excellent storage stability is obtained. The reason for this is presumed to be as follows.

During the storage of the ester compound-containing composition, for example, a radical derived from ultraviolet rays, such as a hydroxyl radical generated by absorption of ultraviolet rays derived from sunlight by oxygen molecules, is generated. Such a radical can cause the generation of a dimer of the ester compound (I) or the generation of the oxidative product of the ester compound (I).

Since the component A2 and the component A6 have a conjugated double bond, the components absorb the ultraviolet light, and an absorption wavelength thereof changes depending on the type of the substituent. The component A2 and the component A6 can absorb ultraviolet light in a wide wavelength range.

Therefore, when the ester compound-containing composition comprises one or more selected from the group consisting of the component A2 and the component A6, the ultraviolet light in a wide wavelength range is absorbed, and the generation of the hydroxyl radical is suppressed. Accordingly, it is considered that the generation of the dimer of the ester compound (I) and the generation of the oxidative product of the ester compound (I) can be efficiently suppressed, and a decrease in purity of the ester compound (I) can be suppressed. Among various organic compounds which absorb ultraviolet light, an $\alpha,\beta$-unsaturated carbonyl compound has a molecular structure similar to that of the ester compound (I). Therefore, when a polymer is produced using the ester compound-containing composition as a raw material, it is possible to reduce the adverse effect of the $\alpha,\beta$-unsaturated carbonyl compound contained as an impurity.

It is preferable that each molecular weight of the component A2 and the component A6 is 1,000 or less. When the molecular weight is 1,000 or less, the number of conjugated double bonds per unit mass in the component A2 and the component A6 can be increased, and thus the effect of the present invention can be obtained with a small mass. Each molecular weight of the component A2 and the component A6 is more preferably 800 or less, still more preferably 600 or less, and particularly preferably 400 or less.

In Formula (a2), $R^{21}$, $R^{22}$, and $R^{23}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{24}$ may be a hydrogen atom, an alkyl group, an alkenyl group, an aryl group, a hydroxy group, an alkoxy group, an amino group, a carbonyl group, or a monovalent group including an ether group, or $R^{21}$, $R^{22}$, $R^{23}$, and $R^{24}$ may be each a divalent group in combination with any of the groups, which may have a substituent. $R^{21}$, $R^{22}$, $R^{23}$, and $R^{24}$ may be the same or different from each other.

In Formula (a6), $R^{61}$, $R^{62}$, and $R^{63}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{64}$ may be a monovalent group selected from an alkyl group, an alkenyl group, an aryl group, a hydroxy group, an alkoxy group, an amino group, a monovalent group including an ether group, an alkylthio group, or an arylthio group, or $R^{61}$, $R^{62}$, $R^{63}$, and $R^{64}$ may be each a divalent group in combination with any of the groups, which may have a substituent. $R^{61}$, $R^{62}$, $R^{63}$, and $R^{64}$ may be the same or different from each other.

When $R^{21}$, $R^{22}$, $R^{23}$, $R^{24}$, $R^{61}$, $R^{62}$, $R^{63}$, and $R^{64}$ satisfy the above-described conditions, a $\pi$-conjugated system of the component A2 and the component A6 is maintained, and thus a property of absorbing ultraviolet light in a wide wavelength range is provided, and the effect of the present invention can be obtained.

$R^{21}$, $R^{22}$, $R^{23}$, $R^{61}$, $R^{62}$, and $R^{63}$ are preferably a hydrogen atom, an alkyl group having 1 to 5 carbon atoms, an alkenyl group having 2 to 5 carbon atoms, an aryl group having 1 to 12 carbon atoms, an alkoxy group having 1 to 6 carbon atoms, an amino group, a monovalent group including a carbonyl group, an alkylthio group having 1 to 5 carbon atoms, or an arylthio group having 1 to 12 carbon atoms. Since these groups are substituents having high stability, the component A2 and the component A6 can be prevented from being changed into other compounds during storage. **In** addition, when these substituents are included, a sufficient amount of ultraviolet light which can be absorbed by one molecule of the component A2 and the component A6 is obtained. $R^{21}$, $R^{22}$, $R^{23}$, $R^{61}$, $R^{62}$, and $R^{63}$ are more preferably a hydrogen atom or an alkyl group having 1 to 5 carbon atoms, and still more preferably a hydrogen atom, a methyl group, an ethyl group, an n-propyl group, or an isopropyl group.

$R^{24}$ is preferably a hydrogen atom, an alkyl group having 1 to 5 carbon atoms, an alkenyl group having 2 to 5 carbon atoms, an aryl group having 1 to 12 carbon atoms, an alkoxy group having 1 to 6 carbon atoms, an amino group, a monovalent group including a carbonyl group, an aromatic alkyl group having 1 to 12 carbon atoms, or a monovalent group having an ether bond and having 2 to 8 carbon atoms. Since these groups are substituents having high stability, the component A2 can be prevented from being changed into other compounds during storage. $R^{24}$ is more preferably an alkyl group having 1 to 5 carbon atoms or an alkenyl group having 1 to 5 carbon atoms, and still more preferably a methyl group, an ethyl group, an n-propyl group, an isopropyl group, an n-butyl group, an isobutyl group, a crotyl group, an n-pentyl group, a 2-methyl-1-butyl group, or an isopentyl group. When $R^{24}$ has the structure, quality stability of the ester compound-containing composition during storage can be improved.

$R^{64}$ is preferably an alkyl group having 1 to 5 carbon atoms, an alkenyl group having 2 to 5 carbon atoms, an aryl group having 1 to 12 carbon atoms, an amino group, an alkylthio group having 1 to 5 carbon atoms, or an arylthio group having 1 to 12 carbon atoms. Since these groups are substituents having high stability, the component A6 can be prevented from being changed into other compounds during storage. $R^{64}$ is more preferably an alkyl group having 1 to 5 carbon atoms, and still more preferably a methyl group, an ethyl group, an n-propyl group, an isopropyl group, an n-butyl group, an isobutyl group, an n-pentyl group, a 2-methyl-1-butyl group, or an isopentyl group. When $R^{64}$ has the structure, quality stability of the ester compound-containing composition during storage can be improved.

The alkyl group is a chain (linear or branched) alkyl group or a cyclic alkyl group. An alkyl group having 1 to 20 carbon atoms is preferable, an alkyl group having 1 to 10 carbon atoms is more preferable, and an alkyl group having 1 to 5 carbon atoms is still more preferable. Examples of the chain alkyl group include a methyl group, an ethyl group, an n-propyl group, an isopropyl group, an n-butyl group, an isobutyl group, an s-butyl group, a t-butyl group, an n-pentyl group, a neopentyl group, an isopentyl group, a 2-methyl-1-butyl group, a hexyl group, an octyl group, and a decyl group. Among these, a methyl group, an ethyl group, an n-propyl group, an isopropyl group, an n-pentyl group, an isopentyl group, or a 2-methyl-1-butyl group is preferable. In addition, examples of the cyclic alkyl group include a cyclopentyl group, a cyclohexyl group, a cyclooctyl group, and an isobornyl group. In addition, examples of the alkyl group including a hydroxy group include a hydroxymethyl group, a 1-hydroxyethyl group, and a 2-hydroxyethyl group.

The alkenyl group is a chain (linear or branched) alkenyl group or a cyclic alkenyl group. An alkenyl group having 2 to 20 carbon atoms is preferable, an alkenyl group having 2 to 10 carbon atoms is more preferable, and an alkenyl group having 2 to 5 carbon atoms is still more preferable. Examples of the chain alkenyl group include a vinyl group, a 1-propenyl group, an isopropenyl group, a crotyl group, a 2-butenyl group, a 1,3-butadienyl group, a 2-pentenyl group, and a 2-hexenyl group. In addition, examples of the cyclic alkenyl group include a cyclopentenyl group and a cyclohexenyl group.

The aryl group is preferably an aryl group having 1 to 20 carbon atoms, and more preferably an aryl group having 1 to 12 carbon atoms. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The aromatic alkyl group is preferably an aromatic alkyl group having 1 to 20 carbon atoms, and more preferably an aromatic alkyl group having 1 to 12 carbon atoms. Examples of the aromatic alkyl group include a benzyl group.

The alkoxy group is preferably an alkoxy group having 1 to 20 carbon atoms, more preferably an alkoxy group having 1 to 10 carbon atoms, and still more preferably an alkoxy group having 1 to 6 carbon atoms. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group includes an amino group ($-NH_2$) having no substituent on a nitrogen atom and an amino group in which a part or all of hydrogen atoms bonded to the nitrogen atom are substituted with carbon atoms. The number of carbon atoms in the amino group substituted with carbon atoms is preferably 1 to 20, more preferably 1 to 10, and still more preferably 1 to 5. Examples of the amino group include a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group include a formyl group, an acyl group, a carboxy group, an amide group, an alkoxycarbonyl group, a thiocarboxy group, and a thioester group.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6. Examples of the acyl group include an acetyl group, a propionyl group, a butylcarbonyl group, a vinylcarbonyl group, and a benzoyl group.

The amide group includes an amide group ($-CONH_2$) having no substituent on a nitrogen atom and an amide group in which a part or all of hydrogen atoms bonded to the nitrogen atom are substituted with carbon atoms. As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called

an ester group. The total number of carbon atoms derived from the carbonyl group and carbon atoms derived from the alkoxy group is preferably 2 to 20, more preferably 2 to 10, and still more preferably 2 to 6. Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group. The total number of carbon atoms derived from the carbonyl group and carbon atoms derived from the alkylthio group or the arylthio group is preferably 2 to 20, more preferably 2 to 10, and still more preferably 2 to 6.

Examples of the thioester group include a methylthiocarbonyl group, an ethylthiocarbonyl group, a butylthiocarbonyl group, and a phenylthiocarbonyl group.

As an example, the monovalent group including a carbonyl group may be a substituent in which one or a plurality of hydrogens of an alkyl group are substituted with carbonyl groups. Examples of such a substituent include a 2-acetoxyethyl group, a 2-acetoethyl group, and a 2-(acetoacetoxy)ethyl group.

The alkylthio group is preferably an alkylthio group having 1 to 20 carbon atoms, more preferably an alkylthio group having 1 to 10 carbon atoms, and still more preferably an alkylthio group having 1 to 5 carbon atoms. Examples of the alkylthio group include a methylthio group, an ethylthio group, a propylthio group, and an isopropylthio group.

The arylthio group is preferably an arylthio group having 1 to 20 carbon atoms, more preferably an arylthio group having 3 to 10 carbon atoms, and still more preferably an arylthio group having 6 to 10 carbon atoms. Examples of the arylthio group include a phenylthio group and a tolylthio group.

Examples of the monovalent group including an ether group include a group having an etheric oxygen between two carbon atoms. The number of etheric oxygens included in the above-described group is preferably 1, but it is not limited thereto and may be 2 or more. In addition, the above-described group may be linear or branched, or may have a ring. As such a substituent, a monovalent group having an etheric oxygen and having 2 to 8 carbon atoms is preferable. Examples of the monovalent group including an ether group include a 2-methoxyethyl group, a 2-(2-methoxyethoxy)ethyl group, a 2-[2-(2-methoxyethoxy)ethoxy]ethyl group, a glycidyl group, and a tetrahydrofurfuryl group.

$R^{21}$ and $R^{22}$, $R^{22}$ and $R^{23}$, or $R^{23}$ and $R^{24}$ may be linked to each other to form a ring. In addition, $R^{61}$ and $R^{62}$, $R^{62}$ and $R^{63}$, or $R^{63}$ and $R^{64}$ may be linked to each other to form a ring. Examples of a compound in which $R^{21}$ and $R^{22}$ are linked to each other to form a ring include methyl 2-cyclohexylidenepropionate and methyl 2-cyclopentylidenepropionate. Examples of a compound in which $R^{22}$ and $R^{23}$ are linked to each other to form a ring include methyl 1-cyclohexene-1-carboxylate and methyl 1-cyclopentene-1-carboxylate. Examples of a compound in which $R^{23}$ and $R^{24}$ are linked to each other to form a ring include α-methylene-δ-valerolactone and α-methylene-γ-butyrolactone.

Among the compounds satisfying the above-described conditions, from the viewpoint of quality stability of the ester compound-containing composition during storage, as the component A2, methyl (meth)acrylate, ethyl (meth) acrylate, n-propyl (meth)acrylate, isopropyl (meth)acrylate, butyl (meth)acrylate, isobutyl (meth)acrylate, crotyl (meth) acrylate, n-pentyl (meth)acrylate, 2-methyl-1-butyl (meth)acrylate, isopentyl (meth)acrylate, butyl crotonate, isobutyl crotonate, cis-butyl crotonate, cis-isobutyl crotonate, butyl 2-methylene-3-butenoate, isobutyl 2-methylene-3-butenoate, butyl 3,3-dimethylacrylate, isobutyl 3,3-dimethylacrylate, butyl 2-ethylacrylate, isobutyl 2-ethylacrylate, butyl 2-pentenoate, isobutyl 2-pentenoate, butyl caprylate, isobutyl caprylate, butyl 3-methoxyacrylate, isobutyl 3-methoxyacrylate, butyl 3-butoxyacrylate, isobutyl 3-butoxyacrylate, dibutyl fumarate, isobutyl fumarate, acrylic acid, methacrylic acid, crotonic acid, cis-crotonic acid, 3,3-dimethylacrylic acid, 2-ethylacrylic acid, 2-methylene-3-butenoic acid, butyl 2-cyclohexylidene propionate, butyl 2-cyclopentylidene propionate, butyl 1-cyclohexen-1-carboxylic acid, butyl 1-cyclopenten-1-carboxylic acid, α-methylene-δ-valerolactone, or α-methylene-γ-butyrolactone or preferable; methyl acrylate, ethyl (meth)acrylate, n-propyl (meth)acrylate, isopropyl (meth)acrylate, crotyl (meth)acrylate, n-pentyl (meth)acrylate, 2-methyl-1-butyl (meth) acrylate, isopentyl (meth)acrylate, acrylic acid, methacrylic acid, or crotonic acid is more preferable; and ethyl (meth) acrylate, n-propyl (meth)acrylate, crotyl (meth)acrylate, n-pentyl (meth)acrylate, 2-methyl-1-butyl (meth)acrylate, or isopentyl (meth)acrylate is still more preferable.

Examples of the component A6 include acrylamide, methacrylamide, trans-3-hexen-2-one, and isopropenyl methyl ketone. Acrylamide, methacrylamide, or isopropenyl methyl ketone is preferable, and isopropenyl methyl ketone is more preferable.

In the following description of the second aspect, the "component A" is a general term for the component A2 and the component A6.

The component A of the ester compound-containing composition according to the second aspect may be one kind or two or more kinds.

(Component B)

The ester compound-containing composition according to the second aspect may further comprise a polymerization inhibitor (component B). The polymerization inhibitor means a compound having a function of suppressing the polymerization reaction of the ester compound (I). Examples of the polymerization inhibitor include a phenol-based

compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound. By the component B, it is possible to suppress the polymerization reaction of the ester compound (I) by a radical polymerization mechanism during storage of the ester compound (I). In addition, the component B can trap the above-described hydroxyl radical generated during the storage of the ester compound (I). That is, when the ester compound-containing composition comprises the component B in addition to the component A, the amount of the hydroxyl radical can be reduced by two different mechanisms of suppressing the generation of the hydroxyl radical by the component A and removing the generated hydroxyl radical by the component B. Accordingly, it is considered that the generation of the dimer of the ester compound (I) and the generation of the oxidative product of the ester compound (I) can be efficiently suppressed, and a decrease in purity of the ester compound (I) can be efficiently suppressed.

Examples of the polymerization inhibitor as the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the polymerization inhibitor as the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor as the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazine.

Examples of the polymerization inhibitor as the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethylpiperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butyl nitroxide, and di-tert-amyl nitroxide.

Examples of the polymerization inhibitor as the amine-based compound include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor as the phosphorus-containing compound include triphenylphosphine,

triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl)phosphite, 4,4'-isopropylidenediphenol alkylphosphite, tris(2,4-di-tert-butylphenyl)phosphite, tris(biphenyl)phosphite, distearyl pentaerythritol diphosphite, di(2,4-di-tert-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-tert-butylphenol) diphosphite, hexa(tridecyl)-1,1,3-tris(2-methyl-4-hydroxy-5-tert-butylphenyl)butanetriphosphite, 3,5-di-tert-butyl-4-hydroxybenzyl phosphate diethyl ester, sodium-bis(4-tert-butylphenyl)phosphate, sodium-2,2'-methylene-bis(4,6-di-tert-butylphenyl)phosphate, and 1,3-bis(diphenoxyphosphoryloxy)benzene.

Examples of the polymerization inhibitor as the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor as the iron-containing compound include iron (III) chloride.

Examples of the polymerization inhibitor as the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor as the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

Among these, from the viewpoint of quality stability of the ester compound-containing composition during storage, as the component B, at least one polymerization inhibitor selected from the group consisting of a phenol-based compound, an N-oxyl-based compound, an amine-based compound, a phosphorus-containing compound, and a sulfur-containing compound is preferable; at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is more preferable; and at least one polymerization inhibitor selected from the group consisting of hydroquinone, 4-methoxyphenol, 2,4-dimethyl-6-t-butylphenol, and 2,6-di-t-butyl-4-methylphenol is still more preferable.

The component B may be one kind or two or more kinds.

When the ester compound-containing composition comprises a compound corresponding to both the component A2 and the component B, the compound is regarded as the component A2. When the ester compound-containing composition comprises the component A2 and the component B, this means that the ester compound-containing composition further comprises the component B different from the compound.

When the ester compound-containing composition comprises two or more kinds of compounds corresponding to both the component A2 and the component B, a compound having the highest molar concentration in the ester compound-containing composition is regarded as the component A2, and the other compounds are regarded as the component B.

Also in the component A6, when the ester compound-containing composition comprises a compound corresponding to both the component A6 and the component B, the compound is regarded as the component A6.

The contained amount of the component A is not particularly limited, but is preferably 1 to 10,000 ppm by mass. When the contained amount of the component A is 1 ppm by mass or more, an effect of suppressing the generation of the dimer of the ester compound (I) and the oxidative product of the ester compound (I) can be sufficiently obtained. In addition, when the contained amount of the component A is 10,000 ppm by mass or less, the amount of impurities when the (meth)acrylic polymer is produced by polymerization of the ester compound-containing composition according to the second aspect can be reduced, and thus it is possible to prevent physical properties of the polymer from being adversely affected. The contained amount of the component A is more preferably 3 ppm by mass or more, still more preferably 5 ppm by mass or more, and particularly preferably 10 ppm by mass or more. The contained amount of the component A is more preferably 7,500 ppm by mass or less, still more preferably 5,000 ppm by mass or less, even more preferably 2,500 ppm by mass or less, even still more preferably 1,500 ppm by mass or less, particularly preferably 1,000 ppm by mass or less, and most preferably 500 ppm by mass or less.

The contained amount of the component A is the total of contained amounts of the component A2 and the component A6.

The contained amount of the component B is not particularly limited, but is preferably 1 to 1,000 ppm by mass. When the contained amount of the component B is 1 ppm by mass or more, an effect of suppressing the generation of the dimer of the ester compound (I) and the oxidative product of the ester compound (I) can be sufficiently obtained. In addition, when the contained amount of the component B is 1,000 ppm by mass or less, the amount of impurities when the (meth)acrylic polymer is produced by polymerization of the ester compound-containing composition according to the second aspect can be reduced, and thus it is possible to prevent physical properties of the polymer from being adversely affected. The contained amount of the component B is more preferably 3 ppm by mass or more, still more preferably 5 ppm by mass or more, and particularly preferably 10 ppm by mass or more. The contained amount of the component B is more preferably 750 ppm by mass or less, still more preferably 500 ppm by mass or less, even more preferably 250 ppm by mass or less, particularly preferably 100 ppm by mass or less, and most preferably 50 ppm by mass or less.

(Contained amount of ester compound (I))

The contained amount of the ester compound (I) is 95.00% to 99.99% by mass. When the contained amount of the ester compound (I) is 90% by mass or more, the amount of impurities when the (meth)acrylic polymer is produced by polymerization of the ester compound-containing composition can be reduced, and thus it is possible to prevent physical properties of the polymer from being adversely affected. In addition, when the contained amount of the ester compound (I) is 99.99% by mass or less, a purification cost can be reduced. The contained amount of the ester compound (I) is preferably 96.00% by mass or more, more preferably 97.00% by mass or more, still more preferably 98.00% by mass or more, particularly preferably 99.00% by mass or more, and most preferably 99.50% by mass or more.

When the ester compound (I) includes the ester compound (1) and does not include the ester compound (2), the ester compound (3), the ester compound (4), and the ester compound (5), the contained amount of the ester compound (I) is the concentration of the ester compound (1).

When the ester compound (I) includes the ester compound (5) and does not include the ester compound (1), the ester compound (2), the ester compound (3), and the ester compound (4), the contained amount of the ester compound (I) is the concentration of the ester compound (5).

When the ester compound (I) includes at least one selected from the group consisting of the ester compound (2), the ester compound (3), and the ester compound (4), and does not include the ester compound (1) and the ester compound (5), the contained amount of the ester compound (I) is the concentration of at least one selected from the group consisting of the ester compound (2), the ester compound (3), and the ester compound (4).

(Component C)

The ester compound-containing composition according to the second aspect may comprise other compounds (component C) as long as the contained amount of the ester compound (I) satisfies 95.00% to 99.99% by mass. Examples thereof include additives such as a release agent, a lubricant, a plasticizer, an antioxidant, an antistatic agent, a light stabilizer, an ultraviolet absorber, a flame retardant, a flame retardant aid, a polymerization inhibitor, a filler, a pigment, a dye, a silane coupling agent, a leveling agent, an antifoaming agent, and a fluorescent agent.

The ester compound-containing composition may comprise unreacted raw materials in the production of the ester compound-containing composition, such as methyl (meth)acrylate, alcohol, and (meth)acrylic acid.

The ester compound-containing composition may comprise impurities generated during the production of the ester compound-containing composition, such as diacetyl, but from the viewpoint of suppressing coloration of the ester compound-containing composition, the concentration of the diacetyl is preferably 5 ppm by mass or less, more preferably 2 ppm by mass or less, still more preferably 1 ppm by mass or less, and particularly preferably 0.1 ppm by mass or less.

The ester compound-containing composition may comprise a (meth)acrylic acid ester other than the ester compound (1).

(Analysis of ester compound-containing composition)

The fact that the ester compound-containing composition comprises the component A, the component B, the component C, and the water can be confirmed by, for example, GC-MS measurement. In a GC-MS chart of the ester compound-containing composition, when a peak is present at the same retention time as a sample of the component A2 and an m/z value detected in a mass spectrum of the peak matches exact mass of the component A2, it can be determined that the ester compound-containing composition comprises the component A. When the sample of the component A2 cannot be obtained, when a pattern of the mass spectrum of the peak appearing in the GC-MS chart of the ester compound-containing composition and a pattern of a mass spectrum of the component A2 in mass spectrum database and match each other, it can be determined that the peak is the peak of the component A2. That is, it can be determined that the ester compound-containing composition comprises the component A. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component A6, the component B, the component C, and the water by the same method.

The contained amount of the ester compound (I) can be calculated, for example, by performing GC-FID

measurement of the ester compound-containing composition, quantifying by an area percentage method, and correcting the quantified moisture content using a Karl Fischer moisture meter.

The concentration of the component A2 can be quantified, for example, by performing GC measurement or GC-MS measurement of the ester compound-containing composition and using an internal standard method or an absolute calibration curve method. When a sample of the component A2 cannot be obtained and the component A2 cannot be quantified by the internal standard method or the absolute calibration curve method, the concentration of the component A2 can be calculated using the following expression by performing GC-FID measurement on any organic compound having a known concentration under the same conditions as those of the ester compound-containing composition.

$$\text{Concentration of component A2 } (\mu\text{mol/L}) = \frac{N}{N_{A2}} \times \frac{S_{A2}}{S} \times M$$

Here, N is the number of carbon atoms in one molecule of the organic compound having a known concentration, $N_{A2}$ is the number of carbon atoms in one molecule of the component A2, $S_{A2}$ is a peak area of the component A2, S is a peak area of the organic compound having a known concentration, and M is the concentration (μmol/L) of the organic compound having a known concentration. When the volatility is low and the quantification cannot be performed by the GC measurement, the quantification can be performed using a chromatography method such as LC.

Concentrations of the component A6, the component B, and the component C can also be calculated by the same method as that for the component A2.

The fact that the ester compound-containing composition comprises water, and the concentration thereof can be confirmed by Karl Fischer method.

(Method for producing ester compound-containing composition)

A method for producing the ester compound-containing composition includes performing an ester exchange reaction between one or more alcohols selected from the group consisting of a monoalcohol having 2 to 20 carbon atoms, an ether bond-containing alcohol having 2 to 8 carbon atoms, a dialcohol having 2 to 8 carbon atoms, and a trialcohol having 2 to 8 carbon atoms, and methyl (meth)acrylate in the presence of one or more compounds selected from the group consisting of the component A2 and the component A6.

Among (meth)acrylic acid esters, the methyl (meth)acrylate tends to particularly easily undergo dimerization or oxidation to produce methyl pyruvate. However, by performing the ester exchange reaction in the presence of the component A, the dimerization of methyl (meth)acrylate or the generation of methyl pyruvate is suppressed, and as a result, the yield of the ester compound (I) is improved.

For example, when obtaining the ester compound-containing composition comprising the ester compound (1) as the ester compound (I), in a reaction vessel, the methyl (meth)acrylate is subjected to an ester exchange with the monoalcohol having 2 to 20 carbon atoms as represented by Formula (II) in the presence of a catalyst and the component A.

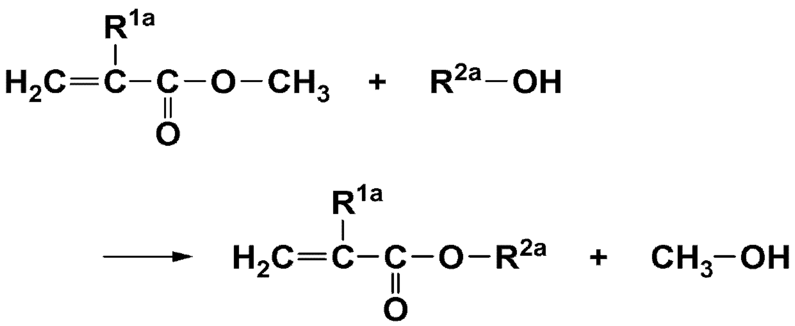

・・・(II)

$R^{1a}$ and $R^{2a}$ in Formula (II) are the same as $R^{1a}$ and $R^{2a}$ in Formula (1).

Examples of the monoalcohol having 2 to 20 carbon atoms include ethanol, n-butanol, isobutanol, t-butanol, 2-ethylhexanol, lauryl alcohol, stearyl alcohol, cyclohexanol, isobornyl alcohol, allyl alcohol, phenol, and benzyl alcohol.

The monoalcohol having 2 to 20 carbon atoms may be used alone or a combination of two or more kinds thereof may be used. The monoalcohol having 2 to 20 carbon atoms preferably includes a linear or branched monoalcohol having 2 to 20 carbon atoms, and more preferably includes n-butanol or isobutanol.

For example, when obtaining the ester compound-containing composition comprising the ester compound (5) as

the ester compound (I), in a reaction vessel, the methyl (meth)acrylate is subjected to an ester exchange with the ether bond-containing alcohol having 2 to 8 carbon atoms as represented by Formula (III) in the presence of a catalyst and the component A.

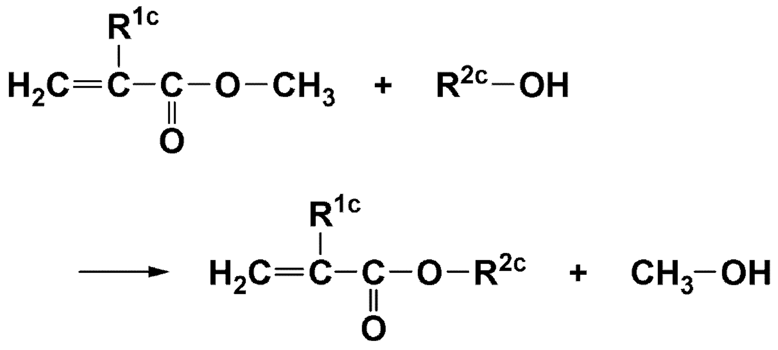

・・・(III)

$R^{1c}$ and $R^{2c}$ in Formula (III) are the same as $R^{1c}$ and $R^{2c}$ in Formula (5).

The number of ether bonds in the ether bond-containing alcohol having 2 to 8 carbon atoms is preferably 1, but is not limited thereto.

Examples of the ether bond-containing alcohol having 2 to 8 carbon atoms include 2-methoxyethanol, glycidyl alcohol, and tetrahydrofurfuryl alcohol. The ether bond-containing alcohol having 2 to 8 carbon atoms may be used alone or a combination of two or more kinds thereof may be used.

The ether bond-containing alcohol having 2 to 8 carbon atoms preferably includes one selected from 2-methoxyethanol, glycidyl alcohol, and tetrahydrofurfuryl alcohol.

For example, when obtaining the ester compound-containing composition comprising at least one or more selected from the group consisting of the ester compound (2), the ester compound (3), and the ester compound (4) as the ester compound (I), in a reaction vessel, the methyl (meth)acrylate is subjected to an ester exchange with one or more alcohols selected from the group consisting of the dialcohol having 2 to 8 carbon atoms and the trialcohol having 2 to 8 carbon atoms as represented by Formula (IV) and Formula (V) in the presence of a catalyst and the component A.

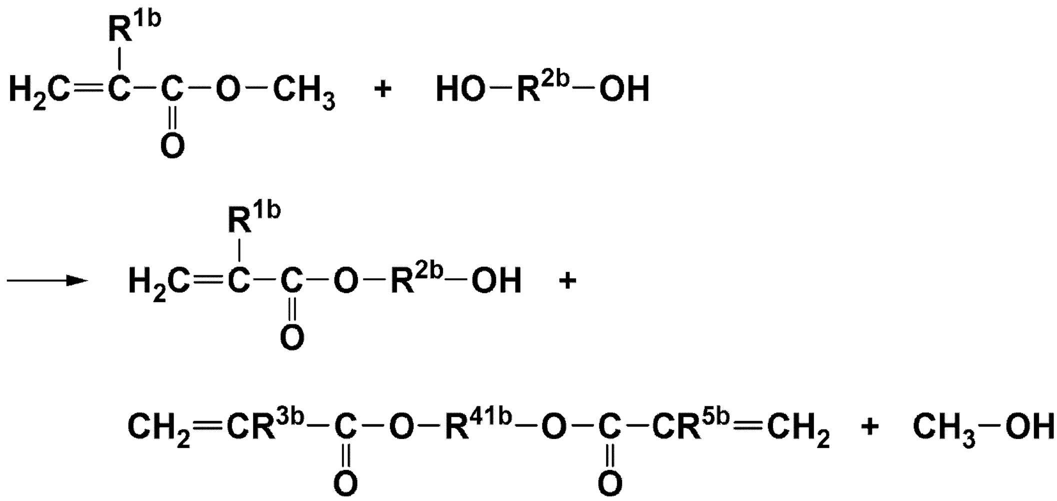

・・・(IV)

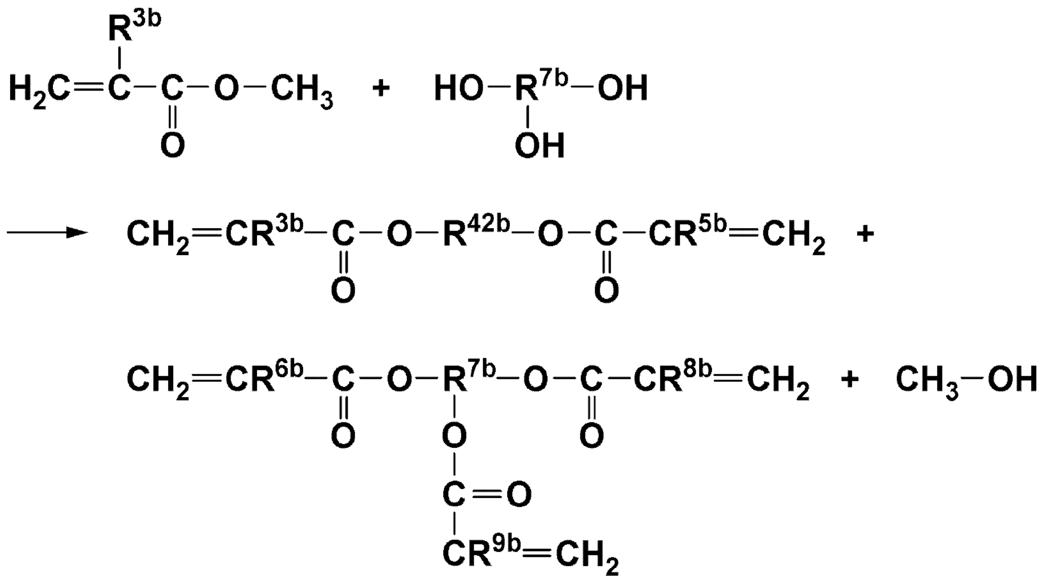

・・・(V)

$R^{1b}$, $R^{2b}$, $R^{3b}$, $R^{5b}$, $R^{6b}$, and $R^{7b}$ in Formula (IV) and Formula (V) are the same as $R^{1b}$, $R^{2b}$, $R^{3b}$, $R^{5b}$, $R^{6b}$, and $R^{7b}$ in Formula (2), Formula (3), and Formula (4). In addition, $R^{41b}$ is a linear or branched alkylene group having 2 to 8 carbon atoms, and $R^{42b}$ is a linear or branched hydroxyalkylene group having 2 to 8 carbon atoms.

Examples of the dialcohol having 2 to 8 carbon atoms and the trialcohol having 2 to 8 carbon atoms include ethylene glycol, 1,2-propanediol, 1,3-propanediol, 1,3-butanediol, 1,6-hexanediol, and trimethylolpropane. Among these, it is preferable that the dialcohol having 2 to 8 carbon atoms and the trialcohol having 2 to 8 carbon atoms include one or more selected from the group consisting of ethylene glycol, 1,2-propanediol, 1,3-propanediol, and trimethylolpropane.

The dialcohol having 2 to 8 carbon atoms and the trialcohol having 2 to 8 carbon atoms may be used alone or a combination of two or more kinds thereof may be used.

The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic mixture with the methyl (meth)acrylate to the outside of the system.

Examples of the reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

The ratio of the amount of the methyl (meth)acrylate to be charged and the amount of the alcohol to be charged can be appropriately determined. From the viewpoint of improving productivity, the ratio of the methyl (meth)acrylate to 1 mol of alcohol is preferably 0.1 mol or more and 10 mol or less, and more preferably 0.3 mol or more and 4 mol or less.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction product during the ester exchange reaction and high catalytic activity, an alkoxide of titanium, dibutyltin oxide, or dioctyltin oxide is preferable. The catalyst may be used alone or a combination of two or more kinds thereof may be used.

The catalyst can be supplied alone to the reaction vessel. In addition, the catalyst can also be supplied to the reaction vessel in a state of being dissolved in the same alcohol as the alcohol as the raw material, or in a state of being dissolved in (meth)acrylic acid ester as the raw material. Examples thereof include a method of directly dissolving the catalyst in the total amount of the alcohol used in the reaction and supplying the catalyst to the reaction vessel, and a method of dissolving the catalyst in a part of the alcohol used in the reaction and supplying the catalyst to the reaction vessel.

The amount of the catalyst used is preferably 0.001 mol% or more and 1 mol% or less, and more preferably 0.01 mol% or more and 0.1 mol% or less with respect to 1 mol of the alcohol.

A solvent may be used for the ester exchange reaction. When a solvent is used, it is preferable to use a solvent which forms an azeotropic composition with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or a combination of two or more kinds thereof may be used.

The reaction temperature for the ester exchange reaction varies depending on the type of the alcohol or the solvent, but is preferably 60°C or higher and 150°C or lower.

The reaction pressure for the ester exchange reaction is not particularly limited, and the reaction may be carried out under any pressure of reduced pressure, normal pressure, or elevated pressure.

The form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, various methods such as distillation, crystallization, extraction, and column chromatography can be performed.

The ester exchange reaction may be performed by blending the component B into the reaction solution. When the component B is present in addition to the component A, the dimerization of methyl (meth)acrylate or the generation of methyl pyruvate is further suppressed, and thus an effect of improving the yield of the ester compound (I) is more easily obtained.

The component B may be blended into a solution containing the ester compound (I) after the ester exchange reaction and the component A. In this case, a solution (B solution) containing the ester compound (I) and the component B is prepared separately from the solution (A solution) containing the ester compound (I) and the component A, and the A solution and the B solution are mixed with each other to obtain the ester compound-containing composition. In addition, the ester compound (I), the A solution, and the B solution may be mixed with each other to obtain the ester compound-containing composition.

The method for producing an ester compound-containing composition is not limited to the above-described method. For example, other (meth)acrylic acid esters other than the methyl (meth)acrylate may be used as the raw material instead of the methyl (meth)acrylate.

An esterification reaction between an alcohol and (meth)acrylic acid may be carried out to produce the ester compound (I).

(Evaluation method for storage stability and thermal stability of ester compound-containing composition)

The ester compound-containing composition has high quality stability during storage. Examples of an evaluation method of the quality stability of the ester compound-containing composition during storage include a method of actually storing the ester compound-containing composition for a long period of time and confirming the generation amount of the dimer of the ester compound (I), the generation amount of the oxidative product of the ester compound (I), and the contained amount of the ester compound (I). In addition, from the viewpoint of ease of work, a method of heating the ester compound-containing composition short period of time and confirming the generation amount of the dimer of the ester compound (I), the generation amount of the oxidative product of the ester compound (I), and the contained amount of the ester compound (I) may be used. When heating short period of time, the heating temperature is preferably 50°C to 100°C and a heating time is preferably 1 to 24 hours. When the ester compound-containing composition is stored at 25°C for 14 days or heated at 70°C for 7 to 20 hours, the quality stability of the ester compound-containing composition during storage is evaluated based on the generation amount of the dimer of the ester compound (I), the generation amount of the oxidative product of the ester compound (I), and the contained amount of the ester compound (I).

[Polymerizable composition]

A polymerizable composition according to the second aspect comprises the above-described ester compound-containing composition according to the second aspect. The polymerizable composition is a polymerizable composition for producing a (meth)acrylic polymer.

As an example, an ester compound-containing composition stored for 1 day or more after production can be used as the polymerizable composition.

The storage time of the ester compound-containing composition can be 1 day or more, 3 days or more, 7 days or more, or 14 days or more. In addition, the storage time of the ester compound-containing composition may be 180 days or less, 150 days or less, 120 days or less, or 90 days or less. The "storage time" means an elapsed time from immediately after the production, which is not limited to a time during which the product is left to stand under a specific environment and

also includes a time of transportation or the like.

The material of a storage container for the ester compound-containing composition is not particularly limited, and for example, a metal container such as stainless steel, a resin container, or a glass container can be used. A transparent container or an opaque container can be used.

The temperature at the time of storing the ester compound-containing composition is preferably -10°C or higher and more preferably 0°C or higher, and is preferably 60°C or lower and more preferably 50°C or lower. By setting the temperature to -10°C or higher, a load on a cooling device can be reduced, and by setting the temperature to 60°C or lower, it is easy to suppress the generation of the dimerized substance or the oxidative product of (meth)acrylic acid ester.

The oxygen concentration of a gas phase portion at the time of storing the ester compound-containing composition is preferably 5% by volume or more and more preferably 7% by volume or more, and is preferably 30% by volume or less and more preferably 22% by volume or less. By setting the oxygen concentration of the gas phase portion to 5% by volume or more, it is easy to prevent the ester compound from being unintentionally polymerized by a polymerization prevention effect of oxygen, and by setting the oxygen concentration of the gas phase portion to 30% by volume or less, it is easy to suppress oxidation of the (meth)acrylic acid ester by oxygen and the generation of various impurities.

As an example, the ester compound-containing composition after the storage may be used in the polymerizable composition without further blending a monomer, or other monomer copolymerizable with the ester compound (I) may be further blended with the ester compound-containing composition after the storage to obtain the polymerizable composition.

As another example, the ester compound-containing composition after the production may be stored in a state in which the other monomer copolymerizable with the ester compound (I) is blended, and used in the polymerizable composition. In this case, it is preferable that a polymerization initiator is not blended during the storage.

The proportion of the ester compound (I) to the total mass of monomers in the polymerizable composition is preferably 10% by mass or more and more preferably 20% by mass or more, and preferably 90% by mass or less and more preferably 80% by mass or less. As a result, a (meth)acrylic polymer having high transparency can be obtained.

The ester compound-containing composition may comprise the other monomer copolymerizable with the ester compound (I), or may not contain the other monomer copolymerizable with the ester compound (I).

Examples of the other monomer copolymerizable with the ester compound (I) include other monomer copolymerizable with the ester compound (1);

other monomer copolymerizable with the ester compound (5); and other monomer copolymerizable with at least one selected from the group consisting of the ester compound (2), the ester compound (3), and the ester compound (4).

(Other monomer copolymerizable with ester compound (1))

Examples of the other monomer copolymerizable with the ester compound (1) include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.

Examples of the unsaturated carboxylic acid include acrylic acid, methacrylic acid, maleic acid, and itaconic acid.

Examples of the unsaturated carboxylic acid anhydride include maleic acid anhydride and itaconic acid anhydride.

Examples of the maleimide include N-phenylmaleimide and N-cyclohexylmaleimide.

Examples of the hydroxy group-containing vinyl monomer include 2-hydroxyethyl acrylate, 2-hydroxyethyl methacrylate, and 2-hydroxypropyl methacrylate. Examples of the vinyl ester include vinyl acetate and vinyl benzoate. Examples of the nitrogen-containing vinyl monomer include methacrylamide and acrylonitrile.

Examples of the epoxy group-containing monomer include glycidyl acrylate and glycidyl methacrylate.

Examples of the aromatic vinyl monomer include styrene and $\alpha$-methylstyrene.

Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.

Examples of the polyoxyalkylene glycol di(meth)acrylate include diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate.

Examples of the vinyl monomer having two or more ethylenically unsaturated bonds in the molecule include divinylbenzene.

As the other monomer, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.

The other monomer may be used alone or a combination of two or more kinds thereof may be used.

(Other monomer copolymerizable with at least one selected from group consisting of ester compound (2), ester compound (3), and ester compound (4))

Examples of the other monomer copolymerizable with at least one selected from the group consisting of the ester compound (2), the ester compound (3), and the ester compound (4) include the same monomers as those of the other monomer copolymerizable with the ester compound (1).

(Other monomer copolymerizable with ester compound (5))

Examples of the other monomer copolymerizable with the ester compound (5) include the same monomers as those of the other monomer copolymerizable with the ester compound (1).
It is preferable that the polymerizable composition further comprises a polymerization initiator.
Examples of the polymerization initiator include an azo compound, an organic peroxide, a persulfate compound, and a redox polymerization initiator. The polymerization initiator may be used alone or a combination of two or more kinds thereof may be used.
Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.
Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.
Examples of the persulfate compound include potassium persulfate.
The additional amountof the polymerization initiator is not particularly limited, and for example, can be 0.005 to 5 parts by mass with respect to 100 parts by mass of the total mass of the monomers in the polymerizable composition.
As an example, the ester compound-containing composition after the storage may be blended with various additives such as a release agent, a lubricant, a plasticizer, an antioxidant, an antistatic agent, a light stabilizer, an ultraviolet absorber, a flame retardant, a flame retardant assistant, a polymerization inhibitor, a filler, a pigment, a dye, a silane coupling agent, a leveling agent, an antifoaming agent, a fluorescent agent, and a chain transfer agent, as necessary.

[Method for producing (meth)acrylic polymer]

A (meth)acrylic polymer can be produced by polymerizing the polymerizable composition.
The polymerization method is not particularly limited, and examples thereof include a bulk polymerization method, a solution polymerization method, an emulsion polymerization method, and a suspension polymerization method. From the viewpoint of environmental load due to the use of the solvent or the like and viewpoint of transparency of the obtained (meth)acrylic polymer, a bulk polymerization method is preferable.
The method of the bulk polymerization method is not particularly limited, and examples thereof include various casting polymerization methods such as a cell casting method and a continuous casting method.
The casting polymerization method is a method in which the (meth)acrylic polymer is obtained by casting and polymerizing the polymerizable composition in a mold made of two inorganic glass plates or metal plates (for example, SUS plates) arranged facing each other at a predetermined interval with the periphery sealed with a gasket such as a soft resin tube.
The mold for the casting polymerization is not particularly limited, and various molds can be used. Examples of a mold for cell casting include a mold in which two plate-shaped products such as an inorganic glass plate, a chromium-plated metal plate, and a stainless steel plate are arranged facing each other at a predetermined interval, and a gasket is disposed on edges of the plates to form a sealed space between the plate-shaped products and the gasket. Examples of the mold for continuous casting include a mold in which a sealed space is formed by opposing surfaces of a pair of endless belts running in the same direction at the same speed and gaskets running at the same speed as the endless belt on both sides of the endless belt.
A gap between cavities of the molds is appropriately adjusted to obtain a resin plate having a desired thickness,

and is generally 1 to 30 mm.

The polymerization temperature is preferably 125°C or higher and 210°C or lower, and more preferably 130°C or higher and 180°C or lower.

The polymerization time is preferably 0.5 hours or more and 24 hours or less.

The weight-average molecular weight (Mw) of the (meth)acrylic polymer is not particularly limited, and can be, for example, 100,000 or more and 1,000,000 or less. As the Mw of the (meth)acrylic polymer is higher, solvent resistance and chemical resistance can be further improved.

The Mw of the (meth)acrylic polymer can be controlled by adjusting the polymerization temperature, the polymerization time, the addition amount of the polymerization initiator, and the like.

The (meth)acrylic polymer according to the second aspect has excellent heat resistance and excellent meltability. For example, among (meth)acrylic polymers, a granular (meth)acrylic polymer having a large amount of resin which can be stored per unit volume and having a small energy cost during storage and transportation needs to be dissolved or melted at the time of use. The (meth)acrylic polymer according to the second aspect is easily melted, has excellent kneading properties with other resins, and has excellent solubility in a monomer, a solvent, or the like. Furthermore, a heat weight loss rate in a high-temperature environment is low.

3. Third aspect

Hereinafter, a third aspect of the embodiment will be described.

[Ester compound-containing composition]

An ester compound-containing composition according to the third aspect comprises an ester compound (I) described later and a compound (component A7) described later. The contained amount of the ester compound (I) is 95.00% to 99.99% by mass.

The ester compound-containing composition may further comprise a polymerization inhibitor (component B) described later, in addition to the ester compound (I) and the component A7.

As long as the effect of the present invention is not impaired, the ester compound-containing composition may comprise the component B as necessary, in addition to the ester compound (I) and the component A7, and may further comprise at least one of a compound other than the ester compound (I), the component A7, and the component B (hereinafter, also referred to as a component C) or water.

(Ester compound (I))

The ester compound (I) is a compound represented by Formula (I).

$$CH_2=CR^{150}\text{-}C(=O)\text{-}O\text{-}R^{200} \cdots \qquad (I)$$

In Formula (I), $R^{150}$ is a hydrogen atom or a methyl group, and $R^{200}$ is a monovalent hydrocarbon group having 2 to 20 carbon atoms, which may have a substituent, or a monovalent group having an ether bond and having 2 to 8 carbon atoms.

The ester compound (I) may be used alone or a combination of two or more kinds thereof may be used.

As the ester compound (I), one or more compounds selected from the group consisting of an ester compound (1) described later, an ester compound (2) described later, an ester compound (3) described later, an ester compound (4) described later, and an ester compound (5) described later are preferable. Hereinafter, the ester compounds will be described.

The ester compound (1) is a (meth)acrylic acid ester represented by Formula (1).

$$CH_2=CR^{1a}\text{-}C(=O)\text{-}O\text{-}R^{2a} \cdots \qquad (1)$$

In Formula (1), $R^{1a}$ is a hydrogen atom or a methyl group. $R^{2a}$ is a hydrocarbon group having 2 to 20 carbon atoms.

The hydrocarbon group of $R^{2a}$ may be a saturated hydrocarbon group or an unsaturated hydrocarbon group.

The hydrocarbon group of $R^{2a}$ may be linear or branched, or may have a ring. When the hydrocarbon group of $R^{2a}$ has a ring, the ring may be an aliphatic ring or an aromatic ring.

The number of carbon atoms in the hydrocarbon group of $R^{2a}$ is 2 to 20, preferably 2 to 18 and more preferably 2 to 12.

Examples of the hydrocarbon group of $R^{2a}$ include an alkyl group having 2 to 20 carbon atoms, a cycloalkyl group

having 3 to 20 carbon atoms, an alkenyl group having 2 to 20 carbon atoms, a cycloalkenyl group having 3 to 20 carbon atoms, an alkynyl group having 2 to 20 carbon atoms, an aryl group having 6 to 20 carbon atoms, and an aromatic alkyl group having 7 to 20 carbon atoms. The "aromatic alkyl group" means a group in which one or more hydrogen atoms of an alkyl group are substituted with an aryl group.

Examples of the alkyl group of $R^{2a}$ include an ethyl group, a propyl group, an isopropyl group, an n-butyl group, an isobutyl group, a t-butyl group, a 2-ethylhexyl group, a lauryl group, and a stearyl group.

Examples of the cycloalkyl group of $R^{2a}$ include a cyclopropyl group, a cyclohexyl group, and an isobornyl group.

Examples of the alkenyl group of $R^{2a}$ include a vinyl group and an allyl group.

Examples of the cycloalkenyl group of $R^{2a}$ include a cyclopentenyl group, a cyclopentadienyl group, and a cyclohexenyl group.

Examples of the alkynyl group of $R^{2a}$ include a propynyl group.

Examples of the aryl group of $R^{2a}$ include a phenyl group and a naphthyl group.

Examples of the aromatic alkyl group of $R^{2a}$ include a benzyl group.

From the viewpoint that the ester compound (1) is relatively easy to obtain and is relatively easy to handle in terms of physical properties, $R^{2a}$ is preferably an alkyl group having 2 to 20 carbon atoms, a cycloalkyl group having 3 to 20 carbon atoms, an alkenyl group having 2 to 20 carbon atoms, an aryl group having 6 to 20 carbon atoms, or an aromatic alkyl group having 7 to 20 carbon atoms; more preferably an ethyl group, an isopropyl group, an n-butyl group, an isobutyl group, a t-butyl group, a 2-ethylhexyl group, a lauryl group, a stearyl group, a cyclohexyl group, an isobornyl group, an allyl group, a phenyl group, or a benzyl group; particularly preferably an n-butyl group or an isobutyl group; and most preferably an n-butyl group.

Examples of the ester compound (1) include ethyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth) acrylate, isobornyl (meth)acrylate, allyl (meth)acrylate, phenyl (meth)acrylate, and benzyl (meth)acrylate.

As the ester compound (1), from the viewpoint that the ester compound (1) is relatively easy to obtain and is relatively easy to handle in terms of physical properties, an alkyl (meth)acrylate in which $R^{2a}$ is a linear or branched alkyl group having 2 to 20 carbon atoms, a cycloalkyl (meth)acrylate in which $R^{2a}$ is a cycloalkyl group having 3 to 20 carbon atoms, an alkenyl (meth)acrylate in which $R^{2a}$ is an alkenyl group having 2 to 20 carbon atoms, an aryl (meth)acrylate in which $R^{2a}$ is an aryl group having 6 to 20 carbon atoms, or an aromatic alkyl (meth)acrylate in which $R^{2a}$ is an aromatic alkyl group having 7 to 20 carbon atoms is preferable; ethyl (meth)acrylate, isopropyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, or benzyl (meth)acrylate is more preferable; butyl (meth)acrylate or isobutyl (meth)acrylate is particularly preferable; and butyl (meth)acrylate is most preferable.

The ester compound (1) may be used alone or a combination of two or more kinds thereof may be used.

The ester compound (2) is a (meth)acrylic acid ester represented by Formula (2). The ester compound (3) is a (meth)acrylic acid ester represented by Formula (3). The ester compound (4) is a (meth)acrylic acid ester represented by Formula (4).

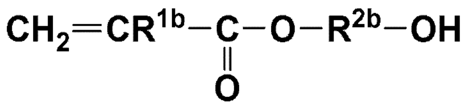

・・・(2)

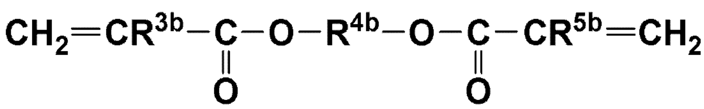

・・・(3)

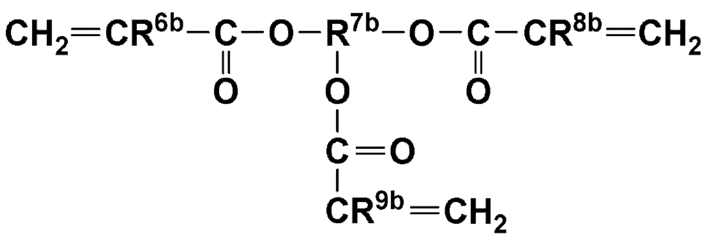

・・・(4)

In Formula (2), Formula (3), and Formula (4), $R^{1b}$, $R^{3b}$, $R^{5b}$, $R^{6b}$, $R^{8b}$, and $R^{9b}$ are each independently a hydrogen atom or a methyl group. $R^{2b}$ and $R^{4b}$ are each independently a linear or branched alkylene group or a hydroxyalkylene

group, having 2 to 8 carbon atoms. $R^{7b}$ is a linear or branched trivalent hydrocarbon group having 2 to 8 carbon atoms.

The number of carbon atoms in the alkylene group or the hydroxyalkylene group of $R^{2b}$ and $R^{4b}$ is 2 to 8, preferably 2 to 6.

Examples of the alkylene group of $R^{2b}$ and $R^{4b}$ include an ethylene group, a propylene group, an isopropylene group, and a butylene group.

Examples of the hydroxyalkylene group of $R^{2b}$ and $R^{4b}$ include a hydroxyethylene group, a hydroxypropylene group, and a hydroxybutylene group.

The number of carbon atoms in the trivalent hydrocarbon group of $R^{7b}$ is 2 to 8, preferably 2 to 4.

Examples of the trivalent hydrocarbon group of $R^{7b}$ include $-(CH_2)-C(-CH_2-)(-CH_3)-CH_2-$.

Examples of the ester compound (2) include 2-hydroxyethyl (meth)acrylate, 2-hydroxypropyl (meth)acrylate, and 3-hydroxypropyl (meth)acrylate.

Examples of the ester compound (3) include ethylene glycol di(meth)acrylate, 1,3-propanediol di(meth)acrylate, 1,2-propanediol di(meth)acrylate, 1,3-butanediol di(meth)acrylate, and 1,6-hexanediol di(meth)acrylate.

Examples of the ester compound (4) include trimethylolpropane tri(meth)acrylate.

From the viewpoint of relatively easy availability, one or more selected from the group consisting of ethylene glycol di(meth)acrylate, 2-hydroxyethyl (meth)acrylate, 3-hydroxypropyl (meth)acrylate, and trimethylolpropane tri(meth) acrylate are preferable as the ester compound (2), the ester compound (3), and the ester compound (4).

The ester compound (2), the ester compound (3), and the ester compound (4) may be used alone or a combination of two or more kinds thereof may be used.

The ester compound (5) is a (meth)acrylic acid ester represented by Formula (5).

$$CH_2=CR^{1c}-C(=O)-O-R^{2c} \cdots \qquad (5)$$

In Formula (5), $R^{1c}$ is a hydrogen atom or a methyl group, and $R^{2c}$ is a monovalent group having an ether bond and having 2 to 8 carbon atoms.

The number of etheric oxygen atoms included in the monovalent group having 2 to 8 carbon atoms of $R^{2c}$ is preferably 1, but it is not limited thereto and may be 2 or more.

The monovalent group having 2 to 8 carbon atoms of $R^{2c}$ may be linear or branched, or may have a ring. When $R^{2c}$ has a ring, the ring may or may not include the etheric oxygen atom.

The number of carbon atoms in the monovalent group having an ether bond of $R^{2c}$ is 2 to 8, preferably 2 to 7.

Examples of $R^{2c}$ include a 2-methoxyethyl group, a 2-(2-methoxyethoxy)ethyl group, a 2-[2-(2-methoxyethoxy) ethoxy]ethyl group, a glycidyl group, and a tetrahydrofurfuryl group.

As $R^{2c}$, from the viewpoint of relatively easy availability and relatively easy handling in terms of physical properties, a 2-methoxyethyl group, a glycidyl group, or a tetrahydrofurfuryl group is preferable.

Examples of the ester compound (5) include 2-methoxyethyl (meth)acrylate, glycidyl (meth)acrylate, 2-(2-methoxyethoxy)ethyl (meth)acrylate, 2-[2-(2-methoxyethoxy)ethoxy]ethyl (meth)acrylate, and tetrahydrofurfuryl (meth) acrylate.

As the ester compound (5), from the viewpoint of relatively easy handling in terms of physical properties, 2-methoxyethyl (meth)acrylate, glycidyl (meth)acrylate, or tetrahydrofurfuryl (meth)acrylate is preferable.

The ester compound (5) may be used alone or a combination of two or more kinds thereof may be used.

(Component A7)

The component A7 is a compound represented by Formula (a7).

When the ester compound-containing composition comprises the component A7, generation of a dimer of the ester compound (I) and generation of an oxidative product of the ester compound (I) are suppressed during storage, and thus an ester compound-containing composition having excellent storage stability is obtained. The reason for this is presumed to be as follows.

During the storage of the ester compound-containing composition, for example, a radical derived from ultraviolet rays, such as a hydroxyl radical generated by absorption of ultraviolet rays derived from sunlight by oxygen molecules, is generated. Such a radical can cause the generation of a dimer of the ester compound (I) or the generation of the oxidative product of the ester compound (I).

Since the component A7 is a $\pi$-conjugated compound having a benzene ring, the component A7 absorbs ultraviolet light, and an absorption wavelength and an absorption intensity thereof change depending on the type of the substituent. In the component A7, since an alkyl ether group having an appropriate bulkiness and an appropriate electron-donating property is bonded to a benzene ring, ultraviolet light in a wide wavelength range can be absorbed. Therefore, when the ester compound-containing composition comprises the component A7, the ultraviolet light having a wide wavelength range is absorbed, and the generation of the hydroxyl radical is suppressed. Accordingly, it is presumed that

the generation of the dimer of the ester compound (I) and the generation of the oxidative product of the ester compound (I) can be suppressed, and a decrease in purity of the ester compound (I) can be suppressed.

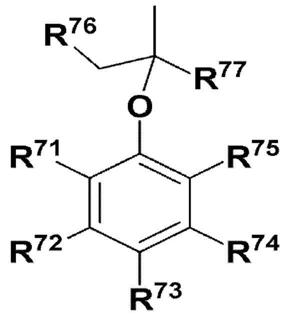

(a 7)

In Formula (a7), $R^{71}$, $R^{72}$, $R^{73}$, $R^{74}$, $R^{75}$, $R^{76}$, and $R^{77}$ are each independently a monovalent group selected from a hydrogen atom, an alkyl group, an alkenyl group, an aryl group, a hydroxy group, an alkoxy group, an amino group, a monovalent group including a carbonyl group, an alkylthio group, or an arylthio group.

$R^{71}$, $R^{72}$, $R^{73}$, $R^{74}$, R75, $R^{76}$, and $R^{77}$ may be the same or different from each other.

The alkyl group of $R^{71}$, $R^{72}$, $R^{73}$, $R^{74}$, $R^{75}$, $R^{76}$, and $R^{77}$ may be linear or branched, or may have a ring.

The number of carbon atoms in the alkyl group is preferably 1 to 20, more preferably 1 to 10, and still more preferably 1 to 5.

Examples of the linear or branched alkyl group (hereinafter, also collectively referred to as "chain alkyl group") include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group. Among these, a methyl group, an ethyl group, an n-propyl group, an isopropyl group, or a t-butyl group is preferable.

Examples of the alkyl group having a ring (hereinafter, also referred to as "cyclic alkyl group") include a cyclopentyl group, a cyclohexyl group, and a cyclooctyl group.

The alkenyl group of $R^{71}$, $R^{72}$, $R^{73}$, $R^{74}$, $R^{75}$, $R^{76}$, and $R^{77}$ may be linear or branched, or may have a ring.

The number of carbon atoms in the alkenyl group is preferably 2 to 20, more preferably 2 to 10, and still more preferably 2 to 5.

Examples of the linear or branched alkenyl group (hereinafter, also collectively referred to as "chain alkenyl group") include a vinyl group, a 1-propenyl group, an isopropenyl group, a 2-butenyl group, a 1,3-butadienyl group, a 2-pentenyl group, and a 2-hexenyl group.

Examples of the alkenyl group having a ring (hereinafter, also referred to as "cyclic alkenyl group") include a cyclopentenyl group and a cyclohexenyl group.

The number of carbon atoms in the aryl group of $R^{71}$, $R^{72}$, $R^{73}$, $R^{74}$, $R^{75}$, $R^{76}$, and $R^{77}$ is preferably 1 to 20 and more preferably 1 to 12.

The aryl group includes a heteroaryl group containing oxygen, nitrogen, sulfur, or the like.

Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The number of carbon atoms in the alkoxy group of $R^{71}$, $R^{72}$, $R^{73}$, $R^{74}$, $R^{75}$, $R^{76}$, and $R^{77}$ is preferably 1 to 20, more preferably 1 to 10, and still more preferably 1 to 6.

Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group of $R^{71}$, $R^{72}$, $R^{73}$, $R^{74}$, $R^{75}$, $R^{76}$, and $R^{77}$ includes an amino group ($-NH_2$) having no substituent on a nitrogen atom and an amino group in which a part or all of hydrogen atoms bonded to the nitrogen atom are substituted with carbon atoms.

The number of carbon atoms in the amino group substituted with carbon atoms is preferably 1 to 20, more preferably 1 to 10, and still more preferably 1 to 5.

Examples of the amino group include an unsubstituted amino group, a methylamino group, an ethylamino group,

a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group of $R^{71}$, $R^{72}$, $R^{73}$, $R^{74}$, $R^{75}$, $R^{76}$, and $R^{77}$ include a formyl group, an acyl group, a carboxy group, an amide group, an alkoxycarbonyl group, a thiocarboxy group, and a thioester group.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6.

Examples of the acyl group include an acetyl group, a propionyl group, a butylcarbonyl group, a vinylcarbonyl group, and a benzoyl group.

The amide group includes an amide group ($-CONH_2$) having no substituent on a nitrogen atom and an amide group in which a part or all of hydrogen atoms bonded to the nitrogen atom are substituted with carbon atoms.

As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group.

The total number of carbon atoms derived from the carbonyl group and carbon atoms derived from the alkoxy group is preferably 2 to 20, more preferably 2 to 10, and still more preferably 2 to 6.

Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group.

The total number of carbon atoms derived from the carbonyl group and carbon atoms derived from the alkylthio group or the arylthio group is preferably 2 to 20, more preferably 2 to 10, and still more preferably 2 to 6.

Examples of the thioester group include a methylthiocarbonyl group, an ethylthiocarbonyl group, a butylthiocarbonyl group, and a phenylthiocarbonyl group.

As an example, the monovalent group including a carbonyl group may be a substituent in which one or a plurality of hydrogen atoms of an alkyl group are substituted with carbonyl groups. Examples of such a substituent include a 2-acetoxyethyl group, a 2-acetoethyl group, and a 2-(acetoacetoxy)ethyl group.

The number of carbon atoms in the alkylthio group of $R^{71}$, $R^{72}$, $R^{73}$, $R^{74}$, $R^{75}$, $R^{76}$, and $R^{77}$ is preferably 1 to 20, more preferably 1 to 10, and still more preferably 1 to 5.

Examples of the alkylthio group include a methylthio group, an ethylthio group, a propylthio group, and an isopropylthio group.

The number of carbon atoms in the arylthio group of $R^{71}$, $R^{72}$, $R^{73}$, $R^{74}$, $R^{75}$, $R^{76}$, and $R^{77}$ is preferably 1 to 20, more preferably 3 to 10, and still more preferably 6 to 10.

Examples of the arylthio group include a phenylthio group and a tolylthio group.

In Formula (a7), when steric hindrance of $R^{71}$, $R^{72}$, $R^{73}$, $R^{74}$, $R^{75}$, $R^{76}$, and $R^{77}$ directly bonded to the benzene ring is large, the benzene ring is distorted, and the $\pi$-conjugated system is not maintained. However, when $R^{71}$, $R^{72}$, $R^{73}$, $R^{74}$, $R^{75}$, $R^{76}$, and $R^{77}$ satisfy the above-described conditions, the $\pi$-conjugated system of the component A7 is maintained due to appropriate bulkiness, and thus a property of absorbing ultraviolet light in a wide wavelength range is provided, and the effect of the present invention can be obtained.

From the viewpoint of absorbing ultraviolet light in a wide wavelength range to suppress the generation of the hydroxyl radical, $R^{71}$, $R^{72}$, $R^{73}$, $R^{74}$, and $R^{75}$ are preferably a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group, or a monovalent group including a carbonyl group; more preferably a hydrogen atom, an alkyl group having 1 to 5 carbon atoms, a hydroxy group, or an alkoxy group having 1 to 5 carbon atoms; still more preferably a hydrogen atom, a methyl group, an ethyl group, a t-butyl group, a hydroxy group, a methoxy group, a propoxy group, or a t-butoxy group; and particularly preferably a hydrogen atom, a methyl group, a t-butyl group, a hydroxy group, or a methoxy group.

From the viewpoint of increasing absorbance of the ultraviolet light to suppress the generation of the hydroxyl radical, $R^{76}$ and $R^{77}$ are preferably a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group, or a monovalent group including a carbonyl group; more preferably a hydrogen atom, an alkyl group having 1 to 5 carbon atoms, a hydroxy group, or an alkoxycarbonyl group having 1 to 5 carbon atoms; still more preferably a hydrogen atom, a methyl group, an ethyl group, a t-butyl group, a hydroxy group, a methoxycarbonyl group, or a butoxycarbonyl group; and particularly preferably a hydrogen atom, a methyl group, a methoxycarbonyl group, or a

butoxycarbonyl group.

The molecular weight of the component A7 is preferably 2,000 or less. When the molecular weight thereof is 2,000 or less, the number of benzene rings per unit mass in the component A7 can be increased, and thus the effect of the present invention can be obtained with a small mass. The molecular weight of the component A7 is more preferably 1,600 or less, still more preferably 1,200 or less, and particularly preferably 800 or less.

Among the compounds satisfying the above-described conditions, from the viewpoint of further improving the storage stability of the ester compound-containing composition, as the component A7, t-butylphenyl ether, isopropylphenyl ether, 1-isopropoxy-3-methylbenzene, 4-isopropoxyphenol, 1-t-butoxy-4-methylbenzene, butyl 2-(2,4-dimethyl-6-t-butylphenoxy)-2-methylpropionate, butyl 2-(2,6-di-t-butyl-4-methylphenoxy)-2-methylpropionate, butyl 2-(4-methoxyphenoxy)-2-methylpropionate, or butyl 2-(4-hydroxyphenoxy)-2-methylpropionate is preferable; and isopropylphenyl ether, butyl 2-(2,4-dimethyl-6-t-butylphenoxy)-2-methylpropionate, butyl 2-(2,6-di-t-butyl-4-methylphenoxy)-2-methylpropionate, butyl 2-(4-methoxyphenoxy)-2-methylpropionate, or butyl 2-(4-hydroxyphenoxy)-2-methylpropionate is more preferable.

The component A7 may be used alone or a combination of two or more kinds thereof may be used.

(Component B)

The component B is a polymerization inhibitor.

When the ester compound-containing composition comprises the component B, the progress of the polymerization reaction of the ester compound (I) by radical polymerization mechanism during storage is suppressed. In addition, during storage, oxygen molecules in the ester compound-containing composition absorb ultraviolet rays derived from sunlight, thereby generating a hydroxyl radical. However, the polymerization inhibitor can trap the hydroxyl radical. Therefore, when the ester compound-containing composition comprises the component A7 and the component B, the amount of the hydroxyl radical can be reduced by two different mechanisms of suppressing the generation of the hydroxyl radical by the component A7 and removing the generated hydroxyl radical by the component B even when the hydroxyl radical is generated. Therefore, the progress of the dimerization of the ester compound (I) and the generation of the oxidative product can be suppressed more efficiently, and a decrease in purity of the ester compound (I) can be suppressed more efficiently.

The polymerization inhibitor means a compound having a function of suppressing the polymerization reaction of the ester compound (I).

Examples of the component B include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

Examples of the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include $\alpha$-tocopherol and 2,3-dihydro-2,2-dimethyl-7-hydroxybenzofuran.

Examples of the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the nitrobenzene-based compound include nitrobenzene, o-dinitrobenzene, m-dinitrobenzene, p-dinitrobenzene, 2,4-dinitrotoluene, dinitrodurene, and 2,2-diphenyl-1-picrylhydrazyl.

Examples of the N-oxyl-based compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethyl-piperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-t-butyl nitroxide, and di-t-amyl nitroxide.

Examples of the amine-based compound include N,N-diphenylamine, alkylated diphenylamine, 4,4'-dicamyldiphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-$\alpha$-naphthylamine, N-phenyl-$\beta$-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl) phosphite, 4,4'-isopropylidenediphenol alkylphosphite, tris(2,4-di-t-butylphenyl)phosphite, tris(biphenyl)phosphite, distearyl pentaerythritol diphosphite, di(2,4-di-t-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-t-butylphenol) diphosphite, hexa(tridecyl)-1,1,3-tris(2-methyl-4-hydroxy-5-t-butylphenyl)butanetriphosphite, 3,5-dit-butyl-4-hydroxybenzyl phosphate diethyl ester, sodium-bis(4-t-butylphenyl)phosphate, sodium-2,2'-methylene-bis(4,6-di-t-butylphenyl)phosphate, and 1,3-bis(diphenoxyphosphoryloxy)benzene.

Examples of the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the iron-containing compound include iron (III) chloride.

Examples of the copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

From the viewpoint of easily exhibiting the effect of further improving the storage stability of the ester compound-containing composition, as the component B, at least one polymerization inhibitor selected from the group consisting of a phenol-based compound, an N-oxyl-based compound, an amine-based compound, a phosphorus-containing compound, and a sulfur-containing compound is preferable; at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is more preferable; and at least one polymerization inhibitor selected from the group consisting of hydroquinone, 4-methoxyphenol, 2,4-dimethyl-6-t-butylphenol, and 2,6-di-t-butyl-4-methylphenol is particularly preferable.

The component B may be used alone or a combination of two or more kinds thereof may be used.

When the ester compound-containing composition comprises a compound corresponding to both the component A7 and the component B, the compound is regarded as the component A7. When the ester compound-containing composition comprises the component A7 and the component B, this means that the ester compound-containing composition further comprises the component B different from the compound.

When the ester compound-containing composition comprises two or more kinds of compounds corresponding to both the component A7 and the component B, a compound having the highest molar concentration in the ester compound-containing composition is regarded as the component A7, and the other compounds are regarded as the component B.

(Component C)

The ester compound-containing composition may comprise other compounds (component C) as long as the contained amount of the ester compound (I) satisfies 95.00% to 99.99% by mass. The component C is a compound other than the ester compound (1), the component A7, and the component B.
Examples of the component C include additives such as a release agent, a lubricant, a plasticizer, an antioxidant, an antistatic agent, a light stabilizer, an ultraviolet absorber, a flame retardant, a flame retardant aid, a polymerization inhibitor, a filler, a pigment, a dye, a silane coupling agent, a leveling agent, an antifoaming agent, and a fluorescent agent.
The ester compound-containing composition may comprise unreacted raw materials in the production of the ester compound-containing composition, such as methyl (meth)acrylate, alcohol, and (meth)acrylic acid.
The ester compound-containing composition may comprise impurities generated during the production of the ester compound-containing composition, such as diacetyl, but from the viewpoint of suppressing coloration of the ester compound-containing composition, the concentration of the diacetyl is preferably 5 ppm by mass or less, more preferably 2 ppm by mass or less, still more preferably 1 ppm by mass or less, and particularly preferably 0.1 ppm by mass or less.
The ester compound-containing composition may comprise a (meth)acrylic acid ester other than the ester compound (1).
The contained amount of the ester compound (I) is 95.00% to 99.99% by mass. When the contained amount of the ester compound (I) is 90.00% by mass or more, the amount of impurities when the (meth)acrylic polymer is produced by polymerization of the ester compound-containing composition can be reduced, and thus it is possible to prevent physical properties of the polymer from being adversely affected. In addition, when the contained amount of the ester compound (I) is 99.99% by mass or less, a purification cost can be reduced. The contained amount of the ester compound (I) is preferably 96.00% by mass or more, more preferably 97.00% by mass or more, still more preferably 98.00% by mass or more, particularly preferably 99.00% by mass or more, and most preferably 99.50% by mass or more.
When the ester compound (I) includes the ester compound (1) and does not include the ester compound (2), the ester compound (3), the ester compound (4), and the ester compound (5), the contained amount of the ester compound (I) is the concentration of the ester compound (1).
When the ester compound (I) includes the ester compound (5) and does not include the ester compound (1), the ester compound (2), the ester compound (3), and the ester compound (4), the contained amount of the ester compound (I) is the concentration of the ester compound (5).
When the ester compound (I) includes at least one selected from the group consisting of the ester compound (2), the ester compound (3), and the ester compound (4), and does not include the ester compound (1) and the ester compound (5), the contained amount of the ester compound (I) is the concentration of at least one selected from the group consisting of the ester compound (2), the ester compound (3), and the ester compound (4).
The contained amount of the component A7 is not particularly limited, but is preferably 1 to 10,000 ppm by mass. When the contained amount of the component A7 is 1 ppm by mass or more, an effect of suppressing the generation of the dimer of the ester compound (I) and the oxidative product of the ester compound (I) can be sufficiently obtained. In addition, when the contained amount thereof is 10,000 ppm by mass or less, the amount of impurities when the (meth)acrylic polymer is produced by polymerization of the ester compound-containing composition according to the third aspect can be reduced, and thus it is possible to prevent physical properties of the polymer from being adversely affected. The contained amount of the component A7 is more preferably 3 ppm by mass or more, still more preferably 5 ppm by mass or more, and particularly preferably 10 ppm by mass or more. The contained amount of the component A7 is more preferably 7,500 ppm by mass or less, still more preferably 5,000 ppm by mass or less, even more preferably 2,500 ppm by mass or less, even still more preferably 1,500 ppm by mass or less, particularly preferably 1,000 ppm by mass or less, and most preferably 500 ppm by mass or less.
The contained amount of the component B is not particularly limited, but is preferably 1 to 1,000 ppm by mass. When the contained amount of the component B is 1 ppm by mass or more, an effect of suppressing the generation of the dimer of the ester compound (I) and the oxidative product of the ester compound (I) can be sufficiently obtained. In addition, when the contained amount thereof is 1,000 ppm by mass or less, the amount of impurities when the (meth)acrylic polymer is produced by polymerization of the ester compound-containing composition according to the third aspect can be reduced, and thus it is possible to prevent physical properties of the polymer from being adversely affected. The contained amount of the component A7 is more preferably 3 ppm by mass or more, still more preferably 5 ppm by mass or more, and particularly preferably 10 ppm by mass or more. The contained amount of the component A7 is more preferably 750 ppm by mass or less, still more preferably 500 ppm by mass or less, even more preferably 250 ppm by mass or less, particularly preferably 100 ppm by mass or less, and most preferably 50 ppm by mass or less.
The moisture content of the ester compound-containing composition is preferably 5,000 ppm by mass or less, more preferably 4,000 ppm by mass or less, still more preferably 3,000 ppm by mass or less, particularly preferably 2,000 ppm by mass or less, and most preferably 1,000 ppm by mass or less with respect to the total mass of the ester compound-containing composition. When the moisture content of the ester compound-containing composition is the above-described

upper limit value or less, the physical properties of the (meth)acrylic polymer obtained by polymerizing the ester compound-containing composition can be maintained more satisfactorily.

The lower limit value of the moisture content of the ester compound-containing composition is 0 ppm by mass.

(Analysis of ester compound-containing composition)

The fact that the ester compound-containing composition comprises the component A7, the component B, the component C, and the water can be confirmed by, for example, GC-MS measurement. In a GC-MS chart of the ester compound-containing composition, when a peak is present at the same retention time as a sample of the component A7 and an m/z value detected in a mass spectrum of the peak matches exact mass of the component A7, it can be determined that the ester compound-containing composition comprises the component A7. When the sample of the component A7 cannot be obtained, when a pattern of the mass spectrum of the peak appearing in the GC-MS chart of the ester compound-containing composition and a pattern of a mass spectrum of the component A7 in mass spectrum database and match each other, it can be determined that the peak is the peak of the component A7. That is, it can be determined that the ester compound-containing composition comprises the component A7. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

The contained amount of the ester compound (I) can be calculated, for example, by performing GC-FID measurement of the ester compound-containing composition, quantifying by an area percentage method, and correcting the quantified moisture content using a Karl Fischer moisture meter.

The contained amount of the component A7 can be quantified, for example, by performing GC measurement or GC-MS measurement of the ester compound-containing composition and using an internal standard method or an absolute calibration curve method. When a sample of the component A7 cannot be obtained and the component A7 cannot be quantified by the internal standard method or the absolute calibration curve method, the contained amount of the component A7 can be calculated using the following expression by performing GC-FID measurement on any organic compound having a known concentration under the same conditions as those of the ester compound-containing composition.

$$\text{Contained amount of component A7 (ppm by mass)} = \frac{N}{N_{A7}} \times \frac{S_{A7}}{S} \times M$$

Here, N is the number of carbon atoms in one molecule of the organic compound having a known concentration, $N_{A7}$ is the number of carbon atoms in one molecule of the component A7, $S_{A7}$ is a peak area of the component A7, S is a peak area of the organic compound having a known concentration, and M is the concentration (ppm by mass) of the organic compound having a known concentration. When the volatility is low and the quantification cannot be performed by the GC measurement, the quantification can be performed using a chromatography method such as LC.

Concentrations of the component B and the component C can also be calculated by the same method as that for the component A7.

The fact that the ester compound-containing composition comprises water, and the concentration thereof can be confirmed by Karl Fischer method.

[Method for producing ester compound-containing composition]

A method for producing the ester compound-containing composition according to the third aspect includes performing an ester exchange reaction between one or more alcohols selected from the group consisting of a monoalcohol having 2 to 20 carbon atoms, an ether bond-containing alcohol having 2 to 8 carbon atoms, a dialcohol having 2 to 8 carbon atoms, and a trialcohol having 2 to 8 carbon atoms, and methyl (meth)acrylate in the presence of the component A7.

Among (meth)acrylic acid esters, the methyl (meth)acrylate tends to particularly easily undergo dimerization or oxidation to produce methyl pyruvate. However, by performing the ester exchange reaction in the presence of the component A7, the dimerization of methyl (meth)acrylate or the generation of methyl pyruvate is suppressed, and as a result, the yield of the ester compound (I) is improved.

For example, when obtaining the ester compound-containing composition comprising the ester compound (1) as the ester compound (I), in a reaction vessel, the methyl (meth)acrylate is subjected to an ester exchange with the monoalcohol having 2 to 20 carbon atoms as represented by Formula (II) in the presence of a catalyst and the component A7.

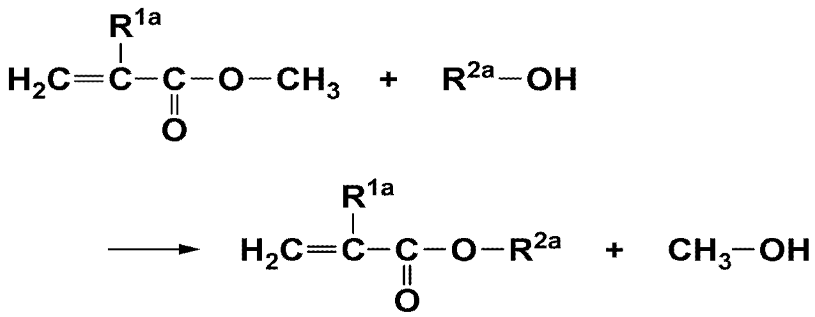

· · · (II)

$R^{1a}$ and $R^{2a}$ in Formula (II) are the same as $R^{1a}$ and $R^{2a}$ in Formula (1).

Examples of the monoalcohol having 2 to 20 carbon atoms include ethanol, n-butanol, isobutanol, t-butanol, 2-ethylhexanol, lauryl alcohol, stearyl alcohol, cyclohexanol, isobornyl alcohol, allyl alcohol, phenol, and benzyl alcohol.

The monoalcohol having 2 to 20 carbon atoms may be used alone or a combination of two or more kinds thereof may be used. The monoalcohol having 2 to 20 carbon atoms preferably includes a linear or branched monoalcohol having 2 to 20 carbon atoms, and more preferably includes n-butanol or isobutanol.

For example, when obtaining the ester compound-containing composition comprising the ester compound (5) as the ester compound (I), in a reaction vessel, the methyl (meth)acrylate is subjected to an ester exchange with the ether bond-containing alcohol having 2 to 8 carbon atoms as represented by Formula (III) in the presence of a catalyst and the component A7.

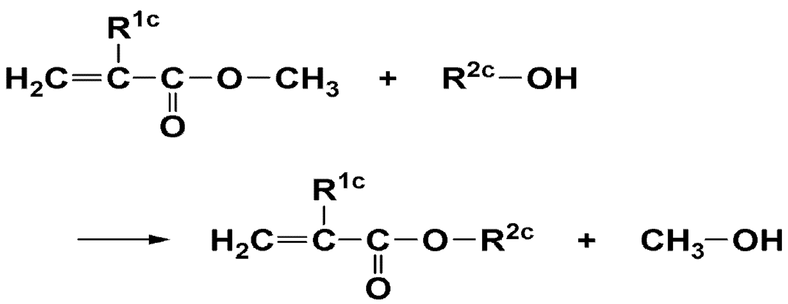

· · · (III)

$R^{1c}$ and $R^{2c}$ in Formula (III) are the same as $R^{1c}$ and $R^{2c}$ in Formula (5).

The number of ether bonds in the ether bond-containing alcohol having 2 to 8 carbon atoms is preferably 1, but is not limited thereto.

Examples of the ether bond-containing alcohol having 2 to 8 carbon atoms include 2-methoxyethanol, glycidyl alcohol, and tetrahydrofurfuryl alcohol. The ether bond-containing alcohol having 2 to 8 carbon atoms may be used alone or a combination of two or more kinds thereof may be used.

The ether bond-containing alcohol having 2 to 8 carbon atoms preferably includes one selected from 2-methoxyethanol, glycidyl alcohol, and tetrahydrofurfuryl alcohol.

For example, when obtaining the ester compound-containing composition comprising at least one or more selected from the group consisting of the ester compound (2), the ester compound (3), and the ester compound (4) as the ester compound (I), in a reaction vessel, the methyl (meth)acrylate is subjected to an ester exchange with one or more alcohols selected from the group consisting of the dialcohol having 2 to 8 carbon atoms and the trialcohol having 2 to 8 carbon atoms as represented by Formula (IV) and Formula (V) in the presence of a catalyst and the component A7.

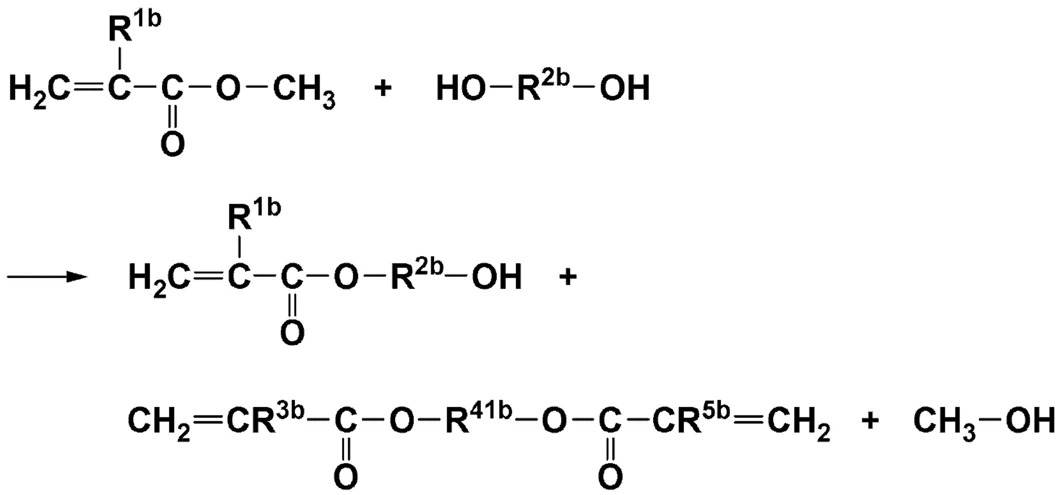

・・・(IV)

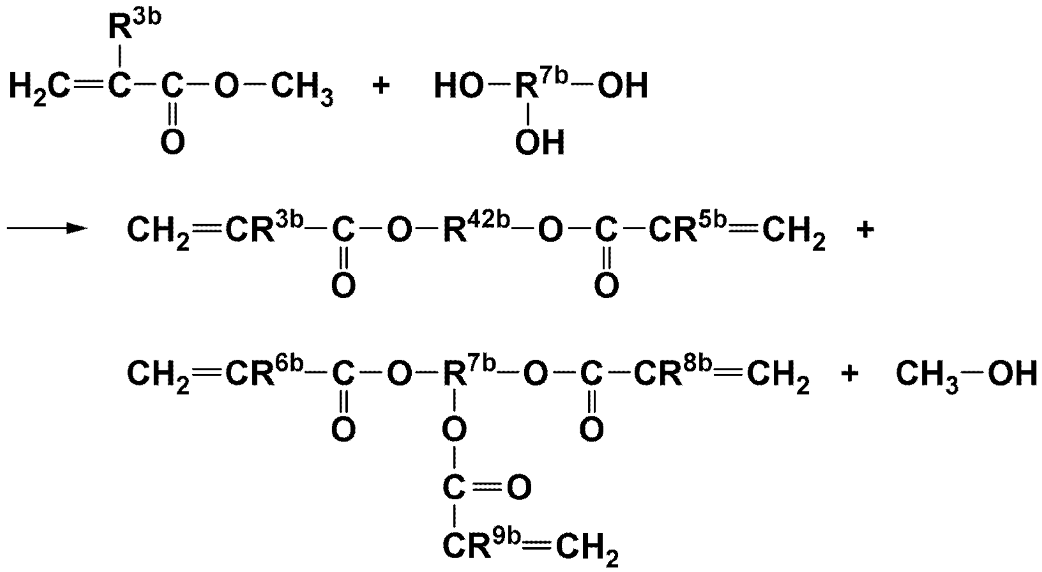

・・・(V)

$R^{1b}$, $R^{2b}$, $R^{3b}$, $R^{5b}$, $R^{6b}$, and $R^{7b}$ in Formula (IV) and Formula (V) are the same as $R^{1b}$, $R^{2b}$, $R^{3b}$, $R^{5b}$, $R^{6b}$, and $R^{7b}$ in Formula (2), Formula (3), and Formula (4). In addition, $R^{41b}$ is a linear or branched alkylene group having 2 to 8 carbon atoms, and $R^{42b}$ is a linear or branched hydroxyalkylene group having 2 to 8 carbon atoms.

Examples of the dialcohol having 2 to 8 carbon atoms and the trialcohol having 2 to 8 carbon atoms include ethylene glycol, 1,2-propanediol, 1,3-propanediol, 1,3-butanediol, 1,6-hexanediol, and trimethylolpropane. Among these, it is preferable that the dialcohol having 2 to 8 carbon atoms and the trialcohol having 2 to 8 carbon atoms include one or more selected from the group consisting of ethylene glycol, 1,2-propanediol, 1,3-propanediol, and trimethylolpropane.

The dialcohol having 2 to 8 carbon atoms and the trialcohol having 2 to 8 carbon atoms may be used alone or a combination of two or more kinds thereof may be used.

The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic mixture with the methyl (meth)acrylate to the outside of the system.

Examples of the reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

The ratio of the amount of the methyl (meth)acrylate to be charged and the amount of the alcohol to be charged can be appropriately determined. From the viewpoint of improving productivity, the ratio of the methyl (meth)acrylate to 1 mol of alcohol is preferably 0.1 mol or more and 10 mol or less, and more preferably 0.3 mol or more and 4 mol or less.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction product during the ester exchange reaction and high catalytic activity, an alkoxide of titanium, dibutyltin oxide, or dioctyltin oxide is preferable. The catalyst may be used alone or a combination of two or more kinds thereof may be used.

The catalyst can be supplied alone to the reaction vessel. In addition, the catalyst can also be supplied to the reaction vessel in a state of being dissolved in the same alcohol as the alcohol as the raw material, or in a state of being dissolved in (meth)acrylic acid ester as the raw material. Examples thereof include a method of directly dissolving the catalyst in the total amount of the alcohol used in the reaction and supplying the catalyst to the reaction vessel, and a method of dissolving the catalyst in a part of the alcohol used in the reaction and supplying the catalyst to the reaction vessel.

The amount of the catalyst used is preferably 0.001 mol% or more and 1 mol% or less, and more preferably 0.01 mol% or more and 0.1 mol% or less with respect to 1 mol of the alcohol.

A solvent may be used for the ester exchange reaction. When a solvent is used, it is preferable to use a solvent which forms an azeotropic composition with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or a combination of two or more kinds thereof may be used.

The reaction temperature for the ester exchange reaction varies depending on the type of the alcohol or the solvent, but is preferably 60°C or higher and 150°C or lower.

The reaction pressure for the ester exchange reaction is not particularly limited, and the reaction may be carried out under any pressure of reduced pressure, normal pressure, or elevated pressure.

The form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, various methods such as distillation, crystallization, extraction, and column chromatography can be performed.

The ester exchange reaction may be performed by blending the component B into the reaction solution. When the component B is present in addition to the component A7, the dimerization of methyl (meth)acrylate or the generation of methyl pyruvate is further suppressed, and thus an effect of improving the yield of the ester compound (I) is more easily obtained.

The component B may be blended into a solution containing the ester compound (I) after the ester exchange reaction and the component A7. In this case, a solution (B solution) containing the ester compound (I) and the component B is prepared separately from the solution (A solution) containing the ester compound (I) and the component A7, and the A solution and the B solution are mixed with each other to obtain the ester compound-containing composition. In addition, the ester compound (I), the A solution, and the B solution may be mixed with each other to obtain the ester compound-containing composition.

The method for producing an ester compound-containing composition is not limited to the above-described method. For example, other (meth)acrylic acid esters other than the methyl (meth)acrylate may be used as the raw material instead of the methyl (meth)acrylate.

An esterification reaction between an alcohol and (meth)acrylic acid may be carried out to produce the ester compound (I).

(Evaluation method for storage stability and thermal stability of ester compound-containing composition)

The ester compound-containing composition has high quality stability during storage. Examples of an evaluation method of the quality stability of the ester compound-containing composition during storage include a method of actually storing the ester compound-containing composition for a long period of time and confirming the generation amount of the dimer of the ester compound (I), the generation amount of the oxidative product of the ester compound (I), and the contained amount of the ester compound (I). In addition, from the viewpoint of ease of work, a method of heating the ester compound-containing composition short period of time and confirming the generation amount of the dimer of the ester

compound (I), the generation amount of the oxidative product of the ester compound (I), and the contained amount of the ester compound (I) may be used. When heating short period of time, the heating temperature is preferably 50°C to 100°C and a heating time is preferably 1 to 24 hours. When the ester compound-containing composition is stored at 25°C for 14 days or heated at 70°C for 7 to 20 hours, the quality stability of the ester compound-containing composition during storage is evaluated based on the generation amount of the dimer of the ester compound (I), the generation amount of the oxidative product of the ester compound (I), and the contained amount of the ester compound (I).

[Polymerizable composition]

A polymerizable composition according to the third aspect is a polymerizable composition for producing a (meth) acrylic polymer, and comprises the above-described ester compound-containing composition according to the third aspect.

As an example, an ester compound-containing composition stored for 1 day or more after production can be used as the polymerizable composition.

The storage time of the ester compound-containing composition can be 1 day or more, 3 days or more, 7 days or more, or 14 days or more. In addition, the storage time of the ester compound-containing composition may be 180 days or less, 150 days or less, 120 days or less, or 90 days or less. The "storage time" means an elapsed time from immediately after the production, which is not limited to a time during which the product is left to stand under a specific environment and also includes a time of transportation or the like.

The material of a storage container for the ester compound-containing composition is not particularly limited, and for example, a metal container such as stainless steel, a resin container, or a glass container can be used. A transparent container or an opaque container can be used.

The temperature at the time of storing the ester compound-containing composition is preferably -10°C or higher and more preferably 0°C or higher, and is preferably 60°C or lower and more preferably 50°C or lower. By setting the temperature to -10°C or higher, a load on a cooling device can be reduced, and by setting the temperature to 60°C or lower, it is easy to suppress the generation of the dimerized substance or the oxidative product of (meth)acrylic acid ester.

The oxygen concentration of a gas phase portion at the time of storing the ester compound-containing composition is preferably 5% by volume or more and more preferably 7% by volume or more, and is preferably 30% by volume or less and more preferably 22% by volume or less. By setting the oxygen concentration of the gas phase portion to 5% by volume or more, it is easy to prevent the ester compound from being unintentionally polymerized by a polymerization prevention effect of oxygen, and by setting the oxygen concentration of the gas phase portion to 30% by volume or less, it is easy to suppress oxidation of the (meth)acrylic acid ester by oxygen and the generation of various impurities.

As an example, the ester compound-containing composition after the storage may be used in the polymerizable composition without further blending a monomer, or a monomer (hereinafter, also referred to as "other monomer") copolymerizable with the ester compound (I) may be further blended with the ester compound-containing composition after the storage to obtain the polymerizable composition.

As another example, the ester compound-containing composition after the production may be stored in a state in which the other monomer is blended, and used in the polymerizable composition. In this case, it is preferable that a polymerization initiator is not blended during the storage.

The proportion of the ester compound (I) to the total mass of monomers in the polymerizable composition is preferably 10% by mass or more and more preferably 20% by mass or more, and preferably 90% by mass or less and more preferably 80% by mass or less. As a result, a (meth)acrylic polymer having high transparency can be obtained.

The ester compound-containing composition may comprise a monomer copolymerizable with the ester compound (I), or may not contain a monomer copolymerizable with the ester compound (I).

Examples of the monomer copolymerizable with the ester compound (I) include other monomer copolymerizable with the ester compound (1);

other monomer copolymerizable with the ester compound (5); and
other monomer copolymerizable with at least one selected from the group consisting of the ester compound (2), the ester compound (3), and the ester compound (4).

(Other monomer copolymerizable with ester compound (1))

Examples of the other monomer copolymerizable with the ester compound (1) include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.

Examples of the unsaturated carboxylic acid include acrylic acid, methacrylic acid, maleic acid, and itaconic acid.

Examples of the unsaturated carboxylic acid anhydride include maleic acid anhydride and itaconic acid anhydride.

Examples of the maleimide include N-phenylmaleimide and N-cyclohexylmaleimide.

Examples of the hydroxy group-containing vinyl monomer include 2-hydroxyethyl acrylate, 2-hydroxyethyl methacrylate, and 2-hydroxypropyl methacrylate.

Examples of the vinyl ester include vinyl acetate and vinyl benzoate.

Examples of the nitrogen-containing vinyl monomer include methacrylamide and acrylonitrile.

Examples of the epoxy group-containing monomer include glycidyl acrylate and glycidyl methacrylate.

Examples of the aromatic vinyl monomer include styrene and $\alpha$-methylstyrene.

Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.

Examples of the polyoxyalkylene glycol di(meth)acrylate include diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate.

Examples of the vinyl monomer having two or more ethylenically unsaturated bonds in the molecule include divinylbenzene.

As the other monomer, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.

The other monomer may be used alone or a combination of two or more kinds thereof may be used.

(Other monomer copolymerizable with at least one selected from group consisting of ester compound (2), ester compound (3), and ester compound (4))

Examples of the other monomer copolymerizable with at least one selected from the group consisting of the ester compound (2), the ester compound (3), and the ester compound (4) include the same monomers as those of the other monomer copolymerizable with the ester compound (1).

(Other monomer copolymerizable with ester compound (5))

Examples of the other monomer copolymerizable with the ester compound (5) include the same monomers as those of the other monomer copolymerizable with the ester compound (1).

When the component A7 is a monomer copolymerizable with the ester compound (I), the component A7 may be used as the monomer copolymerizable with the ester compound (I), or a monomer copolymerizable with the ester compound (I) may be used separately from the component A7.

The polymerizable composition preferably comprises a polymerization initiator.

Examples of the polymerization initiator include an azo compound, an organic peroxide, a persulfate compound, and a redox polymerization initiator.

The polymerization initiator may be used alone or a combination of two or more kinds thereof may be used.

Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.

Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.

Examples of the persulfate compound include potassium persulfate.

The additional amountof the polymerization initiator is not particularly limited, and for example, can be 0.005 to 5 parts by mass with respect to 100 parts by mass of the total mass of the monomers in the polymerizable composition.

The polymerizable composition may comprise, as necessary, other additives such as a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a

leveling agent, an antifoaming agent, and a fluorescent agent.

The other additives may be used alone or a combination of two or more kinds thereof may be used.

[Method for producing (meth)acrylic polymer]

A (meth)acrylic polymer can be produced by polymerizing the polymerizable composition.

The polymerization method is not particularly limited, and examples thereof include a bulk polymerization method, a solution polymerization method, an emulsion polymerization method, and a suspension polymerization method. From the viewpoint of environmental load due to the use of the solvent or the like and viewpoint of transparency of the obtained (meth)acrylic polymer, a bulk polymerization method is preferable.

The method of the bulk polymerization method is not particularly limited, and examples thereof include various casting polymerization methods such as a cell casting method and a continuous casting method.

The casting polymerization method is a method in which the (meth)acrylic polymer is obtained by casting and polymerizing the polymerizable composition in a mold made of two inorganic glass plates or metal plates (for example, SUS plates) arranged facing each other at a predetermined interval with the periphery sealed with a gasket such as a soft resin tube.

The mold for the casting polymerization is not particularly limited, and various molds can be used. Examples of a mold for cell casting include a mold in which two plate-shaped products such as an inorganic glass plate, a chromium-plated metal plate, and a stainless steel plate are arranged facing each other at a predetermined interval, and a gasket is disposed on edges of the plates to form a sealed space between the plate-shaped products and the gasket. Examples of the mold for continuous casting include a mold in which a sealed space is formed by opposing surfaces of a pair of endless belts running in the same direction at the same speed and gaskets running at the same speed as the endless belt on both sides of the endless belt.

A gap between cavities of the molds is appropriately adjusted to obtain a resin plate having a desired thickness, and is generally 1 to 30 mm.

The polymerization temperature is preferably 125°C to 210°C and more preferably 130°C to 180°C.

The polymerization time is preferably 0.5 to 24 hours.

The weight-average molecular weight (Mw) of the (meth)acrylic polymer is not particularly limited, and can be, for example, 100,000 to 1,000,000. As the Mw of the (meth)acrylic polymer is higher, solvent resistance and chemical resistance can be further improved.

The Mw of the (meth)acrylic polymer can be controlled by adjusting the polymerization temperature, the polymerization time, the addition amount of the polymerization initiator, and the like.

The (meth)acrylic polymer according to the third aspect has excellent heat resistance and excellent meltability. For example, among (meth)acrylic polymers, a granular (meth)acrylic polymer having a large amount of resin which can be stored per unit volume and having a small energy cost during storage and transportation needs to be dissolved or melted at the time of use. The (meth)acrylic polymer according to the third aspect is easily melted, has excellent kneading properties with other resins, and has excellent solubility in a monomer, a solvent, or the like.

4. Fourth aspect

Hereinafter, a fourth aspect of the embodiment will be described.

[Ester compound-containing composition]

An ester compound-containing composition according to the fourth aspect comprises an ester compound (I) described later and a compound (component A8) described later. The contained amount of the ester compound (I) is 95.00% to 99.99% by mass.

The ester compound-containing composition may further comprise a polymerization inhibitor (component B) described later, in addition to the ester compound (I) and the component A8.

As long as the effect of the present invention is not impaired, the ester compound-containing composition may further comprise at least one of a compound other than the ester compound (I), the component A8, and the component B (hereinafter, also referred to as "component C") or water as necessary, in addition to the ester compound (I) and the component A8.

(Ester compound (I))

The ester compound (I) is a compound represented by Formula (I).

$$CH_2=CR^{150}-C(=O)-O-R^{200} \cdots \quad (I)$$

In Formula (I), $R^{150}$ is a hydrogen atom or a methyl group, and
$R^{200}$ is a monovalent hydrocarbon group having 2 to 20 carbon atoms, which may have a substituent, or a monovalent group having an ether bond and having 2 to 8 carbon atoms.

The ester compound (I) may be used alone or a combination of two or more kinds thereof may be used.

As the ester compound (I), one or more compounds selected from the group consisting of an ester compound (1) described later, an ester compound (2) described later, an ester compound (3) described later, an ester compound (4) described later, and an ester compound (5) described later are preferable. Hereinafter, the ester compounds will be described.

The ester compound (1) is a (meth)acrylic acid ester represented by Formula (1).

$$CH_2=CR^{1a}-C(=O)-O-R^{2a} \cdots \quad (1)$$

In Formula (1), $R^{1a}$ is a hydrogen atom or a methyl group. $R^{2a}$ is a hydrocarbon group having 2 to 20 carbon atoms.

The hydrocarbon group of $R^{2a}$ may be a saturated hydrocarbon group or an unsaturated hydrocarbon group.

The hydrocarbon group of $R^{2a}$ may be linear or branched, or may have a ring. When the hydrocarbon group of $R^{2a}$ has a ring, the ring may be an aliphatic ring or an aromatic ring.

The number of carbon atoms in the hydrocarbon group of $R^{2a}$ is 2 to 20, preferably 2 to 18 and more preferably 2 to 12.

Examples of the hydrocarbon group of $R^{2a}$ include an alkyl group having 2 to 20 carbon atoms, a cycloalkyl group having 3 to 20 carbon atoms, an alkenyl group having 2 to 20 carbon atoms, a cycloalkenyl group having 3 to 20 carbon atoms, an alkynyl group having 2 to 20 carbon atoms, an aryl group having 6 to 20 carbon atoms, and an aromatic alkyl group having 7 to 20 carbon atoms. The "aromatic alkyl group" means a group in which one or more hydrogen atoms of an alkyl group are substituted with an aryl group.

Examples of the alkyl group of $R^{2a}$ include an ethyl group, a propyl group, an isopropyl group, an n-butyl group, an isobutyl group, a t-butyl group, a 2-ethylhexyl group, a lauryl group, and a stearyl group.

Examples of the cycloalkyl group of $R^{2a}$ include a cyclopropyl group, a cyclohexyl group, and an isobornyl group.

Examples of the alkenyl group of $R^{2a}$ include a vinyl group and an allyl group.

Examples of the cycloalkenyl group of $R^{2a}$ include a cyclopentenyl group, a cyclopentadienyl group, and a cyclohexenyl group.

Examples of the alkynyl group of $R^{2a}$ include a propynyl group.

Examples of the aryl group of $R^{2a}$ include a phenyl group and a naphthyl group.

Examples of the aromatic alkyl group of $R^{2a}$ include a benzyl group.

From the viewpoint that the ester compound (1) is relatively easy to obtain and is relatively easy to handle in terms of physical properties, $R^{2a}$ is preferably an alkyl group having 2 to 20 carbon atoms, a cycloalkyl group having 3 to 20 carbon atoms, an alkenyl group having 2 to 20 carbon atoms, an aryl group having 6 to 20 carbon atoms, or an aromatic alkyl group having 7 to 20 carbon atoms; more preferably an ethyl group, an isopropyl group, an n-butyl group, an isobutyl group, a t-butyl group, a 2-ethylhexyl group, a lauryl group, a stearyl group, a cyclohexyl group, an isobornyl group, an allyl group, a phenyl group, or a benzyl group; particularly preferably an n-butyl group or an isobutyl group; and most preferably an n-butyl group.

Examples of the ester compound (1) include ethyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, and benzyl (meth)acrylate.

As the ester compound (1), from the viewpoint that the ester compound (1) is relatively easy to obtain and is relatively easy to handle in terms of physical properties, an alkyl (meth)acrylate in which $R^{2a}$ is a linear or branched alkyl group having 2 to 20 carbon atoms, a cycloalkyl (meth)acrylate in which $R^{2a}$ is a cycloalkyl group having 3 to 20 carbon atoms, an alkenyl (meth)acrylate in which $R^{2a}$ is an alkenyl group having 2 to 20 carbon atoms, an aryl (meth)acrylate in which $R^{2a}$ is an aryl group having 6 to 20 carbon atoms, or an aromatic alkyl (meth)acrylate in which $R^{2a}$ is an aromatic alkyl group having 7 to 20 carbon atoms is preferable; ethyl (meth)acrylate, isopropyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, or benzyl (meth)acrylate is more preferable; butyl (meth)acrylate or isobutyl (meth)acrylate is particularly preferable; and butyl (meth)acrylate is most preferable.

The ester compound (1) may be used alone or a combination of two or more kinds thereof may be used.

The ester compound (2) is a (meth)acrylic acid ester represented by Formula (2). The ester compound (3) is a (meth)acrylic acid ester represented by Formula (3). The ester compound (4) is a (meth)acrylic acid ester represented by

Formula (4).

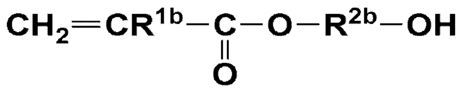

・・・(2)

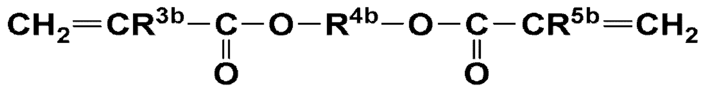

・・・(3)

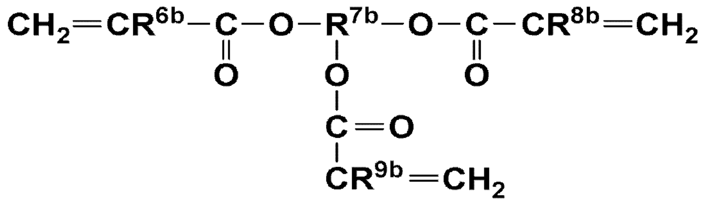

・・・(4)

In Formula (2), Formula (3), and Formula (4), $R^{1b}$ , $R^{3b}$ $R^{5b}$, $R^{6b}$, $R^{8b}$, and $R^{9b}$ are each independently a hydrogen atom or a methyl group. $R^{2b}$ and $R^{4b}$ are each independently a linear or branched alkylene group or a hydroxyalkylene group, having 2 to 8 carbon atoms. $R^{7b}$ is a linear or branched trivalent hydrocarbon group having 2 to 8 carbon atoms.

The number of carbon atoms in the alkylene group or the hydroxyalkylene group of $R^{2b}$ and $R^{4b}$ is 2 to 8, preferably 2 to 6.

Examples of the alkylene group of $R^{2b}$ and $R^{4b}$ include an ethylene group, a propylene group, an isopropylene group, and a butylene group.

Examples of the hydroxyalkylene group of $R^{2b}$ and $R^{4b}$ include a hydroxyethylene group, a hydroxypropylene group, and a hydroxybutylene group.

The number of carbon atoms in the trivalent hydrocarbon group of $R^{7b}$ is 2 to 8, preferably 2 to 4.

Examples of the trivalent hydrocarbon group of $R^{7b}$ include $-(CH_2)-C(-CH_2-)(-CH_3)-CH_2-$.

Examples of the ester compound (2) include 2-hydroxyethyl (meth)acrylate, 2-hydroxypropyl (meth)acrylate, and 3-hydroxypropyl (meth)acrylate.

Examples of the ester compound (3) include ethylene glycol di(meth)acrylate, 1,3-propanediol di(meth)acrylate, 1,2-propanediol di(meth)acrylate, 1,3-butanediol di(meth)acrylate, and 1,6-hexanediol di(meth)acrylate.

Examples of the ester compound (4) include trimethylolpropane tri(meth)acrylate.

From the viewpoint of relatively easy availability, one or more selected from the group consisting of ethylene glycol di(meth)acrylate, 2-hydroxyethyl (meth)acrylate, 3-hydroxypropyl (meth)acrylate, and trimethylolpropane tri(meth) acrylate are preferable as the ester compound (2), the ester compound (3), and the ester compound (4).

The ester compound (2), the ester compound (3), and the ester compound (4) may be used alone or a combination of two or more kinds thereof may be used.

The ester compound (5) is a (meth)acrylic acid ester represented by Formula (5).

$$CH_2=CR^{1c}-C(=O)-O-R^{2c} \cdots \qquad (5)$$

In Formula (5), $R^{1c}$ is a hydrogen atom or a methyl group, and $R^{2c}$ is a monovalent group having an ether bond and having 2 to 8 carbon atoms.

The number of etheric oxygens included in the monovalent group having 2 to 8 carbon atoms of $R^{2c}$ is preferably 1, but it is not limited thereto and may be 2 or more.

The monovalent group having 2 to 8 carbon atoms of $R^{2c}$ may be linear or branched, or may have a ring. When $R^{2c}$ has a ring, the ring may or may not include the etheric oxygen.

The number of carbon atoms in the monovalent group having an ether bond of $R^{2c}$ is 2 to 8, preferably 2 to 7.

Examples of $R^{2c}$ include a 2-methoxyethyl group, a 2-(2-methoxyethoxy)ethyl group, a 2-[2-(2-methoxyethoxy) ethoxy]ethyl group, a glycidyl group, and a tetrahydrofurfuryl group.

As $R^{2c}$, from the viewpoint of relatively easy availability and relatively easy handling in terms of physical properties, a 2-methoxyethyl group, a glycidyl group, or a tetrahydrofurfuryl group is preferable.

Examples of the ester compound (5) include 2-methoxyethyl (meth)acrylate, glycidyl (meth)acrylate, 2-(2-methoxyethoxy)ethyl (meth)acrylate, 2-[2-(2-methoxyethoxy)ethoxy]ethyl (meth)acrylate, and tetrahydrofurfuryl (meth) acrylate.

As the ester compound (5), from the viewpoint of relatively easy handling in terms of physical properties, 2-methoxyethyl (meth)acrylate, glycidyl (meth)acrylate, or tetrahydrofurfuryl (meth)acrylate is preferable.

The ester compound (5) may be used alone or a combination of two or more kinds thereof may be used.

(Component A8)

The component A8 is a compound represented by Formula (a8).

When the ester compound-containing composition comprises the component A8, a dimerization reaction of the ester compound (I) and generation of an oxidative product of the ester compound (I) are suppressed during storage, and thus an ester compound-containing composition having excellent storage stability is obtained. The reason for this is presumed to be as follows.

During the storage of the ester compound-containing composition, for example, a radical derived from ultraviolet rays, such as a hydroxyl radical generated by absorption of ultraviolet rays derived from sunlight by oxygen molecules, is generated. Such a radical can cause the generation of a dimer of the ester compound (I) or the generation of the oxidative product of the ester compound (I). Since the component A8 is a $\pi$-conjugated compound having a benzene ring, the component A8 absorbs ultraviolet light, and an absorption wavelength and an absorption intensity thereof change depending on the type of the substituent. In the component A8, since an alkyl group having an appropriate bulkiness and an appropriate electron-donating property is bonded to a benzene ring, ultraviolet light in a wide wavelength range can be absorbed. Therefore, when the ester compound-containing composition comprises the component A8, the ultraviolet light having a wide wavelength range is absorbed, and the generation of the hydroxyl radical is suppressed. Accordingly, it is presumed that the progress of the dimerization of the ester compound (I) by radical polymerization mechanism and the generation of the oxidative product of the ester compound (I) can be suppressed, and a decrease in purity of the ester compound (I) can be suppressed.

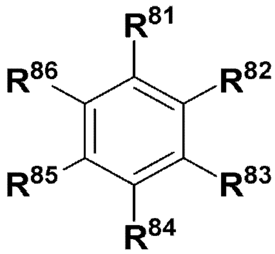

・・・(a8)

In Formula (a8), $R^{81}$, $R^{82}$, $R^{83}$, $R^{84}$, $R^{85}$, and $R^{86}$ are each independently a monovalent group selected from a hydrogen atom, an alkyl group, an alkenyl group, an aryl group, a hydroxy group, an alkoxy group, an amino group, a monovalent group including a carbonyl group, an alkylthio group, or an arylthio group, where the total number of carbon atoms in $R^{81}$, $R^{82}$ , $R^{83}$, $R^{84}$, $R^{85}$, and $R^{86}$ is 2 or more.

The molecular weight of the component A8 is preferably 2,000 or less. When the molecular weight thereof is 2,000 or less, the number of benzene rings per unit mass in the component A8 can be increased, and thus the effect of the present invention can be obtained with a small mass. The molecular weight of the component A8 is more preferably 1,600 or less, still more preferably 1,200 or less, particularly preferably 1,000 or less, and most preferably 800 or less.

in Formula (a8), $R^{81}$, $R^{82}$, $R^{83}$, $R^{84}$, $R^{85}$, and $R^{86}$ are each independently a monovalent group selected from a hydrogen atom, an alkyl group, an alkenyl group, an aryl group, a hydroxy group, an alkoxy group, an amino group, a monovalent group including a carbonyl group, an alkylthio group, or an arylthio group,

$R^{81}$, $R^{82}$, $R^{83}$, $R^{84}$, $R^{85}$, and $R^{86}$ may be the same or different from each other. It is preferable that at least one of $R^{81}$, $R^{82}$, $R^{83}$, $R^{84}$, $R^{85}$, or $R^{86}$ is an alkyl group.

In Formula (a8), when steric hindrance of $R^{81}$, $R^{82}$, $R^{83}$, $R^{84}$, $R^{85}$, and $R^{86}$ is large, the benzene ring is distorted, and the $\pi$-conjugated system is not maintained. However, when $R^{81}$, $R^{82}$, $R^{83}$, $R^{84}$, $R^{85}$, and $R^{86}$ satisfy the above-described conditions, the $\pi$-conjugated system of the component A8 is maintained due to appropriate bulkiness, and thus a property of absorbing ultraviolet light in a wide wavelength range is provided, and the effect of the present invention can be obtained.

From the viewpoint of increasing absorbance of the ultraviolet light to suppress the generation of the hydroxyl radical, $R^{81}$, $R^{82}$, $R^{83}$, $R^{84}$, $R^{85}$, and $R^{86}$ are preferably a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group, or a monovalent group including a carbonyl group; more preferably a hydrogen atom, an alkyl group having 1 to 5 carbon atoms, a hydroxy group, or an alkoxy group having 1 to 6 carbon atoms; still more preferably a hydrogen atom, a methyl group, an ethyl group, a n-propyl group, an isopropyl group, a t-butyl group, a hydroxy group, or a methoxy group; and particularly preferably a hydrogen atom, a methyl group, an ethyl group, a n-propyl group, an isopropyl group, or a t-butyl group.

The alkyl group of $R^{81}$, $R^{82}$, $R^{83}$, $R^{84}$, $R^{85}$, and $R^{86}$ may be linear or branched, or may have a ring.

The number of carbon atoms in the alkyl group is preferably 1 to 20, more preferably 1 to 10, and still more preferably 1 to 5.

Examples of the linear or branched alkyl group (hereinafter, also collectively referred to as "chain alkyl group") include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group. Among these, a methyl group, an ethyl group, an n-propyl group, an isopropyl group, or a t-butyl group is preferable.

Examples of the alkyl group having a ring (hereinafter, also referred to as "cyclic alkyl group") include a cyclopentyl group, a cyclohexyl group, and a cyclooctyl group.

The alkenyl group of $R^{81}$, $R^{82}$, $R^{83}$, $R^{84}$, $R^{85}$, and $R^{86}$ may be linear or branched, or may have a ring.

The number of carbon atoms in the alkenyl group is preferably 2 to 20, more preferably 2 to 10, and still more preferably 2 to 5.

Examples of the linear or branched alkenyl group (hereinafter, also collectively referred to as "chain alkenyl group") include a vinyl group, a 1-propenyl group, an isopropenyl group, a 2-butenyl group, a 1,3-butadienyl group, a 2-pentenyl group, and a 2-hexenyl group.

Examples of the alkenyl group having a ring (hereinafter, also referred to as "cyclic alkenyl group") include a cyclopentenyl group and a cyclohexenyl group.

The number of carbon atoms in the aryl group of $R^{81}$, $R^{82}$, $R^{83}$, $R^{84}$, $R^{85}$, and $R^{86}$ is preferably 1 to 20, more preferably 1 to 12, and still more preferably 6 to 12.

The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The number of carbon atoms in the alkoxy group of $R^{81}$, $R^{82}$, $R^{83}$, $R^{84}$, $R^{85}$, and $R^{86}$ is preferably 1 to 20, more preferably 1 to 10, and still more preferably 1 to 6.

Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group of $R^{81}$, $R^{82}$, $R^{83}$, $R^{84}$, $R^{85}$, and $R^{86}$ includes an amino group ($-NH_2$) having no substituent on a nitrogen atom and an amino group in which a part or all of hydrogen atoms bonded to the nitrogen atom are substituted with carbon atoms.

The number of carbon atoms in the amino group substituted with carbon atoms is preferably 1 to 20, more preferably 1 to 10, and still more preferably 1 to 5.

Examples of the amino group include an unsubstituted amino group, a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group of $R^{81}$, $R^{82}$, $R^{83}$, $R^{84}$, $R^{85}$, and $R^{86}$ include a formyl group, an acyl group, a carboxy group, an amide group, an alkoxycarbonyl group, a thiocarboxy group, and a thioester group.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6.

Examples of the acyl group include an acetyl group, a propionyl group, a butylcarbonyl group, a vinylcarbonyl group, and a benzoyl group.

The amide group includes an amide group ($-CONH_2$) having no substituent on a nitrogen atom and an amide group in which a part or all of hydrogen atoms bonded to the nitrogen atom are substituted with carbon atoms.

As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called

an ester group.

The total number of carbon atoms derived from the carbonyl group and carbon atoms derived from the alkoxy group is preferably 2 to 20, more preferably 2 to 10, and still more preferably 2 to 6.

Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group.

The total number of carbon atoms derived from the carbonyl group and carbon atoms derived from the alkylthio group or the arylthio group is preferably 2 to 20, more preferably 2 to 10, and still more preferably 2 to 6.

Examples of the thioester group include a methylthiocarbonyl group, an ethylthiocarbonyl group, a butylthiocarbonyl group, and a phenylthiocarbonyl group.

As an example, the monovalent group including a carbonyl group may be a substituent in which one or a plurality of hydrogen atoms of an alkyl group are substituted with carbonyl groups. Examples of such a substituent include a 2-acetoxyethyl group, a 2-acetoethyl group, and a 2-(acetoacetoxy)ethyl group.

The number of carbon atoms in the alkylthio group of $R^{81}$, $R^{82}$, $R^{83}$, $R^{84}$, $R^{85}$, and $R^{86}$ is preferably 1 to 20, more preferably 1 to 10, and still more preferably 1 to 5.

Examples of the alkylthio group include a methylthio group, an ethylthio group, a propylthio group, and an isopropylthio group.

The number of carbon atoms in the arylthio group of $R^{81}$, $R^{82}$, $R^{83}$, $R^{84}$, $R^{85}$, and $R^{86}$ is preferably 1 to 20, more preferably 3 to 10, and still more preferably 6 to 10.

Examples of the arylthio group include a phenylthio group and a tolylthio group.

Among the compounds satisfying the above-described conditions, from the viewpoint of further improving the storage stability of the ester compound-containing composition, as the component A8, o-xylene, m-xylene, p-xylene, ethylbenzene, n-propylbenzene, cumene, t-butylbenzene, mesitylene, butyl 2-phenyl isobutyrate, 2-phenylisobutyric acid, 2-isopropylhydroquinone, 2-isopropyl-4-methoxyphenol, 2-methyl-2-phenylpropionamide, butyl 2-(2-hydroxy-5-methoxyphenyl)-2-methylpropionate, or butyl 2-(2,5-dihydroxyphenyl)-2-methylpropionate is preferable; o-xylene, m-xylene, p-xylene, ethylbenzene, n-propylbenzene, cumene, t-butylbenzene, butyl 2-phenyl isobutyrate, 2-phenylisobutyric acid, phenyl isobutyrate, 2-isopropylhydroquinone, or 2-isopropyl-4-methoxyphenol is more preferable; o-xylene, m-xylene, p-xylene, ethylbenzene, n-propylbenzene, cumene, or t-butylbenzene is particularly preferable; and o-xylene, m-xylene, p-xylene, or ethylbenzene is most preferable.

The component A8 may be used alone or a combination of two or more kinds thereof may be used.

(Component B)

The component B is a polymerization inhibitor.

When the ester compound-containing composition comprises the component B, the progress of the polymerization reaction of the ester compound (I) by radical polymerization mechanism during storage is suppressed. In addition, during storage, oxygen molecules in the ester compound-containing composition absorb ultraviolet rays derived from sunlight, thereby generating a hydroxyl radical. However, the polymerization inhibitor can trap the hydroxyl radical. Therefore, when the ester compound-containing composition comprises the component A8 and the component B, the amount of the hydroxyl radical can be reduced by two different mechanisms of suppressing the generation of the hydroxyl radical by the component A8 and removing the generated hydroxyl radical by the component B even when the hydroxyl radical is generated. Therefore, the progress of the dimerization of the ester compound (I) and the generation of the oxidative product can be suppressed more efficiently, and a decrease in purity of the ester compound (I) can be suppressed more efficiently.

The polymerization inhibitor means a compound having a function of suppressing the polymerization reaction of the ester compound (I).

Examples of the component B include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

Examples of the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include $\alpha$-tocopherol and 2,3-dihydro-2,2-dimethyl-7-hydroxybenzofuran.

Examples of the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the nitrobenzene-based compound include nitrobenzene, o-dinitrobenzene, m-dinitrobenzene, p-dinitrobenzene, 2,4-dinitrobenzene, dinitrodurene, and 2,2-diphenyl-1-picrylhydrazine.

Examples of the N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethyl-piperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-t-butyl nitroxide, and di-t-amyl nitroxide.

Examples of the amine-based compound include N,N-diphenylamine, alkylated diphenylamine, 4,4'-dicamyldiphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-$\alpha$-naphthylamine, N-phenyl-$\beta$-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl) phosphite, 4,4'-isopropylidenediphenol alkylphosphite, tris(2,4-di-t-butylphenyl)phosphite, tris(biphenyl)phosphite, distearyl pentaerythritol diphosphite, di(2,4-di-t-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-t-butylphenol) diphosphite, hexa(tridecyl)-1,1,3-tris(2-methyl-4-hydroxy-5-t-butylphenyl)butanetriphosphite, 3,5-dit-butyl-4-hydroxybenzyl phosphate diethyl ester, sodium-bis(4-t-butylphenyl)phosphate, sodium-2,2'-methylene-bis(4,6-di-t-butylphenyl)phosphate, and 1,3-bis(diphenoxyphosphoryloxy)benzene.

Examples of the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the iron-containing compound include iron (III) chloride.

Examples of the copper-containing compound include copper dimethyldithiocarbamate, copper diethylthiocarbamate, copper dibutylthiocarbamate, copper salicylate, copper acetate, copper thiocyanate, copper nitrate, copper chloride, copper carbonate, copper hydroxide, copper acrylate, and copper methacrylate.

Examples of the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

From the viewpoint of easily exhibiting the effect of further improving the storage stability of the ester compound-containing composition, as the component B, at least one polymerization inhibitor selected from the group consisting of a

phenol-based compound, an N-oxyl-based compound, an amine-based compound, a phosphorus-containing compound, and a sulfur-containing compound is preferable; at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine is more preferable; and at least one polymerization inhibitor selected from the group consisting of hydroquinone, 4-methoxyphenol, 2,4-dimethyl-6-t-butylphenol, and 2,6-di-t-butyl-4-methylphenol is particularly preferable.

The component B may be used alone or a combination of two or more kinds thereof may be used.

When the ester compound-containing composition comprises a compound corresponding to both the component A8 and the component B, the compound is regarded as the component A8. When the ester compound-containing composition comprises the component A8 and the component B, this means that the ester compound-containing composition further comprises the component B different from the compound.

When the ester compound-containing composition comprises two or more kinds of compounds corresponding to both the component A8 and the component B, a compound having the highest molar concentration in the ester compound-containing composition is regarded as the component A8, and the other compounds are regarded as the component B.

(Component C)

The ester compound-containing composition may comprise other compounds (component C) as long as the contained amount of the ester compound (I) satisfies 95.00% to 99.99% by mass. The component C is a compound other than the ester compound (1), the component A8, and the component B.

Examples of the component C include additives such as a release agent, a lubricant, a plasticizer, an antioxidant, an antistatic agent, a light stabilizer, an ultraviolet absorber, a flame retardant, a flame retardant aid, a polymerization inhibitor, a filler, a pigment, a dye, a silane coupling agent, a leveling agent, an antifoaming agent, and a fluorescent agent.

The ester compound-containing composition may comprise unreacted raw materials in the production of the ester compound-containing composition, such as methyl (meth)acrylate, alcohol, and (meth)acrylic acid.

The ester compound-containing composition may comprise impurities generated during the production of the ester compound-containing composition, such as diacetyl, but from the viewpoint of suppressing coloration of the ester compound-containing composition, the concentration of the diacetyl is preferably 5 ppm by mass or less, more preferably 2 ppm by mass or less, still more preferably 1 ppm by mass or less, and particularly preferably 0.1 ppm by mass or less.

The ester compound-containing composition may comprise a (meth)acrylic acid ester other than the ester compound (1).

The contained amount of the ester compound (I) is 95.00% to 99.99% by mass with respect to the total mass of the ester compound-containing composition. When the contained amount of the ester compound (I) is 90% by mass or more, the amount of impurities when the (meth)acrylic polymer is produced by polymerization of the ester compound-containing composition can be reduced, and thus it is possible to prevent physical properties of the polymer from being adversely affected. In addition, when the contained amount of the ester compound (I) is 99.99% by mass or less, a purification cost can be reduced. The contained amount of the ester compound (I) is preferably 96.00% by mass or more, more preferably 97.00% by mass or more, still more preferably 98.00% by mass or more, particularly preferably 99.00% by mass or more, and most preferably 99.50% by mass or more.

When the ester compound (I) includes the ester compound (1) and does not include the ester compound (2), the ester compound (3), the ester compound (4), and the ester compound (5), the contained amount of the ester compound (I) is the concentration of the ester compound (1).

When the ester compound (I) includes the ester compound (5) and does not include the ester compound (1), the ester compound (2), the ester compound (3), and the ester compound (4), the contained amount of the ester compound (I) is the concentration of the ester compound (5).

When the ester compound (I) includes at least one selected from the group consisting of the ester compound (2), the ester compound (3), and the ester compound (4), and does not include the ester compound (1) and the ester compound (5), the contained amount of the ester compound (I) is the concentration of at least one selected from the group consisting of the ester compound (2), the ester compound (3), and the ester compound (4).

The contained amount of the component A8 is not particularly limited, but is preferably 1 to 10,000 ppm by mass with respect to the total mass of the ester compound-containing composition. When the contained amount of the component A8 is 1 ppm by mass or more, an effect of suppressing the generation of the dimer of the ester compound (I) and the oxidative product of the ester compound (I) can be sufficiently obtained. In addition, when the contained amount of the component A8 is 10,000 ppm by mass or less, the amount of impurities when the (meth)acrylic polymer is produced by polymerization of the ester compound-containing composition can be reduced, and thus it is possible to prevent physical properties of the polymer from being adversely affected. The contained amount of the component A8 is more preferably 3 ppm by mass or more, still more preferably 5 ppm by mass or more, and particularly preferably 10 ppm by mass or more. The contained amount of the component A8 is more preferably 7,500 ppm by mass or less, still more preferably

5,000 ppm by mass or less, even more preferably 2,500 ppm by mass or less, even still more preferably 1,500 ppm by mass or less, particularly preferably 1,000 ppm by mass or less, and most preferably 500 ppm by mass or less.

The contained amount of the component B is not particularly limited, but is preferably 1 to 1,000 ppm by mass with respect to the total mass of the ester compound-containing composition. When the contained amount of the component B is 1 ppm by mass or more, an effect of suppressing the generation of the dimer of the ester compound (I) and the oxidative product of the ester compound (I) can be sufficiently obtained. In addition, when the contained amount of the component B is 1,000 ppm by mass or less, the amount of impurities when the (meth)acrylic polymer is produced by polymerization of the ester compound-containing composition according to the fourth aspect can be reduced, and thus it is possible to prevent physical properties of the polymer from being adversely affected. The contained amount of the component B is more preferably 3 ppm by mass or more, still more preferably 5 ppm by mass or more, and particularly preferably 10 ppm by mass or more. The contained amount of the component B is more preferably 750 ppm by mass or less, still more preferably 500 ppm by mass or less, even more preferably 250 ppm by mass or less, particularly preferably 100 ppm by mass or less, and most preferably 50 ppm by mass or less.

The moisture content of the ester compound-containing composition is preferably 5,000 ppm by mass or less, more preferably 4,000 ppm by mass or less, still more preferably 3,000 ppm by mass or less, particularly preferably 2,000 ppm by mass or less, and most preferably 1,000 ppm by mass or less with respect to the total mass of the ester compound-containing composition. When the moisture content of the ester compound-containing composition is the above-described upper limit value or less, the physical properties of the (meth)acrylic polymer obtained by polymerizing the ester compound-containing composition can be maintained more satisfactorily.

The lower limit value of the moisture content of the ester compound-containing composition is 0 ppm by mass.

(Analysis of ester compound-containing composition)

The fact that the ester compound-containing composition comprises the component A8, the component B, the component C, and the water can be confirmed by, for example, GC-MS measurement. In a GC-MS chart of the ester compound-containing composition, when a peak is present at the same retention time as a sample of the component A8 and an m/z value detected in a mass spectrum of the peak matches exact mass of the component A8, it can be determined that the ester compound-containing composition comprises the component A8. When the sample of the component A8 cannot be obtained, when a pattern of the mass spectrum of the peak appearing in the GC-MS chart of the ester compound-containing composition and a pattern of a mass spectrum of the component A8 in mass spectrum database and match each other, it can be determined that the peak is the peak of the component A8. That is, it can be determined that the ester compound-containing composition comprises the component A8. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

The contained amount of the ester compound (I) can be calculated, for example, by performing GC-FID measurement of the ester compound-containing composition, quantifying by an area percentage method, and correcting the quantified moisture content using a Karl Fischer moisture meter.

The contained amount (concentration) of the component A8 can be quantified, for example, by performing GC measurement or GC-MS measurement of the ester compound-containing composition and using an internal standard method or an absolute calibration curve method. When a sample of the component A8 cannot be obtained and the component A8 cannot be quantified by the internal standard method or the absolute calibration curve method, the contained amount of the component A8 can be calculated using the following expression by performing GC-FID measurement on any organic compound having a known concentration under the same conditions as those of the ester compound-containing composition.

$$\text{Contained amount of component A8 (ppm by mass)} = \frac{N}{N_{A8}} \times \frac{S_{A8}}{S} \times M$$

Here, N is the number of carbon atoms in one molecule of the organic compound having a known concentration, $N_{A8}$ is the number of carbon atoms in one molecule of the component A8, $S_{A8}$ is a peak area of the component A8, S is a peak area of the organic compound having a known concentration, and M is a contained amount (ppm by mass) of the organic compound having a known concentration. When the volatility is low and the quantification cannot be performed by the GC measurement, the quantification can be performed using a chromatography method such as LC.

Contained amounts of the component B and the component C can also be calculated by the same method as that for the component A8 described above.

The fact that the ester compound-containing composition comprises water, and the concentration thereof can be

confirmed by Karl Fischer method.

[Method for producing ester compound-containing composition]

A method for producing the ester compound-containing composition according to the fourth aspect includes performing an ester exchange reaction between one or more alcohols selected from the group consisting of a monoalcohol having 2 to 20 carbon atoms, an ether bond-containing alcohol having 2 to 8 carbon atoms, a dialcohol having 2 to 8 carbon atoms, and a trialcohol having 2 to 8 carbon atoms, and methyl (meth)acrylate in the presence of the component A8.

Among (meth)acrylic acid esters, the methyl (meth)acrylate tends to particularly easily undergo dimerization or oxidation to produce methyl pyruvate. However, by performing the ester exchange reaction in the presence of the component A8, the dimerization of methyl (meth)acrylate or the generation of methyl pyruvate is suppressed, and as a result, the yield of the ester compound (I) is improved.

For example, when obtaining the ester compound-containing composition comprising the ester compound (1) as the ester compound (I), in a reaction vessel, the methyl (meth)acrylate is subjected to an ester exchange with the monoalcohol having 2 to 20 carbon atoms as represented by Formula (II) in the presence of a catalyst and the component A8.

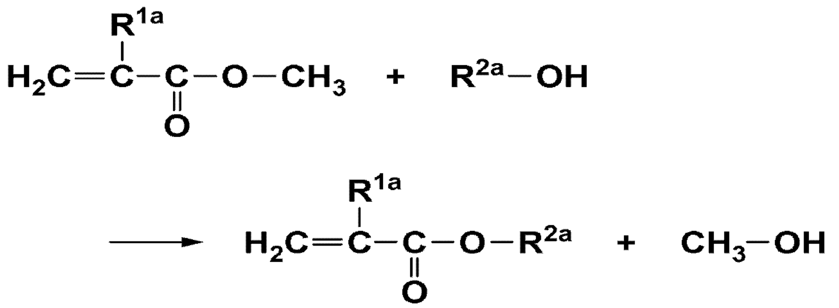

· · · (I I)

$R^{1a}$ and $R^{2a}$ in Formula (II) are the same as $R^{1a}$ and $R^{2a}$ in Formula (1).

Examples of the monoalcohol having 2 to 20 carbon atoms include ethanol, n-butanol, isobutanol, t-butanol, 2-ethylhexanol, lauryl alcohol, stearyl alcohol, cyclohexanol, isobornyl alcohol, allyl alcohol, phenol, and benzyl alcohol.

The monoalcohol having 2 to 20 carbon atoms may be used alone or a combination of two or more kinds thereof may be used. The monoalcohol having 2 to 20 carbon atoms preferably includes a linear or branched monoalcohol having 2 to 20 carbon atoms, and more preferably includes n-butanol or isobutanol.

For example, when obtaining the ester compound-containing composition comprising the ester compound (5) as the ester compound (I), in a reaction vessel, the methyl (meth)acrylate is subjected to an ester exchange with the ether bond-containing alcohol having 2 to 8 carbon atoms as represented by Formula (III) in the presence of a catalyst and the component A8.

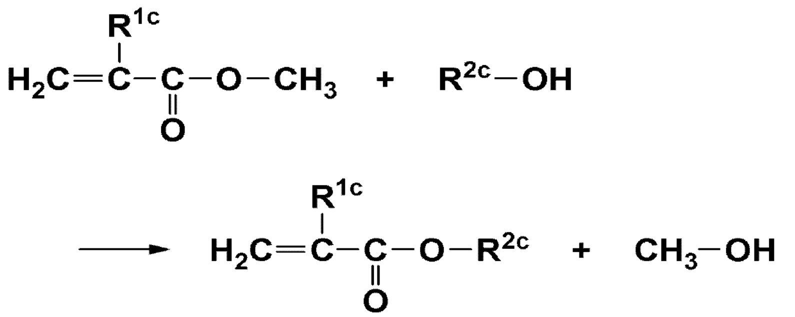

· · · (I I I)

$R^{1c}$ and $R^{2c}$ in Formula (III) are the same as $R^{1c}$ and $R^{2c}$ in Formula (5).

The number of ether bonds in the ether bond-containing alcohol having 2 to 8 carbon atoms is preferably 1, but is not limited thereto.

Examples of the ether bond-containing alcohol having 2 to 8 carbon atoms include 2-methoxyethanol, glycidyl alcohol, and tetrahydrofurfuryl alcohol. The ether bond-containing alcohol having 2 to 8 carbon atoms may be used alone or a combination of two or more kinds thereof may be used.

The ether bond-containing alcohol having 2 to 8 carbon atoms preferably includes one selected from 2-methoxyethanol, glycidyl alcohol, and tetrahydrofurfuryl alcohol.

For example, when obtaining the ester compound-containing composition comprising at least one or more selected from the group consisting of the ester compound (2), the ester compound (3), and the ester compound (4) as the ester compound (I), in a reaction vessel, the methyl (meth)acrylate is subjected to an ester exchange with one or more alcohols selected from the group consisting of the dialcohol having 2 to 8 carbon atoms and the trialcohol having 2 to 8 carbon atoms as represented by Formula (IV) and Formula (V) in the presence of a catalyst and the component A8.

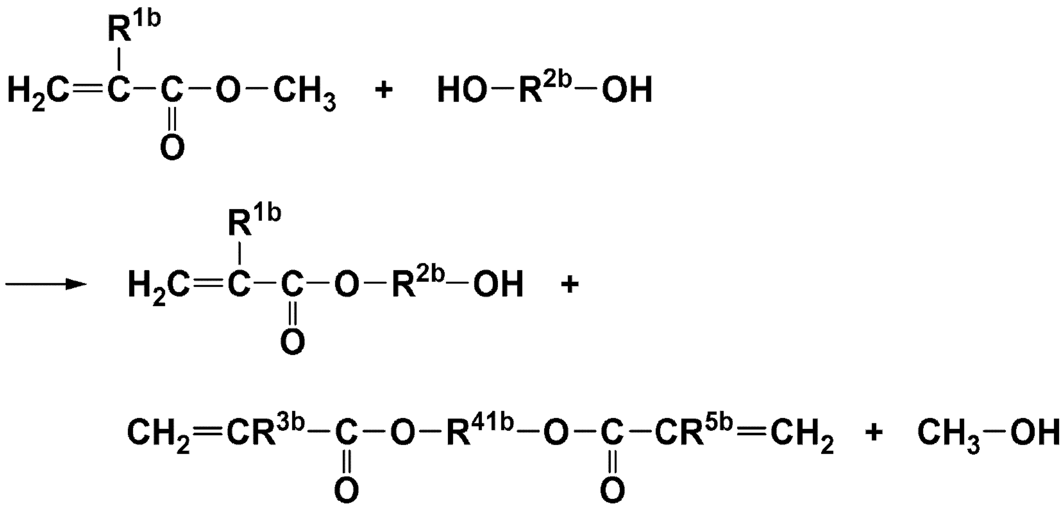

・・・(IV)

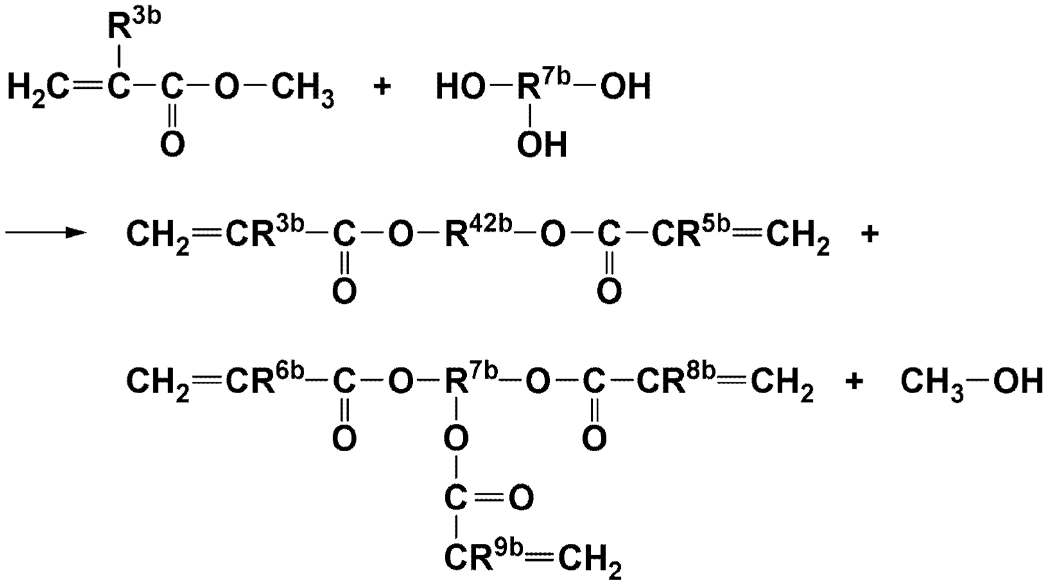

・・・(V)

$R^{1b}$, $R^{2b}$, $R^{3b}$ $R^{5b}$, $R^{6b}$, and $R^{7b}$ in Formula (IV) and Formula (V) are the same as $R^{1b}$, $R^{2b}$, $R^{3b}$ $R^{5b}$, $R^{6b}$, and $R^{7b}$ in Formula (2), Formula (3), and Formula (4). In addition, $R^{41b}$ is a linear or branched alkylene group having 2 to 8 carbon atoms, and $R^{42b}$ is a linear or branched hydroxyalkylene group having 2 to 8 carbon atoms.

Examples of the dialcohol having 2 to 8 carbon atoms and the trialcohol having 2 to 8 carbon atoms include ethylene glycol, 1,2-propanediol, 1,3-propanediol, 1,3-butanediol, 1,6-hexanediol, and trimethylolpropane. Among these, it is preferable that the dialcohol having 2 to 8 carbon atoms and the trialcohol having 2 to 8 carbon atoms include one or more selected from the group consisting of ethylene glycol, 1,2-propanediol, 1,3-propanediol, and trimethylolpropane.

The dialcohol having 2 to 8 carbon atoms and the trialcohol having 2 to 8 carbon atoms may be used alone or a combination of two or more kinds thereof may be used.

The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic

mixture with the methyl (meth)acrylate to the outside of the system.

Examples of the reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

The ratio of the amount of the methyl (meth)acrylate to be charged and the amount of the alcohol to be charged can be appropriately determined. From the viewpoint of improving productivity, the ratio of the methyl (meth)acrylate to 1 mol of alcohol is preferably 0.1 mol or more and 10 mol or less, and more preferably 0.3 mol or more and 4 mol or less.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction product during the ester exchange reaction and high catalytic activity, an alkoxide of titanium, dibutyltin oxide, or dioctyltin oxide is preferable. The catalyst may be used alone or a combination of two or more kinds thereof may be used.

The catalyst can be supplied alone to the reaction vessel. In addition, the catalyst can also be supplied to the reaction vessel in a state of being dissolved in the same alcohol as the alcohol as the raw material, or in a state of being dissolved in (meth)acrylic acid ester as the raw material. Examples thereof include a method of directly dissolving the catalyst in the total amount of the alcohol used in the reaction and supplying the catalyst to the reaction vessel, and a method of dissolving the catalyst in a part of the alcohol used in the reaction and supplying the catalyst to the reaction vessel.

The amount of the catalyst used is preferably 0.001 mol% or more and 1 mol% or less, and more preferably 0.01 mol% or more and 0.1 mol% or less with respect to 1 mol of the alcohol.

A solvent may be used for the ester exchange reaction. When a solvent is used, it is preferable to use a solvent which forms an azeotropic composition with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or a combination of two or more kinds thereof may be used.

The reaction temperature for the ester exchange reaction varies depending on the type of the alcohol or the solvent, but is preferably 60°C or higher and 150°C or lower.

The reaction pressure for the ester exchange reaction is not particularly limited, and the reaction may be carried out under any pressure of reduced pressure, normal pressure, or elevated pressure.

The form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, various methods such as distillation, crystallization, extraction, and column chromatography can be performed.

The ester exchange reaction may be performed by blending the component B into the reaction solution. When the component B is present in addition to the component A8, the dimerization of methyl (meth)acrylate or the generation of methyl pyruvate is further suppressed, and thus an effect of improving the yield of the ester compound (I) is more easily obtained.

The component B may be blended into a solution containing the ester compound (I) after the ester exchange reaction and the component A8. In this case, a solution (B solution) containing the ester compound (I) and the component B is prepared separately from the solution (A solution) containing the ester compound (I) and the component A8, and the A solution and the B solution are mixed with each other to obtain the ester compound-containing composition. In addition, the ester compound (I), the A solution, and the B solution may be mixed with each other to obtain the ester compound-containing composition.

The method for producing an ester compound-containing composition is not limited to the above-described method. For example, other (meth)acrylic acid esters other than the methyl (meth)acrylate may be used as the raw material instead of the methyl (meth)acrylate.

An esterification reaction between an alcohol and (meth)acrylic acid may be carried out to produce the ester compound (I).

(Evaluation method for storage stability and thermal stability of ester compound-containing composition)

The ester compound-containing composition has high quality stability during storage. Examples of an evaluation method of the quality stability of the ester compound-containing composition during storage include a method of actually storing the ester compound-containing composition for a long period of time and confirming the generation amount of the dimer of the ester compound (I), the generation amount of the oxidative product of the ester compound (I), and the contained amount of the ester compound (I). In addition, from the viewpoint of ease of work, a method of heating the ester compound-containing composition short period of time and confirming the generation amount of the dimer of the ester compound (I), the generation amount of the oxidative product of the ester compound (I), and the contained amount of the ester compound (I) may be used. When heating short period of time, the heating temperature is preferably 50°C to 100°C and a heating time is preferably 1 to 24 hours. When the ester compound-containing composition is stored at 25°C for 14 days or heated at 70°C for 7 to 20 hours, the quality stability of the ester compound-containing composition during storage is evaluated based on the generation amount of the dimer of the ester compound (I), the generation amount of the oxidative product of the ester compound (I), and the contained amount of the ester compound (I).

[Polymerizable composition]

A polymerizable composition according to the fourth aspect is a polymerizable composition for producing a (meth)acrylic polymer, and comprises the above-described ester compound-containing composition according to the fourth aspect.
As an example, an ester compound-containing composition stored for 1 day or more after production can be used as the polymerizable composition.
The storage time of the ester compound-containing composition can be 1 day or more, 3 days or more, 7 days or more, or 14 days or more. In addition, the storage time of the ester compound-containing composition may be 180 days or less, 150 days or less, 120 days or less, or 90 days or less. The "storage time" means an elapsed time from immediately after the production, which is not limited to a time during which the product is left to stand under a specific environment and also includes a time of transportation or the like.
The material of a storage container for the ester compound-containing composition is not particularly limited, and for example, a metal container such as stainless steel, a resin container, or a glass container can be used. A transparent container or an opaque container can be used.
The temperature at the time of storing the ester compound-containing composition is preferably -10°C or higher and more preferably 0°C or higher, and is preferably 60°C or lower and more preferably 50°C or lower. By setting the temperature to -10°C or higher, a load on a cooling device can be reduced, and by setting the temperature to 60°C or lower, it is easy to suppress the generation of the dimerized substance or the oxidative product of (meth)acrylic acid ester.
The oxygen concentration of a gas phase portion at the time of storing the ester compound-containing composition is preferably 5% by volume or more and more preferably 7% by volume or more, and is preferably 30% by volume or less and more preferably 22% by volume or less. By setting the oxygen concentration of the gas phase portion to 5% by volume or more, it is easy to prevent the ester compound from being unintentionally polymerized by a polymerization prevention effect of oxygen, and by setting the oxygen concentration of the gas phase portion to 30% by volume or less, it is easy to suppress oxidation of the (meth)acrylic acid ester by oxygen and the generation of various impurities.
As an example, the ester compound-containing composition after the storage may be used in the polymerizable composition without further blending a monomer, or a monomer (hereinafter, also referred to as "other monomer") copolymerizable with the ester compound (1) may be further blended with the ester compound-containing composition after the storage to obtain the polymerizable composition.
As another example, the ester compound-containing composition after the production may be stored in a state in which the other monomer is blended, and used in the polymerizable composition. In this case, it is preferable that a polymerization initiator is not blended during the storage.
The proportion of the ester compound (I) to the total mass of monomers in the polymerizable composition is preferably 10% by mass or more and more preferably 20% by mass or more, and preferably 90% by mass or less and more preferably 80% by mass or less. As a result, a (meth)acrylic polymer having high transparency can be obtained.
The ester compound-containing composition may comprise the other monomer copolymerizable with the ester compound (I), or may not contain the other monomer copolymerizable with the ester compound (I).
Examples of the other monomer copolymerizable with the ester compound (I) include other monomer copolymerizable with the ester compound (1);

other monomer copolymerizable with the ester compound (5); and
other monomer copolymerizable with at least one selected from the group consisting of the ester compound (2), the

ester compound (3), and the ester compound (4).

(Other monomer copolymerizable with ester compound (1))

Examples of the other monomer copolymerizable with the ester compound (1) include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.
Examples of the unsaturated carboxylic acid include acrylic acid, methacrylic acid, maleic acid, and itaconic acid.
Examples of the unsaturated carboxylic acid anhydride include maleic acid anhydride and itaconic acid anhydride.
Examples of the maleimide include N-phenylmaleimide and N-cyclohexylmaleimide.
Examples of the hydroxy group-containing vinyl monomer include 2-hydroxyethyl acrylate, 2-hydroxyethyl methacrylate, and 2-hydroxypropyl methacrylate.
Examples of the vinyl ester include vinyl acetate and vinyl benzoate.
Examples of the nitrogen-containing vinyl monomer include methacrylamide and acrylonitrile.
Examples of the epoxy group-containing monomer include glycidyl acrylate and glycidyl methacrylate.
Examples of the aromatic vinyl monomer include styrene and $\alpha$-methylstyrene.
Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.
Examples of the polyoxyalkylene glycol di(meth)acrylate include diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate.
Examples of the vinyl monomer having two or more ethylenically unsaturated bonds in the molecule include divinylbenzene.
As the other monomer, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.
The other monomer may be used alone or a combination of two or more kinds thereof may be used.

(Other monomer copolymerizable with at least one selected from group consisting of ester compound (2), ester compound (3), and ester compound (4))

Examples of the other monomer copolymerizable with at least one selected from the group consisting of the ester compound (2), the ester compound (3), and the ester compound (4) include the same monomers as those of the other monomer copolymerizable with the ester compound (1).

(Other monomer copolymerizable with ester compound (5))

Examples of the other monomer copolymerizable with the ester compound (5) include the same monomers as those of the other monomer copolymerizable with the ester compound (1).
When the component A8 is a monomer copolymerizable with the ester compound (I), the component A8 may be used as the monomer copolymerizable with the ester compound (I), or a monomer copolymerizable with the ester compound (I) may be used separately from the component A8.
When the ester compound-containing composition comprises other monomer copolymerizable with the ester compound (I), the contained amount of the other monomer copolymerizable with the ester compound (I) is preferably 0.01 parts by mass or more, more preferably 0.1 parts by mass or more, and still more preferably 1 part by mass or more with respect to 100 parts by mass of the ester compound (I). In addition, the contained amount thereof is preferably 50 parts by mass or less, more preferably 40 parts by mass or less, and still more preferably 30 parts by mass or less. As a result, a (meth)acrylic polymer having high transparency can be obtained.
The polymerizable composition preferably comprises a polymerization initiator.
Examples of the polymerization initiator include an azo compound, an organic peroxide, a persulfate compound, and a redox polymerization initiator.
The polymerization initiator may be used alone or a combination of two or more kinds thereof may be used.
Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.

Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.
Examples of the persulfate compound include potassium persulfate.
The additional amountof the polymerization initiator is not particularly limited, and for example, can be 0.005 to 5 parts by mass with respect to 100 parts by mass of the total mass of the monomers in the polymerizable composition.
The polymerizable composition may comprise, as necessary, other additives such as a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, and a fluorescent agent.
The other additives may be used alone or a combination of two or more kinds thereof may be used.

[Method for producing (meth)acrylic polymer]

A (meth)acrylic polymer can be produced by polymerizing the polymerizable composition.
The polymerization method is not particularly limited, and examples thereof include a bulk polymerization method, a solution polymerization method, an emulsion polymerization method, and a suspension polymerization method. From the viewpoint of environmental load due to the use of the solvent or the like and viewpoint of transparency of the obtained (meth)acrylic polymer, a bulk polymerization method is preferable.
The method of the bulk polymerization method is not particularly limited, and examples thereof include various casting polymerization methods such as a cell casting method and a continuous casting method.
The casting polymerization method is a method in which the (meth)acrylic polymer is obtained by casting and polymerizing the polymerizable composition in a mold made of two inorganic glass plates or metal plates (for example, SUS plates) arranged facing each other at a predetermined interval with the periphery sealed with a gasket such as a soft resin tube.
The mold for the casting polymerization is not particularly limited, and various molds can be used. Examples of a mold for cell casting include a mold in which two plate-shaped products such as an inorganic glass plate, a chromium-plated metal plate, and a stainless steel plate are arranged facing each other at a predetermined interval, and a gasket is disposed on edges of the plates to form a sealed space between the plate-shaped products and the gasket. Examples of the mold for continuous casting include a mold in which a sealed space is formed by opposing surfaces of a pair of endless belts running in the same direction at the same speed and gaskets running at the same speed as the endless belt on both sides of the endless belt.
A gap between cavities of the molds is appropriately adjusted to obtain a resin plate having a desired thickness, and is generally 1 to 30 mm.
The polymerization temperature is preferably 125°C to 210°C and more preferably 130°C to 180°C.
The polymerization time is preferably 0.5 to 24 hours.
T weight-average molecular weight (Mw) of the (meth)acrylic polymer is not particularly limited, and can be, for example, 100,000 to 1,000,000. As the Mw of the (meth)acrylic polymer is higher, solvent resistance and chemical resistance can be further improved.
The Mw of the (meth)acrylic polymer can be controlled by adjusting the polymerization temperature, the polymerization time, the addition amount of the polymerization initiator, and the like.
The (meth)acrylic polymer according to the fourth aspect has excellent heat resistance and excellent meltability. For example, among (meth)acrylic polymers, a granular (meth)acrylic polymer having a large amount of resin which can be stored per unit volume and having a small energy cost during storage and transportation needs to be dissolved or melted at the time of use. The (meth)acrylic polymer according to the fourth aspect is easily melted, has excellent kneading properties with other resins, and has excellent solubility in a monomer, a solvent, or the like.

5. Fifth aspect

Hereinafter, a fifth aspect of the embodiment will be described.

[Ester compound-containing composition]

An ester compound-containing composition according to the fifth aspect comprises an ester compound (I) described later and a compound (component A9) described later. The contained amount of the ester compound (I) is 95.00% to 99.99% by mass.

The ester compound-containing composition may further comprise a polymerization inhibitor (component B) described later, in addition to the ester compound (I) and the component A9.

As long as the effect of the present invention is not impaired, the ester compound-containing composition may comprise the component B as necessary, in addition to the ester compound (I) and the component A9, and may further comprise at least one of a compound other than the ester compound (I), the component A9, and the component B (hereinafter, also referred to as "component C") or water.

(Ester compound (I))

The ester compound (I) is a compound represented by Formula (I).

$$CH_2=CR^{150}-C(=O)-O-R^{200} \cdots \qquad (I)$$

In Formula (I), $R^{150}$ is a hydrogen atom or a methyl group, and $R^{200}$ is a monovalent hydrocarbon group having 2 to 20 carbon atoms, which may have a substituent, or a monovalent group having an ether bond and having 2 to 8 carbon atoms.

The ester compound (I) may be used alone or a combination of two or more kinds thereof may be used.

As the ester compound (I), one or more compounds selected from the group consisting of an ester compound (1) described later, an ester compound (2) described later, an ester compound (3) described later, an ester compound (4) described later, and an ester compound (5) described later are preferable. Hereinafter, the ester compounds will be described.

The ester compound (1) is a (meth)acrylic acid ester represented by Formula (1).

$$CH_2=CR^{1a}-C(=O)-O-R^{2a} \cdots \qquad (1)$$

In Formula (1), $R^{1a}$ is a hydrogen atom or a methyl group. $R^{2a}$ is a hydrocarbon group having 2 to 20 carbon atoms.

The hydrocarbon group of $R^{2a}$ may be a saturated hydrocarbon group or an unsaturated hydrocarbon group.

The hydrocarbon group of $R^{2a}$ may be linear or branched, or may have a ring. When the hydrocarbon group of $R^{2a}$ has a ring, the ring may be an aliphatic ring or an aromatic ring.

The number of carbon atoms in the hydrocarbon group of $R^{2a}$ is 2 to 20, preferably 2 to 18 and more preferably 2 to 12.

Examples of the hydrocarbon group of $R^{2a}$ include an alkyl group having 2 to 20 carbon atoms, a cycloalkyl group having 3 to 20 carbon atoms, an alkenyl group having 2 to 20 carbon atoms, a cycloalkenyl group having 3 to 20 carbon atoms, an alkynyl group having 2 to 20 carbon atoms, an aryl group having 6 to 20 carbon atoms, and an aromatic alkyl group having 7 to 20 carbon atoms. The "aromatic alkyl group" means a group in which one or more hydrogen atoms of an alkyl group are substituted with an aryl group.

Examples of the alkyl group of $R^{2a}$ include an ethyl group, a propyl group, an isopropyl group, an n-butyl group, an isobutyl group, a t-butyl group, a 2-ethylhexyl group, a lauryl group, and a stearyl group.

Examples of the cycloalkyl group of $R^{2a}$ include a cyclopropyl group, a cyclohexyl group, and an isobornyl group.

Examples of the alkenyl group of $R^{2a}$ include a vinyl group and an allyl group.

Examples of the cycloalkenyl group of $R^{2a}$ include a cyclopentenyl group, a cyclopentadienyl group, and a cyclohexenyl group.

Examples of the alkynyl group of $R^{2a}$ include a propynyl group.

Examples of the aryl group of $R^{2a}$ include a phenyl group and a naphthyl group.

Examples of the aromatic alkyl group of $R^{2a}$ include a benzyl group.

From the viewpoint that the ester compound (1) is relatively easy to obtain and is relatively easy to handle in terms of physical properties, $R^{2a}$ is preferably an alkyl group having 2 to 20 carbon atoms, a cycloalkyl group having 3 to 20 carbon atoms, an alkenyl group having 2 to 20 carbon atoms, an aryl group having 6 to 20 carbon atoms, or an aromatic alkyl group having 7 to 20 carbon atoms; more preferably an ethyl group, an isopropyl group, an n-butyl group, an isobutyl group, a t-butyl group, a 2-ethylhexyl group, a lauryl group, a stearyl group, a cyclohexyl group, an isobornyl group, an allyl group, a phenyl group, or a benzyl group; particularly preferably an n-butyl group or an isobutyl group; and most preferably an n-butyl group.

Examples of the ester compound (1) include ethyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, and benzyl (meth)acrylate.

As the ester compound (1), from the viewpoint that the ester compound (1) is relatively easy to obtain and is relatively easy to handle in terms of physical properties, an alkyl (meth)acrylate in which $R^{2a}$ is a linear or branched alkyl group having 2 to 20 carbon atoms, a cycloalkyl (meth)acrylate in which $R^{2a}$ is a cycloalkyl group having 3 to 20 carbon atoms, an alkenyl (meth)acrylate in which $R^{2a}$ is an alkenyl group having 2 to 20 carbon atoms, an aryl (meth)acrylate in which $R^{2a}$ is an aryl group having 6 to 20 carbon atoms, or an aromatic alkyl (meth)acrylate in which $R^{2a}$ is an aromatic alkyl group having 7 to 20 carbon atoms is preferable; ethyl (meth)acrylate, isopropyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, or benzyl (meth)acrylate is more preferable; butyl (meth)acrylate or isobutyl (meth)acrylate is particularly preferable; and butyl (meth)acrylate is most preferable.

The ester compound (1) may be used alone or a combination of two or more kinds thereof may be used.

The ester compound (2) is a (meth)acrylic acid ester represented by Formula (2). The ester compound (3) is a (meth)acrylic acid ester represented by Formula (3). The ester compound (4) is a (meth)acrylic acid ester represented by Formula (4).

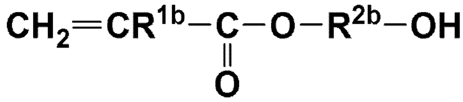

· · · (2)

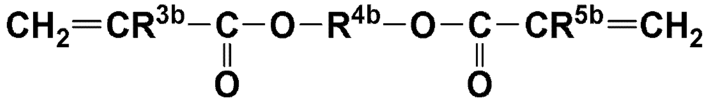

· · · (3)

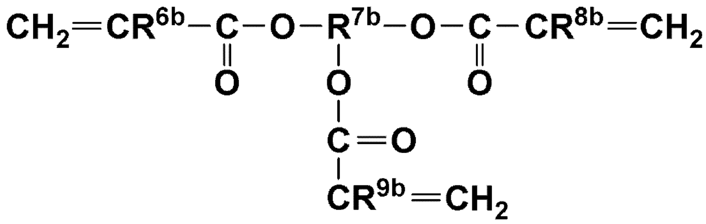

· · · (4)

In Formula (2), Formula (3), and Formula (4), $R^{1b}$, $R^{3b}$ $R^{5b}$, $R^{6b}$, $R^{8b}$, and $R^{9b}$ are each independently a hydrogen atom or a methyl group. $R^{2b}$ and $R^{4b}$ are each independently a linear or branched alkylene group or a hydroxyalkylene group, having 2 to 8 carbon atoms. $R^{7b}$ is a linear or branched trivalent hydrocarbon group having 2 to 8 carbon atoms.

The number of carbon atoms in the alkylene group or the hydroxyalkylene group of $R^{2b}$ and $R^{4b}$ is 2 to 8, preferably 2 to 6.

Examples of the alkylene group of $R^{2b}$ and $R^{4b}$ include an ethylene group, a propylene group, an isopropylene group, and a butylene group.

Examples of the hydroxyalkylene group of $R^{2b}$ and $R^{4b}$ include a hydroxyethylene group, a hydroxypropylene group, and a hydroxybutylene group.

The number of carbon atoms in the trivalent hydrocarbon group of $R^{7b}$ is 2 to 8, preferably 2 to 4.

Examples of the trivalent hydrocarbon group of $R^{7b}$ include $-(CH_2)-C(-CH_2-)(-CH_3)-CH_2-$.

Examples of the ester compound (2) include 2-hydroxyethyl (meth)acrylate, 2-hydroxypropyl (meth)acrylate, and 3-hydroxypropyl (meth)acrylate.

Examples of the ester compound (3) include ethylene glycol di(meth)acrylate, 1,3-propanediol di(meth)acrylate, 1,2-propanediol di(meth)acrylate, 1,3-butanediol di(meth)acrylate, and 1,6-hexanediol di(meth)acrylate.

Examples of the ester compound (4) include trimethylolpropane tri(meth)acrylate.

From the viewpoint of relatively easy availability, one or more selected from the group consisting of ethylene glycol di(meth)acrylate, 2-hydroxyethyl (meth)acrylate, 3-hydroxypropyl (meth)acrylate, and trimethylolpropane tri(meth) acrylate are preferable as the ester compound (2), the ester compound (3), and the ester compound (4).

The ester compound (2), the ester compound (3), and the ester compound (4) may be used alone or a combination of two or more kinds thereof may be used.

The ester compound (5) is a (meth)acrylic acid ester represented by Formula (5).

$CH_2=CR^{1c}-C(=O)-O-R^{2c}$ ⋯ (5)

In Formula (5), $R^{1c}$ is a hydrogen atom or a methyl group, and $R^{2c}$ is a monovalent group having an ether bond and having 2 to 8 carbon atoms.
The number of etheric oxygens included in the monovalent group having 2 to 8 carbon atoms of $R^{2c}$ is preferably 1, but it is not limited thereto and may be 2 or more.
The monovalent group having 2 to 8 carbon atoms of $R^{2c}$ may be linear or branched, or may have a ring. When $R^{2c}$ has a ring, the ring may or may not include the etheric oxygen.
The number of carbon atoms in the monovalent group having an ether bond of $R^{2c}$ is 2 to 8, preferably 2 to 7.
Examples of $R^{2c}$ include a 2-methoxyethyl group, a 2-(2-methoxyethoxy)ethyl group, a 2-[2-(2-methoxyethoxy)ethoxy]ethyl group, a glycidyl group, and a tetrahydrofurfuryl group.
As $R^{2c}$, from the viewpoint of relatively easy availability and relatively easy handling in terms of physical properties, a 2-methoxyethyl group, a glycidyl group, or a tetrahydrofurfuryl group is preferable.
Examples of the ester compound (5) include 2-methoxyethyl (meth)acrylate, glycidyl (meth)acrylate, 2-(2-methoxyethoxy)ethyl (meth)acrylate, 2-[2-(2-methoxyethoxy)ethoxy]ethyl (meth)acrylate, and tetrahydrofurfuryl (meth)acrylate.
As the ester compound (5), from the viewpoint of relatively easy handling in terms of physical properties, 2-methoxyethyl (meth)acrylate, glycidyl (meth)acrylate, or tetrahydrofurfuryl (meth)acrylate is preferable.
The ester compound (5) may be used alone or a combination of two or more kinds thereof may be used.

(Component A9)

The component A9 is a compound represented by Formula (a9).
When the ester compound-containing composition comprises the component A9, a dimerization reaction of the ester compound (I) and generation of an oxidative product of the ester compound (I) are suppressed during storage, and thus an ester compound-containing composition having excellent storage stability is obtained. The reason for this is presumed to be as follows.
The dimerization reaction of the ester compound (I) proceeds by a cationic mechanism or an ene reaction in the presence of an acid catalyst. Since the component A9 has weak basicity, it is considered that the component A9 can trap an acidic substance present in a trace amount in the ester compound-containing composition to inhibit the function as the acid catalyst and suppress the dimerization reaction of the ester compound (I). In addition, the ester compound (I) undergoes hydrolysis under basic conditions, but the component A9 is weakly basic and does not have strong basicity which causes the hydrolysis of the ester compound (I). Therefore, the component A9 is effective as a basic substance which traps the acidic substance present in a trace amount.
On the other hand, the oxidative product of the ester compound (I) is produced by oxidizing the ester compound (I) with a hydroxyl radical and an oxygen molecule. It is considered that the component A9 can trap an intermediate generated by a reaction between the hydroxyl radical and the ester compound (I) and return the intermediate to the ester compound (I), and can suppress the generation of the oxidative product of the ester compound (I).

$R^{91}-C\equiv N$ ⋯ (a 9)

In Formula (a9), $R^{91}$ is an alkyl group having 1 to 5 carbon atoms, an alkenyl group having 2 to 5 carbon atoms, or an aryl group having 1 to 12 carbon atoms, each of which may have a substituent.
The molecular weight of the component A9 is preferably 1,000 or less. When the molecular weight thereof is 1,000 or less, the number of cyano groups per unit mass in the component A9 can be increased, and thus the effect of the present invention can be obtained with a small mass. The molecular weight of the component A9 is more preferably 800 or less, still more preferably 600 or less, and particularly preferably 400 or less.
$R^{91}$ in Formula (a9) is an alkyl group having 1 to 5 carbon atoms, an alkenyl group having 2 to 5 carbon atoms, or an aryl group having 1 to 12 carbon atoms. In addition, $R^{91}$ may further have one or more substituents, and the substituent is a monovalent group having 0 to 18 carbon atoms, independent of the number of carbon atoms in $R^{91}$.
The number of carbon atoms in $R^{91}$ is the number of carbon atoms in the alkyl group, the alkenyl group, or the aryl group, which does not include carbon atoms of substituents of these groups. For example, when the component A9 is 4-(methylthio)benzonitrile, a substituent of $R^{91}$ is a 4-(methylthio)phenyl group, and the 4-(methylthio)phenyl group is regarded as an aryl group having 6 carbon atoms, which has a methylthio group as a substituent of an alkylthio group.
$R^{91}$ is an alkyl group having 1 to 5 carbon atoms, an alkenyl group having 2 to 5 carbon atoms, or an aryl group having 1 to 12 carbon atoms. An alkyl group having 1 to 3 carbon atoms, an alkenyl group having 2 or 3 carbon atoms, or an aryl group having 1 to 8 carbon atoms is preferable; a methyl group, an ethyl group, a 2-alkoxyethyl group, an isopropyl

group, a 2-alkoxyisopropyl group, a vinyl group, an isopropenyl group, or a phenyl group is more preferable; and a methyl group, an ethyl group, a 2-alkoxyethyl group, a 2-alkoxyisopropyl group, a vinyl group, an isopropenyl group, or a phenyl group is still more preferable.

When $R^{91}$ satisfies the above-described condition, the weak basicity of the component A9 and the reactivity with the acidic substance or the radical are maintained, and thus the effect of the present invention can be obtained. In addition, since it is a group having high stability, the component A9 can be prevented from being changed into other compounds during storage.

The alkyl group of $R^{91}$ may be linear or branched, or may have a ring.

The number of carbon atoms in the alkyl group is 1 to 5, preferably 1 to 3.

Examples of the linear or branched alkyl group (hereinafter, also collectively referred to as "chain alkyl group") include a methyl group, an ethyl group, an n-propyl group, an isopropyl group, an n-butyl group, an isobutyl group, an s-butyl group, a t-butyl group, an n-pentyl group, and an isopentyl group. Among these, a methyl group, an ethyl group, an n-propyl group, or an isopropyl group is preferable, and a methyl group, an ethyl group, or an isopropyl group is more preferable.

Examples of the alkyl group having a ring (hereinafter, also referred to as "cyclic alkyl group") include a cyclopropyl group, a cyclobutyl group, and a cyclopentyl group.

The alkenyl group of $R^{91}$ may be linear or branched, or may have a ring.

The number of carbon atoms in the alkenyl group is 2 to 5, preferably 2 or 3.

Examples of the linear or branched alkenyl group (hereinafter, also collectively referred to as "chain alkenyl group") include a vinyl group, a 1-propenyl group, an isopropenyl group, a 2-butenyl group, a 1,3-butadienyl group, and a 2-pentenyl group. Among these, a vinyl group, a 1-propenyl group, or an isopropenyl group is preferable, and a vinyl group or an isopropenyl group is more preferable.

Examples of the alkenyl group having a ring (hereinafter, also referred to as "cyclic alkenyl group") include a cyclopropenyl group, a cyclobutenyl group, and a cyclopentenyl group.

The number of carbon atoms in the aryl group of $R^{91}$ is 1 to 12, preferably 1 to 8. In another aspect, the number of carbon atoms in the aryl group of $R^{91}$ is preferably 6 to 12 and more preferably 6 to 8.

The aryl group includes a heteroaryl group containing oxygen, nitrogen, sulfur, or the like.

Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

When $R^{91}$ is an alkyl group, an alkenyl group, or an aryl group, each of which has a substituent, examples of the substituent include a monovalent group selected from an alkyl group, an alkenyl group, an aryl group, a hydroxy group, an alkoxy group, an amino group, a monovalent group including a carbonyl group, an alkylthio group, or an arylthio group. Among these, a hydroxy group, an alkoxy group, an amino group, a monovalent group including a carbonyl group, or an alkylthio group is preferable, and a hydroxy group, a methoxy group, an n-butoxy group, an isobutoxy group, an amino group, an acetyl group, or a methylthio group is more preferable.

The molecular weight of the substituent is preferably 200 or less, more preferably 100 or less, and still more preferably 50 or less.

The number of carbon atoms in the alkyl group as the substituent is 1 to 18, preferably 1 to 6 and more preferably 1 to 3. It is the same as the above-described alkyl group as long as $R^{91}$ is an alkyl group having 1 to 5 carbon atoms, excluding carbon atoms of the substituent, an alkenyl group having 2 to 5 carbon atoms, excluding carbon atoms of the substituent, or an aryl group having 1 to 12 carbon atoms, excluding carbon atoms of the substituent.

The number of carbon atoms in the alkenyl group as the substituent is 2 to 18, preferably 2 to 6 and more preferably 2 or 3. It is the same as the above-described alkenyl group as long as $R^{91}$ is an alkyl group having 1 to 5 carbon atoms, excluding carbon atoms of the substituent, an alkenyl group having 2 to 5 carbon atoms, excluding carbon atoms of the substituent, or an aryl group having 1 to 12 carbon atoms, excluding carbon atoms of the substituent.

The number of carbon atoms in the aryl group as the substituent is 1 to 18, preferably 6 to 11, more preferably 6 to 9, and particularly preferably 6 or 7. It is the same as the above-described aryl group as long as $R^{91}$ is an alkyl group having 1 to 5 carbon atoms, excluding carbon atoms of the substituent, an alkenyl group having 2 to 5 carbon atoms, excluding carbon atoms of the substituent, or an aryl group having 1 to 12 carbon atoms, excluding carbon atoms of the substituent.

The number of carbon atoms in the alkoxy group as the substituent is 1 to 18, preferably 1 to 6 and more preferably 1 to 4.

Examples of the alkoxy group as the substituent include a methoxy group, an ethoxy group, an n-propoxy group,

an isopropoxy group, an n-butoxy group, an isobutoxy group, an s-butoxy group, a t-butoxy group, an n-pentoxy group, an isopentoxy group, and a phenoxy group.

The amino group as the substituent includes an amino group (-$NH_2$) having no substituent on a nitrogen atom and an amino group in which a part or all of hydrogen atoms bonded to the nitrogen atom are substituted with carbon atoms.

The number of carbon atoms in the amino group substituted with carbon atoms is 1 to 18, preferably 1 to 6 and more preferably 1 to 3.

Examples of the amino group as the substituent include a methylamino group, an ethylamino group, a propylamino group, a butylamino group, a dimethylamino group, a diethylamino group, an anilino group, a toluidino group, an anisidino group, and an N-methyl-N-phenylamino group.

Examples of the monovalent group including a carbonyl group as the substituent include a formyl group, an acyl group, a carboxy group, an amide group, an alkoxycarbonyl group, a thiocarboxy group, and a thioester group.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is 2 to 18, preferably 2 to 7 and more preferably 2 to 4.

Examples of the acyl group include an acetyl group, a propionyl group, a butylcarbonyl group, a vinylcarbonyl group, and a benzoyl group.

The amide group includes an amide group (-$CONH_2$) having no substituent on a nitrogen atom and an amide group in which a part or all of hydrogen atoms bonded to the nitrogen atom are substituted with carbon atoms.

As the number of carbon atoms in the amide group, the total number of carbon atoms derived from the carbonyl group and carbon atoms substituted on the nitrogen atom is 1 to 18, preferably 1 to 7 and more preferably 1 to 4.

Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group.

The total number of carbon atoms derived from the carbonyl group and carbon atoms derived from the alkoxy group is 2 to 18, preferably 2 to 7 and more preferably 2 to 4.

Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group.

The total number of carbon atoms derived from the carbonyl group and carbon atoms derived from the alkylthio group or the arylthio group is 2 to 18, preferably 2 to 7 and more preferably 2 to 4.

Examples of the thioester group include a methylthiocarbonyl group, an ethylthiocarbonyl group, a butylthiocarbonyl group, and a phenylthiocarbonyl group.

As an example, the monovalent group including a carbonyl group may be a substituent in which one or a plurality of hydrogen atoms of an alkyl group are substituted with carbonyl groups. Examples of such a substituent include a 2-acetoxyethyl group, a 2-acetoethyl group, and a 2-(acetoacetoxy)ethyl group.

The number of carbon atoms in the alkylthio group as the substituent is 1 to 18, preferably 1 to 6 and more preferably 1 to 3.

Examples of the alkylthio group as the substituent include a methylthio group, an ethylthio group, a propylthio group, and an isopropylthio group.

The number of carbon atoms in the arylthio group as the substituent is 1 to 18, preferably 6 to 11, more preferably 6 to 9, and still more preferably 6 or 7.

Examples of the arylthio group as the substituent include a phenylthio group and a tolylthio group.

Among the compounds satisfying the above-described conditions, from the viewpoint of further improving the storage stability of the ester compound-containing composition, as the component A9, methacrylonitrile, acetonitrile, propionitrile, acrylonitrile, benzonitrile, cyclohexanecarbonitrile, 3-hydroxypropionitrile, 3-methoxypropionitrile, 3-butoxypropionitrile, 3-isobutoxypropionitrile, 3-hydroxyisobutyronitrile, 3-methoxyisobutyronitrile, 3-butoxyisobutyronitrile, 3-isobutoxyisobutyronitrile, 2-hydroxypropionitrile, 2-aminopropionitrile, 4-cyanophenol, 4-aminobenzonitrile, 4'-cyanoacetophenone, or 4-(methylthio)benzonitrile is preferable; methacrylonitrile, acetonitrile, propionitrile, acrylonitrile, benzonitrile, 3-hydroxypropionitrile, 3-methoxypropionitrile, 3-butoxypropionitrile, 3-isobutoxypropionitrile, 3-hydroxyisobutyronitrile, 3-methoxyisobutyronitrile, 3-butoxyisobutyronitrile, 3-isobutoxyisobutyronitrile, 4-aminobenzonitrile, 4'-cyanoacetophenone, or 4-(methylthio)benzonitrile is more preferable; and methacrylonitrile, acetonitrile, propionitrile, 3-methoxyisobutyronitrile, 3-butoxyisobutyronitrile, 3-isobutoxyisobutyronitrile, acrylonitrile, benzonitrile, 3-hydroxypropionitrile, or 4-(methylthio)benzonitrile is still more preferable.

The component A9 may be used alone or a combination of two or more kinds thereof may be used.

(Component B)

The component B is a polymerization inhibitor.

When the ester compound-containing composition comprises the component B, the progress of the polymerization reaction of the ester compound (I) by radical polymerization mechanism during storage is suppressed, and thus the generation of the dimer of the ester compound (I) is suppressed by a mechanism different from that of the component A9. In addition, during storage, oxygen molecules in the ester compound-containing composition absorb ultraviolet rays derived from sunlight, thereby generating a hydroxyl radical. However, the polymerization inhibitor can trap the hydroxyl radical. Therefore, as described above, when the ester compound-containing composition comprises the component A9 and the component B, the amount of the oxidative product can be reduced by two different mechanisms in which the hydroxyl radical is removed by the component B and in which the component A9 returns the intermediate to the ester compound (I) even when the hydroxyl radical reacts with the ester compound (I). Accordingly, the progress of dimerization of the ester compound (I) and the generation of the oxidative product can be suppressed more efficiently.

The polymerization inhibitor means a compound having a function of suppressing the polymerization reaction of the ester compound (I).

Examples of the component B include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

Examples of the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino)phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include $\alpha$-tocopherol and 2,3-dihydro-2,2-dimethyl-7-hydroxybenzofuran.

Examples of the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the nitrobenzene-based compound include nitrobenzene, o-dinitrobenzene, m-dinitrobenzene, p-dinitrobenzene, 2,4-dinitrobenzene, dinitrodurene, and 2,2-diphenyl-1-picrylhydrazine.

Examples of the N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethyl-piperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-t-butyl nitroxide, and di-t-amyl nitroxide.

Examples of the amine-based compound include N,N-diphenylamine, alkylated diphenylamine, 4,4'-dicamyldiphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl) phosphite, 4,4'-isopropylidenediphenol alkylphosphite, tris(2,4-di-t-butylphenyl)phosphite, tris(biphenyl)phosphite, distearyl pentaerythritol diphosphite, di(2,4-di-t-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-t-butylphenol) diphosphite, hexa(tridecyl)-1,1,3-tris(2-methyl-4-hydroxy-5-t-butylphenyl)butanetriphosphite, 3,5-dit-butyl-4-hydroxybenzyl phosphate diethyl ester, sodium-bis(4-t-butylphenyl)phosphate, sodium-2,2'-methylene-bis(4,6-di-t-butylphenyl)phosphate, and 1,3-bis(diphenoxyphosphoryloxy)benzene.

Examples of the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the iron-containing compound include iron (III) chloride.

Examples of the copper-containing compound include copper dimethyldithiocarbamate, copper diethylthiocarbamate, copper dibutylthiocarbamate, copper salicylate, copper acetate, copper thiocyanate, copper nitrate, copper chloride, copper carbonate, copper hydroxide, copper acrylate, and copper methacrylate.

Examples of the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

From the viewpoint of easily exhibiting the effect of further improving the storage stability of the ester compound-containing composition, as the component B, at least one polymerization inhibitor selected from the group consisting of a phenol-based compound, an N-oxyl-based compound, an amine-based compound, a phosphorus-containing compound, and a sulfur-containing compound is preferable; at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, and phenothiazine is more preferable; and at least one polymerization inhibitor selected from the group consisting of hydroquinone, 4-methoxyphenol, 2,4-dimethyl-6-t-butylphenol, and 2,6-dit-butyl-4-methylphenol is particularly preferable.

The component B may be used alone or a combination of two or more kinds thereof may be used.

When the ester compound-containing composition comprises a compound corresponding to both the component A9 and the component B, the compound is regarded as the component A9. When the ester compound-containing composition comprises the component A9 and the component B, this means that the ester compound-containing composition further comprises the component B different from the compound.

When the ester compound-containing composition comprises two or more kinds of compounds corresponding to both the component A9 and the component B, a compound having the highest molar concentration in the ester compound-containing composition is regarded as the component A9, and the other compounds are regarded as the component B.

(Component C)

The ester compound-containing composition may comprise other compounds (component C) as long as the contained amount of the ester compound (I) satisfies 90.00% to 99.99% by mass. The component C is a compound other than the ester compound (I), the component A9, and the component B.

Examples of the component C include additives such as a release agent, a lubricant, a plasticizer, an antioxidant, an antistatic agent, a light stabilizer, an ultraviolet absorber, a flame retardant, a flame retardant aid, a polymerization inhibitor, a filler, a pigment, a dye, a silane coupling agent, a leveling agent, an antifoaming agent, and a fluorescent agent.

The ester compound-containing composition may comprise unreacted raw materials in the production of the ester compound-containing composition, such as methyl (meth)acrylate, alcohol, and (meth)acrylic acid.

The ester compound-containing composition may comprise impurities generated during the production of the ester compound-containing composition, such as diacetyl, but from the viewpoint of suppressing coloration of the ester compound-containing composition, the concentration of the diacetyl is preferably 5 ppm by mass or less, more preferably 2 ppm by mass or less, still more preferably 1 ppm by mass or less, and particularly preferably 0.1 ppm by mass or less.

The ester compound-containing composition may comprise a (meth)acrylic acid ester other than the ester compound (I).

(Contained amount of ester compound (I))

The contained amount of the ester compound (I) is 95.00% to 99.99% by mass with respect to the total mass of the ester compound-containing composition. When the contained amount of the ester compound (I) is 90% by mass or more, the amount of impurities when the (meth)acrylic polymer is produced by polymerization of the ester compound-containing composition can be reduced, and thus it is possible to prevent physical properties of the polymer from being adversely affected. **In** addition, when the contained amount of the ester compound (I) is 99.99% by mass or less, a purification cost can be reduced. The contained amount of the ester compound (I) is preferably 96.00% by mass or more, more preferably 97.00% by mass or more, still more preferably 98.00% by mass or more, particularly preferably 99.00% by mass or more, and most preferably 99.50% by mass or more.

When the ester compound (I) includes the ester compound (1) and does not include the ester compound (2), the ester compound (3), the ester compound (4), and the ester compound (5), the contained amount of the ester compound (I) is the concentration of the ester compound (1).

When the ester compound (I) includes the ester compound (5) and does not include the ester compound (1), the ester compound (2), the ester compound (3), and the ester compound (4), the contained amount of the ester compound (I) is the concentration of the ester compound (5).

When the ester compound (I) includes at least one selected from the group consisting of the ester compound (2), the ester compound (3), and the ester compound (4), and does not include the ester compound (1) and the ester compound (5), the contained amount of the ester compound (I) is the concentration of at least one selected from the group consisting of the ester compound (2), the ester compound (3), and the ester compound (4).

The contained amount of the component A9 is not particularly limited, but is preferably 1 to 10,000 ppm by mass with respect to the total mass of the ester compound-containing composition. When the contained amount of the component A9 is 1 ppm by mass or more, an effect of suppressing the generation of the dimer of the ester compound (I) and the oxidative product of the ester compound (I) can be sufficiently obtained. In addition, when the contained amount of the component A9 is 10,000 ppm by mass or less, the amount of impurities when the (meth)acrylic polymer is produced by polymerization of the ester compound-containing composition according to the fifth aspect can be reduced, and thus it is possible to prevent physical properties of the polymer from being adversely affected. The contained amount of the component A9 is more preferably 3 ppm by mass or more, still more preferably 5 ppm by mass or more, and particularly preferably 10 ppm by mass or more. The contained amount of the component A9 is more preferably 7,500 ppm by mass or less, still more preferably 5,000 ppm by mass or less, even more preferably 2,500 ppm by mass or less, even still more preferably 1,500 ppm by mass or less, particularly preferably 1,000 ppm by mass or less, and most preferably 500 ppm by mass or less.

The contained amount of the component B is not particularly limited, but is preferably 1 to 1,000 ppm by mass with respect to the total mass of the ester compound-containing composition. When the contained amount of the component B is 1 ppm by mass or more, an effect of suppressing the generation of the dimer of the ester compound (I) and the oxidative product of the ester compound (I) can be sufficiently obtained. In addition, when the contained amount of the component B is 1,000 ppm by mass or less, the amount of impurities when the (meth)acrylic polymer is produced by polymerization of the ester compound-containing composition according to the fifth aspect can be reduced, and thus it is possible to prevent physical properties of the polymer from being adversely affected. The contained amount of the component B is more preferably 3 ppm by mass or more, still more preferably 5 ppm by mass or more, and particularly preferably 10 ppm by mass or more. The contained amount of the component B is more preferably 750 ppm by mass or less, still more preferably 500 ppm by mass or less, even more preferably 250 ppm by mass or less, particularly preferably 100 ppm by mass or less, and most preferably 50 ppm by mass or less.

The moisture content of the ester compound-containing composition is preferably 5,000 ppm by mass or less, more preferably 4,000 ppm by mass or less, still more preferably 3,000 ppm by mass or less, particularly preferably 2,000 ppm by mass or less, and most preferably 1,000 ppm by mass or less with respect to the total mass of the ester compound-containing composition. When the moisture content of the ester compound-containing composition is the above-described upper limit value or less, the physical properties of the (meth)acrylic polymer obtained by polymerizing the ester compound-containing composition can be maintained more satisfactorily.

The lower limit value of the moisture content of the ester compound-containing composition is 0 ppm by mass.

(Analysis of ester compound-containing composition)

The fact that the ester compound-containing composition comprises the component A9, the component B, the component C, and the water can be confirmed by, for example, GC-MS measurement. In a GC-MS chart of the ester compound-containing composition, when a peak is present at the same retention time as a sample of the component A9 and an m/z value detected in a mass spectrum of the peak matches exact mass of the component A9, it can be determined that the ester compound-containing composition comprises the component A9. When the sample of the component A9

cannot be obtained, when a pattern of the mass spectrum of the peak appearing in the GC-MS chart of the ester compound-containing composition and a pattern of a mass spectrum of the component A9 in mass spectrum database and match each other, it can be determined that the peak is the peak of the component A9. That is, it can be determined that the ester compound-containing composition comprises the component A9. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

The contained amount of the ester compound (I) can be calculated, for example, by performing GC-FID measurement of the ester compound-containing composition, quantifying by an area percentage method, and correcting the quantified moisture content using a Karl Fischer moisture meter.

The contained amount (concentration) of the component A9 can be quantified, for example, by performing GC measurement or GC-MS measurement of the ester compound-containing composition and using an internal standard method or an absolute calibration curve method. When a sample of the component A9 cannot be obtained and the component A9 cannot be quantified by the internal standard method or the absolute calibration curve method, the contained amount of the component A9 can be calculated using the following expression by performing GC-FID measurement on any organic compound having a known concentration under the same conditions as those of the ester compound-containing composition.

$$\text{Contained amount of component A9 (ppm by mass)} = \frac{N}{N_{A9}} \times \frac{S_{A9}}{S} \times M$$

Here, N is the number of carbon atoms in one molecule of the organic compound having a known concentration, $N_{A9}$ is the number of carbon atoms in one molecule of the component A9, $S_{A9}$ is a peak area of the component A9, S is a peak area of the organic compound having a known concentration, and M is a contained amount (ppm by mass) of the organic compound having a known concentration. When the volatility is low and the quantification cannot be performed by the GC measurement, the quantification can be performed using a chromatography method such as LC.

Contained amounts of the component B and the component C can also be calculated by the same method as that for the component A9 described above.

The fact that the ester compound-containing composition comprises water, and the concentration thereof can be confirmed by Karl Fischer method.

[Method for producing ester compound-containing composition]

A method for producing the ester compound-containing composition includes performing an ester exchange reaction between one or more alcohols selected from the group consisting of a monoalcohol having 2 to 20 carbon atoms, an ether bond-containing alcohol having 2 to 8 carbon atoms, a dialcohol having 2 to 8 carbon atoms, and a trialcohol having 2 to 8 carbon atoms, and methyl (meth)acrylate in the presence of the component A9.

Among (meth)acrylic acid esters, the methyl (meth)acrylate tends to particularly easily undergo dimerization or oxidation to produce methyl pyruvate. However, by performing the ester exchange reaction in the presence of the component A9, the dimerization of methyl (meth)acrylate or the generation of methyl pyruvate is suppressed, and as a result, the yield of the ester compound (I) is improved.

For example, when obtaining the ester compound-containing composition comprising the ester compound (1) as the ester compound (I), in a reaction vessel, the methyl (meth)acrylate is subjected to an ester exchange with the monoalcohol having 2 to 20 carbon atoms as represented by Formula (II) in the presence of a catalyst and the component A9.

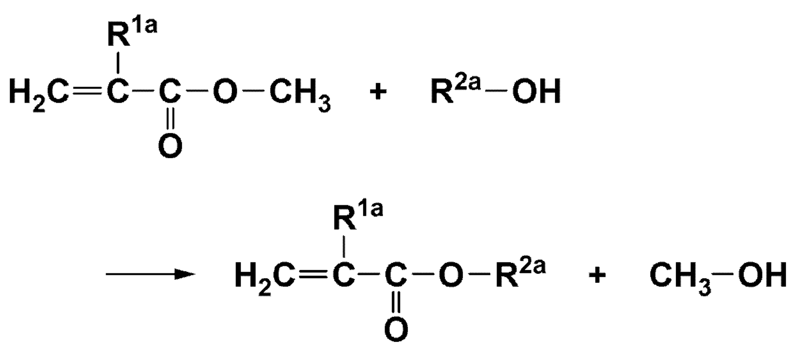

· · · (II)

$R^{1a}$ and $R^{2a}$ in Formula (II) are the same as $R^{1a}$ and $R^{2a}$ in Formula (1).

Examples of the monoalcohol having 2 to 20 carbon atoms include ethanol, n-butanol, isobutanol, t-butanol, 2-ethylhexanol, lauryl alcohol, stearyl alcohol, cyclohexanol, isobornyl alcohol, allyl alcohol, phenol, and benzyl alcohol.

The monoalcohol having 2 to 20 carbon atoms may be used alone or a combination of two or more kinds thereof may be used. The monoalcohol having 2 to 20 carbon atoms preferably includes a linear or branched monoalcohol having 2 to 20 carbon atoms, and more preferably includes n-butanol or isobutanol.

For example, when obtaining the ester compound-containing composition comprising the ester compound (5) as the ester compound (I), in a reaction vessel, the methyl (meth)acrylate is subjected to an ester exchange with the ether bond-containing alcohol having 2 to 8 carbon atoms as represented by Formula (III) in the presence of a catalyst and the component A9.

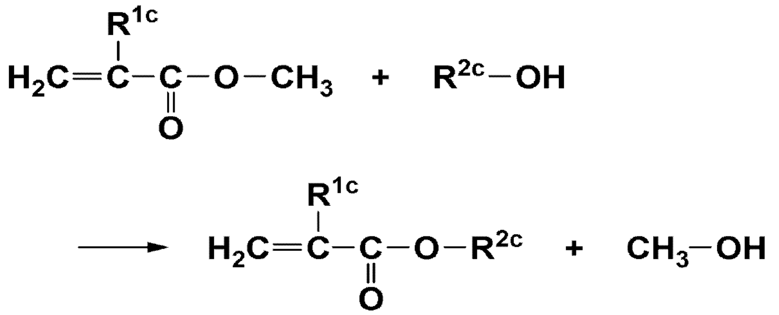

· · · (III)

$R^{1c}$ and $R^{2c}$ in Formula (III) are the same as $R^{1c}$ and $R^{2c}$ in Formula (5).

The number of ether bonds in the ether bond-containing alcohol having 2 to 8 carbon atoms is preferably 1, but is not limited thereto.

Examples of the ether bond-containing alcohol having 2 to 8 carbon atoms include 2-methoxyethanol, glycidyl alcohol, and tetrahydrofurfuryl alcohol. The ether bond-containing alcohol having 2 to 8 carbon atoms may be used alone or a combination of two or more kinds thereof may be used.

The ether bond-containing alcohol having 2 to 8 carbon atoms preferably includes one selected from 2-methoxyethanol, glycidyl alcohol, and tetrahydrofurfuryl alcohol.

For example, when obtaining the ester compound-containing composition comprising at least one or more selected from the group consisting of the ester compound (2), the ester compound (3), and the ester compound (4) as the ester compound (I), in a reaction vessel, the methyl (meth)acrylate is subjected to an ester exchange with one or more alcohols selected from the group consisting of the dialcohol having 2 to 8 carbon atoms and the trialcohol having 2 to 8 carbon atoms as represented by Formula (IV) and Formula (V) in the presence of a catalyst and the component A9.

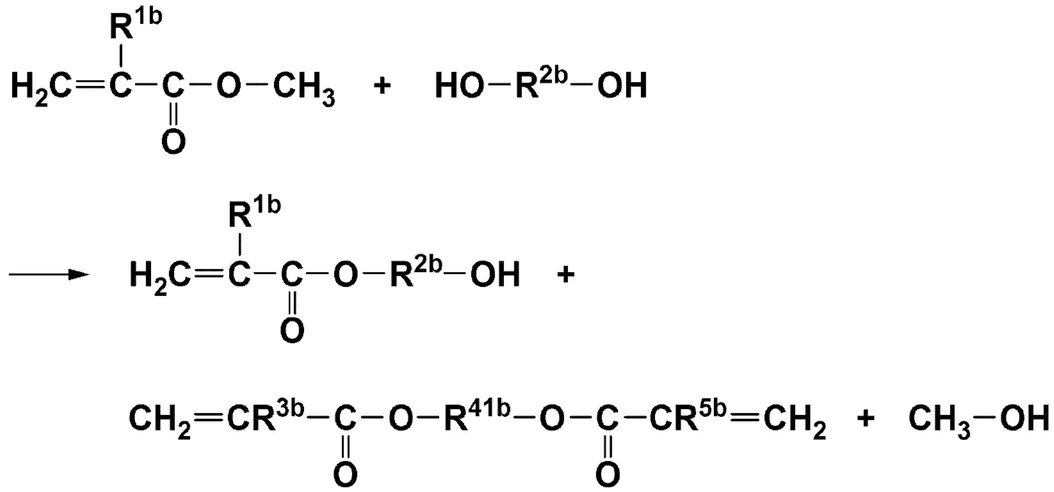

· · · (IV)

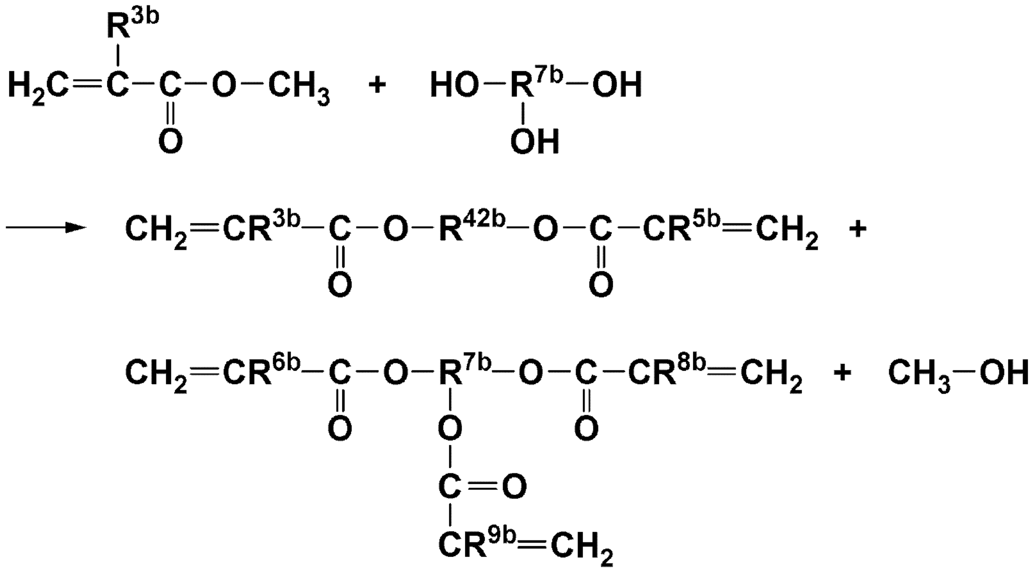

・・・(V)

$R^{1b}$, $R^{2b}$, $R^{3b}$, $R^{5b}$, $R^{6b}$, and $R^{7b}$ in Formula (IV) and Formula (V) are the same as $R^{1b}$, $R^{2b}$, $R^{3b}$, $R^{5b}$, $R^{6b}$, and $R^{7b}$ in Formula (2), Formula (3), and Formula (4). In addition, $R^{41b}$ is a linear or branched alkylene group having 2 to 8 carbon atoms, and $R^{42b}$ is a linear or branched hydroxyalkylene group having 2 to 8 carbon atoms.

Examples of the dialcohol having 2 to 8 carbon atoms and the trialcohol having 2 to 8 carbon atoms include ethylene glycol, 1,2-propanediol, 1,3-propanediol, 1,3-butanediol, 1,6-hexanediol, and trimethylolpropane. Among these, it is preferable that the dialcohol having 2 to 8 carbon atoms and the trialcohol having 2 to 8 carbon atoms include one or more selected from the group consisting of ethylene glycol, 1,2-propanediol, 1,3-propanediol, and trimethylolpropane.

The dialcohol having 2 to 8 carbon atoms and the trialcohol having 2 to 8 carbon atoms may be used alone or a combination of two or more kinds thereof may be used.

The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic mixture with the methyl (meth)acrylate to the outside of the system.

Examples of the reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

The ratio of the amount of the methyl (meth)acrylate to be charged and the amount of the alcohol to be charged can be appropriately determined. From the viewpoint of improving productivity, the ratio of the methyl (meth)acrylate to 1 mol of alcohol is preferably 0.1 mol or more and 10 mol or less, and more preferably 0.3 mol or more and 4 mol or less.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction product during the ester exchange reaction and high catalytic activity, an alkoxide of titanium, dibutyltin oxide, or dioctyltin oxide is preferable. The catalyst may be used alone or a combination of two or more kinds thereof may be used.

The catalyst can be supplied alone to the reaction vessel. In addition, the catalyst can also be supplied to the reaction vessel in a state of being dissolved in the same alcohol as the alcohol as the raw material, or in a state of being dissolved in (meth)acrylic acid ester as the raw material. Examples thereof include a method of directly dissolving the catalyst in the total amount of the alcohol used in the reaction and supplying the catalyst to the reaction vessel, and a method of dissolving the catalyst in a part of the alcohol used in the reaction and supplying the catalyst to the reaction vessel.

The amount of the catalyst used is preferably 0.001 mol% or more and 1 mol% or less, and more preferably 0.01 mol% or more and 0.1 mol% or less with respect to 1 mol of the alcohol.

A solvent may be used for the ester exchange reaction. When a solvent is used, it is preferable to use a solvent which forms an azeotropic composition with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or a combination of two or more kinds thereof may be used.

The reaction temperature for the ester exchange reaction varies depending on the type of the alcohol or the solvent, but is preferably 60°C or higher and 150°C or lower.

The reaction pressure for the ester exchange reaction is not particularly limited, and the reaction may be carried out under any pressure of reduced pressure, normal pressure, or elevated pressure.

The form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, various methods such as distillation, crystallization, extraction, and column chromatography can be performed.

The ester exchange reaction may be performed by blending the component B into the reaction solution. When the component B is present in addition to the component A9, the dimerization of methyl (meth)acrylate or the generation of methyl pyruvate is further suppressed, and thus an effect of improving the yield of the ester compound (I) is more easily obtained.

The component B may be blended into a solution containing the ester compound (I) after the ester exchange reaction and the component A9. In this case, a solution (B solution) containing the ester compound (I) and the component B is prepared separately from the solution (A solution) containing the ester compound (I) and the component A9, and the A solution and the B solution are mixed with each other to obtain the ester compound-containing composition. In addition, the ester compound (I), the A solution, and the B solution may be mixed with each other to obtain the ester compound-containing composition.

The method for producing an ester compound-containing composition is not limited to the above-described method. For example, other (meth)acrylic acid esters other than the methyl (meth)acrylate may be used as the raw material instead of the methyl (meth)acrylate.

An esterification reaction between an alcohol and (meth)acrylic acid may be carried out to produce the ester compound (I).

(Evaluation method for storage stability and thermal stability of ester compound-containing composition)

The ester compound-containing composition has high quality stability during storage. Examples of an evaluation method of the quality stability of the ester compound-containing composition during storage include a method of actually storing the ester compound-containing composition for a long period of time and confirming the generation amount of the dimer of the ester compound (I), the generation amount of the oxidative product of the ester compound (I), and the contained amount of the ester compound (I). In addition, from the viewpoint of ease of work, a method of heating the ester compound-containing composition short period of time and confirming the generation amount of the dimer of the ester compound (I), the generation amount of the oxidative product of the ester compound (I), and the contained amount of the ester compound (I) may be used. When heating short period of time, the heating temperature is preferably 50°C to 100°C and a heating time is preferably 1 to 24 hours. When the ester compound-containing composition is stored at 25°C for 14 days or heated at 70°C for 7 to 20 hours, the quality stability of the ester compound-containing composition during storage is evaluated based on the generation amount of the dimer of the ester compound (I), the generation amount of the oxidative product of the ester compound (I), and the contained amount of the ester compound (I).

[Polymerizable composition]

A polymerizable composition according to the fifth aspect comprises the above-described ester compound-containing composition according to the fifth aspect. The polymerizable composition is a polymerizable composition for producing a (meth)acrylic polymer.

As an example, an ester compound-containing composition stored for 1 day or more after production can be used as the polymerizable composition.

The storage time of the ester compound-containing composition can be 1 day or more, 3 days or more, 7 days or more, or 14 days or more. In addition, the storage time of the ester compound-containing composition may be 180 days or less, 150 days or less, 120 days or less, or 90 days or less. The "storage time" means an elapsed time from immediately after the production, which is not limited to a time during which the product is left to stand under a specific environment and

also includes a time of transportation or the like.

The material of a storage container for the ester compound-containing composition is not particularly limited, and for example, a metal container such as stainless steel, a resin container, or a glass container can be used. A transparent container or an opaque container can be used.

The temperature at the time of storing the ester compound-containing composition is preferably -10°C or higher and more preferably 0°C or higher, and is preferably 60°C or lower and more preferably 50°C or lower. By setting the temperature to -10°C or higher, a load on a cooling device can be reduced, and by setting the temperature to 60°C or lower, it is easy to suppress the generation of the dimerized substance or the oxidative product of (meth)acrylic acid ester.

The oxygen concentration of a gas phase portion at the time of storing the ester compound-containing composition is preferably 5% by volume or more and more preferably 7% by volume or more, and is preferably 30% by volume or less and more preferably 22% by volume or less. By setting the oxygen concentration of the gas phase portion to 5% by volume or more, it is easy to prevent the ester compound from being unintentionally polymerized by a polymerization prevention effect of oxygen, and by setting the oxygen concentration of the gas phase portion to 30% by volume or less, it is easy to suppress oxidation of the (meth)acrylic acid ester by oxygen and the generation of various impurities.

As an example, the ester compound-containing composition after the storage may be used in the polymerizable composition without further blending a monomer, or other monomer copolymerizable with the ester compound (I) may be further blended with the ester compound-containing composition after the storage to obtain the polymerizable composition.

As another example, the ester compound-containing composition after the production may be stored in a state in which the other monomer copolymerizable with the ester compound (I) is blended, and used in the polymerizable composition. In this case, it is preferable that a polymerization initiator is not blended during the storage.

The proportion of the ester compound (I) to the total mass of monomers in the polymerizable composition is preferably 10% by mass or more and more preferably 20% by mass or more, and preferably 90% by mass or less and more preferably 80% by mass or less. As a result, a (meth)acrylic polymer having high transparency can be obtained.

The ester compound-containing composition may comprise the other monomer copolymerizable with the ester compound (I), or may not contain the other monomer copolymerizable with the ester compound (I).

Examples of the other monomer copolymerizable with the ester compound (I) include other monomer copolymerizable with the ester compound (1);

other monomer copolymerizable with the ester compound (5); and
other monomer copolymerizable with at least one selected from the group consisting of the ester compound (2), the ester compound (3), and the ester compound (4).

(Other monomer copolymerizable with ester compound (1))

Examples of the other monomer copolymerizable with the ester compound (1) include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.

Examples of the unsaturated carboxylic acid include acrylic acid, methacrylic acid, maleic acid, and itaconic acid.

Examples of the unsaturated carboxylic acid anhydride include maleic acid anhydride and itaconic acid anhydride.

Examples of the maleimide include N-phenylmaleimide and N-cyclohexylmaleimide.

Examples of the hydroxy group-containing vinyl monomer include 2-hydroxyethyl acrylate, 2-hydroxyethyl methacrylate, and 2-hydroxypropyl methacrylate.

Examples of the vinyl ester include vinyl acetate and vinyl benzoate.

Examples of the nitrogen-containing vinyl monomer include methacrylamide and acrylonitrile.

Examples of the epoxy group-containing monomer include glycidyl acrylate and glycidyl methacrylate.

Examples of the aromatic vinyl monomer include styrene and α-methylstyrene.

Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.

Examples of the polyoxyalkylene glycol di(meth)acrylate include diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate.

Examples of the vinyl monomer having two or more ethylenically unsaturated bonds in the molecule include divinylbenzene.

As the other monomer, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.
The other monomer may be used alone or a combination of two or more kinds thereof may be used.

(Other monomer copolymerizable with at least one selected from group consisting of ester compound (2), ester compound (3), and ester compound (4))

Examples of the other monomer copolymerizable with at least one selected from the group consisting of the ester compound (2), the ester compound (3), and the ester compound (4) include the same monomers as those of the other monomer copolymerizable with the ester compound (1).

(Other monomer copolymerizable with ester compound (5))

Examples of the other monomer copolymerizable with the ester compound (5) include the same monomers as those of the other monomer copolymerizable with the ester compound (1).
When the component A9 is a monomer copolymerizable with the ester compound (I), the component A9 may be used as the monomer copolymerizable with the ester compound (I), or a monomer copolymerizable with the ester compound (I) may be used separately from the component A9.
When the ester compound-containing composition comprises other monomer copolymerizable with the ester compound (I), the contained amount of the other monomer copolymerizable with the ester compound (I) is preferably 0.01 parts by mass or more, more preferably 0.1 parts by mass or more, and still more preferably 1 part by mass or more with respect to 100 parts by mass of the ester compound (I). In addition, the contained amount thereof is preferably 50 parts by mass or less, more preferably 40 parts by mass or less, and still more preferably 30 parts by mass or less. As a result, a (meth)acrylic polymer having high transparency can be obtained.
The polymerizable composition preferably comprises a polymerization initiator.
Examples of the polymerization initiator include an azo compound, an organic peroxide, a persulfate compound, and a redox polymerization initiator.
The polymerization initiator may be used alone or a combination of two or more kinds thereof may be used.
Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.
Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.
Examples of the persulfate compound include potassium persulfate.
The additional amountof the polymerization initiator is not particularly limited, and for example, can be 0.005 to 5 parts by mass with respect to 100 parts by mass of the total mass of the monomers in the polymerizable composition.
The polymerizable composition may comprise, as necessary, other additives such as a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, and a fluorescent agent.
The other additives may be used alone or a combination of two or more kinds thereof may be used.

[Method for producing (meth)acrylic polymer]

A (meth)acrylic polymer can be produced by polymerizing the polymerizable composition.
The polymerization method is not particularly limited, and examples thereof include a bulk polymerization method, a solution polymerization method, an emulsion polymerization method, and a suspension polymerization method. From the viewpoint of environmental load due to the use of the solvent or the like and viewpoint of transparency of the obtained (meth)acrylic polymer, a bulk polymerization method is preferable.
The method of the bulk polymerization method is not particularly limited, and examples thereof include various casting polymerization methods such as a cell casting method and a continuous casting method.

The casting polymerization method is a method in which the (meth)acrylic polymer is obtained by casting and polymerizing the polymerizable composition in a mold made of two inorganic glass plates or metal plates (for example, SUS plates) arranged facing each other at a predetermined interval with the periphery sealed with a gasket such as a soft resin tube.

The mold for the casting polymerization is not particularly limited, and various molds can be used. Examples of a mold for cell casting include a mold in which two plate-shaped products such as an inorganic glass plate, a chromium-plated metal plate, and a stainless steel plate are arranged facing each other at a predetermined interval, and a gasket is disposed on edges of the plates to form a sealed space between the plate-shaped products and the gasket. Examples of the mold for continuous casting include a mold in which a sealed space is formed by opposing surfaces of a pair of endless belts running in the same direction at the same speed and gaskets running at the same speed as the endless belt on both sides of the endless belt.

A gap between cavities of the molds is appropriately adjusted to obtain a resin plate having a desired thickness, and is generally 1 to 30 mm.

The polymerization temperature is preferably 125°C to 210°C and more preferably 130°C to 180°C.

The polymerization time is preferably 0.5 to 24 hours.

The weight-average molecular weight (Mw) of the (meth)acrylic polymer is not particularly limited, and can be, for example, 100,000 to 1,000,000. As the Mw of the (meth)acrylic polymer is higher, solvent resistance and chemical resistance can be further improved.

The Mw of the (meth)acrylic polymer can be controlled by adjusting the polymerization temperature, the polymerization time, the addition amount of the polymerization initiator, and the like.

The (meth)acrylic polymer according to the fifth aspect has excellent heat resistance and excellent meltability. For example, among (meth)acrylic polymers, a granular (meth)acrylic polymer having a large amount of resin which can be stored per unit volume and having a small energy cost during storage and transportation needs to be dissolved or melted at the time of use. The (meth)acrylic polymer according to the fifth aspect is easily melted, has excellent kneading properties with other resins, and has excellent solubility in a monomer, a solvent, or the like.

6. Sixth aspect

Hereinafter, a sixth aspect of the embodiment will be described.

[Ester compound-containing composition]

An ester compound-containing composition according to the sixth aspect comprises an ester compound (I) described later and a compound (component A10) described later. The contained amount of the ester compound (I) is 95.00% to 99.99% by mass.

The ester compound-containing composition may further comprise a polymerization inhibitor (component B) described later, in addition to the ester compound (I) and the component A10.

As long as the effect of the present invention is not impaired, the ester compound-containing composition may further comprise at least one of a compound other than the ester compound (I), the component A10, and the component B (hereinafter, also referred to as "component C") or water as necessary, in addition to the ester compound (I) and the component A10.

(Ester compound (I))

The ester compound (I) is a compound represented by Formula (I).

$$CH_2=CR^{150}\text{-}C(=O)\text{-}O\text{-}R^{200} \cdots \qquad (I)$$

In Formula (I), $R^{150}$ is a hydrogen atom or a methyl group, and
$R^{200}$ is a monovalent hydrocarbon group having 2 to 20 carbon atoms, which may have a substituent, or a monovalent group having an ether bond and having 2 to 8 carbon atoms.

The ester compound (I) may be used alone or a combination of two or more kinds thereof may be used.

As the ester compound (I), one or more compounds selected from the group consisting of an ester compound (1) described later, an ester compound (2) described later, an ester compound (3) described later, an ester compound (4) described later, and an ester compound (5) described later are preferable. Hereinafter, the ester compounds will be described.

The ester compound (1) is a (meth)acrylic acid ester represented by Formula (1).

$CH_2=CR^{1a}-C(=O)-O-R^{2a}$ ⋯ (1)

In Formula (1), $R^{1a}$ is a hydrogen atom or a methyl group. $R^{2a}$ is a hydrocarbon group having 2 to 20 carbon atoms.

The hydrocarbon group of $R^{2a}$ may be a saturated hydrocarbon group or an unsaturated hydrocarbon group.

The hydrocarbon group of $R^{2a}$ may be linear or branched, or may have a ring. When the hydrocarbon group of $R^{2a}$ has a ring, the ring may be an aliphatic ring or an aromatic ring.

The number of carbon atoms in the hydrocarbon group of $R^{2a}$ is 2 to 20, preferably 2 to 18 and more preferably 2 to 12.

Examples of the hydrocarbon group of $R^{2a}$ include an alkyl group having 2 to 20 carbon atoms, a cycloalkyl group having 3 to 20 carbon atoms, an alkenyl group having 2 to 20 carbon atoms, a cycloalkenyl group having 3 to 20 carbon atoms, an alkynyl group having 2 to 20 carbon atoms, an aryl group having 6 to 20 carbon atoms, and an aromatic alkyl group having 7 to 20 carbon atoms. The "aromatic alkyl group" means a group in which one or more hydrogen atoms of an alkyl group are substituted with an aryl group.

Examples of the alkyl group of $R^{2a}$ include an ethyl group, a propyl group, an isopropyl group, an n-butyl group, an isobutyl group, a t-butyl group, a 2-ethylhexyl group, a lauryl group, and a stearyl group.

Examples of the cycloalkyl group of $R^{2a}$ include a cyclopropyl group, a cyclohexyl group, and an isobornyl group.

Examples of the alkenyl group of $R^{2a}$ include a vinyl group and an allyl group.

Examples of the cycloalkenyl group of $R^{2a}$ include a cyclopentenyl group, a cyclopentadienyl group, and a cyclohexenyl group.

Examples of the alkynyl group of $R^{2a}$ include a propynyl group.

Examples of the aryl group of $R^{2a}$ include a phenyl group and a naphthyl group.

Examples of the aromatic alkyl group of $R^{2a}$ include a benzyl group.

From the viewpoint that the ester compound (1) is relatively easy to obtain and is relatively easy to handle in terms of physical properties, $R^{2a}$ is preferably an alkyl group having 2 to 20 carbon atoms, a cycloalkyl group having 3 to 20 carbon atoms, an alkenyl group having 2 to 20 carbon atoms, an aryl group having 6 to 20 carbon atoms, or an aromatic alkyl group having 7 to 20 carbon atoms; more preferably an ethyl group, an isopropyl group, an n-butyl group, an isobutyl group, a t-butyl group, a 2-ethylhexyl group, a lauryl group, a stearyl group, a cyclohexyl group, an isobornyl group, an allyl group, a phenyl group, or a benzyl group; particularly preferably an n-butyl group or an isobutyl group; and most preferably an n-butyl group.

Examples of the ester compound (1) include ethyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, and benzyl (meth)acrylate.

As the ester compound (1), from the viewpoint that the ester compound (1) is relatively easy to obtain and is relatively easy to handle in terms of physical properties, an alkyl (meth)acrylate in which $R^{2a}$ is a linear or branched alkyl group having 2 to 20 carbon atoms, a cycloalkyl (meth)acrylate in which $R^{2a}$ is a cycloalkyl group having 3 to 20 carbon atoms, an alkenyl (meth)acrylate in which $R^{2a}$ is an alkenyl group having 2 to 20 carbon atoms, an aryl (meth)acrylate in which $R^{2a}$ is an aryl group having 6 to 20 carbon atoms, or an aromatic alkyl (meth)acrylate in which $R^{2a}$ is an aromatic alkyl group having 7 to 20 carbon atoms is preferable; ethyl (meth)acrylate, isopropyl (meth)acrylate, butyl (meth)acrylate, isobutyl (meth)acrylate, t-butyl (meth)acrylate, 2-ethylhexyl (meth)acrylate, lauryl (meth)acrylate, stearyl (meth)acrylate, cyclohexyl (meth)acrylate, isobornyl (meth)acrylate, allyl (meth)acrylate, phenyl (meth)acrylate, or benzyl (meth)acrylate is more preferable; butyl (meth)acrylate or isobutyl (meth)acrylate is particularly preferable; and butyl (meth)acrylate is most preferable.

The ester compound (1) may be used alone or a combination of two or more kinds thereof may be used.

The ester compound (2) is a (meth)acrylic acid ester represented by Formula (2). The ester compound (3) is a (meth)acrylic acid ester represented by Formula (3). The ester compound (4) is a (meth)acrylic acid ester represented by Formula (4).

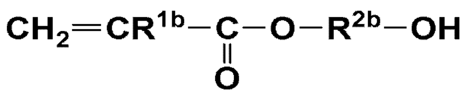

・・・ (2)

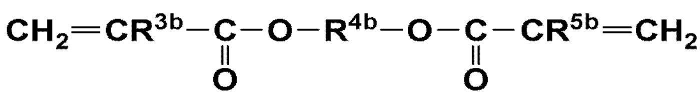

・・・ (3)

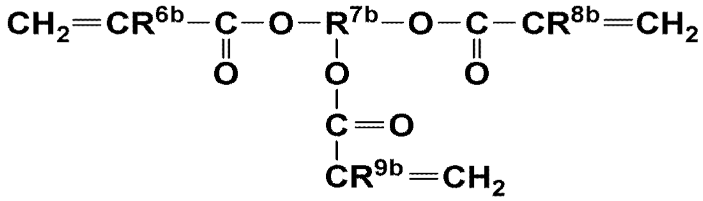

· · · (4)

In Formula (2), Formula (3), and Formula (4), $R^{1b}$, $R^{3b}$, $R^{5b}$, $R^{6b}$, $R^{8b}$, and $R^{9b}$ are each independently a hydrogen atom or a methyl group. $R^{2b}$ and $R^{4b}$ are each independently a linear or branched alkylene group or a hydroxyalkylene group, having 2 to 8 carbon atoms. $R^{7b}$ is a linear or branched trivalent hydrocarbon group having 2 to 8 carbon atoms.

The number of carbon atoms in the alkylene group or the hydroxyalkylene group of $R^{2b}$ and $R^{4b}$ is 2 to 8, preferably 2 to 6.

Examples of the alkylene group of $R^{2b}$ and $R^{4b}$ include an ethylene group, a propylene group, an isopropylene group, and a butylene group.

Examples of the hydroxyalkylene group of $R^{2b}$ and $R^{4b}$ include a hydroxyethylene group, a hydroxypropylene group, and a hydroxybutylene group.

The number of carbon atoms in the trivalent hydrocarbon group of $R^{7b}$ is 2 to 8, preferably 2 to 4.

Examples of the trivalent hydrocarbon group of $R^{7b}$ include $-(CH_2)-C(-CH_2-)(-CH_3)-CH_2-$.

Examples of the ester compound (2) include 2-hydroxyethyl (meth)acrylate, 2-hydroxypropyl (meth)acrylate, and 3-hydroxypropyl (meth)acrylate.

Examples of the ester compound (3) include ethylene glycol di(meth)acrylate, 1,3-propanediol di(meth)acrylate, 1,2-propanediol di(meth)acrylate, 1,3-butanediol di(meth)acrylate, and 1,6-hexanediol di(meth)acrylate.

Examples of the ester compound (4) include trimethylolpropane tri(meth)acrylate.

From the viewpoint of relatively easy availability, one or more selected from the group consisting of ethylene glycol di(meth)acrylate, 2-hydroxyethyl (meth)acrylate, 3-hydroxypropyl (meth)acrylate, and trimethylolpropane tri(meth) acrylate are preferable as the ester compound (2), the ester compound (3), and the ester compound (4).

The ester compound (2), the ester compound (3), and the ester compound (4) may be used alone or a combination of two or more kinds thereof may be used.

The ester compound (5) is a (meth)acrylic acid ester represented by Formula (5).

$$CH_2=CR^{1c}-C(=O)-O-R^{2c} \cdots \quad (5)$$

In Formula (5), $R^{1c}$ is a hydrogen atom or a methyl group, and $R^{2c}$ is a monovalent group having an ether bond and having 2 to 8 carbon atoms.

The number of etheric oxygens included in the monovalent group having 2 to 8 carbon atoms of $R^{2c}$ is preferably 1, but it is not limited thereto and may be 2 or more.

The monovalent group having 2 to 8 carbon atoms of $R^{2c}$ may be linear or branched, or may have a ring. When $R^{2c}$ has a ring, the ring may or may not include the etheric oxygen.

The number of carbon atoms in the monovalent group having an ether bond of $R^{2c}$ is 2 to 8, preferably 2 to 7.

Examples of $R^{2c}$ include a 2-methoxyethyl group, a 2-(2-methoxyethoxy)ethyl group, a 2-[2-(2-methoxyethoxy) ethoxy]ethyl group, a glycidyl group, and a tetrahydrofurfuryl group.

As $R^{2c}$, from the viewpoint of relatively easy availability and relatively easy handling in terms of physical properties, a 2-methoxyethyl group, a glycidyl group, or a tetrahydrofurfuryl group is preferable.

Examples of the ester compound (5) include 2-methoxyethyl (meth)acrylate, glycidyl (meth)acrylate, 2-(2-methoxyethoxy)ethyl (meth)acrylate, 2-[2-(2-methoxyethoxy)ethoxy]ethyl (meth)acrylate, and tetrahydrofurfuryl (meth)acrylate.

As the ester compound (5), from the viewpoint of relatively easy handling in terms of physical properties, 2-methoxyethyl (meth)acrylate, glycidyl (meth)acrylate, or tetrahydrofurfuryl (meth)acrylate is preferable.

The ester compound (5) may be used alone or a combination of two or more kinds thereof may be used.

(Component A10)

The component A10 is a compound represented by Formula (a10).

When the ester compound-containing composition comprises the component A10, a dimerization reaction of the ester compound (I) and generation of an oxidative product of the ester compound (I) are suppressed during storage, and thus an ester compound-containing composition having excellent storage stability is obtained. The reason for this is not clear, but is presumed to be as follows.

During the storage of the ester compound-containing composition, for example, a radical derived from ultraviolet rays, such as a hydroxyl radical generated by absorption of ultraviolet rays derived from sunlight by oxygen molecules, is generated. Such a radical can cause the generation of a dimer of the ester compound (I) or the generation of the oxidative product of the ester compound (I). Since the component A10 is a $\pi$-conjugated compound having a benzene ring, the component A10 absorbs ultraviolet light, and an absorption wavelength and an absorption intensity thereof change depending on the type of the substituent. In the component A10, since a group having an appropriate bulkiness and an appropriate electron-donating property is bonded to a benzene ring, ultraviolet light in a wide wavelength range can be absorbed. Therefore, when the ester compound-containing composition comprises the component A10, the ultraviolet light having a wide wavelength range is absorbed, and the generation of the hydroxyl radical is suppressed. Accordingly, it is presumed that the progress of the dimerization of the ester compound (I) by radical polymerization mechanism and the generation of the oxidative product such as pyruvic acid ester are suppressed.

In addition, it is presumed that the dimerization reaction of the ester compound (I) or the generation of the oxidative product proceeds by hydrolysis of the ester compound (I) with an acidic substance present in a trace amount in the ester compound-containing composition as a catalyst. Since the component A10 has weak basicity, it is considered that the component A10 can trap the acidic substance present in a trace amount in the ester compound-containing composition to inhibit the function as the hydrolysis catalyst and suppress the hydrolysis of the ester compound (I). In addition, the ester compound (I) undergoes hydrolysis under basic conditions, but the component A10 is weakly basic and does not have strong basicity which causes the hydrolysis of the ester compound (I). Therefore, it is considered that, in the ester compound-containing composition, the component A10 functions effectively as a basic substance which traps the acidic substance present in a trace amount, and thus the generation of the oxidative product of the ester compound (I) can be efficiently suppressed.

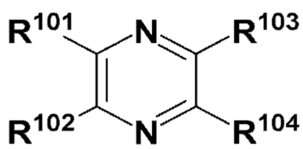

・・・（ａ１０）

In Formula (a10), $R^{101}$, $R^{102}$, $R^{103}$, $R^{104}$ may be each independently a hydrogen atom or a monovalent group selected from an alkyl group, an alkenyl group, an aryl group, a hydroxy group, an alkoxy group, an amino group, a carbonyl group, a monovalent group including a heteroatom and/or an unsaturated bond, an alkylthio group, or an arylthio group, or $R^{101}$, $R^{102}$, $R^{103}$, and $R^{104}$ may be each a divalent group in combination with any of the groups, which may have a substituent and may have a heteroatom and/or an unsaturated bond, where the groups may further have a substituent.

The molecular weight of the component A10 is preferably 1,000 or less. When the molecular weight thereof is 1,000 or less, the number of pyrazine rings per unit mass in the component A10 can be increased, and thus the effect of the present invention can be obtained with a small mass. The molecular weight of the component A10 is more preferably 800 or less, still more preferably 600 or less, and particularly preferably 400 or less.

In addition, the molecular weight of the component A10 is preferably 80 or more, and more preferably 120 or more.

The above-described upper limit and lower limit of the molecular weight of the component A10 can be combined arbitrarily. For example, the molecular weight of the component A10 is preferably 80 to 1,000, more preferably 80 to 800, still more preferably 120 to 600, and particularly preferably 120 to 400.

In Formula (a10), $R^{101}$, $R^{102}$, $R^{103}$, $R^{104}$ may be each independently a hydrogen atom or a monovalent group selected from an alkyl group, an alkenyl group, an aryl group, a hydroxy group, an alkoxy group, an amino group, a carbonyl group, a monovalent group including a heteroatom and/or an unsaturated bond, an alkylthio group, or an arylthio group, or $R^{101}$, $R^{102}$, $R^{103}$, and $R^{104}$ may be each a divalent group in combination with any of the groups, which may have a substituent and may have a heteroatom and/or an unsaturated bond. These groups may further have a substituent.

$R^{101}$, $R^{102}$, $R^{103}$, and $R^{104}$ may be the same or different from each other.

From the viewpoint of increasing absorbance of the ultraviolet light to suppress the generation of the hydroxyl radical and viewpoint of improving the basicity of the component A10, $R^{101}$, $R^{102}$, $R^{103}$, and $R^{104}$ are preferably a hydrogen atom, an alkyl group having 1 to 5 carbon atoms, an alkenyl group having 2 to 5 carbon atoms, an aryl group having 6 to 12 carbon atoms, an alkoxy group having 1 to 6 carbon atoms, an amino group, a monovalent group including a carbonyl group, having 1 to 6 carbon atoms, an alkylthio group having 1 to 5 carbon atoms, or an arylthio group having 6 to 10 carbon atoms; and more preferably a hydrogen atom, an alkyl group having 1 to 5 carbon atoms, or an alkoxy group having 1 to 6 carbon atoms.

The alkyl group of $R^{101}$, $R^{102}$, $R^{103}$, and $R^{104}$ may be linear or branched, or may have a ring.

The number of carbon atoms in the linear or branched alkyl group (hereinafter, also collectively referred to as "chain alkyl group") is preferably 1 to 20, more preferably 1 to 10, and still more preferably 1 to 5.

The number of carbon atoms in the alkyl group having a ring (hereinafter, also referred to as "cyclic alkyl group") is preferably 3 to 20, more preferably 4 to 10, and still more preferably 5 to 7.

Examples of the chain alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, and a decyl group. Among these, a methyl group, an ethyl group, an n-propyl group, an isopropyl group, or a t-butyl group is preferable, and a methyl group, an ethyl group, or an isopropyl group is more preferable.

Examples of the cyclic alkyl group include a cyclopentyl group, a cyclohexyl group, and a cyclooctyl group.

Examples of the alkyl group including a hydroxy group include a hydroxymethyl group, a 1-hydroxyethyl group, and a 2-hydroxyethyl group.

As the alkyl group of $R^{101}$, $R^{102}$, $R^{103}$, and $R^{104}$, the chain alkyl group is preferable.

The alkenyl group of $R^{101}$, $R^{102}$, $R^{103}$, and $R^{104}$ may be linear or branched, or may have a ring.

The number of carbon atoms in the linear or branched alkenyl group (hereinafter, also collectively referred to as "chain alkenyl group") is preferably 2 to 20, more preferably 2 to 10, and still more preferably 2 to 5.

The number of carbon atoms in the alkenyl group having a ring (hereinafter, also referred to as "cyclic alkenyl group") is preferably 3 to 20, more preferably 4 to 10, and still more preferably 5 to 7.

Examples of the chain alkenyl group include a vinyl group, a 1-propenyl group, an isopropenyl group, a 2-butenyl group, a 1,3-butadienyl group, a 2-pentenyl group, and a 2-hexenyl group.

Examples of the cyclic alkenyl group include a cyclopentenyl group and a cyclohexenyl group.

As the alkenyl group of $R^{101}$, $R^{102}$, $R^{103}$, and $R^{104}$, the chain alkenyl group is preferable.

The number of carbon atoms in the aryl group of $R^{101}$, $R^{1^{\circ}2}$, $R^{103}$, and $R^{104}$ is preferably 6 to 20 and more preferably 6 to 12.

The aryl group includes a heteroaryl group containing oxygen, nitrogen, sulfur, or the like.

Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, a thienyl group, an isoxazolyl group, an isothiazolyl group, an imidazolyl group, an oxazolyl group, a thiazolyl group, a thiadiazolyl group, a thienyl group, a thiophenyl group, a triazolyl group, a tetrazolyl group, a pyridyl group, a pyrazinyl group, a pyrimidinyl group, a pyridazinyl group, a pyrazolyl group, a pyrrolyl group, a pyranyl group, a furyl group, a furazanyl group, an imidazolidinyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The number of carbon atoms in the alkoxy group of $R^{101}$, $R^{102}$, $R^{103}$, and $R^{104}$ is preferably 1 to 20, more preferably 1 to 10, and still more preferably 1 to 6.

Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group of $R^{101}$, $R^{102}$, $R^{103}$, and $R^{104}$ includes an amino group ($-NH_2$) having no substituent on a nitrogen atom and an amino group in which a part or all of hydrogen atoms bonded to the nitrogen atom are substituted with carbon atoms.

The number of carbon atoms in the amino group substituted with carbon atoms is preferably 1 to 20, more preferably 1 to 10, and still more preferably 1 to 5.

Examples of the amino group include an unsubstituted amino group, a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group including a carbonyl group of $R^{101}$, $R^{102}$, $R^{103}$, and $R^{104}$ include a formyl group, an acyl group, a carboxy group, an amide group, an alkoxycarbonyl group, a thiocarboxy group, and a thioester group.

The acyl group is a substituent in which a carbonyl group is linked to an alkyl group, an alkenyl group, or an aryl group. The total number of carbon atoms derived from the carbonyl group of the acyl group and carbon atoms derived from the alkyl group, the alkenyl group, or the aryl group is preferably 2 to 20, more preferably 2 to 10, and still more preferably 2 to 6.

Examples of the acyl group include an acetyl group, an ethylcarbonyl group, a propionyl group, a butylcarbonyl group, a vinylcarbonyl group, a phenylcarbonyl group, and a benzoyl group.

The amide group includes an amide group ($-CONH_2$) having no substituent on a nitrogen atom and an amide group in which a part or all of hydrogen atoms bonded to the nitrogen atom are substituted with carbon atoms.

As the number of carbon atoms in the amide group, the total number of carbon atoms derived from the carbonyl group and carbon atoms substituted on the nitrogen atom is preferably 1 to 20, more preferably 1 to 10, and still more preferably 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxycarbonyl group is a substituent in which a carbonyl group is linked to an alkoxy group, and is also called an ester group.

The total number of carbon atoms derived from the carbonyl group and carbon atoms derived from the alkoxy group is preferably 2 to 20, more preferably 2 to 10, and still more preferably 2 to 6.

Examples of the alkoxycarbonyl group include a methoxycarbonyl group, an ethoxycarbonyl group, a butoxycarbonyl group, and a phenoxycarbonyl group.

The thioester group is a substituent in which a carbonyl group is linked to an alkylthio group or an arylthio group.

The total number of carbon atoms derived from the carbonyl group and carbon atoms derived from the alkylthio group or the arylthio group is preferably 2 to 20, more preferably 2 to 10, and still more preferably 2 to 6.

Examples of the thioester group include a methylthiocarbonyl group, an ethylthiocarbonyl group, a butylthiocarbonyl group, and a phenylthiocarbonyl group.

As an example, the monovalent group including a carbonyl group may be a substituent in which one or a plurality of hydrogen atoms of an alkyl group are substituted with carbonyl groups. Examples of such a substituent include a 2-acetoxyethyl group, a 2-acetoethyl group, and a 2-(acetoacetoxy)ethyl group.

The number of carbon atoms in the alkylthio group of $R^{101}$, $R^{102}$, $R^{103}$, and $R^{104}$ is preferably 1 to 20, more preferably 1 to 10, and still more preferably 1 to 5.

Examples of the alkylthio group include a methylthio group, an ethylthio group, a propylthio group, and an isopropylthio group.

The number of carbon atoms in the arylthio group of $R^{101}$, $R^{102}$, $R^{103}$, and $R^{104}$ is preferably 6 to 20 and more preferably 6 to 10.

Examples of the arylthio group include a phenylthio group and a tolylthio group.

When $R^{101}$, $R^{102}$, $R^{103}$, and $R^{104}$ are an alkyl group having a substituent, an alkenyl group having a substituent, an aryl group having a substituent, an alkoxy group having a substituent, an amino group having a substituent, a monovalent group including a carbonyl group, having a substituent, an alkylthio group having a substituent, or an arylthio group having a substituent, the substituent is not particularly limited as long as it does not significantly inhibit the effect of the present invention; and examples thereof include an alkyl group, an alkenyl group, an aryl group, a hydroxy group, an alkoxy group, an amino group, a monovalent group including a carbonyl group, an alkylthio group, and an arylthio group. Among these, a hydroxy group, an alkoxy group, an amino group, a monovalent group including a carbonyl group, or an alkylthio group is preferable, and a hydroxy group, a methoxy group, an amino group, an acetyl group, or a methylthio group is more preferable.

The molecular weight of the substituent is preferably 200 or less, more preferably 100 or less, and still more preferably 50 or less.

The alkyl group, alkenyl group, aryl group, alkoxy group, amino group, monovalent group including a carbonyl group, alkylthio group, and arylthio group as the substituent are the same as the alkyl group, alkenyl group, aryl group, alkoxy group, amino group, monovalent group including a carbonyl group, alkylthio group, and arylthio group, which are described above for $R^{101}$, $R^{102}$, $R^{103}$, and $R^{104}$.

When the alkyl group, the alkenyl group, the aryl group, the alkoxy group, the amino group, the monovalent group including a carbonyl group, the alkylthio group, or the arylthio group has a substituent, the carbon atoms of the substituent are included in the number of carbon atoms of $R^{101}$, $R^{102}$, $R^{103}$, and $R^{104}$

$R^{101}$, $R^{102}$, $R^{103}$, and $R^{104}$ may include a heteroatom or an unsaturated bond such as a double bond and a triple bond. The heteroatom refers to an atom other than hydrogen and carbon, and examples thereof include oxygen, nitrogen, sulfur, and phosphorus.

$R^{101}$ and $R^{102}$, or $R^{103}$ and $R^{104}$ may be linked to each other to form a ring. Examples of a compound forming the ring include 5,6,7,8-tetrahydroquinoxaline, quinoxaline, phenazine, and 1,2,3,4,6,7,8,9-octahydrophenazine.

Among the compounds satisfying the above-described conditions, from the viewpoint of easy availability and easy synthesis, as the component A10, 2,3,5,6-tetramethylpyrazine, pyrazine, 2,3,5-trimethylpyrazine, 2-methoxypyrazine, 2-isopropyl-3-methoxypyrazine, 2,5-dimethylpyrazine, 2,5-diisopropylpyrazine, 2-ethyl-3,5-dimethylpyrazine, 2,5-dimethyl-3-isobutylpyrazine, 2-isopropyl-3-methoxy-5-isobutylpyrazine, 2,5-dimethyl-3-(methylthio)pyrazine, 2-pyrazinemethanol, pyrazine methylamine, 2-pyrazine carboxylic acid methyl, vinylpyrazine, or 2-phenylpyrazine is preferable; and 2,3,5,6-tetramethylpyrazine, pyrazine, 2,3,5-trimethylpyrazine, 2-methoxypyrazine, 2-isopropyl-3-methoxypyrazine, or 2,5-dimethylpyrazine is more preferable.

The component A10 may be used alone or a combination of two or more kinds thereof may be used.

(Component B)

The component B is a polymerization inhibitor.

When the ester compound-containing composition comprises the component B, the progress of the polymerization reaction of the ester compound (I) by radical polymerization mechanism during storage can be suppressed, and a decrease in contained amount of the ester compound (I) can be suppressed. In addition, during storage, oxygen molecules in the ester compound-containing composition absorb ultraviolet rays derived from sunlight, thereby generating a hydroxyl radical. However, the polymerization inhibitor can trap the hydroxyl radical. Therefore, when the ester compound-containing composition comprises the component A10 and the component B, the amount of the hydroxyl radical can be reduced by two different mechanisms of suppressing the generation of the hydroxyl radical by the component A10 and removing the generated hydroxyl radical by the component B even when the hydroxyl radical is generated. Accordingly, the progress of dimerization of the ester compound (I) and the generation of the oxidative product can be suppressed more efficiently.

The polymerization inhibitor means a compound having a function of suppressing the polymerization reaction of the ester compound (I).

Examples of the component B include a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

Examples of the phenol-based compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylenebis(6-t-butyl-4-methylphenol), 2,2'-methylenebis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, benzoquinone, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, di-t-butyl-4-methoxyphenol, pyrogallol-1,2-dimethylether, and hydroquinone monobenzate.

Examples of the tocopherol include α-tocopherol and 2,3-dihydro-2,2-dimethyl-7-hydroxybenzofuran.

Examples of the quinone-based compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the nitrobenzene-based compound include nitrobenzene, o-dinitrobenzene, m-dinitrobenzene, p-dinitrobenzene, 2,4-dinitrobenzene, dinitrodurene, and 2,2-diphenyl-1-picrylhydrazine.

Examples of the N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethenoloxy-2,2,6,6-tetramethyl-piperidine-N-oxyl, 4-benzoyloxy-2,2,6,6-tetramethyl-piperidine-N-oxyl, 2,2,5,5-tetramethyl-piperidine-N-oxyl, 3-amino-2,2,5,5-tetramethyl-piperidine-N-oxyl, 4,4',4"-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-t-butyl nitroxide, and di-t-amyl nitroxide.

Examples of the amine-based compound include an aromatic amine and a hydroxylamine.

Examples of the aromatic amine include N,N-diphenylamine, alkylated diphenylamine, 4,4'-dicamyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (alkyl groups may be the same or different from each other, each independently have 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, 1,4-benzenediamine, N-(1,4-dimethylpentyl)-N'-phenyl-1,4-benzenediamine, aldol-α-naphthylamine, N-phenyl-β-naphthylamine, and 4-benzoyloxy-2,2,6,6-tetramethylpiperidine.

Examples of the hydroxylamine include N,N-diethylhydroxylamine, 6-ethoxy-2,2,4-trimethyl-1,2-dihydroquinoline, a 2,2,4-trimethyl-1,2-dihydroquinoline polymer, 4-hydroxy-2,2,6,6-tetramethylpiperidine, 1,4-dihydroxy-2,2,6,6-tetramethylpiperidine, and 1-hydroxy-4-benzyloxy-2,2,6,6-tetramethylpiperidine.

Examples of the phosphorus-containing compound include triphenylphosphine, triphenylphosphite, triethylphosphite, tris(isodecyl)phosphite, tris(tridecyl)phosphite, phenyldiisooctylphosphite, phenyldiisodecylphosphite, phenyldi(tridecyl)phosphite, diphenyliisooctylphosphite, diphenyldiisodecylphosphite, diphenyldi(tridecyl)phosphite, phosphonic acid [1,1-diphenyl-4,4'-diylbistetraxis-2,4-bis(1,1-dimethylethyl)phenyl] ester, triphenylphosphite, tris(nonylphenyl) phosphite, 4,4'-isopropylidenediphenol alkylphosphite, tris(2,4-di-t-butylphenyl)phosphite, tris(biphenyl)phosphite, distearyl pentaerythritol diphosphite, di(2,4-di-t-butylphenyl)pentaerythritol diphosphite, di(nonylphenyl)pentaerythritol diphosphite, phenyl bisphenol A pentaerythritol diphosphite, tetra(tridecyl)-4,4'-butylidenebis(3-methyl-6-t-butylphenol) diphosphite, hexa(tridecyl)-1,1,3-tris(2-methyl-4-hydroxy-5-t-butylphenyl)butanetriphosphite, 3,5-dit-butyl-4-hydroxybenzyl phosphate diethyl ester, sodium-bis(4-t-butylphenyl)phosphate, sodium-2,2'-methylene-bis(4,6-di-t-butylphenyl)phosphate, and 1,3-bis(diphenoxyphosphoryloxy)benzene.

Examples of the sulfur-containing compound include diphenyl sulfide, phenothiazine, 3-oxophenothiazine, 5-oxophenothiazine, a phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the iron-containing compound include iron (III) chloride.

Examples of the copper-containing compound include copper dimethyldithiocarbamate, copper diethylthiocarbamate, copper dibutylthiocarbamate, copper salicylate, copper acetate, copper thiocyanate, copper nitrate, copper chloride, copper carbonate, copper hydroxide, copper acrylate, and copper methacrylate.

Examples of the manganese-containing compound include manganese dialkyldithiocarbamate (alkyl group is any of a methyl group, an ethyl group, a propyl group, or a butyl group, and the alkyl groups may be the same or different from each other), manganese diphenyldithiocarbamate, manganese formate, manganese acetate, manganese octanoate, manganese naphthenate, manganese permanganate, and manganese salt of ethylenediaminetetraacetic acid.

From the viewpoint of easily exhibiting the effect of further improving the storage stability of the ester compound-containing composition, the component B is preferably at least one polymerization inhibitor selected from the group consisting of a phenol-based compound, an N-oxyl-based compound, an amine-based compound, a phosphorus-containing compound, and a sulfur-containing compound. Among these, in particular, from the viewpoint of making it difficult to inhibit polymerization when producing a (meth)acrylic polymer, the component B is particularly preferably at least one polymerization inhibitor selected from the group consisting of hydroquinone, 4-methoxyphenol, 2,4-dimethyl-6-t-butylphenol, and 2,6-di-t-butyl-4-methylphenol.

The component B may be used alone or a combination of two or more kinds thereof may be used.

When the ester compound-containing composition comprises a compound corresponding to both the component A10 and the component B, the compound is regarded as the component A10. When the ester compound-containing composition comprises the component A10 and the component B, this means that the ester compound-containing composition further comprises the component B different from the compound.

When the ester compound-containing composition comprises two or more kinds of compounds corresponding to both the component A10 and the component B, a compound having the highest molar concentration in the ester compound-containing composition is regarded as the component A10, and the other compounds are regarded as the component B.

(Component C)

The ester compound-containing composition may comprise other compounds (component C) as long as the contained amount of the ester compound (I) satisfies 95.00% to 99.99% by mass. The component C is a compound other than the ester compound (I), the component A10, and the component B.

Examples of the component C include additives such as a release agent, a lubricant, a plasticizer, an antioxidant, an antistatic agent, a light stabilizer, an ultraviolet absorber, a flame retardant, a flame retardant aid, a polymerization inhibitor, a filler, a pigment, a dye, a silane coupling agent, a leveling agent, an antifoaming agent, and a fluorescent agent.

The ester compound-containing composition may comprise unreacted raw materials in the production of the ester compound-containing composition, such as methyl (meth)acrylate, alcohol, and (meth)acrylic acid.

The ester compound-containing composition may comprise impurities generated during the production of the ester compound-containing composition, such as diacetyl, but from the viewpoint of suppressing coloration of the ester compound-containing composition, the concentration of the diacetyl is preferably 5 ppm by mass or less, more preferably 2 ppm by mass or less, still more preferably 1 ppm by mass or less, and particularly preferably 0.1 ppm by mass or less.

The ester compound-containing composition may comprise a (meth)acrylic acid ester other than the ester compound (1).

The contained amount of the ester compound (I) is 95.00% to 99.99% by mass with respect to the total mass of the

ester compound-containing composition. When the contained amount of the ester compound (I) is 90.00% by mass or more, the amount of impurities when the (meth)acrylic polymer is produced by polymerization of the ester compound-containing composition can be reduced, and thus it is possible to prevent physical properties of the polymer from being adversely affected. In addition, when the contained amount of the ester compound (I) is 99.99% by mass or less, a purification cost can be reduced. The contained amount of the ester compound (I) is preferably 96.00% by mass or more, more preferably 97.00% by mass or more, still more preferably 98.00% by mass or more, particularly preferably 99.00% by mass or more, and most preferably 99.50% by mass or more.

When the ester compound (I) includes the ester compound (1) and does not include the ester compound (5), the ester compound (2), the ester compound (3), and the ester compound (4), the contained amount of the ester compound (I) is the contained amount of the ester compound (1).

When the ester compound (I) includes the ester compound (5) and does not include the ester compound (1), the ester compound (2), the ester compound (3), and the ester compound (4), the contained amount of the ester compound (I) is the contained amount of the ester compound (5).

When the ester compound (I) includes at least one selected from the group consisting of the ester compound (2), the ester compound (3), and the ester compound (4), and does not include the ester compound (1) and the ester compound (5), the contained amount of the ester compound (I) is the contained amount of at least one selected from the group consisting of the ester compound (2), the ester compound (3), and the ester compound (4).

The contained amount of the component A10 is not particularly limited, but is preferably 1 to 10,000 ppm by mass with respect to the total mass of the ester compound-containing composition. When the contained amount of the component A10 is 1 ppm by mass or more, an effect of suppressing the generation of the dimer of the ester compound (I) and the oxidative product of the ester compound (I) can be sufficiently obtained. In addition, when the contained amount of the component A10 is 10,000 ppm by mass or less, the amount of impurities when the (meth)acrylic polymer is produced by polymerization of the ester compound-containing composition according to the sixth aspect can be reduced, and thus it is possible to prevent physical properties of the polymer from being adversely affected. The contained amount of the component A10 is more preferably 3 ppm by mass or more, still more preferably 5 ppm by mass or more, and particularly preferably 10 ppm by mass or more. The contained amount of the component A10 is more preferably 7,500 ppm by mass or less, still more preferably 5,000 ppm by mass or less, even more preferably 2,500 ppm by mass or less, even still more preferably 1,500 ppm by mass or less, particularly preferably 1,000 ppm by mass or less, and most preferably 500 ppm by mass or less.

The contained amount of the component B is not particularly limited, but is preferably 1 to 1,000 ppm by mass with respect to the total mass of the ester compound-containing composition. When the contained amount of the component B is 1 ppm by mass or more, an effect of suppressing the generation of the dimer of the ester compound (I) and the oxidative product of the ester compound (I) can be sufficiently obtained. In addition, when the contained amount of the component B is 1,000 ppm by mass or less, the amount of impurities when the (meth)acrylic polymer is produced by polymerization of the ester compound-containing composition according to the sixth aspect can be reduced, and thus it is possible to prevent physical properties of the polymer from being adversely affected. The contained amount of the component B is more preferably 3 ppm by mass or more, still more preferably 5 ppm by mass or more, and particularly preferably 10 ppm by mass or more. The contained amount of the component B is more preferably 750 ppm by mass or less, still more preferably 500 ppm by mass or less, even more preferably 250 ppm by mass or less, particularly preferably 100 ppm by mass or less, and most preferably 50 ppm by mass or less.

The moisture content of the ester compound-containing composition is preferably 5,000 ppm by mass or less, more preferably 4,000 ppm by mass or less, still more preferably 3,000 ppm by mass or less, particularly preferably 2,000 ppm by mass or less, and most preferably 1,000 ppm by mass or less with respect to the total mass of the ester compound-containing composition. When the moisture content of the ester compound-containing composition is the above-described upper limit value or less, the physical properties of the (meth)acrylic polymer obtained by polymerizing the ester compound-containing composition can be maintained more satisfactorily.

The lower limit value of the moisture content of the ester compound-containing composition is 0 ppm by mass.

(Analysis of ester compound-containing composition)

The fact that the ester compound-containing composition comprises the component A10, the component B, the component C, and the water can be confirmed by, for example, GC-MS measurement. In a GC-MS chart of the ester compound-containing composition, when a peak is present at the same retention time as a sample of the component A10 and an m/z value detected in a mass spectrum of the peak matches exact mass of the component A10, it can be determined that the ester compound-containing composition comprises the component A10. When the sample of the component A10 cannot be obtained, when a pattern of the mass spectrum of the peak appearing in the GC-MS chart of the ester compound-containing composition and a pattern of a mass spectrum of the component A10 in mass spectrum database and match each other, it can be determined that the peak is the peak of the component A10. That is, it can be determined

that the ester compound-containing composition comprises the component A10. Examples of the mass spectrum database include NIST 20, NIST 17, NIST 14, and NIST 14s. In addition, when volatility is low and the detection cannot be carried out by the GC-MS measurement, the detection can be carried out by LC-MS. It can be also confirmed that the composition comprises the component B, the component C, and the water by the same method.

The contained amount of the ester compound (I) can be calculated, for example, by performing GC-FID measurement of the ester compound-containing composition, quantifying by an area percentage method, and correcting the quantified moisture content using a Karl Fischer moisture meter.

The contained amount of the component A10 can be quantified, for example, by performing GC or GC-MS measurement of the ester compound-containing composition and using an internal standard method or an absolute calibration curve method. When a sample of the component A10 cannot be obtained and the component A10 cannot be quantified by the internal standard method or the absolute calibration curve method, the contained amount of the component A10 can be calculated using the following expression by performing GC-FID measurement on any organic compound having a known concentration under the same conditions as those of the ester compound-containing composition.

$$\text{Contained amount of component A10 (ppm by mass)} = \frac{N}{N_{A10}} \times \frac{S_{A10}}{S} \times M$$

Here, N is the number of carbon atoms in one molecule of the organic compound having a known concentration, $N_{A10}$ is the number of carbon atoms in one molecule of the component A10, $S_{A10}$ is a peak area of the component A10, S is a peak area of the organic compound having a known concentration, and M is a contained amount (ppm by mass) of the organic component having a known concentration. When the volatility is low and the quantification cannot be performed based on the GC area, the quantification can be performed using a chromatography method such as LC.

Contained amounts of the component B and the component C can also be calculated by the same method as that for the component A10 described above.

The fact that the ester compound-containing composition comprises water, and the concentration thereof can be confirmed by Karl Fischer method.

[Method for producing ester compound-containing composition]

A method for producing the ester compound-containing composition according to the sixth aspect includes performing an ester exchange reaction between one or more alcohols selected from the group consisting of a monoalcohol having 2 to 20 carbon atoms, an ether bond-containing alcohol having 2 to 8 carbon atoms, a dialcohol having 2 to 8 carbon atoms, and a trialcohol having 2 to 8 carbon atoms, and methyl (meth)acrylate in the presence of, for example, a pyrazine compound (component A10).

Among (meth)acrylic acid esters, the methyl (meth)acrylate tends to particularly easily undergo dimerization or oxidation to produce methyl pyruvate. However, by performing the ester exchange reaction in the presence of the component A10, the dimerization of methyl (meth)acrylate or the generation of methyl pyruvate is suppressed, and as a result, the yield of the ester compound (I) is improved.

For example, when obtaining the ester compound-containing composition comprising the ester compound (1) as the ester compound (I), in a reaction vessel, the methyl (meth)acrylate is subjected to an ester exchange with the monoalcohol having 2 to 20 carbon atoms as represented by Formula (II) in the presence of a catalyst and the component A10.

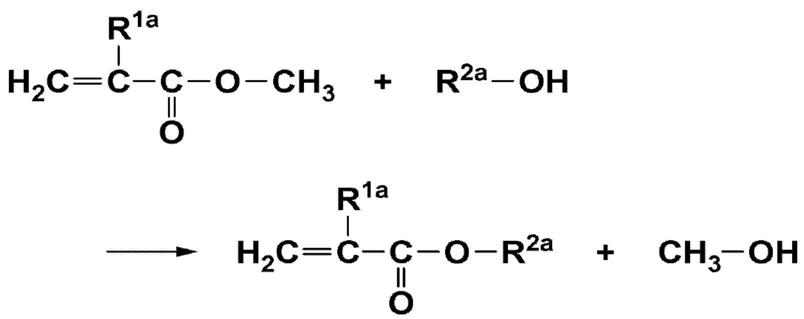

· · · (I I)

$R^{1a}$ and $R^{2a}$ in Formula (II) are the same as $R^{1a}$ and $R^{2a}$ in Formula (1).

Examples of the monoalcohol having 2 to 20 carbon atoms include ethanol, n-butanol, isobutanol, t-butanol, 2-ethylhexanol, lauryl alcohol, stearyl alcohol, cyclohexanol, isobornyl alcohol, allyl alcohol, phenol, and benzyl alcohol.

The monoalcohol having 2 to 20 carbon atoms may be used alone or a combination of two or more kinds thereof may be used. The monoalcohol having 2 to 20 carbon atoms preferably includes a linear or branched monoalcohol having 2 to 20 carbon atoms, and more preferably includes n-butanol or isobutanol.

For example, when obtaining the ester compound-containing composition comprising the ester compound (5) as the ester compound (I), in a reaction vessel, the methyl (meth)acrylate is subjected to an ester exchange with the ether bond-containing alcohol having 2 to 8 carbon atoms as represented by Formula (III) in the presence of a catalyst and the component A10.

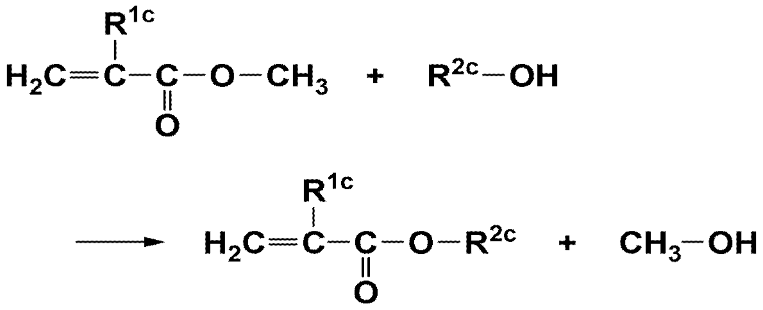

· · · (III)

$R^{1c}$ and $R^{2c}$ in Formula (III) are the same as $R^{1c}$ and $R^{2c}$ in Formula (5).

The number of ether bonds in the ether bond-containing alcohol having 2 to 8 carbon atoms is preferably 1, but is not limited thereto.

Examples of the ether bond-containing alcohol having 2 to 8 carbon atoms include 2-methoxyethanol, glycidyl alcohol, and tetrahydrofurfuryl alcohol. The ether bond-containing alcohol having 2 to 8 carbon atoms may be used alone or a combination of two or more kinds thereof may be used.

The ether bond-containing alcohol having 2 to 8 carbon atoms preferably includes one selected from 2-methoxyethanol, glycidyl alcohol, and tetrahydrofurfuryl alcohol.

For example, when obtaining the ester compound-containing composition comprising at least one or more selected from the group consisting of the ester compound (2), the ester compound (3), and the ester compound (4) as the ester compound (I), in a reaction vessel, the methyl (meth)acrylate is subjected to an ester exchange with one or more alcohols selected from the group consisting of the dialcohol having 2 to 8 carbon atoms and the trialcohol having 2 to 8 carbon atoms as represented by Formula (IV) and Formula (V) in the presence of a catalyst and the component A10.

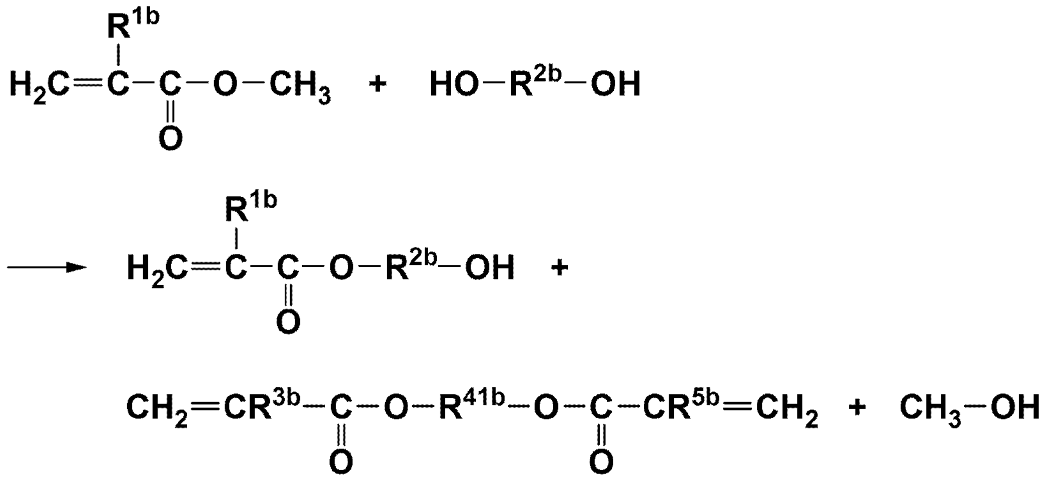

· · · (IV)

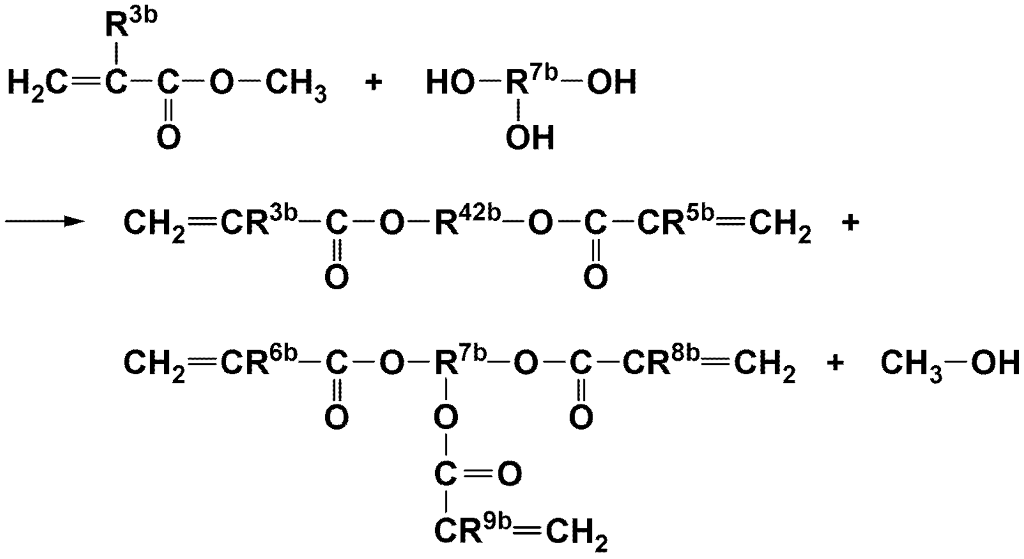

・・・ (V)

$R^{1b}$, $R^{2b}$, $R^{3b}$ $R^{5b}$, $R^{6b}$, and $R^{7b}$ in Formula (IV) and Formula (V) are the same as $R^{1b}$, $R^{2b}$, $R^{3b}$ $R^{5b}$, $R^{6b}$, and $R^{7b}$ in Formula (2), Formula (3), and Formula (4). In addition, $R^{41b}$ is a linear or branched alkylene group having 2 to 8 carbon atoms, and $R^{42b}$ is a linear or branched hydroxyalkylene group having 2 to 8 carbon atoms.

Examples of the dialcohol having 2 to 8 carbon atoms and the trialcohol having 2 to 8 carbon atoms include ethylene glycol, 1,2-propanediol, 1,3-propanediol, 1,3-butanediol, 1,6-hexanediol, and trimethylolpropane. Among these, it is preferable that the dialcohol having 2 to 8 carbon atoms and the trialcohol having 2 to 8 carbon atoms include one or more selected from the group consisting of ethylene glycol, 1,2-propanediol, 1,3-propanediol, and trimethylolpropane.

The dialcohol having 2 to 8 carbon atoms and the trialcohol having 2 to 8 carbon atoms may be used alone or a combination of two or more kinds thereof may be used.

The reaction vessel is preferably a reaction vessel including a distillation column. Since the ester exchange reaction is an equilibrium reaction, productivity is improved by separating a by-produced methanol by the distillation column. For example, it is preferable to perform the ester exchange reaction while separating methanol as a azeotropic mixture with the methyl (meth)acrylate to the outside of the system.

Examples of the reaction vessel include a reaction vessel including a distillation column provided on an upper part of a reaction container called a reaction kettle, and a distillation column in which a distillation can can be used as a reaction container. Examples of the distillation column include a packed column-type distillation column and a tray-type distillation column.

From the viewpoint of high separation ability and stable operation, a theoretical number of column plates of the distillation column is preferably 5 or more, and more preferably 7 or more.

The ratio of the amount of the methyl (meth)acrylate to be charged and the amount of the alcohol to be charged can be appropriately determined. From the viewpoint of improving productivity, the ratio of the methyl (meth)acrylate to 1 mol of alcohol is preferably 0.1 mol or more and 10 mol or less, and more preferably 0.3 mol or more and 4 mol or less.

The catalyst to be used is not particularly limited, and examples thereof include hydroxides, carbonates, and bicarbonates of an alkali metal such as lithium, sodium, and potassium; oxides, hydroxides, and carbonates of an alkaline earth metal such as magnesium and calcium; alkali metal alkoxides such as lithium methoxide, sodium methoxide, sodium ethoxide, and potassium t-butoxide; alkali metal amides such as lithium amide, sodium amide, and potassium amide; titanium alkoxides such as tetramethyl titanate, tetraethyl titanate, tetrapropyl titanate, triisopropyl titanate, tetrabutyl titanate, and tetra(2-ethylhexyl) titanate; and tin-based compounds such as dibutyl tin oxide and dioctyl tin oxide. Among these, from the viewpoint of small amount of by-products of Michael addition reaction product during the ester exchange reaction and high catalytic activity, an alkoxide of titanium, dibutyltin oxide, or dioctyltin oxide is preferable. The catalyst may be used alone or a combination of two or more kinds thereof may be used.

The catalyst can be supplied alone to the reaction vessel. In addition, the catalyst can also be supplied to the reaction vessel in a state of being dissolved in the same alcohol as the alcohol as the raw material, or in a state of being dissolved in the methyl (meth)acrylate as the raw material. Examples thereof include a method of directly dissolving the catalyst in the total amount of the alcohol used in the reaction and supplying the catalyst to the reaction vessel, and a method of dissolving the catalyst in a part of the alcohol used in the reaction and supplying the catalyst to the reaction vessel.

The amount of the catalyst used is preferably 0.001 mol% or more and 1 mol% or less, and more preferably 0.01

mol% or more and 0.1 mol% or less with respect to 1 mol of the alcohol.

A solvent may be used for the ester exchange reaction. When a solvent is used, it is preferable to use a solvent which forms an azeotropic composition with the by-produced methanol.

Examples of the solvent include n-pentane, n-hexane, n-heptane, n-octane, 2,3-dimethylbutane, 2,5-dimethylhexane, 2,2,4-trimethylpentane, cyclohexane, benzene, and toluene. Among these, n-hexane, n-heptane, or cyclohexane is preferable. The solvent may be used alone or a combination of two or more kinds thereof may be used.

The reaction temperature for the ester exchange reaction varies depending on the type of the alcohol or the solvent, but is preferably 60°C to 150°C.

The reaction pressure for the ester exchange reaction is not particularly limited, and the reaction may be carried out under any pressure of reduced pressure, normal pressure, or elevated pressure.

The form of the ester exchange reaction is not particularly limited, and the ester exchange reaction can be performed by a generally used method, for example, a batch method, a continuous method, or the like.

After the ester exchange reaction, unreacted raw materials and by-products may be separated by purifying the reaction solution. For the purification, for example, various methods such as distillation, crystallization, extraction, and column chromatography can be performed.

The ester exchange reaction may be performed by blending the component B into the reaction solution. When the component B is present in addition to the component A10, the dimerization of methyl (meth)acrylate or the generation of methyl pyruvate is further suppressed, and thus an effect of improving the yield of the ester compound (I) is more easily obtained.

The component B may be blended into a solution containing the ester compound (I) after the ester exchange reaction and the component A10. In this case, a solution (B solution) containing the ester compound (I) and the component B is prepared separately from the solution (A solution) containing the ester compound (I) and the component A10, and the A solution and the B solution are mixed with each other to obtain the ester compound-containing composition. In addition, the ester compound (I), the A solution, and the B solution may be mixed with each other to obtain the ester compound-containing composition.

The method for producing the ester compound-containing composition according to the sixth aspect is not limited to the above-described method.

For example, other (meth)acrylic acid esters other than the methyl (meth)acrylate may be used as the raw material instead of the methyl (meth)acrylate.

An esterification reaction between an alcohol and (meth)acrylic acid may be carried out to produce the ester compound (I).

(Evaluation method for storage stability and thermal stability of ester compound-containing composition)

The ester compound-containing composition has high quality stability during storage. Examples of an evaluation method of the quality stability of the ester compound-containing composition during storage include a method of actually storing the ester compound-containing composition for a long period of time and confirming the generation amount of the dimer of the ester compound (I), the generation amount of the oxidative product of the ester compound (I), and the contained amount of the ester compound (I). In addition, from the viewpoint of ease of work, a method of heating the ester compound-containing composition short period of time and confirming the generation amount of the dimer of the ester compound (I), the generation amount of the oxidative product of the ester compound (I), and the contained amount of the ester compound (I) may be used. When heating short period of time, the heating temperature is preferably 50°C to 100°C and a heating time is preferably 1 to 24 hours. When the ester compound-containing composition is stored at 25°C for 14 days or heated at 70°C for 7 to 20 hours, the quality stability of the ester compound-containing composition during storage is evaluated based on the generation amount of the dimer of the ester compound (I), the generation amount of the oxidative product of the ester compound (I), and the contained amount of the ester compound (I).

[Polymerizable composition]

A polymerizable composition according to the sixth aspect is a polymerizable composition for producing a (meth)acrylic polymer, and comprises the above-described ester compound-containing composition according to the sixth aspect.

As an example, an ester compound-containing composition stored for 1 day or more after production can be used as the polymerizable composition.

The storage time of the ester compound-containing composition can be 1 day or more, 3 days or more, 7 days or more, or 14 days or more. In addition, the storage time of the ester compound-containing composition may be 180 days or less, 150 days or less, 120 days or less, or 90 days or less. The "storage time" means an elapsed time from immediately after the production, which is not limited to a time during which the product is left to stand under a specific environment and

also includes a time of transportation or the like.

The material of a storage container for the ester compound-containing composition is not particularly limited, and for example, a metal container such as stainless steel, a resin container, or a glass container can be used. A transparent container or an opaque container can be used.

The temperature at the time of storing the ester compound-containing composition is preferably -10°C or higher and more preferably 0°C or higher, and is preferably 60°C or lower and more preferably 50°C or lower. By setting the temperature to -10°C or higher, a load on a cooling device can be reduced, and by setting the temperature to 60°C or lower, it is easy to suppress the generation of the dimerized substance or the oxidative product of (meth)acrylic acid ester.

The oxygen concentration of a gas phase portion at the time of storing the ester compound-containing composition is preferably 5% by volume or more and more preferably 7% by volume or more, and is preferably 30% by volume or less and more preferably 22% by volume or less. By setting the oxygen concentration of the gas phase portion to 5% by volume or more, it is easy to prevent the ester compound from being unintentionally polymerized by a polymerization prevention effect of oxygen, and by setting the oxygen concentration of the gas phase portion to 30% by volume or less, it is easy to suppress oxidation of the (meth)acrylic acid ester by oxygen and the generation of various impurities.

As an example, the ester compound-containing composition after the storage may be used in the polymerizable composition without further blending a monomer, or a monomer (hereinafter, also referred to as "other monomer") copolymerizable with the ester compound (I) may be further blended with the ester compound-containing composition after the storage to obtain the polymerizable composition.

As another example, the ester compound-containing composition after the production may be stored in a state in which the other monomer is blended, and used in the polymerizable composition. In this case, it is preferable that a polymerization initiator is not blended during the storage.

The proportion of the ester compound (I) to the total mass of monomers in the polymerizable composition is preferably 10% by mass or more and more preferably 20% by mass or more, and preferably 90% by mass or less and more preferably 80% by mass or less. As a result, a (meth)acrylic polymer having high transparency can be obtained.

The ester compound-containing composition may comprise the other monomer copolymerizable with the ester compound (I), or may not contain the other monomer copolymerizable with the ester compound (I).

Examples of the other monomer copolymerizable with the ester compound (I) include other monomer copolymerizable with the ester compound (1);

other monomer copolymerizable with the ester compound (5); and
other monomer copolymerizable with at least one selected from the group consisting of the ester compound (2), the ester compound (3), and the ester compound (4).

(Other monomer copolymerizable with ester compound (1))

Examples of the other monomer copolymerizable with the ester compound (1) include methyl (meth)acrylate, unsaturated carboxylic acid, unsaturated carboxylic acid anhydride, maleimide, a hydroxy group-containing vinyl monomer, vinyl ester, a nitrogen-containing vinyl monomer, an epoxy group-containing monomer, an aromatic vinyl monomer, alkanediol di(meth)acrylate, polyoxyalkylene glycol di(meth)acrylate, and a vinyl monomer having two or more ethylenically unsaturated bonds in the molecule.

Examples of the unsaturated carboxylic acid include acrylic acid, methacrylic acid, maleic acid, and itaconic acid.

Examples of the unsaturated carboxylic acid anhydride include maleic acid anhydride and itaconic acid anhydride.

Examples of the maleimide include N-phenylmaleimide and N-cyclohexylmaleimide.

Examples of the hydroxy group-containing vinyl monomer include 2-hydroxyethyl acrylate, 2-hydroxyethyl methacrylate, and 2-hydroxypropyl methacrylate.

Examples of the vinyl ester include vinyl acetate and vinyl benzoate.

Examples of the nitrogen-containing vinyl monomer include methacrylamide and acrylonitrile.

Examples of the epoxy group-containing monomer include glycidyl acrylate and glycidyl methacrylate.

Examples of the aromatic vinyl monomer include styrene and $\alpha$-methylstyrene.

Examples of the alkanediol di(meth)acrylate include ethylene glycol di(meth)acrylate, 1,2-propylene glycol di(meth)acrylate, 1,3-butylene glycol di(meth)acrylate, and 1,6-hexanediol di(meth)acrylate.

Examples of the polyoxyalkylene glycol di(meth)acrylate include diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate.

Examples of the vinyl monomer having two or more ethylenically unsaturated bonds in the molecule include divinylbenzene.

As the other monomer, vinyl chloride, vinylidene chloride, derivatives thereof, an unsaturated polyester prepolymer obtained from at least one polycarboxylic acid including an ethylenically unsaturated polycarboxylic acid and at least one diol, and a vinyl ester prepolymer obtained by acrylic modification of a terminal of an epoxy group may be used.
The other monomer may be used alone or a combination of two or more kinds thereof may be used.

(Other monomer copolymerizable with at least one selected from group consisting of ester compound (2), ester compound (3), and ester compound (4))

Examples of the other monomer copolymerizable with at least one selected from the group consisting of the ester compound (2), the ester compound (3), and the ester compound (4) include the same monomers as those of the other monomer copolymerizable with the ester compound (1).

(Other monomer copolymerizable with ester compound (5))

Examples of the other monomer copolymerizable with the ester compound (5) include the same monomers as those of the other monomer copolymerizable with the ester compound (1).
When the component A10 is a monomer copolymerizable with the ester compound (I), the component A10 may be used as the monomer copolymerizable with the ester compound (I), or a monomer copolymerizable with the ester compound (I) may be used separately from the component A10.
When the ester compound-containing composition comprises other monomer copolymerizable with the ester compound (I), the contained amount of the other monomer copolymerizable with the ester compound (I) is preferably 0.01 parts by mass or more, more preferably 0.1 parts by mass or more, and still more preferably 1 part by mass or more with respect to 100 parts by mass of the ester compound (I). In addition, the contained amount thereof is preferably 50 parts by mass or less, more preferably 40 parts by mass or less, and still more preferably 30 parts by mass or less. As a result, a (meth)acrylic polymer having high transparency can be obtained.
The polymerizable composition preferably comprises a polymerization initiator.
Examples of the polymerization initiator include an azo compound, an organic peroxide, a persulfate compound, and a redox polymerization initiator.
The polymerization initiator may be used alone or a combination of two or more kinds thereof may be used.
Examples of the azo compound include 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2,2'-azobis(2-methylpropane), 1,1-azobis(cyclohexanecarbonitrile), and dimethyl-2,2'-azobisisobutyrate.
Examples of the organic peroxide include benzoyl peroxide, 2,5-dimethyl-2,5-bis(t-butylperoxy)hexane, 1,1-bis(t-butylperoxy)cyclohexane, 1,1-bis(t-butylperoxy)-3,5,5-trimethylcyclohexane, t-butylperoxy-2-ethylhexanoate, t-butylperoxyisobutyrate, t-butylperoxybenzoate, t-hexylperoxybenzoate, t-butylperoxyisopropyl monocarbonate, t-butylperoxy-3,5,5-trimethylhexanoate, t-butylperoxylaureate, t-butylperoxyacetate, t-hexylperoxyisopropyl monocarbonate, t-hexylperoxy-2-ethylhexanoate, t-amylperoxy-2-ethylhexanoate, 1,1,3,3-tetramethylbutylperoxyethylhexanoate, 1,1,2-trimethylpropylperoxy-2-ethylhexanoate, 1,1,3,3-tetramethylbutylperoxyisopropyl monocarbonate, 1,1,2-trimethylpropylperoxyisopropyl monocarbonate, 1,1,3,3-tetramethylbutylperoxyisononanoate, 1,1,2-trimethylpropylperoxy-isononanoate, di-t-butyl peroxide, di-t-hexyl peroxide, lauroyl peroxide, and dilauroyl peroxide.
Examples of the persulfate compound include potassium persulfate.
The additional amountof the polymerization initiator is not particularly limited, and for example, can be 0.005 to 5 parts by mass with respect to 100 parts by mass of the total mass of the monomers in the polymerizable composition.
The polymerizable composition may comprise, as necessary, other additives such as a chain transfer agent, a release agent, a lubricant, a plasticizer, an antioxidant, an antistatic agent, a light stabilizer, an ultraviolet absorber, a flame retardant aid, a flame retardant assistant, a polymerization inhibitor, a filler, a pigment, a dye, a silane coupling agent, a leveling agent, an antifoaming agent, and a fluorescent agent.
The other additives may be used alone or a combination of two or more kinds thereof may be used.

[Method for producing (meth)acrylic polymer]

A (meth)acrylic polymer can be produced by polymerizing the polymerizable composition.
The polymerization method is not particularly limited, and examples thereof include a bulk polymerization method, a solution polymerization method, an emulsion polymerization method, and a suspension polymerization method. From the viewpoint of environmental load due to the use of the solvent or the like and viewpoint of transparency of the obtained (meth)acrylic polymer, a bulk polymerization method is preferable.
The method of the bulk polymerization method is not particularly limited, and examples thereof include various casting polymerization methods such as a cell casting method and a continuous casting method.

The casting polymerization method is a method in which the (meth)acrylic polymer is obtained by casting and polymerizing the polymerizable composition in a mold made of two inorganic glass plates or metal plates (for example, SUS plates) arranged facing each other at a predetermined interval with the periphery sealed with a gasket such as a soft resin tube.

The mold for the casting polymerization is not particularly limited, and various molds can be used. Examples of a mold for cell casting include a mold in which two plate-shaped products such as an inorganic glass plate, a chromium-plated metal plate, and a stainless steel plate are arranged facing each other at a predetermined interval, and a gasket is disposed on edges of the plates to form a sealed space between the plate-shaped products and the gasket. Examples of the mold for continuous casting include a mold in which a sealed space is formed by opposing surfaces of a pair of endless belts running in the same direction at the same speed and gaskets running at the same speed as the endless belt on both sides of the endless belt.

A gap between cavities of the molds is appropriately adjusted to obtain a resin plate having a desired thickness, and is generally 1 to 30 mm.

The polymerization temperature is preferably 125°C to 210°C and more preferably 130°C to 180°C.

The polymerization time is preferably 0.5 to 24 hours.

The weight-average molecular weight (Mw) of the (meth)acrylic polymer is not particularly limited, and can be, for example, 100,000 to 1,000,000. As the Mw of the (meth)acrylic polymer is higher, solvent resistance and chemical resistance can be further improved.

The Mw of the (meth)acrylic polymer can be controlled by adjusting the polymerization temperature, the polymerization time, the addition amount of the polymerization initiator, and the like.

The (meth)acrylic polymer according to the sixth aspect has excellent heat resistance and excellent meltability. For example, among (meth)acrylic polymers, a granular (meth)acrylic polymer having a large amount of resin which can be stored per unit volume and having a small energy cost during storage and transportation needs to be dissolved or melted at the time of use. The (meth)acrylic polymer according to the sixth aspect is easily melted, has excellent kneading properties with other resins, and has excellent solubility in a monomer, a solvent, or the like. Furthermore, a heat weight loss rate in a high-temperature environment is low.

## Examples

Hereinafter, the embodiments will be described in more detail with reference to Examples, but the present invention is not limited to the following description. In addition, "%" and "ppm" in Examples and Comparative Examples mean "% by mass", "ppm by mass", or "area%" unless otherwise specified.

The moisture content was calculated by Karl Fischer method. A formulation of constituent components of the ester compound-containing composition before storage was calculated from the addition amount of each raw material. A dimer of the ester compound (I) and an oxidative product of the ester compound (I) in the ester compound-containing composition before and after storage were quantified by an absolute calibration curve method using GC-MS. A purity of the ester compound (I) in the ester compound-containing composition after storage was calculated using GC-MS and a trace moisture content measuring device.

[GC-MS measurement]

QP-2010 manufactured by Shimadzu Corporation was used for the GC-MS measurement.

GC conditions were as follows.

Column (product name: DB-FFAP, manufactured by Agilent, length: 30 m, inner diameter: 0.25 mm, film thickness: 0.25 μm)
Injection volume: 1.0 μL
Vaporization chamber temperature: 240°C
Column oven temperature: held at 60°C for 3 minutes, raised from 60°C to 240°C at 10 °C/min, and held at 240°C for 4 minutes
Carrier gas: helium
Injection mode: split (split ratio: 30)
Control mode: constant linear velocity (50.0 cm/sec)
Pressure: 116.3 KPa
Total flow rate: 61.3 mL/min
Purge flow rate: 3.0 mL/min
Column flow rate: 1.88 mL/min

MS conditions were as follows.

Ionization method: electron ionization (EI)
Ion source temperature: 240°C
Interface temperature: 240°C
m/z detection range: 10 to 500
Detection time: 25 minutes

[Measurement of moisture content]

The moisture content was quantified by Karl Fischer method using a trace moisture content measuring device (manufactured by Nittoseiko Analytech Co., Ltd., product name "CA-21").

[Example 1]

0.028 g of butyl isobutyrate as a component A was added to 14.163 g of butyl methacrylate (BMA, manufactured by Tokyo Chemical Industry Co., Ltd., moisture content: 120 ppm by mass) to prepare an A solution.

In addition, 0.022 g of 2,4-dimethyl-6-t-butylphenol as a component B was added to 10.809 g of BMA (moisture content: 120 ppm by mass) to prepare a B solution.

Next, 0.051 g of the A solution and 0.051 g of the B solution were added to 9.850 g of BMA (moisture content: 120 ppm by mass) to prepare an ester compound-containing composition.

The obtained ester compound-containing composition was stored at 25°C for 14 days under white LED illumination, a contained amount (mg/L) of a BMA dimer and a contained amount (mg/L) of butyl pyruvate were determined by the GC-MS measurement using the absolute calibration curve method, and the amount of change (generated amount) before and after the storage was calculated.

[Examples 2 to 64 and Comparative Examples 1 to 6]

An ester compound-containing composition was prepared in the same manner as in Example 1, except that the formulations of the A solution, the B solution, and the ester compound-containing composition were changed as shown in Tables 1 to 3, and the generated amounts of the BMA dimer and the butyl pyruvate were obtained.

[Table 1]

| | A solution | | | | B solution | | | | Ester compound-containing composition | | | |
|---|---|---|---|---|---|---|---|---|---|---|---|---|
| | BMA [g] | Component A | | | BMA [g] | Component B | | | BMA [g] | A solution [g] | B solution [g] | 1-Butanol [g] |
| | | Compound name | Addition amount [g] | Contained amount [ppm by mass] | | Compound name | Addition amount [g] | Contained amount [ppm by mass] | | | | |
| Example 1 | 14.163 | Butyl isobutyrate | 0.028 | 1994 | 10.809 | DMTBP | 0.022 | 1994 | 9.850 | 0.051 | 0.051 | - |
| Example 2 | 13.767 | Butyl butyrate | 0.028 | 1993 | 10.809 | DMTBP | 0.022 | 1994 | 9.860 | 0.052 | 0.050 | - |
| Example 3 | 16.158 | Butyl butyrate | 0.032 | 1995 | 10.809 | DMTBP | 0.022 | 1994 | 9.891 | 0.053 | 0.049 | - |
| Example 4 | 14.163 | Butyl isobutyrate | 0.028 | 1994 | 10.809 | DMTBP | 0.022 | 1994 | 9.438 | 0.499 | 0.050 | - |
| Example 5 | 14.163 | Butyl isobutyrate | 0.028 | 1994 | 10.809 | DMTBP | 0.022 | 1994 | 4.984 | 4.999 | 0.051 | - |
| Example 6 | 9.993 | Butyl isobutyrate | 0.996 | 90655 | 10.809 | DMTBP | 0.022 | 1994 | 8.858 | 1.101 | 0.051 | - |
| Example 7 | 9.993 | Butyl isobutyrate | 0.996 | 90655 | 10.809 | DMTBP | 0.022 | 1994 | 4.995 | 4.951 | 0.050 | - |
| Example 8 | 14.163 | Butyl isobutyrate | 0.028 | 1994 | 10.809 | DMTBP | 0.022 | 1994 | 9.459 | 0.051 | 0.501 | - |
| Example 9 | 14.163 | Butyl isobutyrate | 0.028 | 1994 | 10.809 | DMTBP | 0.022 | 1994 | 4.927 | 0.050 | 5.000 | - |
| Example 10 | 14.163 | Butyl isobutyrate | 0.028 | 1994 | 10.004 | DMTBP | 1.004 | 91224 | 8.892 | 0.049 | 1.107 | - |
| Example 11 | 10.000 | Butyl isobutyrate | 0.020 | 2006 | 10.001 | MEHQ | 0.020 | 2006 | 9.850 | 0.050 | 0.051 | - |
| Example 12 | 10.000 | Butyl isobutyrate | 0.020 | 2006 | 10.002 | IPPDA | 0.020 | 1996 | 9.846 | 0.050 | 0.051 | - |
| Example 13 | 10.000 | Butyl isobutyrate | 0.020 | 2006 | 10.027 | PTZ | 0.020 | 2030 | 9.863 | 0.051 | 0.051 | - |

(continued)

| | A solution | | | | B solution | | | | Ester compound-containing composition | | | |
|---|---|---|---|---|---|---|---|---|---|---|---|---|
| | BMA [g] | Component A | | | BMA [g] | Component B | | | BMA [g] | A solution [g] | B solution [g] | 1-Butanol [g] |
| | | Compound name | Addition amount [g] | Contained amount [ppm by mass] | | Compound name | Addition amount [g] | Contained amount [ppm by mass] | | | | |
| Example 14 | 10.000 | Butyl isobutyrate | 0.020 | 2006 | 10.001 | HO-TEMPO | 0.020 | 1996 | 9.850 | 0.050 | 0.051 | - |
| Example 15 | 14.163 | Butyl isobutyrate | 0.028 | 1994 | 10.000 | - | - | - | 9.906 | 0.051 | - | - |
| Example 16 | 14.852 | Butyl acrylate | 0.030 | 1996 | 12.794 | DMTBP | 0.026 | 1997 | 9.869 | 0.055 | 0.052 | - |
| Example 17 | 14.852 | Butyl acrylate | 0.030 | 1996 | 12.794 | DMTBP | 0.026 | 1997 | 9.437 | 0.499 | 0.046 | - |
| Example 18 | 14.852 | Butyl acrylate | 0.030 | 1996 | 12.794 | DMTBP | 0.026 | 1997 | 4.928 | 4.996 | 0.049 | - |
| Example 19 | 9.962 | Butyl acrylate | 0.997 | 90946 | 12.794 | DMTBP | 0.026 | 1997 | 8.844 | 1.096 | 0.046 | - |
| Example 20 | 9.962 | Butyl acrylate | 0.997 | 90946 | 12.794 | DMTBP | 0.026 | 1997 | 5.009 | 4.955 | 0.053 | - |
| Example 21 | 14.852 | Butyl acrylate | 0.030 | 1996 | 12.794 | DMTBP | 0.026 | 1997 | 9.433 | 0.046 | 0.499 | - |
| Example 22 | 14.852 | Butyl acrylate | 0.030 | 1996 | 12.794 | DMTBP | 0.026 | 1997 | 4.928 | 0.051 | 4.999 | - |
| Example 23 | 10.002 | Butyl acrylate | 0.020 | 1996 | 10.001 | MEHQ | 0.020 | 2006 | 9.851 | 0.050 | 0.050 | - |
| Example 24 | 10.002 | Butyl acrylate | 0.020 | 1996 | 10.002 | IPPDA | 0.020 | 1996 | 9.859 | 0.050 | 0.051 | - |
| Example 25 | 10.002 | Butyl acrylate | 0.020 | 1996 | 10.027 | PTZ | 0.020 | 2030 | 9.850 | 0.051 | 0.052 | - |

[Table 2]

| | A solution | | | | B solution | | | | Ester compound-containing composition | | | |
|---|---|---|---|---|---|---|---|---|---|---|---|---|
| | BMA [g] | Component A | | | BMA [g] | Component B | | | BMA [g] | A solution [g] | B solution [g] | 1-Butanol [g] |
| | | Compound name | Addition amount [g] | Contained amount [ppm by mass] | | Compound name | Addition amount [g] | Contained amount [ppm by mass] | | | | |
| Example 26 | 10.002 | Butyl acrylate | 0.020 | 1996 | 10.001 | HO-TEMPO | 0.020 | 1996 | 9.861 | 0.050 | 0.051 | - |
| Example 27 | 14.852 | Butyl acrylate | 0.030 | 1996 | 10.000 | - | - | - | 9.910 | 0.054 | - | - |
| Example 28 | 11.606 | 2,3.5,6-Tetramethylpyrazine | 0.023 | 1995 | 11.450 | DMTBP | 0.023 | 1996 | 9.849 | 0.051 | 0.053 | - |
| Example 29 | 11.606 | 2,3,5,6-Tetramethylpyrazine | 0.023 | 1995 | 11.450 | DMTBP | 0.023 | 1996 | 9.431 | 0.498 | 0.053 | - |
| Example 30 | 11.606 | Tetramethylpyrazine | 0.023 | 1995 | 11.450 | DMTBP | 0.023 | 1996 | 5.576 | 5.643 | 0.057 | - |
| Example 31 | 10.032 | 2,3,5,6-Tetramethylpyrazine | 1.004 | 90998 | 11.450 | DMTBP | 0.023 | 1996 | 8.843 | 1.110 | 0.051 | - |
| Example 32 | 10.032 | 2,3,5,6-Tetramethylpyrazine | 1.004 | 90998 | 11.450 | DMTBP | 0.023 | 1996 | 5.002 | 4.974 | 0.052 | - |
| Example 33 | 11.606 | 2,3,5,6-Tetramethylpyrazine | 0.023 | 1995 | 11.450 | DMTBP | 0.023 | 1996 | 9.431 | 0.050 | 0.500 | - |
| Example 34 | 11.606 | 2,3,5,6-Tetramethylpyrazine | 0.023 | 1995 | 11.450 | DMTBP | 0.023 | 1996 | 4.930 | 0.053 | 4.998 | - |
| Example 35 | 11.606 | 2,3,5,6-Tetramethylpyrazine | 0.023 | 1995 | 10.017 | DMTBP | 1.002 | 90971 | 8.843 | 0.052 | 1.093 | - |
| Example 36 | 10.001 | 2,3,5,6-Tetramethylpyrazine | 0.020 | 2006 | 10.004 | MEHQ | 0.020 | 2015 | 10.000 | 0.051 | 0.051 | - |
| Example 37 | 10.001 | 2,3,5,6-Tetramethylpyrazine | 0.020 | 2006 | 10.001 | IPPDA | 0.020 | 1996 | 10.001 | 0.050 | 0.050 | - |
| Example 38 | 10.001 | 2,3,5,6-Tetramethylpyrazine | 0.020 | 2006 | 10.002 | PTZ | 0.020 | 2016 | 10.011 | 0.051 | 0.051 | - |

(continued)

| | A solution | | | | B solution | | | | Ester compound-containing composition | | | |
|---|---|---|---|---|---|---|---|---|---|---|---|---|
| | BMA [g] | Component A | | | BMA [g] | Component B | | | BMA [g] | A solution [g] | B solution [g] | 1-Butanol [g] |
| | | Compound name | Addition amount [g] | Contained amount [ppm by mass] | | Compound name | Addition amount [g] | Contained amount [ppm by mass] | | | | |
| Example 39 | 10.001 | 2,3,5,6-Tetramethylpyrazine | 0.020 | 2006 | 10.002 | HO-TEMPO | 0.020 | 1996 | 10.002 | 0.051 | 0.051 | - |
| Example 40 | 11.606 | 2,3,5,6-Tetramethyipyrazine | 0.023 | 1995 | 10.000 | - | - | - | 9.903 | 0.050 | - | - |
| Example 41 | 10.139 | Isopropoxybenzene | 0.020 | 1998 | 11.105 | DMTBP | 0.022 | 2013 | 9.850 | 0.054 | 0.055 | - |
| Example 42 | 10.135 | t-Butoxybenzene | 0.020 | 1999 | 11.105 | DMTBP | 0.022 | 2013 | 9.842 | 0.052 | 0.047 | - |
| Example 43 | 10.139 | Isopropoxybenzene | 0.020 | 1998 | 11.105 | DMTBP | 0.022 | 2013 | 9.435 | 0.538 | 0.051 | - |
| Example 44 | 10.139 | Isopropoxybenzene | 0.020 | 1998 | 11.105 | DMTBP | 0.022 | 2013 | 4.925 | 4.992 | 0.049 | - |
| Example 45 | 9.962 | Isopropoxybenzene | 0.998 | 91015 | 11.105 | DMTBP | 0.022 | 2013 | 8.842 | 1.098 | 0.050 | - |
| Example 46 | 9.962 | Isopropoxybenzene | 0.998 | 91015 | 11.105 | DMTBP | 0.022 | 2013 | 4.996 | 4.950 | 0.052 | - |
| Example 47 | 10.139 | Isopropoxybenzene | 0.020 | 1998 | 11.105 | DMTBP | 0.022 | 2013 | 9.434 | 0.052 | 0.503 | - |
| Example 48 | 10.139 | Isopropoxybenzene | 0.020 | 1998 | 11.105 | DMTBP | 0.022 | 2013 | 4.930 | 0.051 | 4.995 | - |
| Example 49 | 10.139 | Isopropoxybenzene | 0.020 | 1998 | 9.982 | DMTBP | 0.997 | 90829 | 8.852 | 0.050 | 1.111 | - |
| Example 50 | 10.002 | Isopropoxybenzene | 0.020 | 1996 | 10.001 | MEHQ | 0.020 | 2006 | 9.850 | 0.050 | 0.050 | - |

[Table 3]

| | A solution | | | | B solution | | | | Ester compound-containing composition | | | |
|---|---|---|---|---|---|---|---|---|---|---|---|---|
| | BMA [g] | Component A | | | BMA [g] | Component B | | | BMA [g] | A solution [g] | B solution [g] | 1-Butanol [g] |
| | | Compound name | Addition amount [g] | Contained amount [ppm by mass] | | Compound name | Addition amount [g] | Contained amount [ppm by mass] | | | | |
| Example 51 | 10.002 | Isopropoxybenzene | 0.020 | 1996 | 10.002 | IPPDA | 0.020 | 1996 | 9.853 | 0.051 | 0.052 | - |
| Example 52 | 10.002 | Isopropoxybenzene | 0.020 | 1996 | 10.027 | PTZ | 0.020 | 2030 | 9.853 | 0.050 | 0.050 | - |
| Example 53 | 10.002 | Isopropoxybenzene | 0.020 | 1996 | 10.001 | HO-TEMPO | 0.020 | 1996 | 9.851 | 0.050 | 0.050 | - |
| Example 54 | 10.139 | Isopropoxybenzene | 0.020 | 1998 | 10.000 | - | - | - | 9.900 | 0.051 | - | - |
| Example 55 | 10.006 | 3-Methyl-1-butyl methacrylate | 0.020 | 2015 | 10.001 | DMTBP | 0.020 | 2036 | 9.851 | 0.051 | 0.051 | - |
| Example 56 | 10.006 | 3-Methyl-1-butyl methacrylate | 0.020 | 2015 | 10.001 | DMTBP | 0.020 | 2036 | 9.352 | 0.501 | 0.051 | - |
| Example 57 | 10.006 | 3-Methyl-1-butyl methacrylate | 0.020 | 2015 | 10.001 | DMTBP | 0.020 | 2036 | 4.930 | 5.001 | 0.051 | - |
| Example 58 | 10.003 | 3-Methyl-1-butyl methacrylate | 1.000 | 90887 | 10.001 | DMTBP | 0.020 | 2036 | 8.850 | 1.100 | 0.050 | - |
| Example 59 | 10.006 | 3-Methyl-1-butyl methacrylate | 0.020 | 2015 | 10.000 | - | - | - | 9.900 | 0.050 | - | - |
| Example 60 | 10.002 | 1-Pentyl methacrylate | 0.020 | 2026 | 10.001 | DMTBP | 0.020 | 2036 | 9.852 | 0.051 | 0.051 | - |
| Example 61 | 10.002 | 1-Pentyl methacrylate | 0.020 | 2026 | 10.001 | DMTBP | 0.020 | 2036 | 9.439 | 0.501 | 0.051 | - |
| Example 62 | 10.002 | 1-Pentyl methacrylate | 0.020 | 2026 | 10.001 | DMTBP | 0.020 | 2036 | 4.934 | 5.001 | 0.050 | - |
| Example 63 | 10.007 | 1-Pentyl methacrylate | 1.001 | 90907 | 10.001 | DMTBP | 0.020 | 2036 | 8.852 | 1.101 | 0.050 | - |
| Example 64 | 10.002 | 1-Pentyl methacrylate | 0.020 | 2026 | 10.000 | - | - | - | 9.900 | 0.050 | - | - |

(continued)

| | A solution | | | | B solution | | | | Ester compound-containing composition | | | |
|---|---|---|---|---|---|---|---|---|---|---|---|---|
| | BMA [g] | Component A | | | BMA [g] | Component B | | | BMA [g] | A solution [g] | B solution [g] | 1-Butanol [g] |
| | | Compound name | Addition amount [g] | Contained amount [ppm by mass] | | Compound name | Addition amount [g] | Contained amount [ppm by mass] | | | | |
| Comparative Example 1 | 14.163 | Butyl isobutyrate | 0.0283 | 1994 | 10.809 | DMTBP | 0.022 | 1994 | 9.395 | 0.051 | 0.051 | 0.492 |
| Comparative Example 2 | 14.852 | Butyl acrylate | 0.0297 | 1996 | 12.794 | DMTBP | 0.026 | 1997 | 9.527 | 0.052 | 0.054 | 0.505 |
| Comparative Example 3 | 11.606 | 2,3,5,6-Tetramethylpyrazine | 0.0232 | 1995 | 11.450 | DMTBP | 0.023 | 1996 | 9.401 | 0.053 | 0.053 | 0.502 |
| Comparative Example 4 | 10.139 | Isopropoxybenzene | 0.0203 | 1998 | 11.105 | DMTBP | 0.022 | 2013 | 9.395 | 0.053 | 0.049 | 0.507 |
| Comparative Example 5 | - | - | - | - | - | - | - | - | 10.005 | - | - | - |
| Comparative Example 6 | - | - | - | - | 12.198 | DMTBP | 0.024 | 1996 | 9.904 | - | 0.053 | - |

Abbreviations in Tables 1 to 3 have the following meanings.

· BMA: butyl methacrylate
· DMTBP: 2,4-dimethyl-6-t-butylphenol
· MEHQ: 4-methoxyphenol
· IPPDA: 4-isopropylaminodiphenylamine
· PTZ: phenothiazine
· HO-TEMPO: 4-hydroxy-2,2,6,6-tetramethyl-piperidine-N-oxyl

Tables 4 to 6 show the generated amounts of the BMA dimer and the butyl pyruvate in each example.

[Table 4]

| | Formulation of ester compound-containing composition (calculated value) | | | | | | | Analysis result of ester compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generation amount of BMA dimer [mg/L] | Generation amount of butyl pyruvate [mg/L] |
| Example 1 | 99.91 | Butyl isobutyrate | 10 | DMTBP | 10 | - | - | 1.57 | 3.33 |
| Example 2 | 99.91 | Butyl butyrate | 10 | DMTBP | 10 | - | - | 2.05 | 3.70 |
| Example 3 | 99.91 | Butyl butyrate | 11 | DMTBP | 10 | - | - | 1.99 | 3.61 |
| Example 4 | 99.90 | Butyl isobutyrate | 100 | DMTBP | 10 | - | - | 2.17 | 3.40 |
| Example 5 | 99.81 | Butyl isobutyrate | 994 | DMTBP | 10 | - | - | 2.42 | 3.66 |
| Example 6 | 98.91 | Butyl isobutyrate | 9972 | DMTBP | 10 | - | - | 2.13 | 3.68 |
| Example 7 | 95.42 | Butyl isobutyrate | 44901 | DMTBP | 10 | - | - | 2.17 | 3.73 |
| Example 8 | 99.90 | Butyl isobutyrate | 10 | DMTBP | 100 | - | - | 2.40 | 2.97 |
| Example 9 | 99.81 | Butyl isobutyrate | 10 | DMTBP | 1000 | - | - | 2.54 | 2.28 |
| Example 10 | 98.90 | Butyl isobutyrate | 10 | DMTBP | 10047 | - | - | 2.94 | 1.55 |
| Example 11 | 99.91 | Butyl isobutyrate | 10 | MEHQ | 10 | - | - | 2.51 | 3.46 |
| Example 12 | 99.91 | Butyl isobutyrate | 10 | IPPDA | 10 | - | - | 0.90 | 2.14 |
| Example 13 | 99.91 | Butyl isobutyrate | 10 | PTZ | 10 | - | - | 0.46 | 3.35 |
| Example 14 | 99.91 | Butyl isobutyrate | 10 | HO-TEMPO | 10 | - | - | 1.27 | 1.69 |
| Example 15 | 99.91 | Butyl isobutyrate | 10 | - | - | - | - | 2.83 | 3.37 |
| Example 16 | 99.90 | Butyl acrylate | 11 | DMTBP | 10 | - | - | 4.49 | 3.70 |
| Example 17 | 99.89 | Butyl acrylate | 100 | DMTBP | 9 | - | - | 4.47 | 4.36 |
| Example 18 | 99.80 | Butyl acrylate | 1000 | DMTBP | 10 | - | - | 4.41 | 4.25 |
| Example 19 | 98.91 | Butyl acrylate | 9979 | DMTBP | 9 | - | - | 4.55 | 4.09 |
| Example 20 | 95.41 | Butyl acrylate | 44988 | DMTBP | 11 | - | - | 4.57 | 4.41 |
| Example 21 | 99.89 | Butyl acrylate | 9 | DMTBP | 100 | - | - | 4.59 | 3.43 |
| Example 22 | 99.80 | Butyl acrylate | 10 | DMTBP | 1001 | - | - | 4.82 | 3.16 |
| Example 23 | 99.90 | Butyl acrylate | 10 | MEHQ | 10 | - | - | 0.84 | 3.19 |

(continued)

| | Formulation of ester compound-containing composition (calculated value) | | | | | | | Analysis result of ester compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generation amount of BMA dimer [mg/L] | Generation amount of butyl pyruvate [mg/L] |
| Example 24 | 99.90 | Butyl acrylate | 10 | IPPDA | 10 | - | - | 1.02 | 1.82 |
| Example 25 | 99.90 | Butyl acrylate | 10 | PTZ | 11 | - | - | 1.55 | 3.50 |

[Table 5]

| | Formulation of ester compound-containing composition (calculated value) | | | | | | | Analysis result of ester compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generation amount of BMA dimer [mg/L] | Generation amount of butyl pyruvate [mg/L] |
| Example 26 | 99.90 | Butyl acrylate | 10 | HO-TEMPO | 10 | - | - | 0.72 | 1.59 |
| Example 27 | 99.90 | Butyl acrylate | 11 | - | - | - | - | 4.95 | 4.09 |
| Example 28 | 99.89 | 2,3,5,6-Tetramethylpyrazine | 10 | DMTBP | 11 | - | - | 2.84 | 4.06 |
| Example 29 | 99.88 | 2,3,5,6-Tetramethylpyrazine | 100 | DMTBP | 11 | - | - | 2.86 | 4.48 |
| Example 30 | 99.79 | 2,3,5,6-Tetramethylpyrazine | 998 | DMTBP | 10 | - | - | 3.13 | 4.71 |
| Example 31 | 98.88 | 2,3,5,6-Tetramethylpyrazine | 10094 | DMTBP | 10 | - | - | 3.07 | 4.42 |
| Example 32 | 95.38 | 2,3,5,6-Tetramethylpyrazine | 45135 | DMTBP | 10 | - | - | 2.22 | 4.51 |
| Example 33 | 99.88 | 2,3,5,6-Tetramethylpyrazine | 10 | DMTBP | 100 | - | - | 3.18 | 3.64 |
| Example 34 | 99.79 | 2,3,5,6-Tetramethylpyrazine | 11 | DMTBP | 1000 | - | - | 3.40 | 3.17 |
| Example 35 | 98.90 | 2,3,5,6-Tetramethylpyrazine | 10 | DMTBP | 9956 | - | - | 3.69 | 2.54 |
| Example 36 | 99.89 | 2,3,5,6-Tetramethylpyrazine | 10 | MEHQ | 10 | - | - | 1.01 | 3.55 |
| Example 37 | 99.89 | 2,3,5,6-Tetramethylpyrazine | 10 | IPPDA | 10 | - | - | 0.69 | 1.26 |
| Example 38 | 99.89 | 2,3,5,6-Tetramethylpyrazine | 10 | PTZ | 10 | - | - | 0.60 | 3.25 |
| Example 39 | 99.89 | 2,3,5,6-Tetramethylpyrazine | 10 | HO-TEMPO | 10 | - | - | 1.07 | 1.62 |
| Example 40 | 99.89 | 2,3,5,6-Tetramethylpyrazine | 10 | - | - | - | - | 3.34 | 4.55 |
| Example 41 | 99.90 | Isopropoxybenzene | 11 | DMTBP | 11 | - | - | 3.16 | 5.63 |
| Example 42 | 99.90 | t-Butoxybenzene | 10 | DMTBP | 10 | - | - | 3.12 | 5.10 |
| Example 43 | 99.89 | Isopropoxybenzene | 107 | DMTBP | 10 | - | - | 2.99 | 4.99 |
| Example 44 | 99.80 | Isopropoxybenzene | 1001 | DMTBP | 10 | - | - | 2.62 | 4.97 |
| Example 45 | 98.90 | Isopropoxybenzene | 10000 | DMTBP | 10 | - | - | 2.47 | 4.53 |
| Example 46 | 95.40 | Isopropoxybenzene | 45059 | DMTBP | 11 | - | - | 2.59 | 4.94 |
| Example 47 | 99.89 | Isopropoxybenzene | 10 | DMTBP | 101 | - | - | 2.82 | 4.90 |

(continued)

| | Formulation of ester compound-containing composition (calculated value) | | | | | | | Analysis result of ester compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generation amount of BMA dimer [mg/L] | Generation amount of butyl pyruvate [mg/L] |
| Example 48 | 99.80 | Isopropoxybenzene | 10 | DMTBP | 1008 | - | - | 2.36 | 3.64 |
| Example 49 | 98.90 | Isopropoxybenzene | 10 | DMTBP | 10077 | - | - | 3.05 | 2.53 |
| Example 50 | 99.90 | Isopropoxybenzene | 10 | MEHQ | 10 | - | - | 1.47 | 3.41 |

[Table 6]

| | Formulation of ester compound-containing composition (calculated value) | | | | | | | Analysis result of ester compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generation amount of BMA dimer [mg/L] | Generation amount of butyl pyruvate [mg/L] |
| Example 51 | 99.90 | Isopropoxybenzene | 10 | IPPDA | 10 | - | - | 1.07 | 1.70 |
| Example 52 | 99.90 | Isopropoxybenzene | 10 | PTZ | 10 | - | - | 0.70 | 2.93 |
| Example 53 | 99.90 | Isopropoxybenzene | 10 | HO-TEMPO | 10 | - | - | 0.82 | 1.41 |
| Example 54 | 99.90 | Isopropoxybenzene | 10 | - | - | - | - | 2.52 | 5.02 |
| Example 55 | 99.90 | 3-Methyl-1-butyl methacrylate | 10 | DMTBP | 10 | - | - | 2.15 | 1.70 |
| Example 56 | 99.89 | 3-Methyl-1-butyl methacrylate | 102 | DMTBP | 10 | - | - | 2.72 | 1.67 |
| Example 57 | 99.80 | 3-Methyl-1-butyl methacrylate | 1009 | DMTBP | 10 | - | - | 2.14 | 3.07 |
| Example 58 | 98.90 | 3-Methyl-1-butyl methacrylate | 9997 | DMTBP | 10 | - | - | 2.01 | 3.22 |
| Example 59 | 99.90 | 3-Methyl-1-butyl methacrylate | 10 | - | - | - | - | 2.75 | 2.30 |
| Example 60 | 99.90 | 1-Pentyl methacrylate | 10 | DMTBP | 10 | - | - | 3.12 | 2.82 |
| Example 61 | 99.89 | 1-Pentyl methacrylate | 102 | DMTBP | 10 | - | - | 2.85 | 2.21 |
| Example 62 | 99.80 | 1-Pentyl methacrylate | 1015 | DMTBP | 10 | - | - | 3.18 | 2.95 |
| Example 63 | 98.90 | 1-Pentyl methacrylate | 10004 | DMTBP | 10 | - | - | 2.52 | 2.75 |
| Example 64 | 99.90 | 1-Pentyl methacrylate | 10 | - | - | - | - | 2.30 | 2.60 |
| Comparative Example 1 | 94.98 | Butyl isobutyrate | 10 | DMTBP | 10 | 1-Butanol | 49286 | 2.48 | 6.86 |

(continued)

| | Formulation of ester compound-containing composition (calculated value) | | | | | | | | Analysis result of ester compound-containing composition after storage | |
|---|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | | Generation amount of BMA dimer [mg/L] | Generation amount of butyl pyruvate [mg/L] |
| Comparative Example 2 | 94.92 | Butyl acrylate | 10 | DMTBP | 11 | 1-Butanol | 49809 | | 4.61 | 6.80 |
| Comparative Example 3 | 94.88 | 2,3,5,6-Tetramethylpyrazine | 11 | DMTBP | 11 | 1-Butanol | 50149 | | 3.15 | 8.40 |
| Comparative Example 4 | 94.84 | Isopropoxybenzene | 11 | DMTBP | 10 | 1-Butanol | 50657 | | 2.67 | 9.66 |
| Comparative Example 5 | 99.91 | - | - | - | - | - | - | | 5.74 | 7.63 |
| Comparative Example 6 | 99.89 | - | - | DMTBP | 11 | - | - | | 5.38 | 6.27 |

As shown in Tables 4 to 6, in the ester compound-containing compositions of Examples 1 to 64 in which the contained amount of BMA was 95% by mass or more and the component A was contained, the generated amounts of the BMA dimer and the butyl pyruvate after the storage were smaller than those of the ester compound-containing compositions of Comparative Examples 1 to 6 in which the contained amount BMA was less than 95% by mass or the component A was not contained.

[Example 65]

0.019 g of methacrylonitrile as a component A was added to 9.705 g of butyl methacrylate (BMA, manufactured by Tokyo Chemical Industry Co., Ltd., moisture content: 120 ppm by mass) to prepare an A solution.
In addition, 0.024 g of 2,4-dimethyl-6-t-butylphenol as a component B was added to 12.198 g of BMA (moisture content: 120 ppm by mass) to prepare a B solution.
Next, 0.053 g of the A solution and 0.054 g of the B solution were added to 9.850 g of BMA (moisture content: 120 ppm by mass) to prepare an ester compound-containing composition.
The obtained ester compound-containing composition was stored at 70°C for 7 hours under white LED illumination, a contained amount (mg/L) of a BMA dimer and a contained amount (mg/L) of butyl pyruvate were determined by the GC-MS measurement using the absolute calibration curve method, and the amount of change (generated amount) before and after the storage was calculated.

[Examples 66 to 76 and Comparative Examples 7 to 9]

An ester compound-containing composition was prepared in the same manner as in Example 65, except that the formulations of the A solution, the B solution, and the ester compound-containing composition were changed as shown in Table 7, and the generated amounts of the BMA dimer and the butyl pyruvate were obtained.

[Table 7]

| | A solution | | | | B solution | | | | Ester compound-containing composition | | | |
|---|---|---|---|---|---|---|---|---|---|---|---|---|
| | BMA [g] | Component A | | | BMA [g] | Component B | | | BMA [g] | A solution [g] | B solution [g] | 1-Butanol [g] |
| | | Compound name | Addition amount [g] | Contained amount [ppm by mass] | | Compound name | Addition amount [g] | Contained amount [ppm by mass] | | | | |
| Example 65 | 9.705 | Methacrylonitrile | 0.019 | 1995 | 12.198 | DMTBP | 0.024 | 1996 | 9.845 | 0.053 | 0.054 | - |
| Example 66 | 9.705 | Methacrylonitrile | 0.019 | 1995 | 12.198 | DMTBP | 0.024 | 1996 | 9.437 | 0.501 | 0.055 | - |
| Example 67 | 9.705 | Methacrylonitrile | 0.019 | 1995 | 12.198 | DMTBP | 0.024 | 1996 | 4.940 | 5.005 | 0.051 | - |
| Example 68 | 9.994 | Methacrylonitrile | 0.999 | 90870 | 12.198 | DMTBP | 0.024 | 1996 | 8.844 | 1.106 | 0.048 | - |
| Example 69 | 9.994 | Methacrylonitrile | 0.999 | 90870 | 12.198 | DMTBP | 0.024 | 1996 | 5.523 | 5.442 | 0.056 | - |
| Example 70 | 9.705 | Methacrylonitrile | 0.019 | 1995 | 12.198 | DMTBP | 0.024 | 1996 | 9.431 | 0.046 | 0.499 | - |
| Example 71 | 9.705 | Methacrylonitrile | 0.019 | 1995 | 12.198 | DMTBP | 0.024 | 1996 | 4.946 | 0.049 | 5.000 | - |
| Example 72 | 9.705 | Methacrylonitrile | 0.019 | 1995 | 9.958 | DMTBP | 0.996 | 90958 | 8.858 | 0.053 | 1.108 | - |
| Example 73 | 10.000 | Methacrylonitrile | 0.020 | 2016 | 10.004 | MEHQ | 0.020 | 2015 | 10.000 | 0.051 | 0.053 | - |
| Example 74 | 10.000 | Methacrylonitrile | 0.0202 | 2016 | 10.001 | IPPDA | 0.020 | 1996 | 10.004 | 0.051 | 0.051 | - |
| Example 75 | 10.000 | Methacrylonitrile | 0.0202 | 2016 | 10.002 | PTZ | 0.020 | 2016 | 10.003 | 0.051 | 0.051 | - |
| Example 76 | 9.705 | Methacrylonitrile | 0.0194 | 1995 | 10.000 | - | - | - | 9.909 | 0.053 | - | - |
| Comparative Example 7 | 9.705 | Methacrylonitrile | 0.0194 | 1995 | 12.198 | DMTBP | 0.024 | 1996 | 9.400 | 0.054 | 0.046 | 0.504 |
| Comparative Example 8 | - | - | - | - | - | - | - | - | 10.005 | - | - | - |
| Comparative Example 9 | - | - | - | - | 12.198 | DMTBP | 0.024 | 1996 | 9.904 | - | 0.053 | - |

Abbreviations in Table 7 have the following meanings.

- · BMA: butyl methacrylate
- · DMTBP: 2,4-dimethyl-6-t-butylphenol
- · MEHQ: 4-methoxyphenol
- · IPPDA: 4-isopropylaminodiphenylamine
- · PTZ: phenothiazine

Table 8 shows the generated amounts of the BMA dimer and the butyl pyruvate in each example.

[Table 8]

| | Formulation of ester compound-containing composition (calculated value) | | | | | | | Analysis result of ester compound-containing composition after storage | |
|---|---|---|---|---|---|---|---|---|---|
| | BMA [% by mass] | Component A compound name | Component A [ppm by mass] | Component B compound name | Component B [ppm by mass] | Component C compound name | Component C [ppm by mass] | Generation amount of BMA dimer [mg/L] | Generation amount of butyl pyruvate [mg/L] |
| Example 65 | 99.89 | Methacrylonitrile | 11 | DMTBP | 11 | - | - | 8.31 | 0.47 |
| Example 66 | 99.88 | Methacrylonitrile | 100 | DMTBP | 11 | - | - | 9.06 | 0.40 |
| Example 67 | 99.79 | Methacrylonitrile | 999 | DMTBP | 10 | - | - | 8.85 | 0.52 |
| Example 68 | 98.89 | Methacrylonitrile | 10056 | DMTBP | 10 | - | - | 9.77 | 0.58 |
| Example 69 | 95.41 | Methacrylonitrile | 44873 | DMTBP | 10 | - | - | 5.74 | 0.64 |
| Example 70 | 99.88 | Methacrylonitrile | 9 | DMTBP | 100 | - | - | 924 | 0.32 |
| Example 71 | 99.79 | Methacrylonitrile | 10 | DMTBP | 999 | - | - | 8.75 | 0.13 |
| Example 72 | 98.89 | Methacrylonitrile | 10 | DMTBP | 10056 | - | - | 10.13 | 0.14 |
| Example 73 | 99.89 | Methacrylonitrile | 10 | MQ | 11 | - | - | 12.61 | 0.24 |
| Example 74 | 99.89 | Methacrylonitrile | 10 | MEHQ | 10 | - | - | 12.27 | 0.36 |
| Example 75 | 99.89 | Methacrylonitrile | 10 | PTZ | 10 | - | - | 10.94 | 0.28 |
| Example 76 | 99.89 | Methacrylonitrile | 11 | - | - | - | - | 9.31 | 0.45 |
| Comparative Example 7 | 94.86 | Methacrylonitrile | 11 | DMTBP | 9 | 1-Butanol | 50348 | 10.65 | 2.07 |
| Comparative Example 8 | 99.91 | - | - | - | - | - | - | 13.55 | 0.93 |
| Comparative Example 9 | 99.89 | - | - | DMTBP | 11 | - | - | 12.94 | 0.96 |

As shown in Table 8, in the ester compound-containing compositions of Examples 65 to 76 in which the contained amount of BMA was 95% by mass or more and the component A was contained, the generated amounts of the BMA dimer and the butyl pyruvate after the storage were smaller than those of the ester compound-containing compositions of Comparative Examples 7 to 9 in which the contained amount of BMA was less than 95% by mass or the component A was not contained.

[Example 77]

0.021 g of 2-methyl-1-butyl methacrylate as a component A was added to 10.001 g of butyl methacrylate (BMA, manufactured by Tokyo Chemical Industry Co., Ltd., moisture content: 120 ppm by mass) to prepare an A solution.
In addition, 0.020 g of 2,4-dimethyl-6-t-butylphenol as a component B was added to 10.002 g of BMA (moisture content: 120 ppm by mass) to prepare a B solution.
Next, 0.050 g of the A solution and 0.051 g of the B solution were added to 9.854 g of BMA (moisture content: 120 ppm by mass) to prepare an ester compound-containing composition.
The obtained ester compound-containing composition was stored at 70°C for 20 hours under white LED illumination, a contained amount (mg/L) of a BMA dimer and a contained amount (mg/L) of butyl pyruvate were determined by the GC-MS measurement using the absolute calibration curve method, and the amount of change (generated amount) before and after the storage was calculated.

[Examples 78 to 84 and Comparative Examples 10 and 11]

An ester compound-containing composition was prepared in the same manner as in Example 77, except that the formulations of the A solution, the B solution, and the ester compound-containing composition were changed as shown in Table 9, and the generated amounts of the BMA dimer and the butyl pyruvate were obtained.

[Table 9]

| | A solution | | | | B solution | | | | Ester compound-containing composition | | |
|---|---|---|---|---|---|---|---|---|---|---|---|
| | BMA [g] | Component A | | | BMA [g] | Component B | | | BMA [g] | A solution [g] | B solution [g] |
| | | Compound name | Addition amount [g] | Contained amount [ppm by mass] | | Compound name | Addition amount [g] | Contained amount [ppm by mass] | | | |
| Example 77 | 10.001 | 2-Methyl-1-butyl methacrylate | 0.021 | 2046 | 10.002 | DMTBP | 0.020 | 2015 | 9.854 | 0.050 | 0.051 |
| Example 78 | 10.001 | 2-Methyl-1-butyl methacrylate | 0.021 | 2046 | 10.002 | DMTBP | 0.020 | 2015 | 9.436 | 0.502 | 0.050 |
| Example 79 | 10.001 | 2-Methyl-1-butyl methacrylate | 0.021 | 2046 | 10.002 | DMTBP | 0.020 | 2015 | 4.942 | 5.001 | 0.051 |
| Example 80 | 10.004 | 2-Methyl-1-butyl methacrylate | 1.001 | 90916 | 10.002 | DMTBP | 0.020 | 2015 | 8.852 | 1.101 | 0.050 |
| Example 81 | 10.004 | 2-Methyl-1-butyl methacrylate | 1.001 | 90916 | 10.002 | DMTBP | 0.020 | 2015 | 5.001 | 4.951 | 0.050 |
| Example 82 | 10.001 | 2-Methyl-1-butyl methacrylate | 0.021 | 2046 | 10.002 | DMTBP | 0.020 | 2015 | 9.436 | 0.051 | 0.500 |
| Example 83 | 10.001 | 2-Methyl-1-butyl methacrylate | 0.021 | 2046 | 10.017 | MEHQ | 0.020 | 2013 | 9.851 | 0.051 | 0.051 |
| Example 84 | 10.001 | 2-Methyl-1-butyl methacrylate | 0.021 | 2046 | 10.000 | - | - | - | 9.902 | 0.050 | - |
| Comparative Example 10 | - | - | - | - | - | - | - | - | 10.005 | - | - |

(continued)

| | A solution | | | | B solution | | | | Ester compound-containing composition | | |
|---|---|---|---|---|---|---|---|---|---|---|---|
| | BMA [g] | Component A | | | BMA [g] | Component B | | | BMA [g] | A solution [g] | B solution [g] |
| | | Compound name | Addition amount [g] | Contained amount [ppm by mass] | | Compound name | Addition amount [g] | Contained amount [ppm by mass] | | | |
| Comparative Example 11 | - | - | - | - | 12.198 | DMTBP | 0.024 | 1996 | 9.904 | - | 0.053 |

Abbreviations in Table 9 have the following meanings.

· BMA: butyl methacrylate

· DMTBP: 2,4-dimethyl-6-t-butylphenol

· MEHQ: 4-methoxyphenol

Table 10 shows the generated amounts of the BMA dimer and the butyl pyruvate in each example.

[Table 10]

| | Formulation of ester compound-containing composition (calculated value) | | | | | Analysis result of ester compound-containing composition after storage | |
|---|---|---|---|---|---|---|---|
| | BMA [% by mass] | Component A compound name | Component A [ppm by mass] | Component B compound name | Component B [ppm by mass] | Generation amount of BMA dimer [mg/L] | Generation amount of butyl pyruvate [mg/L] |
| Example 77 | 99.90 | 2-Methyl-1-butyl methacrylate | 10 | DMTBP | 10 | 28.33 | 0.18 |
| Example 78 | 99.89 | 2-Methyl-1-butyl methacrylate | 103 | DMTBP | 10 | 27.88 | 0.17 |
| Example 79 | 99.80 | 2-Methyl-1-butyl methacrylate | 1024 | DMTBP | 10 | 29.65 | 0.23 |
| Example 80 | 98.90 | 2-Methyl-1-butyl methacrylate | 10009 | DMTBP | 10 | 31.09 | 0.31 |
| Example 81 | 95.41 | 2-Methyl-1-butyl methacrylate | 45001 | DMTBP | 10 | 26.57 | 0.34 |
| Example 82 | 99.89 | 2-Methyl-1-butyl methacrylate | 10 | DMTBP | 101 | 31.85 | 0.17 |
| Example 83 | 99.90 | 2-Methyl-1-butyl methacrylate | 10 | MEHQ | 10 | 32.32 | 0.19 |
| Example 84 | 99.90 | 2-Methyl-1-butyl methacrylate | 10 | - | - | 30.61 | 0.23 |
| Comparative Example 10 | 99.91 | - | - | - | - | 36.63 | 2.36 |
| Comparative Example 11 | 99.89 | - | - | DMTBP | 11 | 34.72 | 2.18 |

As shown in Table 10, in the ester compound-containing compositions of Examples 77 to 84 in which the component A was contained, the generated amounts of the BMA dimer and the butyl pyruvate after the storage were smaller than those of the ester compound-containing compositions of Comparative Examples 10 and 11 in which the component A was not contained.

[Example 85]

0.020 g of isobutyl isobutyrate as a component A was added to 10.001 g of isobutyl methacrylate (IBMA, manufactured by Tokyo Chemical Industry Co., Ltd., moisture content: 110 ppm by mass) to prepare an A solution.

In addition, 0.020 g of 2,4-dimethyl-6-t-butylphenol as a component B was added to 10.000 g of IBMA (moisture content: 110 ppm by mass) to prepare a B solution.

Next, 0.051 g of the A solution and 0.051 g of the B solution were added to 9.853 g of IBMA (moisture content: 110 ppm by mass) to prepare an ester compound-containing composition.

The obtained ester compound-containing composition was stored at 25°C for 14 days under white LED illumination, a contained amount (mg/L) of an IBMA dimer and a contained amount (mg/L) of isobutyl pyruvate were determined by the GC-MS measurement using the absolute calibration curve method, and the amount of change (generated amount) before and after the storage was calculated.

[Examples 86 to 102 and Comparative Examples 12 to 15]

An ester compound-containing composition was prepared in the same manner as in Example 85, except that the formulations of the A solution, the B solution, and the ester compound-containing composition were changed as shown in Table 11, and the generated amounts of the IBMA dimer and the isobutyl pyruvate were obtained.

[Table 11]

| | A solution | | | | B solution | | | | Ester compound-containing composition | | | |
|---|---|---|---|---|---|---|---|---|---|---|---|---|
| | IBMA [g] | Component A | | | IBMA [g] | Component B | | | IBMA [g] | A solution [g] | B solution [g] | Isobutanol [g] |
| | | Compound name | Addition amount [g] | Contained amount [ppm by mass] | | Compound name | Addition amount [g] | Contained amount [ppm by mass] | | | | |
| Example 85 | 10.001 | Isobutyl isobutyrate | 0.020 | 2036 | 10.000 | DMTBP | 0.020 | 2016 | 9.853 | 0.051 | 0.051 | - |
| Example 86 | 15.005 | Ethylbenzene | 0.030 | 1995 | 12.494 | DMTBP | 0.025 | 1997 | 9.850 | 0.052 | 0.051 | - |
| Example 87 | 15.813 | tert-Butvlbenzene | 0.032 | 1994 | 12.494 | DMTBP | 0.025 | 1997 | 9.852 | 0.051 | 0.054 | - |
| Example 88 | 15.005 | Ethylbenzene | 0.030 | 1995 | 12.494 | DMTBP | 0.025 | 1997 | 9.429 | 0.500 | 0.054 | - |
| Example 89 | 9.991 | Ethylbenzene | 0.999 | 90877 | 12.494 | DMTBP | 0.025 | 1997 | 8.855 | 1.099 | 0.053 | - |
| Example 90 | 15.005 | Ethylbenzene | 0.030 | 1995 | 12.494 | DMTBP | 0.025 | 1997 | 9.430 | 0.052 | 0.505 | - |
| Example 91 | 15.005 | Ethylbenzene | 0.030 | 1995 | 12.494 | DMTBP | 0.025 | 1997 | 4.936 | 0.055 | 5.003 | - |
| Example 92 | 15.005 | Ethylbenzene | 0.030 | 1995 | 10.000 | DMTBP | 1.000 | 90909 | 8.846 | 0.051 | 1.098 | - |
| Example 93 | 15.005 | Ethylbenzene | 0.030 | 1995 | 10.000 | - | - | - | 9.898 | 0.053 | - | - |
| Example 94 | 13.301 | Toluene | 0.027 | 1996 | 12.494 | DMTBP | 0.025 | 1997 | 9.431 | 0.501 | 0.052 | - |
| Example 95 | 9.991 | Ethylbenzene | 0.999 | 90877 | 12.494 | DMTBP | 0.025 | 1997 | 4.996 | 4.948 | 0.051 | - |
| Example 96 | 10.003 | 2-Methyl-1-butyl methacrylate | 0.021 | 2065 | 10.002 | DMTBP | 0.020 | 2015 | 9.851 | 0.051 | 0.050 | - |
| Example 97 | 10.003 | 2-Methyl-1-butyl methacrylate | 0.021 | 2065 | 10.002 | DMTBP | 0.020 | 2015 | 9.437 | 0.502 | 0.050 | - |
| Example 98 | 10.003 | 2-Methyl-1-butyl methacrylate | 0.021 | 2065 | 10.002 | DMTBP | 0.020 | 2015 | 4.931 | 5.003 | 0.051 | - |
| Example 99 | 10.001 | 2-Methyl-1-butyl methacrylate | 1.001 | 90959 | 10.002 | DMTBP | 0.020 | 2015 | 5.005 | 4.951 | 0.051 | - |

(continued)

| | A solution | | | | B solution | | | | Ester compound-containing composition | | | |
|---|---|---|---|---|---|---|---|---|---|---|---|---|
| | IBMA [g] | Component A | | | IBMA [g] | Component B | | | IBMA [g] | A solution [g] | B solution [g] | Isobutanol [g] |
| | | Compound name | Addition amount [g] | Contained amount [ppm by mass] | | Compound name | Addition amount [g] | Contained amount [ppm by mass] | | | | |
| Example 100 | 10.003 | 2-Methyl-1-butyl methacrylate | 0.021 | 2065 | 10.002 | DMTBP | 0.020 | 2015 | 9.439 | 0.050 | 0.502 | - |
| Example 101 | 10.003 | 2-Methyl-1-butyl methacrylate | 0.021 | 2065 | 10.001 | MEHQ | 0.020 | 2036 | 9.854 | 0.051 | 0.050 | - |
| Example 102 | 10.003 | 2-Methyl-1-butyl methacrylate | 0.021 | 2065 | 10.000 | - | - | - | 9.901 | 0.051 | - | - |
| Comparative Example 12 | 15.005 | Ethylbenzene | 0.030 | 1995 | 12.494 | DMTBP | 0.025 | 1997 | 9.392 | 0.048 | 0.051 | 0.501 |
| Comparative Example 13 | 10.003 | 2-Methyl-1-butyl methacrylate | 0.021 | 2065 | 10.002 | DMTBP | 0.020 | 2015 | 9.417 | 0.050 | 0.050 | 0.501 |
| Comparative Example 14 | 10.000 | - | - | - | 10.000 | - | - | - | 10.005 | - | - | - |
| Comparative Example 15 | 10.000 | - | - | - | 12.494 | DMTBP | 0.025 | 1997 | 9.905 | - | 0.051 | - |

Abbreviations in Table 11 have the following meanings.

· IBMA: isobutyl methacrylate

· DMTBP: 2,4-dimethyl-6-t-butylphenol

· MEHQ: 4-methoxyphenol

Table 12 shows the generated amounts of the IBMA dimer and the isobutyl pyruvate in each example.

[Table 12]

| | Formulation of ester compound-containing composition (calculated value) | | | | | | | Analysis result of ester compound-containing composition after storage | |
|---|---|---|---|---|---|---|---|---|---|
| | IBMA [ % by mass] | Component A compound name | Component A [ppm by mass] | Component B compound name | Component B [ppm by mass] | Component C compound name | Component C [ppm by mass] | Generation amount of IBMA dimer [mg/L] | Generation amount of isobutyl pyruvate [me/L] |
| Example 85 | 99.69 | Isobutyl isobutyrate | 10 | DMTBP | 10 | - | - | 2.30 | 2.38 |
| Example 86 | 99.69 | Ethylbenzene | 10 | DMTBP | 10 | - | - | 2.30 | 2.93 |
| Example 87 | 99.69 | tert-Butylbenzene | 10 | DMTBP | 11 | - | - | 1.99 | 2.98 |
| Example 88 | 99.68 | Ethylbenzene | 100 | DMTBP | 11 | - | - | 2.21 | 2.88 |
| Example 89 | 98.69 | Ethylbenzene | 9979 | DMTBP | 11 | - | - | 2.42 | 2.86 |
| Example 90 | 99.68 | Ethylbenzene | 10 | DMTBP | 101 | - | - | 2.34 | 2.27 |
| Example 91 | 99.59 | Ethylbenzene | 11 | DMTBP | 1000 | - | - | 2.81 | 1.50 |
| Example 92 | 98.69 | Ethylbenzene | 10 | DMTBP | 9987 | - | - | 2.89 | 0.86 |
| Example 93 | 99.69 | Ethylbenzene | 11 | - | - | - | - | 2.90 | 2.93 |
| Example 94 | 99.68 | Toluene | 100 | DMTBP | 10 | - | - | 2.80 | 2.85 |
| Example 95 | 95.20 | Ethylbenzene | 44990 | DMTBP | 10 | - | - | 2.64 | 2.90 |
| Example 96 | 99.69 | 2-Methyl-1-butyl methacrylate | 10 | DMTBP | 10 | - | - | 1.28 | 2.37 |
| Example 97 | 99.68 | 2-Methyl-1-butyl methacrylate | 104 | DMTBP | 10 | - | - | 2.42 | 1.61 |
| Example 98 | 99.58 | 2-Methyl-1-butyl methacrylate | 1035 | DMTBP | 10 | - | - | 2.74 | 1.81 |
| Example 99 | 95.20 | 2-Methyl-1-butyl methacrylate | 45004 | DMTBP | 10 | - | - | 2.77 | 1.77 |

(continued)

| | Formulation of ester compound-containing composition (calculated value) | | | | | | | Analysis result of ester compound-containing composition after storage | |
|---|---|---|---|---|---|---|---|---|---|
| | IBMA [ % by mass] | Component A compound name | Component A [ppm by mass] | Component B compound name | Component B [ppm by mass] | Component C compound name | Component C [ppm by mass] | Generation amount of IBMA dimer [mg/L] | Generation amount of isobutyl pyruvate [me/L] |
| Example 100 | 99.68 | 2-Methyl-1-butyl methacrylate | 10 | DMTBP | 101 | - | - | 2.77 | 1.49 |
| Example 101 | 99.69 | 2-Methyl-1-butyl methacrylate | 10 | MEHQ | 10 | - | - | 2.58 | 1.67 |
| Example 102 | 99.69 | 2-Methyl-1-butyl methacrylate | 11 | - | - | - | - | 2.78 | 1.58 |
| Comparative Example 12 | 94.69 | Ethylbenzene | 10 | DMTBP | 10 | Isobutanol | 50121 | 2.49 | 5.88 |
| Comparative Example 13 | 94.70 | 2-Methyl-1-butyl methacrylate | 10 | DMTBP | 10 | Isobutanol | 50010 | 2.35 | 5.55 |
| Comparative Example 14 | 99.69 | - | - | - | - | - | - | 2.90 | 3.13 |
| Comparative Example 15 | 99.69 | - | - | DMTBP | 10 | - | - | 3.09 | 3.03 |

As shown in Table 12, in the ester compound-containing compositions of Examples 85 to 102 in which the contained amount of IBMA was 95% by mass or more and the component A was contained, the generated amounts of the IBMA dimer and the isobutyl pyruvate after the storage were smaller than those of the ester compound-containing compositions of Comparative Examples 12 to 15 in which the contained amount of IBMA was less than 95% by mass or the component A was not contained.

[Example 103]

0.021 g of o-xylene as a component A was added to 10.002 g of isobutyl methacrylate (IBMA, manufactured by Tokyo Chemical Industry Co., Ltd., moisture content: 110 ppm by mass) to prepare an A solution.

In addition, 0.020 g of 2,4-dimethyl-6-t-butylphenol as a component B was added to 10.001 g of IBMA (moisture content: 110 ppm by mass) to prepare a B solution.

Next, 0.050 g of the A solution and 0.051 g of the B solution were added to 9.852 g of IBMA (moisture content: 110 ppm by mass) to prepare an ester compound-containing composition.

The obtained ester compound-containing composition was stored at 70°C for 20 hours under white LED illumination, a contained amount (mg/L) of butyl pyruvate was determined by the GC-MS measurement using the absolute calibration curve method, and the amount of change (generated amount) before and after the storage was calculated.

[Examples 104 to 115 and Comparative Examples 16 to 19]

An ester compound-containing composition was prepared in the same manner as in Example 103, except that the formulations of the A solution, the B solution, and the ester compound-containing composition were changed as shown in Table 13, and the generated amount of the isobutyl pyruvate was obtained.

[Table 13]

| | A solution | | | | B solution | | | | Ester compound-containing composition | | | |
|---|---|---|---|---|---|---|---|---|---|---|---|---|
| | IBMA [g] | Component A | | | IBMA [g] | Component B | | | IBMA [g] | A solution [g] | B solution [g] | Isobutanol [g] |
| | | Compound name | Addition amount [g] | Contained amount [ppm by mass] | | Compound name | Addition amount [g] | Contained amount [ppm by mass] | | | | |
| Example 103 | 10.002 | o-Xylene | 0.021 | 2045 | 10.001 | DMTBP | 0.020 | 2016 | 9.852 | 0.050 | 0.051 | - |
| Example 104 | 10.002 | o-Xylene | 0.021 | 2045 | 10.001 | DMTBP | 0.020 | 2016 | 9.435 | 0.502 | 0.050 | - |
| Example 105 | 10.002 | o-Xylene | 0.021 | 2045 | 10.001 | DMTBP | 0.020 | 2016 | 4.932 | 5.002 | 0.050 | - |
| Example 106 | 10.001 | o-Xylene | 1.000 | 90906 | 10.001 | DMTBP | 0.020 | 2016 | 8.853 | 1.101 | 0.050 | - |
| Example 107 | 10.001 | o-Xylene | 1.000 | 90906 | 10.001 | DMTBP | 0.020 | 2016 | 5.029 | 4.957 | 0.050 | - |
| Example 108 | 10.002 | o-Xylene | 0.021 | 2045 | 10.000 | - | - | - | 9.901 | 0.050 | - | - |
| Example 109 | 10.000 | m-Xylene | 0.020 | 2026 | 10.001 | DMTBP | 0.020 | 2016 | 9.851 | 0.051 | 0.051 | - |
| Example 110 | 10.000 | m-Xylene | 0.020 | 2026 | 10.001 | DMTBP | 0.020 | 2016 | 9.436 | 0.501 | 0.050 | - |
| Example 111 | 10.000 | m-Xylene | 0.020 | 2026 | 10.001 | DMTBP | 0.020 | 2016 | 4.999 | 5.001 | 0.050 | - |
| Example 112 | 10.001 | m-Xylene | 1.000 | 90921 | 10.001 | DMTBP | 0.020 | 2016 | 8.852 | 1.102 | 0.051 | - |
| Example 113 | 10.001 | m-Xylene | 1.000 | 90921 | 10.001 | DMTBP | 0.020 | 2016 | 5.002 | 4.951 | 0.051 | - |
| Example 114 | 10.000 | m-Xylene | 0.020 | 2026 | 10.001 | DMTBP | 0.020 | 2016 | 9.401 | 0.050 | 0.050 | 0.051 |
| Example 115 | 10.000 | m-Xylene | 0.020 | 2026 | 10.000 | - | - | - | 9.902 | 0.050 | - | - |
| Comparative Example 16 | 10.002 | o-Xylene | 0.021 | 2045 | 10.001 | DMTBP | 0.020 | 2016 | 9.400 | 0.051 | 0.051 | 0.501 |
| Comparative Example 17 | 10.002 | m-Xylene | 0.020 | 2026 | 10.003 | DMTBP | 0.020 | 2005 | 9.406 | 0.050 | 0.051 | 0.501 |
| Comparative Example 18 | 10.000 | - | - | - | 10.000 | - | - | - | 10.005 | - | - | - |
| Comparative Example 19 | 10.000 | - | - | - | 12.494 | DMTBP | 0.025 | 1997 | 9.905 | - | 0.051 | - |

Abbreviations in Table 13 have the following meanings.

· IBMA: isobutyl methacrylate

· DMTBP: 2,4-dimethyl-6-t-butylphenol

Table 14 shows the generated amount of the isobutyl pyruvate in each example.

[Table 14]

| | Formulation of ester compound-containing composition (calculated value) | | | | | | | Analysis result of ester compound-containing composition after storage |
|---|---|---|---|---|---|---|---|---|
| | IBMA [% by mass] | Component A compound name | Component A [ppm by mass] | Component B compound name | Component B [ppm by mass] | Component C compound name | Component C [ppm by mass] | Generation amount of isobutyl pyruvate [mg/L] |
| Example 103 | 99.69 | o-Xylene | 10 | DMTBP | 10 | - | - | 0.48 |
| Example 104 | 99.68 | o-Xylene | 103 | DMTBP | 10 | - | - | 0.33 |
| Example 105 | 99.59 | o-Xylene | 1025 | DMTBP | 10 | - | - | 0.40 |
| Example 106 | 98.69 | o-Xylene | 10001 | DMTBP | 10 | - | - | 0.28 |
| Example 107 | 95.21 | o-Xylene | 44899 | DMTBP | 10 | - | - | 0.39 |
| Example 108 | 99.69 | o-Xylene | 10 | - | - | - | - | 0.38 |
| Example 109 | 99.69 | m-Xylene | 10 | DMTBP | 10 | - | - | 0.33 |
| Example 110 | 99.68 | m-Xylene | 102 | DMTBP | 10 | - | - | 0.33 |
| Example 111 | 99.59 | m-Xylene | 1008 | DMTBP | 10 | - | - | 0.42 |
| Example 112 | 98.69 | m-Xylene | 10017 | DMTBP | 10 | - | - | 0.29 |
| Example 113 | 95.20 | m-Xylene | 44998 | DMTBP | 10 | - | - | 0.33 |
| Example 114 | 99.15 | m-Xylene | 11 | DMTBP | 11 | Isobutanol | 5339 | 1.16 |
| Example 115 | 99.69 | m-Xylene | 10 | - | - | - | - | 0.33 |
| Comparative Example 16 | 94.69 | o-Xylene | 10 | DMTBP | 10 | Isobutanol | 50098 | 7.02 |
| Comparative Example 17 | 94.70 | m-Xylene | 10 | DMTBP | 10 | Isobutanol | 50062 | 6.72 |
| Comparative Example 18 | 99.69 | - | - | - | - | - | - | 1.83 |
| Comparative Example 19 | 99.69 | - | - | DMTBP | 10 | - | - | 1.57 |

As shown in Table 14, in the ester compound-containing compositions of Examples 103 to 115 in which the contained amount of IBMA was 95% by mass or more and the component A was contained, the generated amount of the isobutyl pyruvate after the storage was smaller than that of the ester compound-containing compositions of Comparative Examples 16 to 19 in which the contained amount of IBMA was less than 95% by mass or the component A was not contained.

[Example 116]

0.020 g of butyl acetate as a component A was added to 10.029 g of ethylene glycol dimethacrylate (EDMA, manufactured by Tokyo Chemical Industry Co., Ltd., moisture content: 91 ppm by mass) to prepare an A solution.
In addition, 0.024 g of 2,4-dimethyl-6-t-butylphenol as a component B was added to 10.014 g of EDMA (moisture content: 91 ppm by mass) to prepare a B solution.
Next, 0.033 g of the A solution and 0.032 g of the B solution were added to 5.005 g of EDMA (moisture content: 91 ppm by mass) to prepare an ester compound-containing composition.
The obtained ester compound-containing composition was stored at 25°C for 14 days under fluorescent lamp illumination, and a purity (%) of EDMA was calculated from the proportion (area%) of an area value of EDMA to the total area value of all components detected by the GC-MS measurement. In the calculation, a value obtained by subtracting the moisture content (% by mass) from 100% was multiplied by the area% of EDMA to calculate the purity of EDMA.

[Example 117 and Comparative Examples 19 to 21]

An ester compound-containing composition was prepared in the same manner as in Example 116, except that the formulations of the A solution, the B solution, and the ester compound-containing composition were changed as shown in Table 15, and the purity of EDMA was obtained.

[Table 15]

| | A solution | | | | B solution | | | | Ester compound-containing composition | | |
|---|---|---|---|---|---|---|---|---|---|---|---|
| | EDMA [g] | Component A | | | EDMA [g] | Component B | | | EDMA [g] | A solution [g] | B solution [g] |
| | | Compound name | Addition amount [g] | Contained amount [ppm by mass] | | Compound name | Addition amount [g] | Contained amount [ppm by mass] | | | |
| Example 116 | 10.029 | Butyl butyrate | 0.020 | 1994 | 10.014 | DMTBP | 0.024 | 2347 | 5.005 | 0.033 | 0.032 |
| Example 117 | 10.029 | Butyl butyrate | 0.020 | 1994 | 10.014 | DMTBP | 0.024 | 2347 | 5.005 | 0.064 | 0.032 |
| Comparative Example 19 | - | - | - | - | 10.014 | DMTBP | 0.024 | 2347 | 5.012 | - | 0.030 |
| Comparative Example 20 | - | - | - | - | 10.011 | DMTBP | 0.024 | 2377 | 5.019 | - | 0.066 |
| Comparative Example 21 | - | - | - | - | - | - | - | - | 5.016 | - | - |

Abbreviations in Table 15 have the following meanings.

· EDMA: ethylene glycol dimethacrylate

· DMTBP: 2,4-dimethyl-6-t-butylphenol

Table 16 shows the measurement results of the purity of EDMA in each example.

[Table 16]

| | Formulation of ester compound-containing composition (calculated value) | | | | | Analysis result of ester compound-containing composition after storage |
|---|---|---|---|---|---|---|
| | EDMA [% by mass] | Component A compound name | Component A [ppm by mass] | Component B compound name | Component B [ppm by mass] | Purity of EDMA [%] |
| Example 116 | 99.98 | Butyl butyrate | 13 | DMTBP | 15 | 92.13 |
| Example 117 | 99.98 | Butyl butyrate | 25 | DMTBP | 15 | 92.14 |
| Comparative Example 19 | 99.98 | - | - | DMTBP | 14 | 91.22 |
| Comparative Example 20 | 99.98 | - | - | DMTBP | 31 | 91.05 |
| Comparative Example 21 | 99.98 | - | - | - | - | 91.43 |

As shown in Table 16, in the ester compound-containing compositions of Examples 116 and 117 in which the component A was contained, the purity of EDMA after the storage was higher than that of the ester compound-containing compositions of Comparative Examples 19 to 21 in which the component A was not contained, and it can be said that quality stability during storage was high.

[Example 118 and Comparative Examples 22 to 24]

An ester compound-containing composition was prepared in the same manner as in Example 116, except that 2-methoxyethyl methacrylate (MTMA, moisture content: 575 ppm by mass) was used instead of the EDMA and the formulations of the A solution, the B solution, and the ester compound-containing composition were changed as shown in Table 17, and the purity of MTMA was obtained.

[Table 17]

| | A solution | | | | B solution | | | | Ester compound-containing composition | | |
|---|---|---|---|---|---|---|---|---|---|---|---|
| | MTMA [g] | Component A | | | MTMA [g] | Component B | | | MTMA [g] | A solution [g] | B solution [g] |
| | | Compound name | Addition amount [g] | Contained amount [ppm by mass] | | Compound name | Addition amount [g] | Contained amount [ppm by mass] | | | |
| Example 118 | 10.011 | Butyl butyrate | 0.023 | 2248 | 10.019 | DMTBP | 0.247 | 24653 | 5.014 | 0.035 | 0.030 |
| Comparative Example 22 | - | - | - | - | 10.007 | DMTBP | 0.247 | 24683 | 5.016 | - | 0.053 |
| Comparative Example 23 | - | - | - | - | 10.019 | DMTBP | 0.247 | 24653 | 5.016 | - | 0.059 |
| Comparative Example 24 | - | - | - | - | - | - | - | - | 5.021 | - | - |

Table 18 shows the measurement results of the purity of MTMA in each example.

[Table 18]

| | Formulation of ester compound-containing composition (calculated value) | | | | | Analysis result of ester compound-containing composition after storage |
|---|---|---|---|---|---|---|
| | MTMA [% by mass] | Component A compound name | Component A [ppm by mass] | Component B compound name | Component B [ppm by mass] | Purity of MTMA [%] |
| Example 118 | 99.87 | Butyl butyrate | 16 | DMTBP | 15 | 99.34 |
| Comparative Example 22 | 99.86 | - | - | DMTBP | 26 | 99.19 |
| Comparative Example 23 | 99.86 | - | - | DMTBP | 29 | 99.17 |
| Comparative Example 24 | 99.89 | - | - | - | - | 99.22 |

As shown in Table 18, in the ester compound-containing compositions of Example 118 in which the component A was contained, the purity of MTMA after the storage was higher than that of the ester compound-containing compositions of Comparative Examples 22 to 24 in which the component A was not contained, and it can be said that quality stability during storage was high.

[Example 119]

0.024 g of 2-ethylhexyl acrylate as a component A was added to 10.007 g of ethylene glycol dimethacrylate (EDMA, manufactured by Tokyo Chemical Industry Co., Ltd., moisture content: 91 ppm by mass) to prepare an A solution.

In addition, 0.024 g of 2,4-dimethyl-6-t-butylphenol as a component B was added to 5.006 g of EDMA (moisture content: 91 ppm by mass) to prepare a B solution.

Next, 0.032 g of the A solution and 0.030 g of the B solution were added to 5.006 g of EDMA (moisture content: 91 ppm by mass) to prepare an ester compound-containing composition.

The obtained ester compound-containing composition was stored at 25°C for 14 days under fluorescent lamp illumination, and a purity (%) of EDMA was calculated from the proportion (area%) of an area value of EDMA to the total area value of all components detected by the GC-MS measurement. In the calculation, a value obtained by subtracting the moisture content (% by mass) from 100% was multiplied by the area% of EDMA to calculate the purity of EDMA.

[Examples 120 to 123 and Comparative Examples 25 to 27]

An ester compound-containing composition was prepared in the same manner as in Example 119, except that the formulations of the A solution, the B solution, and the ester compound-containing composition were changed as shown in Table 19, and the purity of EDMA was obtained.

[Table 19]

| | A solution | | | | B solution | | | | Ester compound-containing composition | | |
|---|---|---|---|---|---|---|---|---|---|---|---|
| | EDMA [g] | Component A | | | EDMA [g] | Component B | | | EDMA [g] | A solution [g] | B solution [g] |
| | | Compound name | Addition amount [g] | Contained amount [ppm by mass] | | Compound name | Addition amount [g] | Contained amount [ppm by mass] | | | |
| Example 119 | 10.007 | 2-Ethylhexyl acrylate | 0.024 | 2358 | 5.006 | DMTBP | 0.024 | 2377 | 5.006 | 0.032 | 0.030 |
| Example 120 | 10.007 | 2-Ethylhexyl acrylate | 0.024 | 2358 | 5.006 | DMTBP | 0.024 | 2377 | 5.006 | 0.032 | 0.062 |
| Example 121 | 10.010 | Butyl crotonate | 0.024 | 2368 | 5.012 | DMTBP | 0.024 | 2377 | 5.012 | 0.031 | 0.032 |
| Example 122 | 10.006 | Methacrylamide | 0.023 | 2279 | 5.008 | DMTBP | 0.024 | 2377 | 5.008 | 0.065 | 0.060 |
| Example 123 | 10.007 | 2-Ethylhexyl acrylate | 0.024 | 2358 | 5.008 | - | - | - | 5.008 | 0.030 | - |
| Comparative Example 25 | - | - | - | - | 5.012 | DMTBP | 0.024 | 2347 | 5.012 | - | 0.033 |
| Comparative Example 26 | - | - | - | - | 5.004 | DMTBP | 0.024 | 2346 | 5.004 | - | 0.061 |
| Comparative Example 27 | - | - | - | - | 5.033 | DMTBP | - | - | 5.033 | - | - |

Table 20 shows the measurement results of the purity of EDMA in each example.

[Table 20]

| | Formulation of ester compound-containing composition (calculated value) | | | | | Analysis result of ester compound-containing composition after storage |
|---|---|---|---|---|---|---|
| | EDMA [% by mass] | Component A compound name | Component A [ppm by mass] | Component B compound name | Component B [ppm by mass] | Purity of EDMA [%] |
| Example 119 | 99.98 | 2-Ethylhexyl acrylate | 15 | DMTBP | 14 | 91.92 |
| Example 120 | 99.98 | 2-Ethylhexyl acrylate | 15 | DMTBP | 29 | 91.33 |
| Example 121 | 99.98 | Butyl crotonate | 14 | DMTBP | 15 | 91.26 |
| Example 122 | 99.98 | Methacrylamide | 29 | DMTBP | 28 | 91.14 |
| Example 123 | 99.98 | 2-Ethylhexyl acrylate | 14 | DMTBP | - | 91.42 |
| Comparative Example 25 | 99.98 | - | - | DMTBP | 15 | 90.40 |
| Comparative Example 26 | 99.98 | - | - | DMTBP | 28 | 90.83 |
| Comparative Example 27 | 99.98 | - | - | DMTBP | - | 90.94 |

As shown in Table 20, in the ester compound-containing compositions of Examples 119 to 123 in which the component A was contained, the purity of EDMA after the storage was higher than that of the ester compound-containing compositions of Comparative Examples 25 to 27 in which the component A was not contained, and it can be said that quality stability during storage was high.

[Example 124]

0.028 g of t-butylphenyl ether as a component A was added to 10.009 g of ethylene glycol dimethacrylate (EDMA, manufactured by Tokyo Chemical Industry Co., Ltd., moisture content: 91 ppm by mass) to prepare an A solution.
In addition, 0.024 g of 2,4-dimethyl-6-t-butylphenol as a component B was added to 10.017 g of EDMA (moisture content: 91 ppm by mass) to prepare a B solution.
Next, 0.030 g of the A solution and 0.031 g of the B solution were added to 5.016 g of EDMA (moisture content: 91 ppm by mass) to prepare an ester compound-containing composition.
The obtained ester compound-containing composition was stored at 25°C for 14 days under fluorescent lamp illumination, and a purity (%) of EDMA was calculated from the proportion (area%) of an area value of EDMA to the total area value of all components detected by the GC-MS measurement. In the calculation, a value obtained by subtracting the moisture content (% by mass) from 100% was multiplied by the area% of EDMA to calculate the purity of EDMA.

[Examples 125 to 128 and Comparative Examples 28 and 29]

An ester compound-containing composition was prepared in the same manner as in Example 124, except that the formulations of the A solution, the B solution, and the ester compound-containing composition were changed as shown in Table 21, and the purity of EDMA was obtained.

[Table 21]

| | A solution | | | | B solution | | | | Ester compound-containing composition | | |
|---|---|---|---|---|---|---|---|---|---|---|---|
| | EDMA [g] | Component A | | | EDMA [g] | Component B | | | EDMA [g] | A solution [g] | B solution [g] |
| | | Compound name | Addition amount [g] | Contained amount [ppm by mass] | | Compound name | Addition amount [g] | Contained amount [ppm by mass] | | | |
| Example 124 | 10.009 | t-Butyl phenyl ether | 0.028 | 2837 | 10.017 | DMTBP | 0.024 | 2346 | 5.016 | 0.030 | 0.031 |
| Example 125 | 10.009 | t-Butyl phenyl ether | 0.028 | 2837 | 10.017 | DMTBP | 0.024 | 2346 | 5.013 | 0.063 | 0.064 |
| Example 126 | 10.018 | Isopropoxybenzene | 0.023 | 2266 | 10.017 | DMTBP | 0.024 | 2346 | 5.017 | 0.031 | 0.030 |
| Example 127 | 10.009 | t-Butyl phenyl ether | 0.028 | 2837 | 10.017 | DMTBP | 0.024 | 2346 | 4.742 | 0.254 | 0.030 |
| Example 128 | 10.009 | t-Butyl phenyl ether | 0.028 | 2837 | - | - | - | - | 5.005 | 0.031 | - |
| Comparative Example 28 | - | - | - | - | 10.014 | DMTBP | 0.024 | 2347 | 5.012 | - | 0.033 |
| Comparative Example 29 | - | - | - | - | 10.017 | DMTBP | 0.024 | 2346 | 5.004 | - | 0.061 |

Abbreviations in Table 21 have the following meanings.

· EDMA: ethylene glycol dimethacrylate

· DMTBP: 2,4-dimethyl-6-t-butylphenol

Table 22 shows the measurement results of the purity of EDMA in each example.

[Table 22]

| | Formulation of ester compound-containing composition (calculated value) | | | | | Analysis result of ester compound-containing composition after storage |
|---|---|---|---|---|---|---|
| | EDMA [% by mass] | Component A compound name | Component A [ppm by mass] | Component B compound name | Component B [ppm by mass] | Purity of EDMA [%] |
| Example 124 | 99.98 | t-Butyl phenyl ether | 17 | DMTBP | 15 | 91.13 |
| Example 125 | 99.98 | t-Butyl phenyl ether | 35 | DMTBP | 29 | 90.93 |
| Example 126 | 99.98 | Isopropoxybenzene | 14 | DMTBP | 14 | 90.98 |
| Example 127 | 99.97 | t-Butyl phenyl ether | 143 | DMTBP | 14 | 90.68 |
| Example 128 | 99.98 | t-Butyl phenyl ether | 18 | DMTBP | - | 90.93 |
| Comparative Example 28 | 99.98 | - | - | DMTBP | 15 | 90.40 |
| Comparative Example 29 | 99.98 | - | - | DMTBP | 28 | 90.83 |

As shown in Table 22, in the ester compound-containing compositions of Examples 124 to 128 in which the component A was contained, the purity of EDMA after the storage was higher than that of the ester compound-containing compositions of Comparative Examples 28 and 29 in which the component A was not contained.

[Examples 129 to 132 and Comparative Examples 30 to 32]

An ester compound-containing composition was prepared in the same manner as in Example 124, except that 2-methoxyethyl methacrylate (MTMA, moisture content: 575 ppm by mass) was used instead of the EDMA and the formulations of the A solution, the B solution, and the ester compound-containing composition were changed as shown in Table 23, and the purity of MTMA was obtained.

[Table 23]

| | A solution | | | | B solution | | | | Ester compound-containing composition | | |
|---|---|---|---|---|---|---|---|---|---|---|---|
| | MTMA [g] | Component A | | | MTMA [g] | Component B | | | MTMA [g] | A solution [g] | B solution [g] |
| | | Compound name | Addition amount [g] | Contained amount [ppm by mass] | | Compound name | Addition amount [g] | Contained amount [ppm by mass] | | | |
| Example 129 | 10.026 | t-Butyl phenyl ether | 0.024 | 2364 | 10.022 | DMTBP | 0.024 | 2345 | 5.011 | 0.030 | 0.036 |
| Example 130 | 10.026 | t-Butyl phenyl ether | 0.024 | 2364 | 10.022 | DMTBP | 0.024 | 2345 | 5.028 | 0.063 | 0.065 |
| Example 131 | 10.013 | Isopropoxybenzene | 0.023 | 2327 | 10.022 | DMTBP | 0.024 | 2345 | 5.001 | 0.036 | 0.061 |
| Example 132 | 10.026 | t-Butyl phenyl ether | 0.024 | 2364 | - | - | - | - | 5.015 | 0.030 | - |
| Comparative Example 30 | - | - | - | - | 10.007 | DMTBP | 0.024 | 2348 | 5.016 | - | 0.053 |
| Comparative Example 31 | - | - | - | - | 10.022 | DMTBP | 0.024 | 2345 | 5.016 | - | 0.065 |
| Comparative Example 32 | - | - | - | - | - | - | - | - | 5.021 | - | - |

Table 24 shows the measurement results of the purity of MTMA in each example.

[Table 24]

| | Formulation of ester compound-containing composition (calculated value) | | | | | Analysis result of ester compound-containing composition after storage |
|---|---|---|---|---|---|---|
| | MTMA [% by mass] | Component A compound name | Component A [ppm by mass] | Component B compound name | Component B [ppm by mass] | Purity of MTMA [%] |
| Example 129 | 99.98 | t-Butyl phenyl ether | 14 | DMTBP | 17 | 99.43 |
| Example 130 | 99.98 | t-Butyl phenyl ether | 29 | DMTBP | 30 | 99.38 |
| Example 131 | 99.98 | Isopropoxybenzene | 16 | DMTBP | 28 | 99.35 |
| Example 132 | 99.98 | t-Butyl phenyl ether | 14 | - | - | 99.41 |
| Comparative Example 30 | 99.98 | - | - | DMTBP | 24 | 99.24 |
| Comparative Example 31 | 99.98 | - | - | DMTBP | 30 | 99.22 |
| Comparative Example 32 | 99.98 | - | - | - | - | 99.27 |

As shown in Table 24, in the ester compound-containing compositions of Examples 129 to 132 in which the component A was contained, the purity of MTMA after the storage was higher than that of the ester compound-containing compositions of Comparative Examples 30 to 32 in which the component A was not contained.

[Example 134]

0.023 g of 2-phenylisobutyric acid as a component A was added to 10.030 g of ethylene glycol dimethacrylate (EDMA, manufactured by Tokyo Chemical Industry Co., Ltd., moisture content: 91 ppm by mass) to prepare an A solution.
In addition, 0.020 g of 2,4-dimethyl-6-t-butylphenol as a component B was added to 10.007 g of EDMA (moisture content: 91 ppm by mass) to prepare a B solution.
Next, 0.031 g of the A solution and 0.031 g of the B solution were added to 5.005 g of EDMA (moisture content: 91 ppm by mass) to prepare an ester compound-containing composition.
The obtained ester compound-containing composition was stored at 25°C for 14 days under fluorescent lamp illumination, and a purity (%) of EDMA was calculated from the proportion (area%) of an area value of EDMA to the total area value of all components detected by the GC-MS measurement. In the calculation, a value obtained by subtracting the moisture content (% by mass) from 100% was multiplied by the area% of EDMA to calculate the purity of EDMA.

[Examples 135 to 137 and Comparative Examples 33 to 35]

An ester compound-containing composition was prepared in the same manner as in Example 134, except that the formulations of the A solution, the B solution, and the ester compound-containing composition were changed as shown in Table 25, and the purity of EDMA was obtained.

[Table 25]

| | A solution | | | | B solution | | | | Ester compound-containing composition | | |
|---|---|---|---|---|---|---|---|---|---|---|---|
| | EDMA [g] | Component A | | | EDMA [g] | Component B | | | EDMA [g] | A solution [g] | B solution [g] |
| | | Compound name | Addition amount [g] | Contained amount [ppm by mass] | | Compound name | Addition amount [g] | Contained amount [ppm by mass] | | | |
| Example 134 | 10.030 | 2-Phenyl isobutyrate | 0.023 | 2333 | 10.007 | DMTBP | 0.020 | 2029 | 5.005 | 0.031 | 0.031 |
| Example 135 | 10.030 | 2-Phenyl isobutyrate | 0.023 | 2333 | 10.007 | DMTBP | 0.020 | 2029 | 5.021 | 0.060 | 0.059 |
| Example 136 | 10.012 | 2-Isopropyl-4-methoxyphenol | 0.031 | 3076 | 10.007 | DMTBP | 0.020 | 2029 | 5.014 | 0.635 | 0.040 |
| Example 137 | 10.030 | 2-Phenyl isobutyrate | 0.023 | 2333 | - | - | - | - | 5.009 | 0.028 | - |
| Comparative Example 33 | - | - | - | - | 10.014 | DMTBP | 0.024 | 2347 | 5.012 | - | 0.033 |
| Comparative Example 34 | - | - | - | - | 10.017 | DMTBP | 0.024 | 2346 | 5.004 | - | 0.061 |
| Comparative Example 35 | - | - | - | - | - | - | - | - | 5.033 | - | - |

Abbreviations in Table 25 have the following meanings.

· EDMA: ethylene glycol dimethacrylate

· DMTBP: 2,4-dimethyl-6-t-butylphenol

Table 26 shows the measurement results of the purity of EDMA in each example.

[Table 26]

| | Formulation of ester compound-containing composition (calculated value) | | | | | Analysis result of ester compound-containing composition after storage |
|---|---|---|---|---|---|---|
| | EDMA [% by mass] | Component A compound name | Component A [ppm by mass] | Component B compound name | Component B [ppm by mass] | Purity of EDMA [%] |
| Example 134 | 99.98 | 2-Phenyl isobutyrate | 14 | DMTBP | 12 | 91.65 |
| Example 135 | 99.98 | 2-Phenyl isobutyrate | 27 | DMTBP | 23 | 91.07 |
| Example 136 | 99.95 | 2-Isopropyl-4-methoxyphenol | 343 | DMTBP | 14 | 91.06 |
| Example 137 | 99.98 | 2-Phenyl isobutyrate | 13 | - | - | 91.22 |
| Comparative Example 33 | 99.98 | - | - | DMTBP | 15 | 90.40 |
| Comparative Example 34 | 99.98 | - | - | DMTBP | 28 | 90.83 |
| Comparative Example 35 | 99.98 | - | - | - | - | 90.94 |

As shown in Table 26, in the ester compound-containing compositions of Examples 134 to 137 in which the component A was contained, the purity of EDMA after the storage was higher than that of the ester compound-containing compositions of Comparative Examples 33 to 35 in which the component A was not contained, and it can be said that quality stability during storage was high.

[Examples 139 to 144 and Comparative Examples 36 to 38]

An ester compound-containing composition was prepared in the same manner as in Example 134, except that 2-methoxyethyl methacrylate (MTMA, moisture content: 575 ppm by mass) was used instead of the EDMA and the formulations of the A solution, the B solution, and the ester compound-containing composition were changed as shown in Table 27, and the purity of MTMA was obtained.

[Table 27]

| | A solution | | | | B solution | | | | Ester compound-containing composition | | |
|---|---|---|---|---|---|---|---|---|---|---|---|
| | MTMA [g] | Component A | | | MTMA [g] | Component B | | | MTMA [g] | A solution [g] | B solution [g] |
| | | Compound name | Addition amount [g] | Contained amount [ppm by mass] | | Compound name | Addition amount [g] | Contained amount [ppm by mass] | | | |
| Example 139 | 10.007 | 2-Phenyl isobutyrate | 0.022 | 2238 | 10.017 | DMTBP | 0.028 | 2825 | 5.012 | 0.031 | 0.031 |
| Example 140 | 10.007 | 2-Phenyl isobutyrate | 0.022 | 2238 | 10.017 | DMTBP | 0.028 | 2825 | 5.005 | 0.060 | 0.060 |
| Example 141 | 10.013 | 2-Phenylisobutyric acid methyl ester | 0.024 | 2407 | 10.017 | DMTBP | 0.028 | 2825 | 5.007 | 0.032 | 0.040 |
| Example 142 | 10.017 | 2-Isopropyl-4-methoxyphenol | 0.030 | 2945 | 10.017 | DMTBP | 0.028 | 2825 | 5.003 | 0.032 | 0.027 |
| Example 143 | 10.007 | 2-Phenyl isobutyrate | 0.022 | 2238 | 10.017 | DMTBP | 0.028 | 2825 | 4.744 | 0.257 | 0.031 |
| Example 144 | 10.007 | 2-Phenyl isobutyrate | 0.022 | 2238 | - | - | - | - | 5.001 | 0.030 | - |
| Comparative Example 36 | - | - | - | - | 10.007 | DMTBP | 0.028 | 2828 | 5.016 | - | 0.053 |
| Comparative Example 37 | - | - | - | - | 10.017 | DMTBP | 0.028 | 2825 | 5.016 | - | 0.058 |
| Comparative Example 38 | - | - | - | - | - | - | - | - | 5.021 | - | - |

Abbreviations in Table 27 have the following meanings.

· MTMA: 2-methoxyethyl methacrylate

· DMTBP: 2,4-dimethyl-6-t-butylphenol

Table 28 shows the measurement results of the purity of MTMA in each example.

[Table 28]

| | Formulation of ester compound-containing composition (calculated value) | | | | | Analysis result of ester compound-containing composition after storage |
|---|---|---|---|---|---|---|
| | MTMA [% by mass] | Component A compound name | Component A [ppm by mass] | Component B compound name | Component B [ppm by mass] | Purity of MTMA [%] |
| Example 139 | 99.98 | 2-Phenyl isobutyrate | 14 | DMTBP | 17 | 99.40 |
| Example 140 | 99.98 | 2-Phenyl isobutyrate | 26 | DMTBP | 33 | 99.37 |
| Example 141 | 99.98 | 2-Phenylisobutyric acid methyl ester | 15 | DMTBP | 22 | 99.37 |
| Example 142 | 99.98 | 2-Isopropyl-4-methoxyphenol | 19 | DMTBP | 15 | 99.38 |
| Example 143 | 99.97 | 2-Phenyl isobutyrate | 114 | DMTBP | 17 | 99.34 |
| Example 144 | 99.98 | 2-Phenyl isobutyrate | 13 | - | - | 99.41 |
| Comparative Example 36 | 99.98 | - | - | DMTBP | 29 | 99.24 |
| Comparative Example 37 | 99.98 | - | - | DMTBP | 33 | 99.22 |
| Comparative Example 38 | 99.98 | - | - | - | - | 99.27 |

As shown in Table 28, in the ester compound-containing compositions of Examples 139 to 144 in which the component A was contained, the purity of MTMA after the storage was higher than that of the ester compound-containing compositions of Comparative Examples 36 to 38 in which the component A was not contained, and it can be said that quality stability during storage was high.

[Example 145]

0.010 g of 2,3,5,6-tetramethylpyrazine as a component A was added to 5.015 g of ethylene glycol dimethacrylate (EDMA, manufactured by Tokyo Chemical Industry Co., Ltd., moisture content: 91 ppm by mass) to prepare an A solution.
In addition, 0.024 g of 2,4-dimethyl-6-t-butylphenol as a component B was added to 10.014 g of EDMA (moisture content: 91 ppm by mass) to prepare a B solution.
Next, 0.030 g of the A solution and 0.030 g of the B solution were added to 5.011 g of EDMA (moisture content: 91 ppm by mass) to prepare an ester compound-containing composition.
The obtained ester compound-containing composition was stored at 25°C for 14 days under fluorescent lamp illumination, and a purity (%) of EDMA was calculated from the proportion (area%) of an area value of EDMA to the total area value of all components detected by the GC-MS measurement. In the calculation, a value obtained by subtracting the moisture content (% by mass) from 100% was multiplied by the area% of EDMA to calculate the purity of EDMA.

[Example 146 and Comparative Examples 39 and 40]

An ester compound-containing composition was prepared in the same manner as in Example 145, except that the formulations of the A solution, the B solution, and the ester compound-containing composition were changed as shown in Table 29, and the purity of EDMA was obtained.

[Table 29]

| | A solution | | | | B solution | | | | Ester compound-containing composition | | |
|---|---|---|---|---|---|---|---|---|---|---|---|
| | EDMA [g] | Component A | | | EDMA [g] | Component B | | | EDMA [g] | A solution [g] | B solution [g] |
| | | Compound name | Addition amount [g] | Contained amount [ppm by mass] | | Compound name | Addition amount [g] | Contained amount [ppm by mass] | | | |
| Example 145 | 5.015 | 2,3,5,6-Tetramethylpyrazine | 0.010 | 2014 | 10.014 | DMTBP | 0.024 | 2347 | 5.011 | 0.030 | 0.030 |
| Example 146 | 5.015 | 2,3,5,6-Tetramethylpyrazine | 0.010 | 2014 | 10.014 | DMTBP | 0.024 | 2347 | 5.011 | 0.060 | 0.062 |
| Comparative Example 39 | - | - | - | - | 10.014 | DMTBP | 0.024 | 2347 | 5.012 | - | 0.033 |
| Comparative Example 40 | - | - | - | - | 10.017 | DMTBP | 0.024 | 2346 | 5.004 | - | 0.061 |

Abbreviations in Table 29 have the following meanings.

· EDMA: ethylene glycol dimethacrylate
· DMTBP: 2,4-dimethyl-6-t-butylphenol

Table 30 shows the measurement results of the purity of EDMA in each example.

[Table 30]

| | Formulation of ester compound-containing composition (calculated value) | | | | | Analysis result of ester compound-containing composition after storage |
|---|---|---|---|---|---|---|
| | EDMA [% by mass] | Component A compound name | Component A [ppm by mass] | Component B compound name | Component B [ppm by mass] | Purity of EDMA [%] |
| Example 145 | 99.98 | 2,3,5,6-Tetramethylpyrazine | 12 | DMTBP | 14 | 90.92 |
| Example 146 | 99.98 | 2,3,5,6-Tetramethylpyrazine | 23 | DMTBP | 28 | 91.00 |
| Comparative Example 39 | 99.98 | - | - | DMTBP | 15 | 90.40 |
| Comparative Example 40 | 99.98 | - | - | DMTBP | 28 | 90.83 |

As shown in Table 30, in the ester compound-containing compositions of Examples 145 and 146 in which the component A was contained, the purity of EDMA after the storage was higher than that of the ester compound-containing compositions of Comparative Examples 39 to 40 in which the component A was not contained, and it can be said that quality stability during storage was high.

[Example 147 and Comparative Examples 41 to 43]

An ester compound-containing composition was prepared in the same manner as in Example 145, except that 2-methoxyethyl methacrylate (MTMA, moisture content: 575 ppm by mass) was used instead of the EDMA and the formulations of the A solution, the B solution, and the ester compound-containing composition were changed as shown in Table 31, and the purity of MTMA was obtained.

[Table 31]

| | A solution | | | | B solution | | | | Ester compound-containing composition | | |
|---|---|---|---|---|---|---|---|---|---|---|---|
| | MTMA [g] | Component A | | | MTMA [g] | Component B | | | MTMA [g] | A solution [g] | B solution [g] |
| | | Compound name | Addition amount [g] | Contained amount [ppm by mass] | | Compound name | Addition amount [g] | Contained amount [ppm by mass] | | | |
| Example 147 | 5.008 | 2,3,5,6-Tetramethylpyrazine | 0.010 | 2077 | 10.014 | DMTBP | 0.024 | 2347 | 5.011 | 0.033 | 0.034 |
| Comparative Example 41 | - | - | - | - | 10.007 | DMTBP | 0.024 | 2348 | 5.016 | - | 0.053 |
| Comparative Example 42 | - | - | - | - | 10.014 | DMTBP | 0.024 | 2347 | 5.016 | - | 0.063 |
| Comparative Example 43 | - | - | - | - | - | - | - | - | 5.021 | - | - |

Abbreviations in Table 31 have the following meanings.

· MTMA: 2-methoxyethyl methacrylate

· DMTBP: 2,4-dimethyl-6-t-butylphenol

Table 32 shows the measurement results of the purity of MTMA in each example.

[Table 32]

| | Formulation of ester compound-containing composition (calculated value) | | | | | Analysis result of ester compound-containing composition after storage |
|---|---|---|---|---|---|---|
| | MTMA [% by mass] | Component A compound name | Component A [ppm by mass] | Component B compound name | Component B [ppm by mass] | Purity of MTMA [%] |
| Example 147 | 99.98 | 2,3,5,6-Tetramethylpyrazine | 13 | DMTBP | 16 | 99.32 |
| Comparative Example 41 | 99.98 | - | - | DMTBP | 24 | 99.24 |
| Comparative Example 42 | 99.98 | - | - | DMTBP | 29 | 99.22 |
| Comparative Example 43 | 99.98 | - | - | - | - | 99.27 |

As shown in Table 32, in the ester compound-containing compositions of Example 147 in which the component A was contained, the purity of MTMA after the storage was higher than that of the ester compound-containing compositions of Comparative Examples 41 to 43 in which the component A was not contained, and it can be said that quality stability during storage was high.

INDUSTRIAL APPLICABILITY

According to the present invention, an ester compound-containing composition which can be used as a raw material or the like of a (meth)acrylic polymer can be stably stored for a long period of time, which is industrially useful.

**Claims**

**1.** An ester compound-containing composition comprising:

an ester compound (I) represented by Formula (I); and
one or more compounds selected from the group consisting of a compound represented by Formula (a1), a compound represented by Formula (a2), a compound represented by Formula (a6), a compound represented by Formula (a7), a compound represented by Formula (a8), a compound represented by Formula (a9), and a compound represented by Formula (a10),
wherein a contained amount of the ester compound (I) is 95.00% to 99.99% by mass,

$$CH_2=CR^{150}\text{-}C(=O)\text{-}O\text{-}R^{200} \cdots \qquad (I),$$

in Formula (I), $R^{150}$ is a hydrogen atom or a methyl group, and
$R^{200}$ is a monovalent hydrocarbon group having 2 to 20 carbon atoms, which may have a substituent, or a monovalent group having an ether bond and having 2 to 8 carbon atoms,

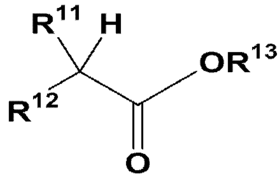

(a 1)

in Formula (a1), $R^{11}$, $R^{12}$, $R^{13}$ may be each independently a group selected from a hydrogen atom, an alkyl group, an alkenyl group, an aryl group, a hydroxy group, an alkoxy group, an amino group, a carbonyl group, and a monovalent group having an ether bond and/or a sulfide bond, or $R^{11}$, $R^{12}$, and $R^{13}$ may be each a divalent group in combination with any of the groups, which may have a substituent,

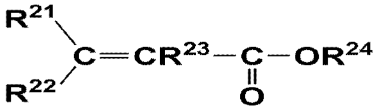

・・・(a 2)

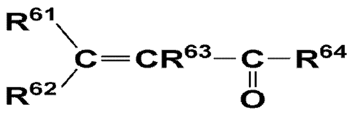

・・・(a 6)

in Formula (a2), $R^{21}$, $R^{22}$, and $R^{23}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{24}$ may be a hydrogen atom, an alkyl group, an alkenyl group, an aryl group, a hydroxy group, an alkoxy group, an amino group, a carbonyl group, or a monovalent group including an ether group, or $R^{21}$, $R^{22}$, $R^{23}$, and $R^{24}$ may be each a divalent group in combination with any of the groups, which may have a substituent,

provided that a case where $R^{21}$ = H, $R^{22}$ = H, $R^{23}$ = $R^{150}$, and $R^{24}$ = $R^{200}$, that is, a case where the compound represented by Formula (a2) is the same as the ester compound (I) is excluded,

in Formula (a6), $R^{61}$, $R^{62}$, and $R^{63}$ may be each independently a hydrogen atom or a monovalent group selected from an alkyl group, an alkenyl group, an aryl group, a hydroxy group, an alkoxy group, an amino group, a monovalent group including a carbonyl group, an alkylthio group, or an arylthio group, $R^{64}$ may be a monovalent group selected from an alkyl group, an alkenyl group, an aryl group, a hydroxy group, an alkoxy group, an amino group, a monovalent group including an ether group, an alkylthio group, or an arylthio group, or $R^{61}$, $R^{62}$, $R^{63}$, and $R^{64}$ may be each a divalent group in combination with any of the groups, which may have a substituent,

provided that a case where $R^{61}$ = H, $R^{62}$ = H, $R^{63}$ = $R^{150}$, and $R^{64}$ = $OR^{200}$, that is, a case where the compound represented by Formula (a6) is the same as the ester compound (I) is excluded,

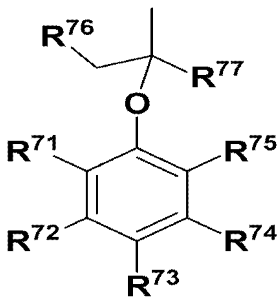

(a 7)

in Formula (a7), $R^{71}$, $R^{72}$, $R^{73}$, $R^{74}$, $R^{75}$, $R^{76}$, and $R^{77}$ are each independently a monovalent group selected from a hydrogen atom, an alkyl group, an alkenyl group, an aryl group, a hydroxy group, an alkoxy group, an amino group, a monovalent group including a carbonyl group, an alkylthio group, or an arylthio group,

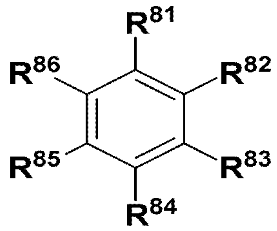

・・・（a８）

in Formula (a8), $R^{81}$, $R^{82}$, $R^{83}$, $R^{84}$, $R^{85}$, and $R^{86}$ are each independently a monovalent group selected from a hydrogen atom, an alkyl group, an alkenyl group, an aryl group, a hydroxy group, an alkoxy group, an amino group, a monovalent group including a carbonyl group, an alkylthio group, or an arylthio group, where the total number of carbon atoms in $R^{81}$, $R^{82}$ , $R^{83}$, $R^{84}$, $R^{85}$, and $R^{86}$ is 2 or more,
in Formula (a9), $R^{91}$ is an alkyl group having 1 to 5 carbon atoms, an alkenyl group having 2 to 5 carbon atoms, or an aryl group having 1 to 12 carbon atoms, each of which may have a substituent,

$$R^{91}-C\equiv N$$ ・・・（a９）

in Formula (a10), $R^{101}$, $R^{102}$, $R^{103}$, $R^{104}$ may be each independently a hydrogen atom or a monovalent group selected from an alkyl group, an alkenyl group, an aryl group, a hydroxy group, an alkoxy group, an amino group, a carbonyl group, a monovalent group including a heteroatom and/or an unsaturated bond, an alkylthio group, or an arylthio group, or $R^{101}$, $R^{102}$, $R^{103}$, and $R^{104}$ may be each a divalent group in combination with any of the groups, which may have a substituent and may have a heteroatom and/or an unsaturated bond, where the groups may further have a substituent.

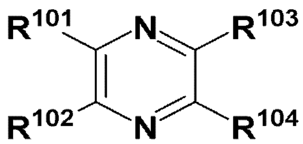

・・・（a１０）

2. The ester compound-containing composition according to Claim 1, further comprising:
a polymerization inhibitor.

3. The ester compound-containing composition according to Claim 1,
wherein a total contained amount of the compound represented by Formula (a1), the compound represented by Formula (a2), the compound represented by Formula (a6), the compound represented by Formula (a7), the compound represented by Formula (a8), the compound represented by Formula (a9), and the compound represented by Formula (a10) is 1 ppm by mass or more and 10000 ppm by mass or less.

4. The ester compound-containing composition according to Claim 3,
wherein the total contained amount of the compound represented by Formula (a1), the compound represented by Formula (a2), the compound represented by Formula (a6), the compound represented by Formula (a7), the compound represented by Formula (a8), the compound represented by Formula (a9), and the compound represented by Formula (a10) is 5 ppm by mass or more and 1500 ppm by mass or less.

5. The ester compound-containing composition according to Claim 2,
wherein a total contained amount of the polymerization inhibitor is 1 ppm by mass or more and 1000 ppm by mass or less.

6. The ester compound-containing composition according to Claim 5,
wherein the total contained amount of the polymerization inhibitor is 5 ppm by mass or more and 100 ppm by mass or less.

7. The ester compound-containing composition according to Claim 1,
wherein the molecular weight of one or more compounds selected from the group consisting of the compound represented by Formula (a1), the compound represented by Formula (a2), the compound represented by Formula (a6), the compound represented by Formula (a7), the compound represented by Formula (a8), the compound

represented by Formula (a9), and the compound represented by Formula (a10) is 1000 or less.

**8.** The ester compound-containing composition according to Claim 2,
wherein the polymerization inhibitor is at least one polymerization inhibitor selected from the group consisting of a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

**9.** The ester compound-containing composition according to Claim 2,
wherein the polymerization inhibitor is at least one polymerization inhibitor selected from the group consisting of a phenol-based compound, an N-oxyl-based compound, an amine-based compound, a phosphorus-containing compound, and a sulfur-containing compound.

**10.** The ester compound-containing composition according to Claim 2,
wherein the polymerization inhibitor is at least one polymerization inhibitor selected from the group consisting of hydroquinone, 4-methoxyphenol, 2,4-dimethyl-6-t-butylphenol, and 2,6-di-t-butyl-4-methylphenol.

**11.** The ester compound-containing composition according to Claim 1,
wherein $R^{11}$ and $R^{12}$ in Formula (a1) are each independently a hydrogen atom or an alkyl group having 1 to 5 carbon atoms.

**12.** The ester compound-containing composition according to Claim 1,
wherein $R^{13}$ in Formula (a1) is a chain alkyl group having 1 to 10 carbon atoms, an allyl group, a 2-hydroxyethyl group, a 2-hydroxypropyl group, a 2-methoxyethyl group, a glycidyl group, or a tetrahydrofurfuryl group.

**13.** The ester compound-containing composition according to Claim 1,

wherein, in Formula (a2) and Formula (a6), $R^{21}$, $R^{22}$, $R^{23}$, $R^{60}$, $R^{61}$, $R^{62}$, and $R^{63}$ are each independently a hydrogen atom, an alkyl group having 1 to 5 carbon atoms, an alkenyl group having 2 to 5 carbon atoms, or an aryl group having 1 to 12 carbon atoms,
$R^{24}$ is a hydrogen atom, an alkyl group having 1 to 5 carbon atoms, an alkenyl group having 2 to 5 carbon atoms, an aryl group having 1 to 12 carbon atoms, an aromatic alkyl group having 1 to 12 carbon atoms, or a group having an etheric oxygen between carbon atoms of a hydrocarbon group having 2 to 8 carbon atoms, and
$R^{64}$ is an alkyl group having 1 to 5 carbon atoms, an alkenyl group having 2 to 5 carbon atoms, an aryl group having 1 to 12 carbon atoms, an amino group, an alkylthio group having 1 to 5 carbon atoms, or an arylthio group having 1 to 12 carbon atoms.

**14.** The ester compound-containing composition according to Claim 1,

wherein, in Formula (a2) and Formula (a6), $R^{21}$, $R^{22}$, $R^{23}$, $R^{60}$, $R^{61}$, $R^{62}$, and $R^{63}$ are each independently a hydrogen atom or an alkyl group having 1 to 5 carbon atoms,
$R^{24}$ is an alkyl group having 1 to 5 carbon atoms or an alkenyl group having 2 to 5 carbon atoms, and
$R^{64}$ is an alkyl group having 1 to 5 carbon atoms.

**15.** The ester compound-containing composition according to Claim 1,

wherein, in Formula (a2), $R^{21}$, $R^{22}$, and $R^{23}$ are each independently a hydrogen atom, a methyl group, an ethyl group, an n-propyl group, or an isopropyl group, and
$R^{24}$ is a methyl group, an ethyl group, an n-propyl group, an isopropyl group, an n-butyl group, an isobutyl group, a crotyl group, an n-pentyl group, a 2-methyl-1-butyl group, or an isopentyl group.

**16.** The ester compound-containing composition according to Claim 1,
wherein $R^{71}$, $R^{72}$, $R^{73}$, $R^{74}$, $R^{75}$, $R^{76}$, and $R^{77}$ in Formula (a7) are each independently a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group, or a monovalent group including a carbonyl group.

**17.** The ester compound-containing composition according to Claim 1,

wherein $R^{71}$, $R^{72}$, $R^{73}$, $R^{74}$, and $R^{75}$ in Formula (a7) are each independently a hydrogen atom, an alkyl group having 1 to 5 carbon atoms, a hydroxy group, an alkoxy group having 1 to 5 carbon atoms, or an alkoxycarbonyl group having 2 to 6 carbon atoms.

**18.** The ester compound-containing composition according to Claim 1,
wherein $R^{71}$, $R^{72}$, $R^{73}$, $R^{74}$, and $R^{75}$ in Formula (a7) are each independently a hydrogen atom, a methyl group, a t-butyl group, a hydroxy group, or a methoxy group.

**19.** The ester compound-containing composition according to Claim 1,
wherein $R^{76}$ and $R^{77}$ in Formula (a7) are each independently a hydrogen atom, an alkyl group having 1 to 5 carbon atoms, a hydroxy group, or an alkoxycarbonyl group having 1 to 5 carbon atoms.

**20.** The ester compound-containing composition according to Claim 1,
wherein $R^{76}$ and $R^{77}$ in Formula (a7) are each independently a hydrogen atom, a methyl group, a methoxycarbonyl group, or a butoxycarbonyl group.

**21.** The ester compound-containing composition according to Claim 1,
wherein $R^{81}$, $R^{82}$, $R^{83}$, $R^{84}$, $R^{85}$, and $R^{86}$ in Formula (a8) are each independently a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group, or a monovalent group including a carbonyl group.

**22.** The ester compound-containing composition according to Claim 1,
wherein $R^{81}$, $R^{82}$, $R^{83}$, $R^{84}$, $R^{85}$, and $R^{86}$ in Formula (a8) are each independently a hydrogen atom, an alkyl group having 1 to 5 carbon atoms, a hydroxy group, or an alkoxy group having 1 to 6 carbon atoms.

**23.** The ester compound-containing composition according to Claim 1,
wherein $R^{81}$, $R^{82}$, $R^{83}$, $R^{84}$, $R^{85}$, and $R^{86}$ in Formula (a8) are each independently a hydrogen atom, a methyl group, an ethyl group, an n-propyl group, an isopropyl group, or a t-butyl group.

**24.** The ester compound-containing composition according to Claim 1,
wherein $R^{91}$ in Formula (a9) is an alkyl group having 1 to 3 carbon atoms, which may have a substituent, an alkenyl group having 2 to 3 carbon atoms, which may have a substituent, or an aryl group having 1 to 8 carbon atoms, which may have a substituent.

**25.** The ester compound-containing composition according to Claim 1,
wherein $R^{91}$ in Formula (a9) is a methyl group, an ethyl group, a 2-alkoxyethyl group, a 2-alkoxyisopropyl group, a vinyl group, an isopropenyl group, or a phenyl group.

**26.** The ester compound-containing composition according to Claim 1,
wherein $R^{101}$, $R^{102}$, $R^{103}$, and $R^{104}$ in Formula (a10) are each independently a hydrogen atom, an alkyl group having 1 to 5 carbon atoms, or an alkoxy group having 1 to 6 carbon atoms.

**27.** The ester compound-containing composition according to Claim 1,
wherein $R^{101}$, $R^{102}$, $R^{103}$, and $R^{104}$ in Formula (a10) are each independently a hydrogen atom, a methyl group, an ethyl group, an isopropyl group, a methoxy group, an ethoxy group, or an isopropoxy group.

**28.** The ester compound-containing composition according to Claim 1,
wherein the contained amount of the ester compound (I) is 98% to 99.99% by mass.

**29.** The ester compound-containing composition according to Claim 1,
wherein the ester compound (I) includes the ester compound in which $R^{200}$ is a linear or branched alkyl group having 2 to 20 carbon atoms.

**30.** The ester compound-containing composition according to Claim 29,
wherein $R^{200}$ is an n-butyl group or an isobutyl group.

**31.** The ester compound-containing composition according to Claim 1,

wherein the ester compound (I) includes one or more selected from the group consisting of ethylene glycol di(meth) acrylate, 2-hydroxyethyl (meth)acrylate, 3-hydroxypropyl (meth)acrylate, and trimethylolpropane tri(meth)acrylate.

**32.** The ester compound-containing composition according to Claim 1,
wherein the ester compound (I) includes the ester compound in which $R^{200}$ is a 2-methoxyethyl group, a glycidyl group, or a tetrahydrofurfuryl group.

**33.** The ester compound-containing composition according to Claim 1,
wherein the ester compound-containing composition is stored for 1 day or longer.

**34.** A method for producing the ester compound-containing composition according to Claim 1, the production method comprising:
performing an ester exchange reaction between one or more alcohols selected from the group consisting of a monoalcohol having 2 to 20 carbon atoms, an ether bond-containing alcohol having 2 to 8 carbon atoms, a dialcohol having 2 to 8 carbon atoms, and a trialcohol having 2 to 8 carbon atoms, and methyl (meth)acrylate in a presence of at least one or more compounds selected from the group consisting of the compound represented by Formula (a1), the compound represented by Formula (a2), the compound represented by Formula (a6), the compound represented by Formula (a7), the compound represented by Formula (a8), the compound represented by Formula (a9), and the compound represented by Formula (a10).

**35.** The production method according to Claim 34,
wherein the ester exchange reaction is performed in a presence of a polymerization inhibitor, in addition to the at least one or more compounds selected from the group consisting of the compound represented by Formula (a1), the compound represented by Formula (a2), the compound represented by Formula (a6), the compound represented by Formula (a7), the compound represented by Formula (a8), the compound represented by Formula (a9), and the compound represented by Formula (a10).

**36.** The production method according to Claim 35,
wherein the polymerization inhibitor is at least one polymerization inhibitor selected from the group consisting of a phenol-based compound, a quinone-based compound, a nitrobenzene-based compound, an N-oxyl-based compound, an amine-based compound, a phosphorus-containing compound, a sulfur-containing compound, an iron-containing compound, a copper-containing compound, and a manganese-containing compound.

**37.** The production method according to Claim 35,
wherein the polymerization inhibitor is at least one polymerization inhibitor selected from the group consisting of a phenol-based compound, an N-oxyl-based compound, an amine-based compound, a phosphorus-containing compound, and a sulfur-containing compound.

**38.** The production method according to Claim 35,
wherein the polymerization inhibitor is at least one polymerization inhibitor selected from the group consisting of hydroquinone, 4-methoxyphenol, 2,4-dimethyl-6-t-butylphenol, and 2,6-di-t-butyl-4-methylphenol.

**39.** The production method according to Claim 34,
wherein $R^{11}$ and $R^{12}$ in Formula (a1) are each independently a hydrogen atom or an alkyl group having 1 to 5 carbon atoms.

**40.** The production method according to Claim 34,
wherein $R^{13}$ in Formula (a1) is a chain alkyl group having 1 to 10 carbon atoms, an allyl group, a 2-hydroxyethyl group, a 2-hydroxypropyl group, a 2-methoxyethyl group, a glycidyl group, or a tetrahydrofurfuryl group.

**41.** The production method according to Claim 34,

wherein, in Formula (a2) and Formula (a6), $R^{21}$, $R^{22}$, $R^{23}$, $R^{60}$, $R^{61}$, $R^{62}$, and $R^{63}$ are each independently a hydrogen atom, an alkyl group having 1 to 5 carbon atoms, an alkenyl group having 2 to 5 carbon atoms, or an aryl group having 1 to 12 carbon atoms,
$R^{24}$ is a hydrogen atom, an alkyl group having 1 to 5 carbon atoms, an alkenyl group having 2 to 5 carbon atoms, an aryl group having 1 to 12 carbon atoms, an aromatic alkyl group having 1 to 12 carbon atoms, or a group having an etheric oxygen between carbon atoms of a hydrocarbon group having 2 to 8 carbon atoms, and

$R^{64}$ is an alkyl group having 1 to 5 carbon atoms, an alkenyl group having 2 to 5 carbon atoms, an aryl group having 1 to 12 carbon atoms, an amino group, an alkylthio group having 1 to 5 carbon atoms, or an arylthio group having 1 to 12 carbon atoms.

**42.** The production method according to Claim 34,

wherein, in Formula (a2) and Formula (a6), $R^{21}$, $R^{22}$, $R^{23}$, $R^{60}$, $R^{61}$, $R^{62}$, and $R^{63}$ are each independently a hydrogen atom or an alkyl group having 1 to 5 carbon atoms,
$R^{24}$ is an alkyl group having 1 to 5 carbon atoms or an alkenyl group having 2 to 5 carbon atoms, and
$R^{64}$ is an alkyl group having 1 to 5 carbon atoms.

**43.** The production method according to Claim 34,

wherein, in Formula (a2), $R^{21}$, $R^{22}$, and $R^{23}$ are each independently a hydrogen atom, a methyl group, an ethyl group, an n-propyl group, or an isopropyl group, and
$R^{24}$ is a methyl group, an ethyl group, an n-propyl group, an isopropyl group, an n-butyl group, an isobutyl group, a crotyl group, an n-pentyl group, a 2-methyl-1-butyl group, or an isopentyl group.

**44.** The production method according to Claim 34,
wherein $R^{71}$, $R^{72}$, $R^{73}$, $R^{74}$, $R^{75}$, $R^{76}$, and $R^{77}$ in Formula (a7) are each independently a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group, or a monovalent group including a carbonyl group.

**45.** The production method according to Claim 34,
wherein $R^{71}$, $R^{72}$, $R^{73}$, $R^{74}$, and $R^{75}$ in Formula (a7) are each independently a hydrogen atom, an alkyl group having 1 to 5 carbon atoms, a hydroxy group, or an alkoxy group having 1 to 5 carbon atoms.

**46.** The production method according to Claim 34,
wherein $R^{71}$, $R^{72}$, $R^{73}$, $R^{74}$, and $R^{75}$ in Formula (a7) are each independently a hydrogen atom, a methyl group, a t-butyl group, a hydroxy group, or a methoxy group.

**47.** The production method according to Claim 34,
wherein $R^{76}$ and $R^{77}$ in Formula (a7) are each independently a hydrogen atom, an alkyl group having 1 to 5 carbon atoms, a hydroxy group, or an alkoxycarbonyl group having 1 to 5 carbon atoms.

**48.** The production method according to Claim 34,
wherein $R^{76}$ and $R^{77}$ in Formula (a7) are each independently a hydrogen atom, a methyl group, a methoxycarbonyl group, or a butoxycarbonyl group.

**49.** The production method according to Claim 34,
wherein $R^{81}$, $R^{82}$, $R^{83}$, $R^{84}$, $R^{85}$, and $R^{86}$ in Formula (a8) are each independently a hydrogen atom, an alkyl group having 1 to 5 carbon atoms, an alkenyl group having 2 to 5 carbon atoms, an aryl group having 1 to 12 carbon atoms, a hydroxy group, an alkoxy group having 1 to 6 carbon atoms, an amino group, or a monovalent group including a carbonyl group.

**50.** The production method according to Claim 34,
wherein $R^{81}$, $R^{82}$, $R^{83}$, $R^{84}$, $R^{85}$, and $R^{86}$ in Formula (a8) are each independently a hydrogen atom, an alkyl group having 1 to 5 carbon atoms, a hydroxy group, or an alkoxy group having 1 to 6 carbon atoms.

**51.** The production method according to Claim 34,
wherein $R^{81}$, $R^{82}$, $R^{83}$, $R^{84}$, $R^{85}$, and $R^{86}$ in Formula (a8) are each independently a hydrogen atom, a methyl group, an ethyl group, an n-propyl group, an isopropyl group, or a t-butyl group.

**52.** The production method according to Claim 34,
wherein $R^{91}$ in Formula (a9) is an alkyl group having 1 to 3 carbon atoms, which may have a substituent, an alkenyl group having 2 to 3 carbon atoms, which may have a substituent, or an aryl group having 1 to 8 carbon atoms, which may have a substituent.

**53.** The production method according to Claim 34,
wherein $R^{91}$ in Formula (a9) is a methyl group, an ethyl group, a 2-alkoxyethyl group, a 2-alkoxyisopropyl group, a vinyl group, an isopropenyl group, or a phenyl group.

**54.** The production method according to Claim 34,
wherein $R^{101}$, $R^{102}$, $R^{103}$, and $R^{104}$ in Formula (a10) are each independently a hydrogen atom, an alkyl group having 1 to 5 carbon atoms, or an alkoxy group having 1 to 6 carbon atoms.

**55.** The production method according to Claim 34,
wherein $R^{101}$, $R^{102}$, $R^{103}$, and $R^{104}$ in Formula (a10) are each independently a hydrogen atom, a methyl group, an ethyl group, an isopropyl group, a methoxy group, an ethoxy group, or an isopropoxy group.

**56.** A polymerizable composition comprising:
the ester compound-containing composition according to any one of Claims 1 to 33.

**57.** A (meth)acrylic polymer obtained by polymerizing the polymerizable composition according to Claim 56.

**58.** The (meth)acrylic polymer according to Claim 57,
wherein the polymerizable composition comprises 0.01 parts by mass or more of a monomer copolymerizable with the ester compound (I), with respect to 100 parts by mass of the ester compound (I).

**59.** A method for producing a (meth)acrylic polymer, comprising:
polymerizing the polymerizable composition according to Claim 56.

**60.** The production method according to Claim 59,
wherein the polymerizable composition comprises 0.01 parts by mass or more of a monomer copolymerizable with the ester compound (I), with respect to 100 parts by mass of the ester compound (I).

**INTERNATIONAL SEARCH REPORT**

International application No.
**PCT/JP2023/039088**

**A. CLASSIFICATION OF SUBJECT MATTER**

***C08F 20/18***(2006.01)i; ***C07C 67/03***(2006.01)i; ***C07C 69/54***(2006.01)i; ***C08F 20/10***(2006.01)i
FI: C08F20/18; C07C67/03; C08F20/10; C07C69/54 Z CSP

According to International Patent Classification (IPC) or to both national classification and IPC

**B. FIELDS SEARCHED**

Minimum documentation searched (classification system followed by classification symbols)

C08F20/18; C07C67/03; C07C69/54; C08F20/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 47-037929 B1 (NIPPON KAYAKU CO., LTD.) 25 September 1972 (1972-09-25) claims, examples | 1, 3-4, 7, 11-29, 33, 56-60 |
| Y | | 2, 5-6, 8-10, 56-60 |
| X | JP 51-146418 A (NIPPON ZEON CO.) 16 December 1976 (1976-12-16) claims, examples | 1, 3-4, 7, 11-29, 33, 56-60 |
| Y | | 2, 5-6, 8-10, 34-50, 52-60 |
| Y | JP 2021-081725 A (ASAHI KASEI CORP.) 27 May 2021 (2021-05-27) claims, examples | 2, 5-6, 8-10, 34-50, 52-60 |
| Y | JP 2009-274986 A (MITSUBISHI RAYON CO., LTD.) 26 November 2009 (2009-11-26) claims, paragraphs [0023]-[0025], examples | 2, 5-6, 8-10, 34-50, 52-60 |
| X | WO 2011/049138 A1 (DENKI KAGAKU KOGYO KK) 28 April 2011 (2011-04-28) claims, examples | 1-5, 8-9, 11-21, 24-28, 31, 33, 56-60 |
| Y | | 34-50, 52-55 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 January 2024** | **16 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2023/039088

C. DOCUMENTS CONSIDERED TO BE RELEVANT

| Category* | Citation of document, with indication, where appropriate, of the relevant passages | Relevant to claim No. |
|---|---|---|
| X<br>Y | JP 2018-083896 A (SOKEN CHEM. & ENG. CO., LTD.) 31 May 2018 (2018-05-31) claims, paragraph [0022], examples | 1, 7, 11-27, 29-30, 33, 56-60<br>2, 5-6, 8-10, 34-50, 52-60 |
| X<br>Y | JP 2021-113317 A (SEKISUI CHEM. CO., LTD.) 05 August 2021 (2021-08-05) claims, examples | 1, 7, 11-13, 16-27, 32-33, 56-60<br>2, 5-6, 8-10, 34-50, 52-60 |
| X<br>Y | WO 2020/174787 A1 (NITTO DENKO CORP.) 03 September 2020 (2020-09-03) claims, examples | 1, 3, 7, 11-12, 16-30, 33, 56-60<br>2, 5-6, 8-10, 34-50, 52-60 |
| X<br>Y | JP 2011-506517 A (EVONIK ROEHM GMBH) 03 March 2011 (2011-03-03) claims, paragraph [0022], examples | 1, 4-5, 7, 11-29, 33-50, 52-60<br>2, 5-6, 8-10, 34-50, 52-60 |
| X<br>Y | WO 2012/018007 A1 (MITSUBISHI RAYON CO., LTD.) 09 February 2012 (2012-02-09) claims, paragraph [0039], examples | 1, 7, 11-30, 33, 56-60<br>2, 5-6, 8-10, 34-50, 52-60 |
| X<br>Y | JP 2005-336139 A (MITSUBISHI CHEMICALS CORP.) 08 December 2005 (2005-12-08) claims, examples | 1, 3-4, 7, 11-13, 16-29, 33, 56-60<br>2, 5-6, 8-10, 34-50, 52-60 |
| X | WO 2022/219969 A1 (SOKEN CHEM. & ENG. CO., LTD.) 20 October 2022 (2022-10-20) claims, examples | 56-60 |
| X | WO 2022/185715 A1 (SOKEN CHEM. & ENG. CO., LTD.) 09 September 2022 (2022-09-09) claims, examples | 56-60 |
| A | WO 2015/119233 A1 (KURARAY CO., LTD.) 13 August 2015 (2015-08-13) claims, examples | 1-60 |
| A | JP 2022-056754 A (SUMITOMO CHEMICAL CO., LTD.) 11 April 2022 (2022-04-11) claims, examples | 1-60 |
| A | WO 2022/158190 A1 (SUMITOMO CHEMICAL CO., LTD.) 28 July 2022 (2022-07-28) claims, examples | 1-60 |
| A | JP 2022-066090 A (KURARAY CO., LTD.) 28 April 2022 (2022-04-28) claims, examples | 1-60 |
| A | JP 2022-103861 A (NIPPON ZEON CO.) 08 July 2022 (2022-07-08) claims, examples | 1-60 |
| A | JP 2019-099751 A (NITTO DENKO CORP.) 24 June 2019 (2019-06-24) claims, examples | 1-60 |
| A | JP 2022-134862 A (NITTO DENKO CORP.) 15 September 2022 (2022-09-15) claims, examples | 1-60 |
| A | JP 2022-102363 A (MITSUBISHI CHEMICAL CORP.) 07 July 2022 (2022-07-07) claims, examples | 1-60 |
| P, A | WO 2022/230596 A1 (MITSUBISHI CHEMICAL CORP.) 03 November 2022 (2022-11-03) claims, examples | 1-60 |
| P, A | WO 2022/230913 A1 (MITSUBISHI CHEMICAL CORP.) 03 November 2022 (2022-11-03) claims, examples | 1-60 |

Form PCT/ISA/210 (second sheet) (January 2015)

**INTERNATIONAL SEARCH REPORT**

International application No.
**PCT/JP2023/039088**

**C. DOCUMENTS CONSIDERED TO BE RELEVANT**

| Category* | Citation of document, with indication, where appropriate, of the relevant passages | Relevant to claim No. |
|---|---|---|
| P, A | WO 2022/230914 A1 (MITSUBISHI CHEMICAL CORP.) 03 November 2022 (2022-11-03) claims, examples | 1-60 |
| P, A | WO 2022/230915 A1 (MITSUBISHI CHEMICAL CORP.) 03 November 2022 (2022-11-03) claims, examples | 1-60 |
| P, A | WO 2022/230916 A1 (MITSUBISHI CHEMICAL CORP.) 03 November 2022 (2022-11-03) claims, examples | 1-60 |
| P, A | WO 2023/100867 A1 (MITSUBISHI CHEMICAL CORP.) 08 June 2023 (2023-06-08) claims, examples | 1-60 |

Form PCT/ISA/210 (second sheet) (January 2015)

INTERNATIONAL SEARCH REPORT

International application No.
PCT/JP2023/039088

**Box No. III Observations where unity of invention is lacking (Continuation of item 3 of first sheet)**

This International Searching Authority found multiple inventions in this international application, as follows:

(Invention 1)
Inventions in claim 1 containing a compound represented by formula (a1) in claim 1 and inventions in dependent claims thereof
(Invention 2)
Inventions in claim 1 containing a compound represented by formula (a2) or (a6) in claim 1 and inventions in dependent claims thereof
(Invention 3)
Inventions in claim 1 containing a compound represented by formula (a7) in claim 1 and inventions in dependent claims thereof
(Invention 4)
Inventions in claim 1 containing a compound represented by formula (a8) in claim 1 and inventions in dependent claims thereof
(Invention 5)
Inventions in claim 1 containing a compound represented by formula (a9) in claim 1 and inventions in dependent claims thereof
(Invention 6)
Inventions in claim 1 containing a compound represented by formula (a10) in claim 1 and inventions in dependent claims thereof

The inventions 1-6 share the technical feature of an "ester compound-containing composition comprising an ester compound (I) represented by formula (I) indicates below ..., wherein the composition contains the ester compound (I) at 95.00-99.99% by mass of the total mass" (claim 1 of the present application). However, the technical feature does not make a contribution over the prior art in light of the disclosures in document 5 (WO 2011/049138 A1, the claims, the examples (particularly, example 1), etc.), and thus is not considered to be a special technical feature.

Although the compound represented by formula (a1), the compound represented by formula (a2) or (a6), the compound represented by formula (a7), the compound represented by formula (a8), the compound represented by formula (a9) and the compound represented by formula (a10) are considered to share some nature or activity, no common structure is present and the compounds cannot be considered to belong to a recognized class (PCT International Search and Preliminary Examination Guidelines 10.17) Thus, the compounds are not considered to share a same nature or a similar nature.

Accordingly, the inventions 1-6 cannot be said to share a same or corresponding special technical feature.

Form PCT/ISA/210 (continuation of first sheet) (January 2015)

**INTERNATIONAL SEARCH REPORT**

International application No.
**PCT/JP2023/039088**

**Box No. III Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2023/039088**

| Patent document cited in search report | Publication date (day/month/year) | Patent family member(s) | Publication date (day/month/year) |
|---|---|---|---|
| JP 47-037929 B1 | 25 September 1972 | (Family: none) | |
| JP 51-146418 A | 16 December 1976 | US 4070254 A<br>claims, examples | |
| JP 2021-081725 A | 27 May 2021 | (Family: none) | |
| JP 2009-274986 A | 26 November 2009 | (Family: none) | |
| WO 2011/049138 A1 | 28 April 2011 | US 2012/0205043 A1<br>claims, examples<br>CN 102574951 A<br>KR 10-2012-0099053 A | |
| JP 2018-083896 A | 31 May 2018 | (Family: none) | |
| JP 2021-113317 A | 05 August 2021 | (Family: none) | |
| WO 2020/174787 A1 | 03 September 2020 | CN 113474690 A<br>KR 10-2021-0134319 A | |
| JP 2011-506517 A | 03 March 2011 | US 2010/0280205 A1<br>claims, paragraph [0038], examples<br>WO 2009/080380 A2<br>EP 2220023 A2<br>CN 101462958 A | |
| WO 2012/018007 A1 | 09 February 2012 | (Family: none) | |
| JP 2005-336139 A | 08 December 2005 | US 2008/0011385 A1<br>claims, examples<br>WO 2005/115967 A1<br>CN 1697824 A | |
| WO 2022/219969 A1 | 20 October 2022 | (Family: none) | |
| WO 2022/185715 A1 | 09 September 2022 | TW 202302796 A | |
| WO 2015/119233 A1 | 13 August 2015 | US 2016/0347879 A1<br>claims, examples<br>EP 3103819 A1<br>CN 105980414 A<br>KR 10-2016-0118206 A | |
| JP 2022-056754 A | 11 April 2022 | US 2022/0098344 A1<br>claims, examples<br>EP 3978541 A1<br>CN 114316122 A<br>KR 10-2022-0044107 A | |
| WO 2022/158190 A1 | 28 July 2022 | (Family: none) | |
| JP 2022-066090 A | 28 April 2022 | (Family: none) | |
| JP 2022-103861 A | 08 July 2022 | (Family: none) | |
| JP 2019-099751 A | 24 June 2019 | CN 111433305 A<br>KR 10-2020-0093634 A | |
| JP 2022-134862 A | 15 September 2022 | TW 202246818 A | |
| JP 2022-102363 A | 07 July 2022 | (Family: none) | |
| WO 2022/230596 A1 | 03 November 2022 | (Family: none) | |
| WO 2022/230913 A1 | 03 November 2022 | (Family: none) | |
| WO 2022/230914 A1 | 03 November 2022 | (Family: none) | |
| WO 2022/230915 A1 | 03 November 2022 | (Family: none) | |
| WO 2022/230916 A1 | 03 November 2022 | (Family: none) | |

Form PCT/ISA/210 (patent family annex) (January 2015)

**INTERNATIONAL SEARCH REPORT**
**Information on patent family members**

International application No.
**PCT/JP2023/039088**

| Patent document cited in search report | Publication date (day/month/year) | Patent family member(s) | Publication date (day/month/year) |
|---|---|---|---|
| WO 2023/100867 A1 | 08 June 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022174242 A
- JP 2022174342 A
- JP 2022174417 A
- JP 2022174628 A
- JP 2022174460 A
- JP 2022174760 A
- JP 2009274986 A